# EUROPEAN PATENT APPLICATION

(11) **EP 2 116 618 A1**
(43) Date of publication of application: **11.11.2009**
(21) Application number: 09251278.9
(22) Date of filing: 08.05.2009
(51) Int. Cl.: C12Q 1/68

(54) **Diagnosis and treatment of Kawasaki disease**

(30) Priority: 09.05.2008 US 51721 P
(71) Applicant: Agency for Science, Technology And Research, Singapore 138632 (SG); Emma Children's Hospital, 1105 AZ Amsterdam (NL); THE UNIVERSITY OF WESTERN AUSTRALIA, Crawley, Western Australia 6009 (AU)
(72) Inventor: Kuijpers, Taco, 1105 AZ Amsterdam (NL); Hibberd, Martin Lloyd, 138672 Singapore (SG); Burgner, David Paul, 6840 Perth (AU); Dominguez, Sonia Maria Davila, 138672 Singapore (SG)
(74) Representative: Richards, William John

(57) **Abstract**

A molecule for use in a method of treatment or diagnosis of Kawasaki Disease in an individual, the molecule comprising: (a) CACNA2D3; (b) CAMK2D; (c) KCNIP4; (d) ANGPT1; (e) NAALADL2; (f) ZFHX3; (g) MPHOSPH10; (h) a sequence having at least 90% sequence identity to any of (a) to (g); or (i) a modulator of any of (a) to (e). We also describe the use of Pregabalin ((3S)-3-(aminomethyl)-5-methyl-hexanoic acid) in the treatment or prevention of Kawasaki Disease in an individual.

## Description

### FIELD

The present invention relates to the fields of medicine, cell biology, molecular biology and genetics. More particularly, the invention relates to methods of diagnosis and treatment of Kawasaki Disease.

### BACKGROUND

Kawasaki disease (KD, MIM:611775) is a common inflammatory vasculitis predominantly affecting young children. It is characterized by a striking propensity for coronary artery damage, which occurs in approximately 25% of untreated and 3-5% of treated children.

Kawasaki Disease is the commonest cause of heart disease acquired in childhood in developed nations and in those who manifest coronary artery damage, Kawasaki Disease may be associated with increased likelihood of later atherosclerotic damage. The long-term cardiovascular implications of Kawasaki Disease in those without overt coronary artery lesions are unclear. The etiology of Kawasaki Disease is unknown, but it is thought to reflect an abnormal and sustained immunological response to one or more infectious triggers in genetically susceptible individuals. No consistent etiologic agent for Kawasaki Disease has been identified, hampering accurate and timely diagnosis and the development of optimal management strategies.

The incidence of Kawasaki Disease varies markedly in different ethnic groups, with the highest incidence in North East Asian populations; Kawasaki Disease affects approximately 1 in 150 Japanese children and is responsible for 1-2% of all pediatric hospital admissions in South Korea. There are strong epidemiologic data to support a substantial genetic contribution to Kawasaki Disease susceptibility. The Japanese incidence (135-200/100 000 <5 years of age) is 10-15 times greater than the Caucasian incidence (9-17/100 000 <5 years of age) and this difference is maintained in American children of Japanese descent resident in the US. Other Asian populations in the UK (Harnden et al, unpublished data) and the US also show significantly higher incidence than non-Asians residing in the same geographic location. The familial inheritance pattern of Kawasaki Disease is in keeping with a polygenic complex disease and in multi-case pedigrees, Kawasaki Disease occurs in family members at different times and geographic locations. Across all populations Kawasaki Disease is approximately 1.6 times more common in males. The sibling risk ratio for Kawasaki Disease in the Japanese is approximately 10 during an epidemic and 6 overall. Kawasaki Disease is over twice as common in the children of parents who themselves had Kawasaki Disease in childhood, with multi-generational pedigrees often having more than one child affected, earlier age of onset and a more severe phenotype.

Epidemiological, clinical and immunological data strongly support the belief that Kawasaki Disease is due to one or more widely distributed infectious agent(s). These infectious agents are thought to evoke a markedly abnormal immunological inflammation response in genetically susceptible individuals.

Despite 40 years of research, there is no diagnostic test and effective treatment. Treatment by administration of adult non-specific antibodies (gamma globulin) is non-specific and sub-optimal, failing to prevent coronary artery lesions (CAL) in up to 10%. Such treatment also fails to prevent pro-atherosclerotic changes in many more.

### SUMMARY

The present invention is based on a genome-wide association study (GWAS) to identify novel loci that might mediate susceptibility to Kawasaki Disease. This is described in detail in the Examples.

We performed the initial GWAS in a well-characterized Dutch Caucasian population and verified the most significantly associated SNPs and haplotypes in a large independent cohort of predominantly Caucasian trios from three countries.

We identified a number of novel loci that are associated with Kawasaki Disease susceptibility, many of which show differential gene expression in acute versus convalescent Kawasaki Disease and which contribute to a plausible biological network.

According to a 1^{st} aspect of the present invention, we provide a molecule for use in a method of treatment or diagnosis of Kawasaki Disease in an individual. The molecule may comprise any one or more of the following: (a) CACNA2D3; (b) CAMK2D; (c) KCNIP4; (d) ANGPT1; (e) NAALADL2; (f) ZFHX3; (g) MPHOSPH10. The molecule may also comprise (h) a sequence having at least 90% sequence identity to any of (a) to (g). It may comprise (i) a modulator of any of (a) to (e). The molecule may, for example, be in the form of a pharmaceutical composition comprising the molecule together with a pharmaceutically acceptable excipient, carrier or diluent.

The molecule may comprise an antagonist of CACNA2D3. The antagonise of CACNA2D3 may comprise Pregabalin ((3S)-3-(aminomethyl)-5-methyl-hexanoic acid). The method may preferably comprise administering the antagonist of CACNA2D3 to an individual.

There is provided, according to a 2^{nd} aspect of the present invention, a method of identifying a molecule suitable for the treatment, prophylaxis or alleviation of Kawasaki Disease. The method may comprise determining if a candidate molecule is an agonist or antagonist of a polypeptide. The polypeptide may be selected from the group consisting of: (a) CACNA2D3; (b) CAMK2D; (c) KCNIP4; (d) ANGPT1; (e) NAALADL2; (f) ZFHX3; (g) MPHOSPH10; and (h) a sequence having at least 90% sequence identity to any of (a) to (g). The method may comprise exposing the candidate molecule to the polypeptide, or a cell expressing the polypeptide, in order to determine if the candidate molecule is a modulator thereof.

We provide, according to a 3^{rd} aspect of the present invention, a method of identifying a modulator of a polypeptide selected from the group consisting of: (a) CACNA2D3; (b) CAMK2D; (c) KCNIP4; (d) ANGPT1; (e) NAALADL2; (f) ZFHX3; (g) MPHOSPH10; and (h) a sequence having at least 90% sequence identity to any of (a) to (g). The method may comprise administering a candidate molecule to an animal, such as a rodent, for example a mouse, suffering from Kawasaki Disease and determining whether the animal exhibits an alleviated symptom of Kawasaki Disease.

As a 4^{th} aspect of the present invention, there is provided a molecule or modulator of a polypeptide identified by a method set out above. The molecule identified may be for use in the treatment of Kawasaki Disease.

We provide, according to a 5^{th} aspect of the present invention, Pregabalin ((3S)-3-(aminomethyl)-5-methyl-hexanoic acid) for use in the treatment or prevention of Kawasaki Disease in an individual.

The present invention, in a 6^{th} aspect, provides use of a molecule as identified for the preparation of a pharmaceutical composition for the treatment of Kawasaki Disease in an individual.

In a 7^{th} aspect of the present invention, there is provided a pharmaceutical composition for the treatment or prevention of Kawasaki Disease in an individual. The pharmaceutical composition may comprise Pregabalin ((3S)-3-(aminomethyl)-5-methyl-hexanoic acid) together with a pharmaceutically acceptable excipient, carrier or diluent.

According to an 8^{th} aspect of the present invention, we provide a method for the diagnosis or detection of Kawasaki Disease. The method may comprise (a) detecting a polymorphism in or around a gene selected from NAALADL2, ANGPT1 and ZFHX3 or a sequence having at least 90% sequence identity thereto. The method may comprise (b) detecting a single nucleotide polymorphism (SNP) selected from the group consisting of: rs17531088, rs1010824, rs6469101, rs7199343, rs10852516 and rs11075953 as set out in Table 2 or Table S1. The method may comprise (c) detecting a haplotype in or around a gene selected from MPHOSPH10, RBMS1 and LOC441938 or a sequence having at least 90% sequence identity thereto. The method may comprise (d) detecting a haplotype selected from the group consisting of: rs7558220, rs357752; and rs7558220, rs357752, rs1458868, rs357729 and rs11674899 as set out in Table 2 or Table S2. The method may comprise (e) any combination of (a) to (d) above.

The method may comprise any one or more of the following features. The method may comprise (a) detecting a plurality of polymorphisms, such as 5 or more polymorphisms, for example all the polymorphisms such as single nucleotide polymorphisms (SNPs) as specified in (b) above. The method may comprise (b) detecting a plurality of haplotypes, such as 5 or more haplotypes, for example all the haplotypes as specified in (d) above. The method may comprise (c) detecting a plurality of genes, polypeptides encoded by the genes or markers. The method may comprise (d) generating a polymorphism profile, a haplotype profile or an expression profile of a plurality of genes. The method may comprise (e) detecting, or deriving a profile from, a gene, polypeptide encoded by a gene, marker, polymorphism or haplotype, by microarray hybridisation such as hybridisation to an Affymetrix microarray, or by real time polymerase chain reaction (RT-PCR). The method may comprise (f) performing a computer implemented method comprising processing expression or polymorphism data for one or more genes, polypeptides encoded by the genes, markers, polymorphisms or haplotypes set out above in a computer.

We provide, according to a 9^{th} aspect of the invention, a method for the diagnosis or detection of Kawasaki Disease. The method may comprise detecting expression, such as a change in the expression pattern or level, of a gene comprising NAALADL2 or CAMK2D, or a polypeptide encoded by such a gene or a sequence having at least 90% sequence identity thereto.

There is provided, in accordance with a 10^{th} aspect of the present invention, a diagnostic kit for Kawasaki Disease or susceptibility thereto. The diagnostic kit may comprise any one or more of the following. It may comprise a molecule capable of binding to a polypeptide (a) CACNA2D3; (b) CAMK2D; (c) KCNIP4; (d) ANGPT1; (e) NAALADL2; (f) ZFHX3; (g) MPHOSPH10 or a sequence having at least 90% sequence identity thereto. The molecule may comprise an antibody. The diagnostic kit may comprise a nucleic acid capable of encoding such. The diagnostic kit may comprise a nucleic acid capable of binding to a nucleic acid selected from the group consisting of: (a) CACNA2D3; (b) CAMK2D; (c) KCNIP4; (d) ANGPT1; (e) NAALADL2; (f) ZFHX3; (g) MPHOSPH10 or a sequence having at least 90% sequence identity thereto. The nucleic acid may be one which is capable of discriminating between polymorphisms for example by being capable of binding to one polymorphism at a particular location, but not capable of binding to another polymorphism at that location.

As an 11^{th} aspect of the invention, we provide a combination comprising a plurality of markers, polymorphisms or haplotypes set out in (a) to (d) above such as in the form of an array, such as a microarray.

We provide, according to a 12^{th} aspect of the invention, there is provided a data carrier comprising information set out in (a) to (d) above.

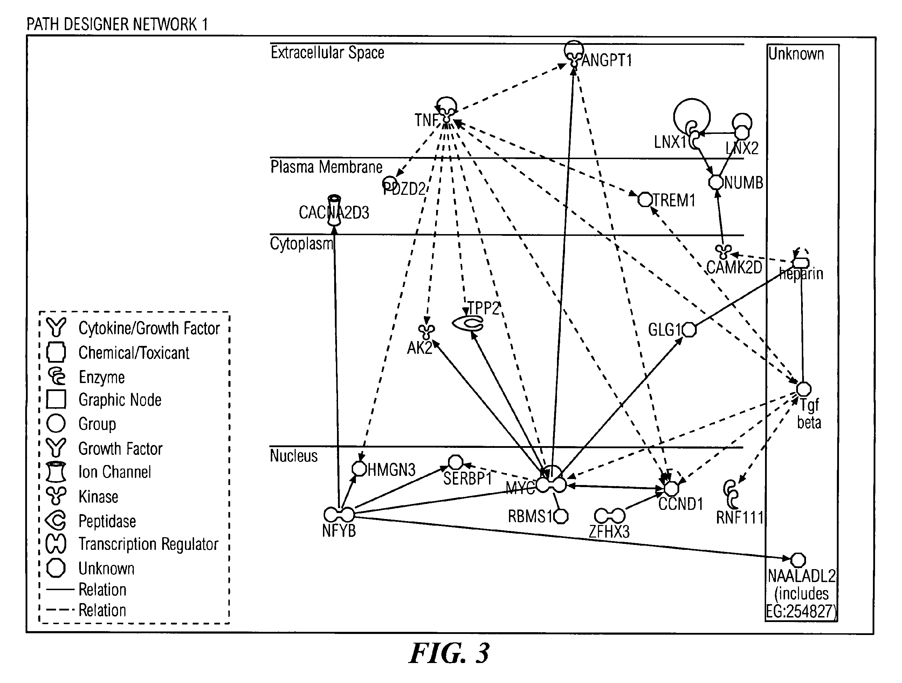

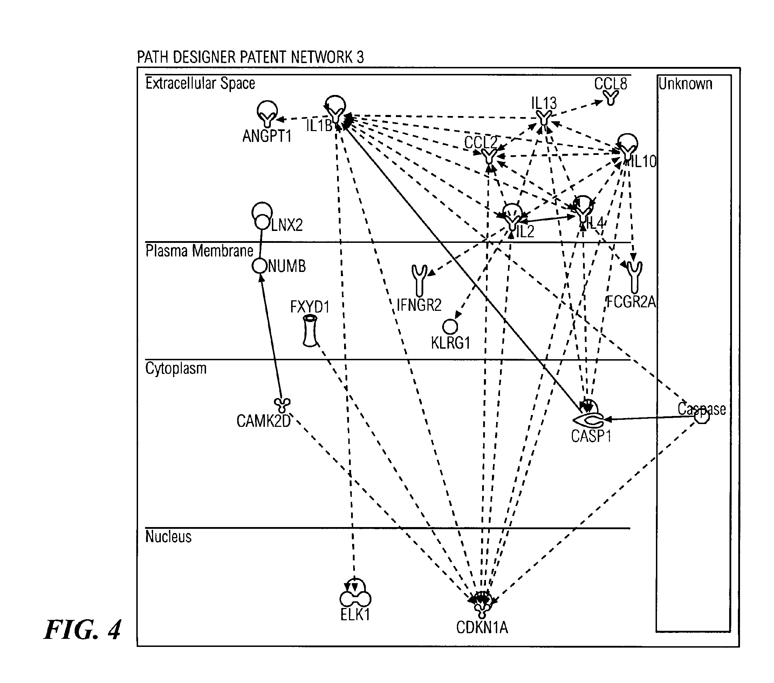

The method may further comprise detecting or modulating a gene, polypeptide encoded by a gene, marker, polymorphism or haplotype shown in Table 2, Table 3, Table 4, Table S1, Table S2, Figure 3 or Figure 4 or a sequence having at least 90% sequence identity thereto

We describe a method for the treatment or prevention of Kawasaki Disease in an individual, in which the method comprises modulating the expression of a gene selected from the group consisting of: (a) CACNA2D3; (b) CAMK2D; (c) KCNIP4; (d) ANGPT1; (e) NAALADL2; (f) ZFHX3; (g) MPHOSPH10 or a sequence having at least 90% sequence identity thereto, or the amount or activity of a polypeptide encoded by such a gene.

We further describe such a method which comprises administering an agonist or antagonist of a polypeptide (a) CACNA2D3; (b) CAMK2D; (c) KCNIP4; (d) ANGPT1; (e) NAALADL2; (f) ZFHX3; (g) MPHOSPH10 or a sequence having at least 90% sequence identity thereto.

The method may comprise administering a therapeutically effective amount of Pregabalin ((3S)-3-(aminomethyl)-5-methyl-hexanoic acid), a compound similar in structure to, a compound functionally equivalent to, a chemical derivative of, a chemical modification of, a substituted, a pharmaceutically acceptable salt of, a polymorphic form of, an isotopic variation of, a prodrug of, a pro-moiety of or a salt of Pregabalin, to an individual.

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; Using Antibodies : A Laboratory Manual : Portable Protocol NO. I by Edward Harlow, David Lane, Ed Harlow (1999, Cold Spring Harbor Laboratory Press, ISBN 0-87969-544-7); Antibodies : A Laboratory Manual by Ed Harlow (Editor), David Lane (Editor) (1988, Cold Spring Harbor Laboratory Press, ISBN 0-87969-314-2), 1855. Handbook of Drug Screening, edited by Ramakrishna Seethala, Prabhavathi B. Fernandes (2001, New York, NY, Marcel Dekker, ISBN 0-8247-0562-9); and Lab Ref: A Handbook of Recipes, Reagents, and Other Reference Tools for Use at the Bench, Edited Jane Roskams and Linda Rodgers, 2002, Cold Spring Harbor Laboratory, ISBN 0-87969-630-3. Each of these general texts is herein incorporated by reference.

### BRIEF DESCRIPTION OF THE FIGURES

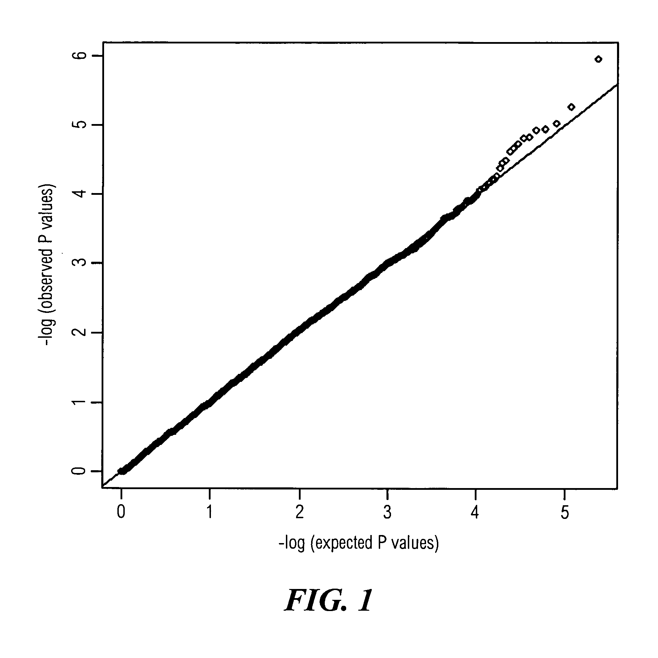

Figure 1. Quantile-quantile plot for allelic distributions. Allelic association analysis of expected versus observed P-values of 223,922 SNPs in 107 Kawasaki Disease cases and 134 controls. Red dots showing deviations from the line of equality indicate either that the theoretical distribution is incorrect, or that the sample is contaminated with values generated by a true association.

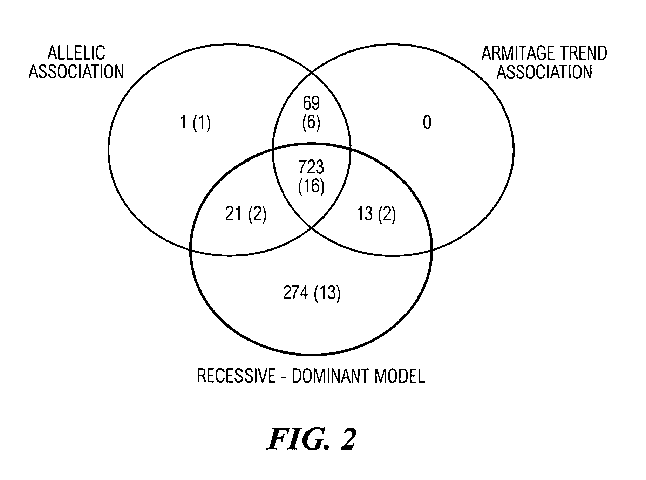

Figure 2. Selection and verification of SNPs and haplotypes from the GWAS analysis. GWAS SNPs and haplotypes are ranked by P-value and the top 1101 (of 14,065 total associated) SNPs and 35 (of 3,549 non-overlapping total associated) haplotypes are carried through to the follow-up stage. Numbers in parenthesis indicate the number of variants selected from the GWAS by each method of selection that are subsequently replicated in the family-based study.
Figure 3. Putative gene network derived from Ingenuity Pathway Analysis software. Edges are displayed with labels that describe the nature of the relationship between the nodes. The lines in between genes represent known interactions, with solid lines representing direct interactions and dashed lines representing indirect interactions. Nodes are displayed using various shapes that represent the functional class of the gene product. Orange highlighting indicates significantly associated genes and non-highlighted represent genes identified in the network. Genes which showed differential expression in acute versus convalescent Kawasaki Disease are colored red if expression is significantly higher during acute Kawasaki Disease, green if expression is significantly lower and white if there is no significant change in expression. Color intensity is related to fold change.
Figure 4. Putative gene network derived from Ingenuity Pathway Analysis software.

### DETAILED DESCRIPTION

### KAWASAKI DISEASE GENES

The present invention is based on the demonstration that certain genes comprise genetic determinants of Kawasaki Disease susceptibility. We performed a genome wide association study (GWAS) in 119 Caucasian Kawasaki Disease cases and 135 ethnically matched controls using an Affymetrix 250K SNP chip.

Significant associations of 40 SNPs and 6 haplotypes, identifying 31 genes, were confirmed in 583 independent Kawasaki Disease families. Accordingly, we describe a method for the diagnosis or detection of Kawasaki Disease, the method comprising detecting a gene, polypeptide encoded by a gene; marker, polymorphism or haplotype shown in Table 2, Table 3, Table 4, Table S1, Table S2, Figure 3 or Figure 4 or a sequence having at least 90% sequence identity thereto. In particular, any one or more of the 31 genes mentioned above may be detected for this purpose.

15 of these 31 genes were differentially expressed in peripheral blood in acute vs. convalescent Kawasaki Disease. We therefore describe a method for the treatment or alleviation of Kawasaki Disease, the method comprising detecting or modulating a gene, polypeptide encoded by a gene, marker, polymorphism or haplotype shown in Table 2, Table 3, Table 4, Table S1, Table S2, Figure 3 or Figure 4 or a sequence having at least 90% sequence identity thereto, including the 15 genes mentioned above.

An intronic SNP in *NAALADL2* showed the strongest disease association (p_{combined}= 1.1x10⁻⁶) and the largest fold change (FC) in acute KD (FC = -6.7, p = 0.0003). Three SNPs in *ZFHX3* (alias *ATBF1*), involved in regulation of innate immunity, were also highly associated (p_{combined}= 5.3x10⁻⁴, 7.1x10⁻⁵ and 2.4x10⁻⁶), as were two SNPs within the angiogenic mediator *ANGPT1* gene (p_{combined}= 3.4x10⁻⁵, 5.4x10⁻⁵). *ANGPT1* was differentially expressed in acute Kawasaki Disease (FC = -3.2, p = 1.6x10⁻⁵).

We therefore describe a method for the diagnosis or detection of Kawasaki Disease, in which the method comprises detecting a polymorphism in or around a gene selected from NAALADL2, ANGPT1 and ZFHX3 or a sequence having at least 90% sequence identity thereto.

We further describe a method for the diagnosis or detection of Kawasaki Disease, in which the method comprises detecting a single nucleotide polymorphism (SNP) selected from the group consisting of: rs17531088, rs1010824, rs6469101, rs7199343, rs10852516 and rs11075953 as set out in Table 2 or Table S 1.

Disease associations were also detected in the genes MPHOSPH10, RBMS1 and LOC441938. We therefore describe a method for the diagnosis or detection of Kawasaki Disease, in which the method comprises detecting a haplotype in or around a gene selected from MPHOSPH10, RBMS1 and LOC441938 or a sequence having at least 90% sequence identity thereto. Such a method may comprise detecting a haplotype selected from the group consisting of: rs7558220, rs357752; and rs7558220, rs357752, rs1458868, rs357729 and rs11674899 as set out in Table 2 or Table S2.

Investigation of functional relationships between the associated genes using Ingenuity Pathway Analysis^{™} identified a single highly significant network of 35 genes related to cardiovascular disease, including twelve of the GWAS-associated genes.

Ten network-identified genes were also differentially expressed in acute Kawasaki Disease.

Detection of expression of any of the above genes, including those in Table 2, Table 3, Table 4, Table S1, Table S2, Figure 3 or Figure 4 or a sequence having at least 90% sequence identity thereto, such as detection of modulation of expression of such genes, may be used for the diagnosis or detection of Kawasaki Disease.

This is the first GWAS in an infectious disease and has identified novel and biologically plausible variants associated with Kawasaki Disease susceptibility that also highlight pathways common to other cardiovascular diseases.

**Table 4. List of genes associated with Kawasaki Disease (35)**

| Gene Name | Gene Transcript ID | Fold change | P value | Network ? | Genetic |
|---|---|---|---|---|---|
| ANGPT1 | NM 001146 | -3.2 | 1.6*10-5 | y | y |
| CAMK2D | NM 001221 | -2.6 | 6.3*10-8 | y | y |
| CCL2 | NM 002982 | -13.7 | 0.0000151 | y | |
| CCL8 | NM 005623 | -6.8 | 0.00232 | y | |
| CDKN1A | NM 078467 | 1.6 | 0.00183 | y | |
| ELK1 | NM 005229 | -2.1 | 0.00911 | y | |
| FCGR2A | NM 021642 | 2.5 | 0.0000545 | y | y |
| FXYD1 | NM 005031 | 7.3 | 0.0022 | y | |
| IFNGR2 | NM 005534 | 1.8 | 0.00000363 | y | y |
| IL10 | NM 000572 | 2.8 | 0.00000187 | y | y |
| IL13 | NM 002188 | -3.5 | 0.0122 | y | |
| IL1B | NM 000576 | 3 | 0.0000156 | y | |
| IL2 | NM 000586 | -10.5 | 0.00000588 | y | |
| IL4 | NM 172348 | -3.5 | 0.0199 | y | |
| KLRG1 | NM 005810 | -3.3 | 0.00000112 | y | |
| LNX2 | NM 153371 | -1.8 | 1.0*10-2 | y | |
| NUMB | NM 001005743 | -1.7 | 1.5*10-2 | y | |
| ACE | NM 152830 | -3.7 | 0.0000757 | n | |
| AK2 | NM 013411 | -1.7 | 6.7*10-3 | n | |
| CEP68 | NM 015147 | -1.7 | 9.8*10-6 | n | |
| CFH | NM 000186 | 5 | 0.0000231 | n | |
| COL28A1 | NM 001037763 | -3.4 | 1.3*10-2 | n | |
| CYLD | NM 015247 | -1.5 | 0.000129 | n | |
| FARP1 | NM 005766 | -3.2 | 7.3*10-3 | n | |
| GLG1 | NM 012201 | -1.5 | 7.9*10-3 | n | |
| HD | NM 032778 | -1.8 | 2.48074E-06 | n | |
| MARK1 | NM 018650 | -2.1 | 3.6*10-3 | n | |
| MINA | NM 033292 | -1.9 | 2.55246E-07 | n | |
| MPHOSPH10 | NM 005791 | -1.8 | 2.9*10-4 | n | |
| NAALADL2 | M 207015 | -6.6 | 3.5*10-4 | n | |
| PI16 | NM 153370 | -3.5 | 9.2*10-6 | n | |
| RBMS1 | NM 016839 | 1.9 | 3.9*10-7 | n | |
| RIOK1 | NM 153005 | -2.1 | 1.4*10-3 | n | |
| RPS16 | NM 001020 | -1.5 | 0.00526 | n | |
| TIFAB | NM 130848 | -2.8 | 0.00522 | n | |
| TPP2 | NM 003291 | -1.7 | 3.0*10-5 | n | |

### ZFHX3

*ZFHX3* encodes a large enhancer-binding transcription factor that is known to be polymorphic. ²⁵ ZFHX3 interacts with PIAS3 (protein inhibitor of activated STAT) that inhibits STAT3 (signal transducer and activator of transcription-3). ²⁶ STAT3 is activated by interleukin 6 (IL-6) a pro-inflammatory cytokine that involved in the early innate inflammatory response and in Kawasaki Disease pathogenesis. ²⁷

"ZFHX3" as the term is used in this document should be understood to refer to a nucleic acid sequence having GenBank Accession number N_006885.3 or a polypeptide sequence having GenBank Accession number NP_008816.3, as the case may be. Homologues, fragments, variants and derivatives of each of these sequences are also included, as explained below.

The following text is adapted from OMIM entry 104155.

ZFHX3 is also known as Zinc Finger Homeobox 3, At Motif-Binding Factor 1; Atbfl, At-Binding Transcription Factor I and Alpha-Fetoprotein Enhancer-Binding Protein. The chromosomal location of ZFHX3 is at gene map locus 16q22.3-q23.1.

Tissue-specific expression of the human alpha-fetoprotein (AFP) gene (OMIM reference 104150) is strongly stimulated by an enhancer present 3.3 to 4.9 kb upstream of the transcription initiation site. One of the enhancer elements contains an AT-rich core sequence (AT motif). To determine the nuclear factor in hepatoma cell lines that interacts with the human AFP enhancer AT motif, Morinaga et al. (1991) screened a hepatoma cDNA expression library with an AFP enhancer fragment that bore the AT motif. They succeeded in isolating a cDNA that can code for an AT motif-binding factor, which they termed ATBF1. This was the largest DNA-binding protein identified to that time and the first protein shown to contain multiple homeodomains and multiple zinc finger motifs. The protein had a predicted mass of 306 kD and contained 4 homeodomains and 17 zinc finger motifs.

Qi et al. (2008) identified 3 highly conserved regulatory elements in the promoter region of the mouse Pit1 gene (POU1F1; OMIM reference 173110) and found that Atbfl bound and activated Pit1 from 1 of these elements, EE-alpha. Pituitaries of mice with a hypomorphic Atbf1 allele showed decreased expression of the somatotrope marker, Gh (OMIM reference 139250), and almost no expression of the thyrotrope marker, Tshb (OMIM reference 188540). Qi et al. (2008) concluded that ATBF1 is required for early PIT1 transcriptional activation.

By fluorescence in situ hybridization, Yamada et al. (1995) mapped the ATBF1 gene to 16q22.3-q23.1. Yamada et al. (1996) used fluorescence in situ hybridization to assign the Atbf1 gene to mouse chromosome 8E1.

The long arm of chromosome 16 is frequently deleted in human cancers. Sun et al. (2005) presented evidence that the ATBF1 gene is a good candidate for the 16q22 tumor suppressor gene. They narrowed the region of deletion at 16q22 to 861 kb containing ATBF1. ATBF I mRNA was abundant in normal prostates but more scarce in approximately half of prostate cancers tested. In 24 of 66 (36%) cancers examined, Sun et al. (2005) identified 22 unique somatic mutations, many of which impaired ATBF1 function. Furthermore, ATBF1 inhibited cell proliferation. Sun et al. (2005) concluded that loss of ATBF1 is one mechanism that defines the absence of growth control in prostate cancer.

In 4 cases of low- to midgrade primary prostate cancer (OMIM reference176807), Sun et al. (2005) identified a 24-bp deletion in exon 10 of the ATBF1 gene, 10814de124, resulting in loss of 8 amino acids, beginning with codon 3381, in a glutamine-rich domain. The deletion was also identified in a microdissected high-grade primary metastasis.

### [End of text adapted from OMIM]

### ANGPT1

Two associated SNPs are located in the 3'UTR of *ANGPT1*, which encodes angiopoietin-1 (ANGPT1). *ANGPT1* showed significant down-regulated expression during acute Kawasaki Disease. ANGPT1 is an angiogenic mediator with important functions in vascular development; *Angpt*1-/- knockout mice have dilated vessels that are structurally abnormal and prone to rupture ²⁸; coronary artery dilatation is the hallmark of Kawasaki Disease and giant coronary aneurysms may rupture. ²⁹ In mature blood vessels ANGPT1 maintains the integrity of vascular endothelium, suppressing plasma leakage, inhibiting vascular inflammation and preventing endothelial cell apoptosis. ²⁸ In Kawasaki Disease, vascular leakage is strongly predictive of coronary artery damage and poor outcome. ³⁰ ANGPT1 and other angiogenic mediators such as ANGPT2 and vascular endothelial growth factor (VEGF) are central to angiogenesis, although their exact roles are unclear. ³¹ Interestingly, serum from Kawasaki Disease cases with coronary artery aneurysms has reduced angiogenic activity ³², although ANGPT1 levels have never been reported in Kawasaki Disease. Marked inflammation and angiogenesis is observed in coronary artery aneurysms and in the myocardium of Kawasaki Disease patients in fatal cases. ³³ Preclinical studies indicate that ANGPT1 may have a therapeutic potential in the treatment of endotoxemia and transplant artherosclerosis. ²⁸

"ANGPT1" as the term is used in this document should be understood to refer to a nucleic acid sequence having GenBank Accession number NM_001146.3 or a polypeptide sequence having GenBank Accession number NP_001137.2, as the case may be. Homologues, fragments, variants and derivatives of each of these sequences are also included, as explained below.

The following text is adapted from OMIM entry 601667.

ANGPT1 is also known as Angiopoietin 1. The chromosomal location of ANGPT1 is at gene map locus 8q22.

TIE2 (TEK; 600221) is a receptor-like tyrosine kinase expressed almost exclusively in endothelial cells and early hematopoietic cells and required for the normal development of vascular structures during embryogenesis. Davis et al. (1996) identified a secreted ligand for TIE2, termed angiopoietin-1, using a novel expression cloning technique that involved intracellular trapping and protection of the ligand in COS cells. The human gene encodes a 498-amino acid polypeptide with predicted coiled-coil and fibrinogen-like domains. The structure of angiopoietin-1 differs from that of known angiogenic factors or other ligands for receptor tyrosine kinases. Although angiopoietin-1 bound and induced the tyrosine phosphorylation of TIE2, it did not directly promote the growth of cultured endothelial cells. However, its expression and close proximity to developing blood vessels implicated angiopoietin-1 in endothelial developmental processes. See also angiopoietin-2 (601922).

Folkman and D'Amore (1996) pointed out that vascular abnormality in both mice and humans is defined by a receptor-ligand system on the vascular endothelial cell. An apparent defect in vascular remodeling can result from an activating mutation of the receptor (Vikkula et al., 1996), from the absence of the ligand (Suri et al., 1996), or from a deficiency of the TIE2 receptor itself (Sato et al., 1995). However, while each situation reveals a general abnormality in vascular remodeling, there may be subtle but important differences.

To explore the possibility that VEGF (192240) and angiopoietins collaborate during tumor angiogenesis, Holash et al. (1999) analyzed several different murine and human tumor models. The apparent association of tumor vessel regression, apoptosis, and disruption of endothelial cell interactions with support cells in rat C6 gliomas raised the possibility that blockade of the stabilizing action of Ang1 might be contributing to tumor vessel regression. Consistent with this possibility, Holash et al. (1999) noted that angiopoietin-1 was antiapoptotic for cultured endothelial cells and expression of its antagonist angiopoietin-2 was induced in the endothelium of co-opted tumor vessels before their regression. Diffuse angiopoietin-1 expression in human tumors resembled that seen in the rat model. Holash et al. (1999) suggested that a subset of tumors rapidly co-opts existing host vessels to form an initially well vascularized tumor mass. Perhaps as part of a host defense mechanism there is widespread regression of these initially co-opted vessels, leading to a secondarily avascular tumor and a massive tumor cell loss. However, the remaining tumor is ultimately rescued by robust angiogenesis at the tumor margin.

Loughna and Sato (2001) showed that the combinatorial function of angiopoietin-1 and the orphan receptor TIE1 (600222) is critical for the development of the right-hand side venous system but is dispensable for the left-hand side venous system. This finding revealed the existence of a distinct genetic program for the establishment of the right-hand side and left-hand side vascular networks well before the network asymmetry becomes morphologically discernible.

Geva et al. (2002) investigated VEGFA, ANGPT1, and ANGPT2 transcript profiles, and the protein products that they encode, in placentas from normotensive pregnancies throughout pregnancy. Quantitative real-time PCR analysis demonstrated that VEGFA and ANGPT1 mRNA increased in a linear pattern by 2.5% (not significant) and 2.8%/week (P = 0.034), respectively, whereas ANGPT2 decreased logarithmically by 3.5%/week (P = 0.0003). ANGPT2 mRNA was 400- and 100-fold higher than that of ANGPT1 and VEGFA, respectively, in the first trimester and declined to 20-fold and 7-fold in the third. In situ hybridization and immunohistochemical studies revealed that VEGFA was localized in cyto- and syncytiotrophoblast and perivascular cells, whereas ANGPT1 and ANGPT2 were only in syncytiotrophoblast and perivascular cells in the immature intermediate villi during the first and second trimesters, and mature intermediate and terminal villi during the third trimester. The authors concluded that these molecules may play important roles in placental biology and chorionic villus vascular development and remodeling in an autocrine/paracrine manner.

Interaction of hematopoietic stem cells (HSCs) with their particular microenvironments, known as stem cell niches, is critical for adult hematopoiesis in bone marrow. Arai et al. (2004) demonstrated that HSCs expressing the receptor tyrosine kinase TIE2 are quiescent and antiapoptotic and comprise a side population of HSCs that adhere to osteoblasts in the bone marrow niche. The interaction of TIE2 with its ligand, ANG 1, induced cobblestone formation of HSCs in vitro and maintained in vivo long-term repopulating activity of HSCs. Furthermore, ANG I enhanced the ability of HSCs to become quiescent and induced adhesion to bone, resulting in protection of the HSC compartment from myelosuppressive stress. These data suggested that the TIE2/ANG I signaling pathway plays a critical role in the maintenance of HSCs in a quiescent state in the bone marrow niche.

Ward et al. (2001) determined that the ANGPT1 gene contains 9 exons and spans 48.3 kb. Exons 1 to 5 encode the N terminus, the coiled-coil domain, and part of the hinge region, and exons 5 to 9 encode the remainder of the hinge region, the fibrinogen (see 134820)-like domain, and the C terminus.

By FISH and radiation hybrid analysis, Cheung et al. (1998) mapped the ANGPT1 gene to 8q22.3-q23. Using radiation hybrid analysis and FISH, Grosios et al. (1999) also mapped the ANGPT1 gene to chromosome 8q22.3-q23. By FISH, Valenzuela et al. (1999) mapped the ANGPT1 gene to 8q22 in a region that shows homology of synteny to mouse chromosome 15, where they mapped the mouse Angpt1 gene. However, by indirect in situ PCR and FISH, Marziliano et al. (1999) mapped the Angpt1 gene in the mouse to chromosome 9E2.

Suri et al. (1996) showed that mice engineered to lack angiopoietin-1 display angiogenic defects reminiscent of those previously seen in mice lacking Tie2, demonstrating that angiopoietin-1 is a primary physiologic ligand for Tie2 and that it has critical in vivo angiogenic actions that are distinct from vascular endothelial growth factor (VEGF; 192240) and that are not reflected in the classic in vitro assays used to characterize VEGF. They concluded that angiopoietin-1 appears to play a crucial role in mediating reciprocal interactions between the endothelium and surrounding matrix and mesenchyme.

Targeted gene inactivation studies in mice show that vascular endothelial growth factor is necessary for the early stages of vascular development and that angiopoietin-1 is required for the later stages of vascular remodeling. Suri et al. (1998) showed that transgenic overexpression of angiopoietin-1 in the skin of mice produces larger, more numerous, and more highly branched vessels. These results raised the possibility that angiopoietins can be used, alone or in combination with VEGF, to promote therapeutic angiogenesis.

Thurston et al. (1999) compared transgenic mice overexpressing either Vegf or Ang1 in the skin. Vegf-induced blood vessels were leaky, whereas those induced by Ang1 were not. Moreover, vessels in Ang1-overexpressing mice were resistant to leaks caused by inflammatory agents. Coexpression of Ang1 and Vegf had an additive effect on angiogenesis but resulted in leakage-resistant vessels typical of Ang1. Thurston et al. (1999) concluded that ANG1, therefore, may be useful for reducing microvascular leakage in diseases in which the leakage results from chronic inflammation or elevated VEFG and, in combination with VEGF, for promoting growth of nonleaky vessels.

### [End of text adapted from OMIM]

### NAALADL2

The functions of other genes identified by associated SNPs and haplotypes are less well understood. The most significantly associated gene (*NAALADL2*), which also showed the greatest change in expression between acute and convalescent Kawasaki Disease, is a very large gene of 32 exons spanning 1.37Mb. *NAALADL2* undergoes extensive alternative splicing leading to multiple 5' and 3' untranslated regions and variable coding sequences. Its function is largely unknown. ³⁴

"NAALADL2" as the term is used in this document should be understood to refer to a nucleic acid sequence having GenBank Accession number NM_207015.2 or a polypeptide sequence having GenBank Accession number NP_996898.2 , as the case may be. Homologues, fragments, variants and derivatives of each of these sequences are also included, as explained below.

The following text is adapted from OMIM entry 608806.

NAALADL2 is also known as N-Acetylated Alpha-Linked Acidic Dipeptidase-Like 2. The chromosomal location of NAALADL2 is at gene map locus 3q26.3.

In the course of studying a Cornelia de Lange (122470)-associated translocation involving chromosomes 3 and 17, Tonkin et al. (2004) identified in the breakpoint on 3q26.3 a previously uncharacterized gene, designated N-acetylated alpha-linked acidic dipeptidase-like-2 (NAALADL2). Northern blot analysis identified up to 6 different transcripts in the 1- to 10-kb range with strongest expression in kidney and placenta; embryonic expression was largely confined to duodenal and stomach endoderm, mesonephros, metanephros, and pancreas. Transcript analysis identified extensive alternative splicing leading to multiple 5-prime and 3-prime untranslated regions and variable coding sequences. Multiple protein isoforms were defined by different N-terminal regions (with at least 4 alternative initiating methionine codons), and by differential protein truncation/use of alternative C-terminal sequences attributable to alternative splicing/polyadenylation. Outside the N-terminal regions, the predicted proteins showed significant homology to N-acetylated alpha-linked acidic dipeptidase and transferrin receptors. Full-length NAALADL2 encodes a deduced 795-amino acid protein which shares 74% sequence identity with the mouse ortholog.

Tonkin et al. (2004) determined that the NAALADL2 gene contains at least 32 exons spanning 1.37 Mb.

In a cell line from a patient with Cornelia de Lange syndrome, Tonkin et al. (2004) used FISH to map the breakpoints of a t(3;17) translocation to chromosomes 3q26.3 and 17q23.1, respectively. The NAALADL2 gene was disrupted by the 3q26.3 breakpoint.

In a panel of DNA samples from patients with Cornelia de Lange syndrome, Tonkin et al. (2004) performed mutation screening of the NAALADL2 gene but failed to identify patient-specific mutations.

### [End of text adapted from OMIM]

### KCNIP4

*KCNIP4* (voltage-dependent potassium (Kv) channel interacting protein 4, KChIP-4,) encodes one of a family of potassium channel-interacting proteins that are expressed predominantly in the heart and brain. It interacts with A-type potassium channels and modulates neuronal excitability in response to changes in intracellular calcium. ³⁵ KCNIP proteins bind cardiotoxins. ³⁶ *In vitro* cultured cardiomyocytes exposed to TNF have significantly down-regulated Kv4.2 and KChIP-2 expression, which has been suggested as a mechanism of electrical remodeling of cardiomyocytes under inflammatory conditions ³⁷ and cardiac arrythmias are reported in acute Kawasaki Disease.³⁸

"KCNIP4 " as the term is used in this document should be understood to refer to a nucleic acid sequence having GenBank Accession number NM_001035003.1or a polypeptide sequence having GenBank Accession number NP_001030175.1, as the case may be. Homologues, fragments, variants and derivatives of each of these sequences are also included, as explained below.

The following text is adapted from OMIM entry 608182.

KCNIP4 is also known as Potassium Channel-Interacting Protein 4, Kchip4, Calsenilin-Like Protein; CALP. The chromosomal location of KCNIP4 is at gene map locus 4p15.3.

K channel-interacting proteins, such as KCNIP4, specifically modulate the activity of Kv4 A-type potassium channels (see KCND1; 300281). Kv4 A-type channels contribute to the frequency of slow repetitive firing and back-propagation of action potentials in neurons and shape the action potential in heart (Holmqvist et al., 2002).

Holmqvist et al. (2002) cloned mouse Kcnip4, which they designated Kchip4. By analyzing human and rodent databases, they identified several KCNIP4 splice variants that differ from each other mostly in the N-terminal domain. Human and mouse variants that include an N-terminal 34-amino acid K channel inactivation suppressor (KIS) domain share 100% amino acid identity. Northern blot analysis of rat tissues detected Kcnip4 expression in brain, but not in heart or any other tissue examined. RT-PCR of mouse tissues detected expression of 2 splice variants in brain, but not in atrium or ventricle.

Using the C terminus of presenilin-2 (PS2; 600759) as bait in a yeast 2-hybrid screen, followed by library screening and PCR of a brain cDNA library, Morohashi et al. (2002) cloned 2 splice variants of KCNIP4, which they designated CALP. One variant encodes a 216-amino acid protein, and the other encodes a 250-amino acid protein with an N-terminal insert. The N-terminal sequence of KCNIP4 diverges from the N termini of other KCNIP proteins, but its C terminus shares 79.6% and 77.6% identity with the C termini of KCNIP2 (604661) and KCNIP3 (CSEN; 604662), respectively. Northern blot analysis detected KCNIP4 expression limited to brain, with expression in all specific brain regions examined, including frontal, temporal, parietal, and occipital cortices and cerebellum. KCNIP4 diffusely distributed in the cytoplasm and nuclei of transiently transfected COS cells. However, upon cotransfection with PS2, KCNIP4 redistributed in a reticular pattern that overlapped with PS2 in the perinuclear area and endoplasmic reticulum membranes. Western blot analysis detected endogenous mouse Kcnip4 expressed predominantly as a protein of about 28 kD.

Holmqvist et al. (2002) determined that coexpression of mouse Kcnip4 with Kv4 alpha subunits abolished fast inactivation of the Kv4 current in various cell types, including rat cerebellar granule neurons. The KIS domain of Kcnip4 delayed Kv4.3 (KCND3; 605411) opening, but, once the channel opened, it disrupted rapid inactivation and slowed Kv4.3 closing. Accordingly, Kcnip4 increased the open probability of single Kv4.3 channels. The net effects of Kcnip4 and other Kcnip subunits on Kv4 gating were quite different. When both Kcnip4 and Kcnip1 (604660) were present, the Kv4.3 current showed mixed inactivation profiles depending on the Kcnip4/Kcnip1 ratios. The KIS domain converted the A-type Kv4 current to a slowly inactivating delayed rectifier-type potassium current.

By yeast 2-hybrid analysis, Morohashi et al. (2002) determined that KCNIP4 interacts with the C termini of PS2 and PS1 (104311). By Ca(2+) overlay assay, they determined that the EF-hand motif of KCNIP4 binds calcium. Using mutation analysis and coimmunoprecipitation experiments, they showed that the EF-hand motif mediates the interaction between KCNIP4 and rat Kv4. Overexpression of KCNIP4 did not alter the metabolism or stability of PS, but overexpression of KCNIP4 with rat Kv4.2 (KCND2; 605410) reconstituted the features of A-type K+ currents. Morohashi et al. (2002) concluded that KCNIP4 is a novel EF-hand protein that interacts with both PS and Kv4.

By genomic sequence analysis, Morohashi et al. (2002) mapped the KCNIP4 gene to chromosome 4.

### [End of text adapted from OMIM]

### CACNA2D3 and CAMK2D

CACNA2D3 and CAMK2D are two genes associated with MYC and NFYB and are particularly appealing candidates in Kawasaki Disease.

CACNA2D3 (calcium channel, voltage-dependent, alpha 2/delta 3 subunit), is expressed in the heart and may bind plasma ligands such as cytokines and lead to calcium signaling ⁴⁵

CAMK2D (calcium/calmodulin-dependent protein kinase (CaM kinase) II delta), is also expressed in the heart and implicated in calcium signaling.

CAMK2D is involved in regulation of class II histone deacetylases, which are repressors of cardiac hypertrophy, suggesting broad roles in signaling pathways within the heart. ⁴⁶ In addition, CAMK2D regulates endotoxin- and TNF-mediated apopotosis in human promonocytic cells ⁴⁷ and delayed apoptosis of leukocytes is characteristic of acute Kawasaki Disease and may contribute to pathogenesis. ⁴⁸

CAMK2D interacts with LNX1 (ligand of numb-protein X 1), which act as scaffolds for the NUMB family of proteins, including NUMBL (numb homolog (Drosophila)-like), whose gene interestingly lies in the same small haplotype block recently associated with Kawasaki Disease susceptibility by a linkage study. ⁴⁹ LNX1 binds the Coxsackievirus and adenovirus receptor (CAR). ⁵⁰ CAR is the receptor for coxsackievirus B3, which causes myocarditis in humans and which can be prevented in animal models by antagonizing viral binding to CAR. ⁵¹ Coxsackievirus B3 has also been implicated in acute myocardial infarction. ⁵²

Interestingly, the human endogenous retrovirus K protein Np9 also interacts with LNX1 ⁵³ and therefore a number of viruses may theoretically bind the NUMB/CAR/LNX1 complex, leading to internalization and CAMK2D activity. This suggests a possible mechanism where more than one infectious trigger may result in cardiovascular damage in Kawasaki Disease in genetically susceptible individuals.

### CACNA2D3

"CACNA2D3" as the term is used in this document should be understood to refer to a nucleic acid sequence having GenBank Accession number NM_018398.2or a polypeptide sequence having GenBank Accession number NP_060868.2, as the case may be. Homologues, fragments, variants and derivatives of each of these sequences are also included, as explained below.

The following text is adapted from OMIM entry 606399.

CACNA2D3 is also known as Calcium Channel, Voltage-Dependent, Alpha-2/Delta Subunit 3. The chromosomal location of CACNA2D3 is at gene map locus 3p21.1.

Deletion of chromosome 3p is the most frequent genetic change in sporadic and von Hippel-Lindau disease (VHL; 193300)-associated conventional renal cell carcinomas (RCCs). Hanke et al. (2001) detected loss of heterozygosity (LOH) of approximately 1 cM at 3p21.1 in an RCC. By shotgun BAC sequencing and analysis of novel microsatellites, followed by 5-prime and 3-prime RACE, they obtained a cDNA encoding CACNA2D3, a calcium channel subunit gene. The predicted 997-amino acid protein has significant homology to mouse Cacna2d3 but lacks the N-terminal 44 amino acids of the mouse protein. RT-PCR analysis detected CACNA2D3 expression in all fetal tissues and most adult tumors tested. The authors found that the LOH involved exclusively intronic sequences.

Hanke et al. (2001) determined that the CACNA2D3 gene spans 500 kb.

By BAC and microsatellite analyses, Hanke et al. (2001) mapped the CACNA2D3 gene to 3p21.1.

### [End of text adapted from OMIM]

### CAMK2D

"CAMK2D" as the term is used in this document should be understood to refer to a nucleic acid sequence haying GenBank Accession number NM_001221.3or a polypeptide sequence having GenBank Accession number NP_001212.2, as the case may be. Homologues, fragments, variants and derivatives of each of these sequences are also included, as explained below.

The following text is adapted from OMIM entry 607708.

CAMK2D is also known as Calcium/Calmodulin-Dependent Protein Kinase II-Delta. The chromosomal location of CAMK2D is at gene map locus Chr.4.

CAMK2D belongs to a family of type II multifunctional calcium- and calmodulin (see 114180)-dependent protein kinases. There are at least 4 CAMK2 genes, and each can yield several isoforms through alternative splicing. CAMK2 isoforms assemble into homo- or heteromultimeric holoenzymes composed of 8 to 12 subunits.

Tombes and Krystal (1997) identified a CAMK2D variant that was the predominant CAMK2 clone found in a screen of several human tumor cell line and brain cDNA libraries. This variant was expressed in every nonneuronal cell line examined, regardless of malignant status. Another CAMK2D variant was expressed exclusively in rodent and human cerebral cortex.

Hoch et al. (1999) cloned several isoforms of CAMK2D from human cardiac and skeletal muscle. Both cardiac and skeletal muscle expressed unique dominant isoforms. The deduced protein encoded by I major variant has 499 amino acids.

By semiquantitative RT-PCR of left ventricular tissues from normal hearts and from patients suffering from dilated cardiomyopathy, Hoch et al. (1999) determined that expression of the major cardiac CAMK2D transcript was significantly increased with disease. Transcript levels of the other CAMK2 isoforms remained at control levels.

The transition from juvenile to adult life is accompanied by programmed remodeling in many tissues and organs, which is key for organisms to adapt to the demand of the environment. Xu et al. (2005) reported a regulated alternative splicing program that is crucial for postnatal heart remodeling in the mouse. They identified the essential splicing factor Asf/Sf2 (SFRS1; 600812) as a key component of the program, regulating a restricted set of tissue-specific alternative splicing events during heart remodeling. Cardiomyocytes deficient in Asf/Sf2 displayed an unexpected hypercontraction phenotype due to a defect in postnatal splicing switch of Camk2d transcript. This failure resulted in mistargeting of the kinase to sarcolemmal membranes, causing severe excitation-contraction coupling defects.

In mouse cardiomyocytes, Zhu et al. (2003) demonstrated that beta-1-adrenergic receptor (ADRB1; 109630)-induced apoptosis was resistant to inhibition of PKA (see 176911). Rather, the Adrb1 proapoptotic effect was associated with non-Pka-dependent increases in intracellular Ca(2+) and Camk2 activity. Blocking the L-type Ca(2+) channel (see 114205), buffering intracellular Ca(2+), or inhibiting Camk2 activity fully protected cardiomyocytes against Adrb1-induced apoptosis; overexpressing the c splice variant of Camk2d markedly exaggerated the Adrb1 apoptotic effect. Zhu et al. (2003) concluded that CAMK2 constitutes a PKA-independent linkage of ADRB1 stimulation to cardiomyocyte apoptosis.

The International Radiation Hybrid Mapping Consortium mapped the CAMK2D gene to chromosome 4 (SHGC-8542).

Zhang et al. (2003) generated transgenic mice that expressed the c splice variant of Camk2d (Camk2d-c) under the control of the cardiac-specific alpha-myosin heavy chain promoter. The mice developed a dilated cardiomyopathy with a decrease in fractional shortening of up to 65% and died prematurely. Isolated myocytes were enlarged and exhibited reduced contractility and altered Ca(2+) handling. Phosphorylation of the ryanodine receptor-2 (RYR2; 180902) at the Camk2 site was increased even before the development of heart failure, and Camk2 was found associated with Ryr2 in immunoprecipitates from the Camk2 transgenic mice. Phosphorylation of phospholamban (172405) was also increased specifically at the Camk2, but not the Pka, phosphorylation site. These findings demonstrated that Camk2d-c can mediate phosphorylation of Ca(2+) regulatory proteins in vivo, and provided evidence for the involvement of CAMK2D-C activation in the pathogenesis of dilated cardiomyopathy and heart failure.

Using gene targeting, Zhang et al. (2005) developed a mouse model of cardiac Camk2 inhibition and demonstrated substantial prevention of maladaptive remodeling from excessive beta-adrenergic receptor (see 109630) stimulation and myocardial infarction, and induction of balanced changes in excitation-contraction coupling that preserved baseline and beta-adrenergic receptor-stimulated physiologic increases in cardiac function. Zhang et al. (2005) concluded that CAMK2 is a determinant of clinically important heart disease phenotypes.

### [End of text adapted from OMIM]

### MPHOSPH10

"MPHOSPH 10" as the term is used in this document should be understood to refer to a nucleic acid sequence having GenBank Accession number NM_005791.1or a polypeptide sequence having GenBank Accession number NP_005782.1, as the case may be. Homologues, fragments, variants and derivatives of each of these sequences are also included, as explained below.

The following text is adapted from OMIM entry 605503.

MPHOSPH10 is also known as M-Phase Phosphoprotein 10 or MPP10.

Progression of cells from interphase to mitosis involves alterations in cell structures and activities. The transition from G2 to M phase is induced by M phase-promoting factor, or MPF (see CCNB1; 123836). In M phase, many proteins are phosphorylated directly by MPF or indirectly by kinases activated by MPF. These M-phase phosphoproteins (MPPs, or MPHOSPHs) permit disassembly of interphase structures and generation of M-phase enzymatic activities and structures.

By treating bacterial expression libraries with M-phase cytosol containing the relevant kinases in the presence of a phosphatase inhibitor, followed by immunoscreening with MPM2 antibody, Matsumoto-Taniura et al. (1996) isolated cDNAs for a number of MPHOSPHs, including MPHOSPH10, which they called MPP10. Immunoblot analysis showed that when phosphorylated, MPHOSPH10 is expressed as a 120-kD protein in M-phase cells. Immunofluorescence microscopy localized MPHOSPH10 in the nucleus, almost entirely in nucleoli, in interphase. In mitotic cells, MPHOSPH10 adhered to chromosomes in a manner similar to fibrillarin (FBL; 134795).

Westendorf et al. (1998) obtained a full-length cDNA encoding MPHOSPH10 by screening cDNA libraries, PCR, and 5-prime RACE. Sequence analysis predicted that the 681-amino acid protein has a PEST sequence and 6 coiled-coil alpha helices containing acidic and basic stretches found in many nucleolar proteins. Cell fractionation and immunoblot analysis confirmed the nuclear and nucleolar localization of MPHOSPH10. Detailed examination of different phases of the cell cycle by immunofluorescence microscopy showed that MPHOSPH10 colocalizes with fibrillarin and is a component of U3 small nucleolar ribonucleoproteins (snoRNPs). However, Northern blot analysis established that MPHOSPH10 is not a major component of ribonuclear particles containing snoRNAs other than U3. Confocal microscopy demonstrated that MPHSPH10 does not colocalize in coiled bodies with FBL or with p80 coilin (COIL; 600272). MPHOSPH10 was found in telophase bodies distinct from most of the FBL-containing bodies. Westendorf et al. (1998) concluded that MPHOSPH10 is involved in rRNA processing.

### [End of text adapted from OMIM]

### RBMS1

RBMS1 is RNA binding motif, single stranded interacting protein 1 isoform c. "RBMS1" as the term is used in this document should be understood to refer to a nucleic acid sequence having GenBank Accession number NM_002897.4 or a polypeptide sequence having GenBank Accession number NP_002888.1, as the case may be. Homologues, fragments, variants and derivatives of each of these sequences are also included, as explained below.

### LOC441938

LOC441938 is similar to signal-regulatory protein beta 1.

"LOC441938" as the term is used in this document should be understood to refer to a sequence having GenBank Accession number NC_000002 (range 1664616..1593952) or NT_011387.8 (range 1656616..1585952). Homologues, fragments, variants and derivatives of each of these sequences are also included, as explained below.

### PREGABALIN

We provide for the use of a modulator, such as an antagonist, of CACNA2D3 in the treatment of Kawasaki Disease. The antagonise of CACNA2D3 may comprise Pregabalin ((3S)-3-(aminomethyl)-5-methyl-hexanoic acid). The method may preferably comprise administering the antagonist of CACNA2D3 to an individual.

Pregabalin, which is (3S)-3-(aminomethyl)-5-methyl-hexanoic acid, is also known as 148553-50-8, 3-isobutyl GABA, CI-1008, hexanoic acid, 3-(aminomethyl)-5-methyl-, (3S)-, hexanoic acid, 3-(aminomethyl)-5-methyl-, (S)-, PD 144723 and S-(+)-3-isobutylgaba. It is commercially available under the name "Lyrica".

Pregabalin has the systematic name hexanoic acid, 3-(aminomethyl)-5-methyl-, (3S)-. Its IUPAC name is (3S)-3-(aminomethyl)-5-methyl-hexanoic acid. The CAS Registry Number of Pregabalin is 148553-50-8 and the SMILES notation of Pregabalin is CC(C)C[C@@H](CC(=O)O)CN. The InChI of Pregabalin is InChI=1/C8H17NO2/c1-6(2)3-7(5-9)4-8(10)11/h6-7H,3-5,9H2,1-2H3,(H,10,11)/t7-/m0/s1/f7h10H. It has a Chemical Formula: C8H17NO2 and molecular weight 159.226. The PubChem Link for Pregabalin is 5486971.

Pregabalin is thought to be involved in allodynia. Therapeutic categories for Pregabalin are antiepileptic drug; non-narcotic analgesic. Indication (Status) for Pregabalin include fibromyalgia (Phase III) , HIV infection (Phase III) , osteoarthritis (Phase III) , pain (Phase III) , partial seizure (Approved) , peripheral neuropathy (Phase III) , postherpetic neuralgia (Approved), seizures (Phase III). The Target (Action) is thought to be CACNA2D1 (binder) , CACNA2D2 (binder) , CACNA2D3 (binder) , CACNA2D4 (binder).

As the term is used in this document, "Pregabalin", where the context allows, should be treated as including a compound similar in structure to, a compound functionally equivalent to, a chemical derivative of, a chemical modification of, a substituted, a pharmaceutically acceptable salt of, a polymorphic form of, an isotopic variation of, a prodrug of, a pro-moiety of or a salt of Pregabalin.

Pregabalin may be obtained commercially as Lyrica (Pfizer, Inc). It may be produced by a number of methods known in the art, for example chemical synthesis as described in US Patent 6,127,418. US Patent 6891059 describes a method of asymmetric synthesis of pregabalin.

Pregabalin, its synthesis and its properties, are described in for example US Patents 7,524,864, 7,517,910, 7,514,457, 7,511,158, 7,507,742, 7,507,732, 7,507,717, 7,507,546, 7,501,414, 7,494,998, 7,491,835, 7,488,846, 7,488,740, 7,485,636, 7,482,460, 7,482,375, 7,476,665, 7,470,812, 7,465,826, 7,462,738, 7,462,737, 7,456,218, 7,449,585, 7,446,220, 7,442,701, 7,442,370, 7,438,927, 7,438,893, 7,432,299, 7,429,580, 7,427,600, 7,425,631, 7,425,569, 7,423,169, 7,423,159, 7,423,054, 7,420,002, 7,419,981, 7,417,165, 7,414,156, 7,414,134, 7,410,945, 7,402,687, 7,393,873, 7,393,872, 7,393,852, 7,390,931, 7,390,798, 7,381,747, 7,374,779, 7,368,477, 7,368,472, 7,365,095, 7,354,937, 7,354,929, 7,354,925, 7,351,724, 7,338,968, 7,332,504, 7,332,499, 7,329,664, 7,329,659, 7,329,658, 7,326,729, 7,326,721, 7,326,414, 7,319,106, 7,312,233, 7,309,799, 7,309,790, 7,309,719, 7,309,712, 7,285,563, 7,279,486, 7,268,133, 7,262,192, 7,261,529, 7,256,216, 7,247,628, 7,241,788, 7,235,667, 7,235,657, 7,235,363, 7,232,924, 7,232,823, 7,230,135, 7,230,024, 7,227,028, 7,217,721, 7,214,824, 7,205,295, 7,199,244, 7,199,141, 7,196,079, 7,189,747, 7,186,855, 7,183,259, 7,176,210, 7,164,034, 7,151,097, 7,146,205, 7,145,012, 7,141,695, 7,141,669, 7,138,542, 7,138,406, 7,135,484, 7,129,239, 7,125,848, 7,122,683, 7,109,149, 7,074,814, 7,071,339, 7,067,262, 7,060,727, 7,060,708, 7,053,122, 7,049,305, 7,041,704, 7,038,085, 7,030,119, 7,026,505, 7,026,351, 7,026,332, 7,022,678, 6,992,110, 6,992,109, 6,992,076, 6,984,659, 6,984,634, 6,984,496, 6,977,070, 6,972,341, 6,962,973, 6,958,218, 6,955,888, 6,942,876, 6,927,036, 6,924,377, 6,923,988, 6,916,841, 6,900,192, 6,894,047, 6,891,059, 6,887,902, 6,855,849, 6,855,724, 6,849,629, 6,833,370, 6,833,140, 6,828,344, 6,828,328, 6,825,217, 6,818,787, 6,809,105, 6,797,708, 6,774,132, 6,761,903, 6,750,171, 6,730,674, 6,720,348, 6,720,001, 6,713,490, 6,703,522, 6,689,915, 6,680,343, 6,642,398, 6,635,675, 6,630,496, 6,630,490, 6,620,829, 6,605,745, 6,602,911, 6,596,900, 6,593,368, 6,579,879, 6,569,463, 6,566,400, 6,562,865, 6,544,998, 6,521,650, 6,500,853, 6,488,964, 6,451,857, 6,436,974, 6,426,368, 6,372,792, 6,359,005, 6,329,429, 6,326,374, 6,310,098, 6,309,663, 6,306,910, 6,267,985, 6,248,363, 6,242,488, 6,194,459 and 6,127,418.

### KAWASAKI DISEASE

The following references relate to Kawasaki syndrome: ICD-10 M30.3; ICD-9 446:1; OMIM 611775; DiseasesDB 7121; MedlinePlus 000989; eMedicine ped/1236; MeSH D009080.

Kawasaki disease (also known as lymph node syndrome, mucocutaneous node disease, infantile polyarteritis and Kawasaki syndrome) is an inflammation (vasculitis) of the middle-sized arteries. It affects many organs, including the skin, mucous membranes, lymph nodes, and blood vessel walls, but the most serious effect is on the heart where it can cause severe aneurysmal dilations. Without treatment, mortality may approach 1%, usually within 6 weeks of onset. With treatment, mortality is <0.01% in the U.S.[1] There is often a pre-existing viral infection that may play some role in pathogenesis. The conjunctival and oral mucosa, along with the epidermis (skin), become erythmatous (red and inflammed). Edema is often seen in the hands and feet and the cervical lymph nodes are often enlarged. Also, some degree of fever is often noted. Twenty percent of children affected have cardiovascular sequelae.

It was first described in 1967 by Dr. Tomisaku Kawasaki in Japan.[2]

### Incidence and risk factors

By far the highest incidence of Kawasaki disease occurs in Japan (175 per 100,000), though its incidence in the United States is increasing. Kawasaki disease is predominantly a disease of young children, with 80% of patients younger than 5 years of age. The disease affects boys more than girls. Approximately 2000-4000 cases are identified in the United States each year. [3] [4]

### Causes

Like all autoimmune diseases, the cause of Kawasaki disease is presumably the interaction of genetic and environmental factors, possibly including an infection. The specific cause is unknown,[5][6][7] but current theories center primarily on immunological causes for the disease. Evidence increasingly points to an infectious etiology,[8] but debate continues on whether the cause is a conventional antigenic substance or a superantigen.[9] Children's Hospital Boston reports that "[s]ome studies have found associations between the occurrence of Kawasaki disease and recent exposure to carpet cleaning or residence near a body of stagnant water; however, cause and effect have not been established."[4]

An association has been identified with a SNP in the ITPKC gene, which codes an enzyme that negatively regulates T-cell activation.[10] An additional factor that suggests genetic susceptibility is the fact that regardless of where they are living, Japanese children are more likely than other children to contract the disease.[4] The HLA-B51 serotype has been found to be associated with endemic instances of the disease.[11]

### Presentation

The cardiac complications are, by far, the most important aspect of the disease. Kawasaki disease can cause vasculitic changes (inflammation of blood vessels) in the coronary arteries and subsequent coronary artery aneurysms. These aneurysms can lead to myocardial infarction (heart attack) even in young children. Overall, about 10-18% of children with Kawasaki disease develop coronary artery aneurysms with much higher prevalence among patients who are not treated early in the course of illness. Kawasaki disease and rheumatic fever are most common causes of acquired heart disease among children in the United States.[3][4]

### Symptoms

Kawasaki disease often begins with a high and persistent fever that is not very responsive to normal doses of paracetamol (acetaminophen) or ibuprofen. The fever may persist steadily for up to two weeks and is normally accompanied by irritability. Affected children develop red eyes, red mucous membranes in the mouth, red cracked lips, a "strawberry tongue",[12] iritis, keratic precipitates (detectable by an ophthalmologist but usually too small to be seen by the unaided eye), and swollen lymph nodes. Skin rashes occur early in the disease, and peeling of the skin in the genital area, hands, and feet (especially around the nails and on the palms and soles) may occur in later phases. Some of these symptoms may come and go during the course of the illness. If left untreated, the symptoms will eventually relent, but coronary artery aneurysms will not improve, resulting in a significant risk of death or disability due to myocardial infarction (heart attack). If treated in a timely fashion, this risk can be mostly avoided and the course of illness cut short.

High-grade fever (greater than 39 °C or 102 °F; often as high as 40 °C or 104 °F) that normally lasts for more than 5 days if left untreated.
- Red eyes (conjunctivitis) without pus or drainage, also known as "conjunctival injection"
- Bright red, chapped, or cracked lips
- Red mucous membranes in the mouth
- Strawberry tongue, white coating on the tongue or prominent red bumps (papillae) on the back of the tongue
- Red palms of the hands and the soles of the feet
- Rash which may take many forms, but not vesicular (blister-like), on the trunk
- Swollen lymph nodes (frequently only one lymph node is swollen), particularly in the neck area
- Joint pain (arthralgia) and swelling, frequently symmetrical
- Irritability
- Tachycardia (rapid heart beat)
- Peeling (desquamation) palms and soles (later in the illness); peeling may begin around the nails
- Beau's lines (transverse grooves on nails)

### Signs and tests

A physical examination will demonstrate many of the features listed above.

### Blood tests

- Complete blood count (CBC) may reveal normocytic anemia and eventually thrombocytosis
- Erythrocyte sedimentation rate (ESR) will be elevated
- C-reactive protein (CRP) will be elevated
- Liver function tests may show evidence of hepatic inflammation and low serum albumin

### Other tests (may or may not be performed)

- Electrocardiogram may show evidence of ventricular dysfunction or, occasionally, arrhythmia due to myocarditis
- Echocardiogram may show subtle coronary artery changes or, later, true aneurysms.
- Ultrasound or computerized tomography may show hydrops (enlargement) of the gallbladder
- Urinalysis may show white blood cells and protein in the urine (pyuria and proteinuria) without evidence of bacterial growth
- Lumbar puncture may show evidence of aseptic meningitis
- Angiography was historically used to detect coronary artery aneurysms and remains the gold standard for their detection, but is rarely used today unless coronary artery aneurysms have already been detected by echocardiography.

### Diagnosis

Kawasaki disease can only be diagnosed clinically (by medical signs and symptoms), as there exists no specific laboratory test that can tell if someone has it. It is normally difficult to establish the diagnosis, especially early in the course of illness, and frequently children are not diagnosed until they have seen their doctor several times, or visited a number of different health care providers. Many other serious illnesses can cause similar symptoms, and must be considered in the differential diagnosis, including scarlet fever, toxic shock syndrome, and juvenile idiopathic arthritis.

Classically, five days of fever[13] plus four of five diagnostic criteria must be met in order to establish the diagnosis. The criteria are: (1) erythema of the lips or oral cavity or cracking of the lips; (2) rash on the trunk; (3) swelling or erythema of the hands or feet; (4) red eyes (conjunctival injection) (5) swollen lymph node in the neck of at least 15 millimeters.

Many children, especially infants, eventually diagnosed with Kawasaki disease do not exhibit all of the above criteria. In fact, many experts now recommend treating for Kawasaki disease even if only three days of fever have passed and at least three diagnostic criteria are present, especially if other tests reveal abnormalities consistent with Kawasaki disease. In addition, the diagnosis can be made purely by the detection of coronary artery aneurysms in the proper clinical setting.

### Treatment

Children with Kawasaki disease should be hospitalized and cared for by a physician who has experience with this disease. When in an academic medical center, care is often shared between pediatric cardiology and pediatric infectious disease specialists (although no specific infectious agent has been identified yet[4]). It is imperative that treatment be started as soon as the diagnosis is made to prevent damage to the coronary arteries.

Intravenous immunoglobulin (IVIG) is the standard treatment for Kawasaki disease[14] and is administered in high doses with marked improvement usually noted within 24 hours. If the fever does not respond, an additional dose may have to be considered. IVIG by itself is most useful within the first 7 days of onset of fever, in terms of preventing coronary artery aneurysm.

Salicylate therapy, particularly aspirin, remains an important part of the treatment (though questioned by some)[15] but salicylates alone are not as effective as Intravenous immunoglobulin. Aspirin therapy is started at high doses until the fever subsides, and then is continued at a low dose when the patient returns home, usually for 2 months to prevent blood clots from forming. Except for Kawasaki disease and a few other indications, aspirin is otherwise normally not recommended for children due to its association with Reye's syndrome.

Corticosteroids have also been used,[16] especially when other treatments fail or symptoms recur, but in a randomized controlled trial, the addition of corticosteroid to immune globulin and aspirin did not improve outcome. [17]

There are also treatments for iritis and other eye symptoms.

### Prognosis

With early treatment, rapid recovery from the acute symptoms can be expected and the risk of coronary artery aneurysms greatly reduced. Untreated, the acute symptoms of Kawasaki disease are self-limited (i.e. the patient will recover eventually), but the risk of coronary artery involvement is much greater. Overall, about 2% of patients die from complications of coronary vasculitis. Patients who have had Kawasaki disease should have an echocardiogram initially every few weeks, and then every 1-2 years to screen for progression of cardiac involvement.

It is also not uncommon that a relapse of symptoms may occur soon after initial treatment with IVIG. This usually requires re-hospitalization and retreatment. Treatment with IVIG can cause allergic and non-allergic acute reactions, aseptic meningitis, fluid overload and, rarely, other serious reactions. Aspirin may increase the risk of bleeding from other causes and may be associated with Reye's syndrome. Overall, life-threatening complications resulting from therapy for Kawasaki disease are exceedingly rare, especially compared with the risk of non-treatment.

The text in this section is adapted from the Wikipedia entry for "Kawasaki Disease". Kawasaki disease. (2009, April 23). In Wikipedia, The Free Encyclopedia. Retrieved 09:34, April 28, 2009, from http://en.wikipedia.org/w/index.php?title=Kawasaki_disease&oldid=285754914

### POLYPEPTIDES

The methods and compositions described here make use of CACNA2D3, CAMK2D, KCNIP4, ANGPT1, NAALADL2, ZFHX3 and MPHOSPH10 polypeptides, which are described in detail below.

Homologues variants and derivatives thereof of any, some or all of these polypeptides are also included.

CACNA2D3, CAMK2D, KCNIP4, ANGPT1, NAALADL2, ZFHX3 and MPHOSPH10 polypeptides may be used for a variety of means, for example, administration to an individual suffering from, or suspected to be suffering from, Kawasaki Disease, for the treatment thereof. They may also be used for production or screening of anti-CACNA2D3, CAMK2D, KCNIP4, ANGPT1, NAALADL2, ZFHX3 or MPHOSPH10 agents such as specific CACNA2D3, CAMK2D, KCNIP4, ANGPT1, NAALADL2, ZFHX3 or MPHOSPH10 binding agents, in particular, anti-CACNA2D3, CAMK2D, KCNIP4, ANGPT1, NAALADL2, ZFHX3 or MPHOSPH10 antibodies. These are described in further detail below. The expression of CACNA2D3, CAMK2D, KCNIP4, ANGPT1, NAALADL2, ZFHX3 or MPHOSPH10 polypeptides may be detected for diagnosis or detection of Kawasaki Disease.

A "polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids.

"Polypeptides" include amino acid sequences modified either by natural processes, such as post-translational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications.

Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-inking, cyclization, disulfide bond formation, demethylation, formation of covalent cross-inks, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, Proteins - Structure and Molecular Properties, 2nd Ed., T. E. Creighton, W. H. Freeman and Company, New York, 1993 and Wold, F., Posttranslational Protein Modifications: Perspectives and Prospects, pgs. 1-12 in Posttranslational Covalent Modification of Proteins, B. C. Johnson, Ed., Academic Press, New York, 1983; Seifter et al., "Analysis for protein modifications and nonprotein cofactors", Meth Enzymol (1990) 182:626-646 and Rattan et aL, "Protein Synthesis: Posttranslational Modifications and Aging", Ann NY Acad Sci (1992) 663:48-62.

The term "polypeptide" includes the various synthetic peptide variations known in the art, such as a retroinverso D peptides. The peptide may be an antigenic determinant and/or a T-cell epitope. The peptide may be immunogenic *in vivo*. The peptide may be capable of inducing neutralising antibodies *in vivo*.

As applied to CACNA2D3, CAMK2D, KCNIP4, ANGPT1, NAALADL2, ZFHX3 or MPHOSPH 10, the resultant amino acid sequence may have one or more activities, such as biological activities in common with a CACNA2D3, CAMK2D, KCNIP4, ANGPT1, NAALADL2, ZFHX3 or MPHOSPH10 polypeptide, for example a human CACNA2D3, CAMK2D, KCNIP4, ANGPT1, NAALADL2, ZFHX3 or MPHOSPH10 polypeptide. For example, a CACNA2D3, CAMK2D, KCNIP4, ANGPT1, NAALADL2, ZFHX3 or MPHOSPH 10 homologue may have an increased expression level in Kawasaki Disease cells compared to normal cells. In particular, the term "homologue" covers identity with respect to structure and/or function providing the resultant amino acid sequence has CACNA2D3, CAMK2D, KCNIP4, ANGPT1, NAALADL2, ZFHX3 or MPHOSPH10 activity. With respect to sequence identity (i.e. similarity), there may be at least 70%, such as at least 75%, such as at least 85%, such as at least 90% sequence identity. There may be at least 95%, such as at least 98%, sequence identity. These terms also encompass polypeptides derived from amino acids which are allelic variations of the CACNA2D3, CAMK2D, KCNIP4, ANGPT1, NAALADL2, ZFHX3 or MPHOSPH 10 nucleic acid sequence.

Where reference is made to the "activity" or "biological activity" of a polypeptide such as CACNA2D3, CAMK2D, KCNIP4, ANGPT1, NAALADL2, ZFHX3 or MPHOSPH10, these terms are intended to refer to the metabolic or physiological function of CACNA2D3, CAMK2D, KCNIP4, ANGPT1, NAALADL2, ZFHX3 or MPHOSPH10, including similar activities or improved activities or these activities with decreased undesirable side effects. Also included are antigenic and immunogenic activities of CACNA2D3, CAMK2D, KCNIP4, ANGPT I, NAALADL2, ZFHX3 or MPHOSPH10. Examples of such activities, and methods of assaying and quantifying these activities, are known in the art, and are described in detail elsewhere in this document.

### Other Polypeptides

CACNA2D3, CAMK2D, KCNIP4, ANGPT1, NAALADL2, ZFHX3 and MPHOSPH10 variants, homologues, derivatives and fragments are also of use in the methods and compositions described here.

The terms "variant", "homologue", "derivative" or "fragment" in relation to CACNA2D3, CAMK2D, KCNIP4, ANGPT1, NAALADL2, ZFHX3 or MPHOSPH10 include any substitution of, variation of, modification of, replacement of, deletion of or addition of one (or more) amino acid from or to a sequence. Unless the context admits otherwise, references to "CACNA2D3", "CAMK2D", "KCNIP4", "ANGPT1", "NAALADL2", "ZFHX3" or "MPHOSPH10" include references to such variants, homologues, derivatives and fragments of CACNA2D3, CAMK2D, KCNIP4, ANGPT1, NAALADL2, ZFHX3 or MPHOSPH10, as the case may be.

As used herein a "deletion" is defined as a change in either nucleotide or amino acid sequence in which one or more nucleotides or amino acid residues, respectively, are absent. As used herein an "insertion" or "addition" is that change in a nucleotide or amino acid sequence which has resulted in the addition of one or more nucleotides or amino acid residues, respectively, as compared to the naturally occurring substance. As used herein "substitution" results from the replacement of one or more nucleotides or amino acids by different nucleotides or amino acids, respectively.

CACNA2D3, CAMK2D, KCNIP4, ANGPT1, NAALADL2, ZFHX3 and MPHOSPH10 polypeptides as described here may also have deletions, insertions or substitutions of amino acid residues which produce a silent change and result in a functionally equivalent amino acid sequence. Deliberate amino acid substitutions may be made on the basis of similarity in polarity, charge, solubility, hydrophobicity, hydrophilicity, and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the table below. Amino acids in the same block in the second column and in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

CACNA2D3, CAMK2D, KCNIP4, ANGPT1, NAALADL2, ZFHX3 and MPHOSPH10 polypeptides may further comprise heterologous amino acid sequences, typically at the N-terminus or C-terminus, such as the N-terminus. Heterologous sequences may include sequences that affect intra or extracellular protein targeting (such as leader sequences). Heterologous sequences may also include sequences that increase the immunogenicity of the CACNA2D3, CAMK2D, KCNIP4, ANGPT1, NAALADL2, ZFHX3 or MPHOSPH10 polypeptide and/or which facilitate identification, extraction and/or purification of the polypeptides. Another heterologous sequence that may be used is a polyamino acid sequence such as polyhistidine which may be N-terminal. A polyhistidine sequence of at least 10 amino acids, such as at least 17 amino acids but fewer than 50 amino acids may be employed.

The CACNA2D3, CAMK2D, KCNIP4, ANGPT1, NAALADL2, ZFHX3 or MPHOSPH 10 polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

CACNA2D3, CAMK2D, KCNIP4, ANGPT1, NAALADL2, ZFHX3 or MPHOSPH10 polypeptides as described here are advantageously made by recombinant means, using known techniques. However they may also be made by synthetic means using techniques well known to skilled persons such as solid phase synthesis. Such polypeptides may also be produced as fusion proteins, for example to aid in extraction and purification. Examples of fusion protein partners include glutathione-S-transferase (GST), 6xHis, GAL4 (DNA binding and/or transcriptional activation domains) and β-galactosidase. It may also be convenient to include a proteolytic cleavage site between the fusion protein partner and the protein sequence of interest to allow removal of fusion protein sequences, such as a thrombin cleavage site. The fusion protein may be one which does not hinder the function of the protein of interest sequence.

The CACNA2D3, CAMK2D, KCNIP4, ANGPT1, NAALADL2, ZFHX3 or MPHOSPH10 polypeptides may be in a substantially isolated form. This term is intended to refer to alteration by the hand of man from the natural state. If an "isolated" composition or substance occurs in nature, it has been changed or removed from its original environment, or both. For example, a polynucleotide, nucleic acid or a polypeptide naturally present in a living animal is not "isolated," but the same polynucleotide, nucleic acid or polypeptide separated from the coexisting materials of its natural state is "isolated", as the term is employed herein.

It will however be understood that the CACNA2D3, CAMK2D, KCNIP4, ANGPT1, NAALADL2, ZFHX3 or MPHOSPH10 protein may be mixed with carriers or diluents which will not interfere with the intended purpose of the protein and still be regarded as substantially isolated. A CACNA2D3, CAMK2D, KCNIP4, ANGPT1, NAALADL2, ZFHX3 or MPHOSPH10 polypeptide may also be in a substantially purified form, in which case it will generally comprise the protein in a preparation in which more than 90%, for example, 95%, 98% or 99% of the protein in the preparation is a CACNA2D3, CAMK2D, KCNIP4, ANGPT1, NAALADL2, ZFHX3 or MPHOSPH10 polypeptide.

By aligning CACNA2D3, CAMK2D, KCNIP4, ANGPT1, NAALADL2, ZFHX3 or MPHOSPH 10 sequences from different species, it is possible to determine which regions of the amino acid sequence are conserved between different species ("homologous regions"), and which regions vary between the different species ("heterologous regions").

The CACNA2D3, CAMK2D, KCNIP4, ANGPT1, NAALADL2, ZFHX3 or MPHOSPH10 polypeptides may therefore comprise a sequence which corresponds to at least part of a homologous region. A homologous region shows a high degree of homology between at least two species. For example, the homologous region may show at least 70%, at least 80%, at least 90% or at least 95% identity at the amino acid level using the tests described above. Peptides which comprise a sequence which corresponds to a homologous region may be used in therapeutic strategies as explained in further detail below. Alternatively, the CACNA2D3, CAMK2D, KCNIP4, ANGPT1, NAALADL2, ZFHX3 or MPHOSPH10 peptide may comprise a sequence which corresponds to at least part of a heterologous region. A heterologous region shows a low degree of homology between at least two species.

### Homologues

The CACNA2D3, CAMK2D, KCNIP4, ANGPT1, NAALADL2, ZFHX3 or MPHOSPH 10 polypeptides disclosed for use include homologous sequences obtained from any source, for example related viral/bacterial proteins, cellular homologues and synthetic peptides, as well as variants or derivatives thereof. Thus polypeptides also include those encoding homologues of CACNA2D3, CAMK2D, KCNIP4, ANGPT1, NAALADL2, ZFHX3 or MPHOSPH10 from other species including animals such as mammals (e.g. mice, rats or rabbits), especially primates, more especially humans. More specifically, homologues include human homologues.

In the context of this document, a homologous sequence is taken to include an amino acid sequence which is at least 15, 20, 25, 30, 40, 50, 60, 70, 80 or 90% identical, such as at least 95 or 98% identical at the amino acid level, for example over at least 50 or 100, 110, 115, 120, 125, 130, 135, 140, 141, 142, 143, 144, 145, 146, 147, 148 or 149 amino acids with the sequence of a relevant CACNA2D3, CAMK2D, KCNIP4, ANGPT1, NAALADL2, ZFHX3 or MPHOSPH10 sequence.

In particular, homology should typically be considered with respect to those regions of the sequence known to be essential for protein function rather than non-essential neighbouring sequences. This is especially important when considering homologous sequences from distantly related organisms. An example is the cysteine residue at or corresponding to residue number 95 of the human CACNA2D3, CAMK2D, KCNIP4, ANGPT1, NAALADL2, ZFHX3 or MPHOSPH 10 protein, shown to be essential for phosphatase function, and surrounding residues.

Although homology can also be considered in terms of similarity (i.e. amino acid residues having similar chemical properties/functions), in the context of the present document homology may be expressed in terms of sequence identity.

Homology comparisons can be conducted by eye, or more usually, with the aid of readily available sequence comparison programs. These publicly and commercially available computer programs can calculate % identity between two or more sequences.

% identity may be calculated over contiguous sequences, i.e. one sequence is aligned with the other sequence and each amino acid in one sequence directly compared with the corresponding amino acid in the other sequence, one residue at a time. This is called an "ungapped" alignment. Typically, such ungapped alignments are performed only over a relatively short number of residues (for example less than 50 contiguous amino acids).

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local identity or similarity.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, the default values may be used when using such software for sequence comparisons. For example when using the GCG Wisconsin Bestfit package (see below) the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U.S.A; Devereux et al., 1984, Nucleic Acids Research 12:387). Examples of other software than can perform sequence comparisons include, but are not limited to, the BLAST package (see Ausubel *et al*., 1999 *ibid -* Chapter 18), FASTA (Altschul et al., 1990, J. Mol. Biol., 403-410) and the GENEWORKS suite of comparison tools. Both BLAST and FASTA are available for offline and online searching (see Ausubel *et al*., 1999 *ibid*, pages 7-58 to 7-60). The GCG Bestfit program may be used.

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). The public default values for the GCG package may be used, or in the case of other software, the default matrix, such as BLOSUM62.

Once the software has produced an optimal alignment, it is possible to calculate % homology, such as % sequence identity. The software typically does this as part of the sequence comparison and generates a numerical result.

The terms "variant" or "derivative" in relation to amino acid sequences includes any substitution of, variation of, modification of, replacement of, deletion of or addition of one (or more) amino acids from or to the sequence providing the resultant amino acid sequence retains substantially the same activity as the unmodified sequence, such as having at least the same activity as the CACNA2D3, CAMK2D, KCNIP4, ANGPT1, NAALADL2, ZFHX3 or MPHOSPH 10 polypeptides.

Polypeptides having the CACNA2D3, CAMK2D, KCNIP4, ANGPT1, NAALADL2, ZFHX3 or MPHOSPH 10 amino acid sequence disclosed here, or fragments or homologues thereof may be modified for use in the methods and compositions described here. Typically, modifications are made that maintain the biological activity of the sequence. Amino acid substitutions may be made, for example from 1, 2 or 3 to 10, 20 or 30 substitutions provided that the modified sequence retains the biological activity of the unmodified sequence. Alternatively, modifications may be made to deliberately inactivate one or more functional domains of the polypeptides described here. Amino acid substitutions may include the use of non-naturally occurring analogues, for example to increase blood plasma half-life of a therapeutically administered polypeptide.

### Fragments

Polypeptides for use in the methods and compositions described here also include fragments of the full length sequence of any of the CACNA2D3, CAMK2D, KCNIP4, ANGPT1, NAALADL2, ZFHX3 or MPHOSPH10 polypeptides identified above. Fragments may comprise at least one epitope. Methods of identifying epitopes are well known in the art. Fragments will typically comprise at least 6 amino acids, such as at least 10, 20, 30, 50 or 100 amino acids.

Included are fragments comprising or consisting of, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 105, 110, 115, 120, 125, 130, 135, 140, 145 or more residues from a relevant CACNA2D3, CAMK2D, KCNIP4, ANGPT1, NAALADL2, ZFHX3 or MPHOSPH10 amino acid sequence.

We further describe peptides comprising a portion of a CACNA2D3, CAMK2D, KCNIP4, ANGPT1, NAALADL2, ZFHX3 or MPHOSPH10 polypeptide as described here. Thus, fragments of CACNA2D3, CAMK2D, KCNIP4, ANGPT1, NAALADL2, ZFHX3 or MPHOSPH10 and its homologues, variants or derivatives are included. The peptides may be between 2 and 200 amino acids, such as between 4 and 40 amino acids in length. The peptide may be derived from a CACNA2D3, CAMK2D, KCNIP4, ANGPT1, NAALADL2, ZFHX3 or MPHOSPH10 polypeptide as disclosed here, for example by digestion with a suitable enzyme, such as trypsin. Alternatively the peptide, fragment, etc may be made by recombinant means, or synthesised synthetically.

Such fragments may be used to generate probes to preferentially detect expression of the relevant polypeptide, for example, through antibodies generated against such fragments. These antibodies would be expected to bind specifically to the polypeptide, and are useful in the methods of diagnosis and treatment disclosed here.

CACNA2D3, CAMK2D, KCNIP4, ANGPT1, NAALADL2, ZFHX3 or MPHOSPH10 and its fragments, homologues, variants and derivatives, may be made by recombinant means. However they may also be made by synthetic means using techniques well known to skilled persons such as solid phase synthesis. The proteins may also be produced as fusion proteins, for example to aid in extraction and purification. Examples of fusion protein partners include glutathione-S-transferase (GST), 6xHis, GAL4 (DNA binding and/or transcriptional activation domains) and β-galactosidase. It may also be convenient to include a proteolytic cleavage site between the fusion protein partner and the protein sequence of interest to allow removal of fusion protein sequences. The fusion protein may be one which will not hinder the function of the protein of interest sequence. Proteins may also be obtained by purification of cell extracts from animal cells.

The CACNA2D3, CAMK2D, KCNIP4, ANGPT1, NAALADL2, ZFHX3 or MPHOSPH10 polypeptides, variants, homologues, fragments and derivatives disclosed here may be in a substantially isolated form. It will be understood that such polypeptides may be mixed with carriers or diluents which will not interfere with the intended purpose of the protein and still be regarded as substantially isolated. A CACNA2D3, CAMK2D, KCNIP4, ANGPT1, NAALADL2, ZFHX3 or MPHOSPH10 variant, homologue, fragment or derivative may also be in a substantially purified form, in which case it will generally comprise the protein in a preparation in which more than 90%, e.g. 95%, 98% or 99% of the protein in the preparation is a protein.

The CACNA2D3, CAMK2D, KCNIP4, ANGPT1, NAALADL2, ZFHX3 or MPHOSPH10 polypeptides, variants, homologues, fragments and derivatives disclosed here may be labelled with a revealing label. The revealing label may be any suitable label which allows the polypeptide, etc to be detected. Suitable labels include radioisotopes, e.g. ¹²⁵I, enzymes, antibodies, polynucleotides and linkers such as biotin. Labelled polypeptides may be used in diagnostic procedures such as immunoassays to determine the amount of a polypeptide in a sample. Polypeptides or labelled polypeptides may also be used in serological or cell-mediated immune assays for the detection of immune reactivity to said polypeptides in animals and humans using standard protocols.

CACNA2D3, CAMK2D, KCNIP4, ANGPT1, NAALADL2, ZFHX3 or MPHOSPH10 polypeptides, variants, homologues, fragments and derivatives disclosed here, optionally labelled, may also be fixed to a solid phase, for example the surface of an immunoassay well or dipstick. Such labelled and/or immobilised polypeptides may be packaged into kits in a suitable container along with suitable reagents, controls, instructions and the like. Such polypeptides and kits may be used in methods of detection of antibodies to the polypeptides or their allelic or species variants by immunoassay.

Immunoassay methods are well known in the art and will generally comprise: (a) providing a polypeptide comprising an epitope bindable by an antibody against said protein; (b) incubating a biological sample with said polypeptide under conditions which allow for the formation of an antibody-antigen complex; and (c) determining whether antibody-antigen complex comprising said polypeptide is formed.

The polypeptides, variants, homologues, fragments and derivatives disclosed here may be used in *in vitro* or *in vivo* cell culture systems to study the role of their corresponding genes and homologues thereof in cell function, including their function in disease. For example, truncated or modified polypeptides may be introduced into a cell to disrupt the normal functions which occur in the cell. The polypeptides may be introduced into the cell by *in situ* expression of the polypeptide from a recombinant expression vector (see below). The expression vector optionally carries an inducible promoter to control the expression of the polypeptide.

The use of appropriate host cells, such as insect cells or mammalian cells, is expected to provide for such post-translational modifications (e.g. myristolation, glycosylation, truncation, lapidation and tyrosine, serine or threonine phosphorylation) as may be needed to confer optimal biological activity on recombinant expression products. Such cell culture systems in which the polypeptides, variants, homologues, fragments and derivatives disclosed here are expressed may be used in assay systems to identify candidate substances which interfere with or enhance the functions of the polypeptide in the cell.

### NUCLEIC ACIDS

The methods and compositions described here may employ, as a means for detecting expression levels of CACNA2D3, CAMK2D, KCNIP4, ANGPT1, NAALADL2, ZFHX3 or MPHOSPH10, CACNA2D3, CAMK2D, KCNIP4, ANGPT1, NAALADL2, ZFHX3 or MPHOSPH10 polynucleotides, CACNA2D3, CAMK2D, KCNIP4, ANGPT1, NAALADL2, ZFHX3 or MPHOSPH10 nucleotides and CACNA2D3, CAMK2D, KCNIP4, ANGPT1, NAALADL2, ZFHX3 or MPHOSPH10 nucleic acids, as well as variants, homologues, derivatives and fragments of any of these. In addition, we disclose particular fragments useful for the methods of diagnosis described here. These nucleic acids may also be used for the methods of treatment or prophylaxis described.

The terms "polynucleotide", "nucleotide" and "nucleic acid" may be used interchangeably, and should be understood to specifically include both cDNA and genomic sequences. These terms are also intended to include a nucleic acid sequence capable of encoding a relevant polypeptide and/or a fragment, derivative, homologue or variant of this.

Where reference is made to a CACNA2D3, CAMK2D, KCNIP4, ANGPT1, NAALADL2, ZFHX3 or MPHOSPH 10 nucleic acid, this should be taken as a reference to any member of the CACNA2D3, CAMK2D, KCNIP4, ANGPT1, NAALADL2, ZFHX3 or MPHOSPH10 family of nucleic acids.

Also included are any one or more of the nucleic acid sequences set out as "Other nucleic acid sequences" below.

The nucleic acids described here may be used for a variety of means, for example, administration to an individual suffering from, or suspected to be suffering from, Kawasaki Disease, for the treatment thereof. The expression of such nucleic acids may be detected for diagnosis or detection of Kawasaki Disease. CACNA2D3, CAMK2D, KCNIP4, ANGPT1, NAALADL2, ZFHX3 or MPHOSPH10 nucleic acids may also be used for the expression or production of the corresponding polypeptides.

"Polynucleotide" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single-and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

It will be understood by the skilled person that numerous nucleotide sequences can encode the same polypeptide as a result of the degeneracy of the genetic code.

As used herein, the term "nucleotide sequence" refers to nucleotide sequences, oligonucleotide sequences, polynucleotide sequences and variants, homologues, fragments and derivatives thereof (such as portions thereof). The nucleotide sequence may be DNA or RNA of genomic or synthetic or recombinant origin which may be double-stranded or single-stranded whether representing the sense or antisense strand or combinations thereof. The term nucleotide sequence may be prepared by use of recombinant DNA techniques (for example, recombinant DNA).

The term "nucleotide sequence" may means DNA.

### Other Nucleic Acids

We also provide nucleic acids which are fragments, homologues, variants or derivatives of CACNA2D3, CAMK2D, KCNIP4, ANGPT1, NAALADL2, ZFHX3 or MPHOSPH10 nucleic acids. The terms "variant", "homologue", "derivative" or "fragment" in relation to such nucleic acids include any substitution of, variation of, modification of, replacement of, deletion of or addition of one (or more) nucleic acids from or to the sequence of a relevant nucleotide sequence. Unless the context admits otherwise, references to "CACNA2D3", "CAMK2D", "KCNIP4", "ANGPT1", "NAALADL2", "ZFHX3" and "MPHOSPH10" include references to such variants, homologues, derivatives and fragments.

The resultant nucleotide sequence may encode a polypeptide having any one or more relevant activity. The term "homologue" may be intended to cover identity with respect to structure and/or function such that the resultant nucleotide sequence encodes a polypeptide which has that activity. For example, a homologue etc of CACNA2D3, CAMK2D, KCNIP4, ANGPT1, NAALADL2, ZFHX3 or MPHOSPH10 may have a increased expression level in Kawasaki Disease cells compared to normal cells. With respect to sequence identity (i.e. similarity), there may be at least 70%, at least 75%, at least 85% or at least 90% sequence identity. There may be at least 95%, such as at least 98%, sequence identity to a relevant sequence. These terms also encompass allelic variations of the sequences.

### Variants, Derivatives and Homologues

CACNA2D3, CAMK2D, KCNIP4, ANGPT1, NAALADL2, ZFHX3 or MPHOSPH10 nucleic acid variants, fragments, derivatives and homologues may comprise DNA or RNA. They may be single-stranded or double-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of this document, it is to be understood that the polynucleotides may be modified by any method available in the art. Such modifications may be carried out in order to enhance the *in vivo* activity or life span of polynucleotides of interest.

Where the polynucleotide is double-stranded, both strands of the duplex, either individually or in combination, are encompassed by the methods and compositions described here. Where the polynucleotide is single-stranded, it is to be understood that the complementary sequence of that polynucleotide is also included.

The terms "variant", "homologue" or "derivative" in relation to a nucleotide sequence include any substitution of, variation of, modification of, replacement of, deletion of or addition of one (or more) nucleic acid from or to the sequence. Said variant, homologues or derivatives may code for a polypeptide having biological activity. Such fragments, homologues, variants and derivatives of CACNA2D3, CAMK2D, KCNIP4, ANGPT1, NAALADL2, ZFHX3 or MPHOSPH10 may comprise modulated activity, as set out above.

As indicated above, with respect to sequence identity, a "homologue" may have at least 5% identity, at least 10% identity, at least 15% identity, at least 20% identity, at least 25% identity, at least 30% identity, at least 35% identity, at least 40% identity, at least 45% identity, at least 50% identity, at least 55% identity, at least 60% identity, at least 65% identity, at least 70% identity, at least 75% identity, at least 80% identity, at least 85% identity, at least 90% identity, or at least 95% identity to the relevant CACNA2D3, CAMK2D, KCNIP4, ANGPT1, NAALADL2, ZFHX3 or MPHOSPH10 sequence.

There may be at least 95% identity, at least 96% identity, at least 97% identity, at least 98% identity or at least 99% identity. Nucleotide identity comparisons may be conducted as described above. A sequence comparison program which may be used is the GCG Wisconsin Bestfit program described above. The default scoring matrix has a match value of 10 for each identical nucleotide and -9 for each mismatch. The default gap creation penalty is -50 and the default gap extension penalty is -3 for each nucleotide.

### Hybridisation

We further describe nucleotide sequences that are capable of hybridising selectively to any of the sequences presented herein, or any variant, fragment or derivative thereof, or to the complement of any of the above. Nucleotide sequences may be at least 15 nucleotides in length, such as at least 20, 30, 40 or 50 nucleotides in length.

The term "hybridization" as used herein shall include "the process by which a strand of nucleic acid joins with a complementary strand through base pairing" as well as the process of amplification as carried out in polymerase chain reaction technologies.

Polynucleotides capable of selectively hybridising to the nucleotide sequences presented herein, or to their complement, may be at least 40% homologous, at least 45% homologous, at least 50% homologous, at least 55% homologous, at least 60% homologous, at least 65% homologous, at least 70% homologous, at least 75% homologous, at least 80% homologous, at least 85% homologous, at least 90% homologous, or at least 95% homologous to the corresponding nucleotide sequences presented herein. Such polynucleotides may be generally at least 70%, at least 80 or 90% or at least 95% or 98% homologous to the corresponding nucleotide sequences over a region of at least 20, such as at least 25 or 30, for instance at least 40, 60 or 100 or more contiguous nucleotides.

The term "selectively hybridizable" means that the polynucleotide used as a probe is used under conditions where a target polynucleotide is found to hybridize to the probe at a level significantly above background. The background hybridization may occur because of other polynucleotides present, for example, in the cDNA or genomic DNA library being screening. In this event, background implies a level of signal generated by interaction between the probe and a non-specific DNA member of the library which is less than 10 fold, such as less than 100 fold as intense as the specific interaction observed with the target DNA. The intensity of interaction may be measured, for example, by radiolabelling the probe, e.g. with ³²P or ³³P or with non-radioactive probes (e.g., fluorescent dyes, biotin or digoxigenin).

Hybridization conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA), and confer a defined "stringency" as explained below.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridization can be used to identify or detect identical polynucleotide sequences while an intermediate (or low) stringency hybridization can be used to identify or detect similar or related polynucleotide sequences.

We provide nucleotide sequences that may be able to hybridise to the CACNA2D3, CAMK2D, KCNIP4, ANGPT1, NAALADL2, ZFHX3 or MPHOSPH10 nucleic acids, fragments, variants, homologues or derivatives under stringent conditions (e.g. 65°C and 0.1xSSC (1xSSC = 0.15 M NaCl, 0.015 M Na₃ Citrate pH 7.0)).

### Generation of Homologues, Variants and Derivatives

Polynucleotides which are not 100% identical to the relevant CACNA2D3, CAMK2D, KCNIP4, ANGPT1, NAALADL2, ZFHX3 or MPHOSPH10 sequences but which are also included, as well as homologues, variants and derivatives of these can be obtained in a number of ways. Other variants of the sequences may be obtained for example by probing DNA libraries made from a range of individuals, for example individuals from different populations. For example, such homologues may be identified from other individuals, or other species. Further recombinant nucleic acids and polypeptides may be produced by identifying corresponding positions in the homologues, and synthesising or producing the molecule as described elsewhere in this document.

In addition, other viral/bacterial, or cellular homologues, particularly cellular homologues found in mammalian cells (e.g. rat, mouse, bovine and primate cells), may be obtained and such homologues and fragments thereof in general will be capable of selectively hybridising to human CACNA2D3, CAMK2D, KCNIP4, ANGPT1, NAALADL2, ZFHX3 or MPHOSPH10. Such homologues may be used to design non-human nucleic acids, fragments, variants and homologues. Mutagenesis may be carried out by means known in the art to produce further variety.

Sequences of such homologues may be obtained by probing cDNA libraries made from or genomic DNA libraries from other animal species, and probing such libraries with probes comprising all or part of any of the nucleic acids, fragments, variants and homologues, or other fragments described here under conditions of medium to high stringency.

Similar considerations apply to obtaining species homologues and allelic variants of the polypeptide or nucleotide sequences disclosed here.

Variants and strain/species homologues may also be obtained using degenerate PCR which will use primers designed to target sequences within the variants and homologues encoding conserved amino acid sequences within the sequences of the nucleic acids. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences. It will be appreciated by the skilled person that overall nucleotide homology between sequences from distantly related organisms is likely to be very low and thus in these situations degenerate PCR may be the method of choice rather than screening libraries with labelled fragments the relevant sequences.

In addition, homologous sequences may be identified by searching nucleotide and/or protein databases using search algorithms such as the BLAST suite of programs.

Alternatively, such polynucleotides may be obtained by site directed mutagenesis of characterised sequences, for example, CACNA2D3, CAMK2D, KCNIP4, ANGPT1, NAALADL2, ZFHX3 or MPHOSPH 10 nucleic acids, or variants, homologues, derivatives or fragments thereof. This may be useful where for example silent codon changes are required to sequences to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides.

The polynucleotides described here may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 8, 9, 10, or 15, such as at least 20, for example at least 25, 30 or 40 nucleotides in length, and are also encompassed by the term "polynucleotides" as used herein.

Polynucleotides such as a DNA polynucleotides and probes may be produced recombinantly, synthetically, or by any means available to those of skill in the art. They may also be cloned by standard techniques.

In general, primers will be produced by synthetic means, involving a step wise manufacture of the desired nucleic acid sequence one nucleotide at a time. Techniques for accomplishing this using automated techniques are readily available in the art.

Primers comprising fragments of CACNA2D3, CAMK2D, KCNIP4, ANGPT1, NAALADL2, ZFHX3 or MPHOSPH10 are particularly useful in the methods of detection of expression of the relevant gene, such as up-regulation of expression, for example, as associated with Kawasaki Disease. Suitable primers for amplification of CACNA2D3, CAMK2D, KCNIP4, ANGPT1, NAALADL2, ZFHX3 or MPHOSPH10 may be generated from any suitable stretch of the relevant nucleic acid. Primers which may be used include those capable of amplifying a sequence of the nucleic acid which is specific.

Although such primers may be provided on their own, they are most usefully provided as primer pairs, comprising a forward primer and a reverse primer.

Longer polynucleotides will generally be produced using recombinant means, for example using a PCR (polymerase chain reaction) cloning techniques. This will involve making a pair of primers (e.g. of about 15 to 30 nucleotides), bringing the primers into contact with mRNA or cDNA obtained from an animal or human cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector

Polynucleotides or primers may carry a revealing label. Suitable labels include radioisotopes such as ³²P or ³⁵S, digoxigenin, fluorescent dyes, enzyme labels, or other protein labels such as biotin. Such labels may be added to polynucleotides or primers and may be detected using by techniques known *per se*. Polynucleotides or primers or fragments thereof labelled or unlabeled may be used by a person skilled in the art in nucleic acid-based tests for detecting or sequencing polynucleotides in the human or animal body.

Such tests for detecting generally comprise bringing a biological sample containing DNA or RNA into contact with a probe comprising a polynucleotide or primer under hybridising conditions and detecting any duplex formed between the probe and nucleic acid in the sample. Such detection may be achieved using techniques such as PCR or by immobilising the probe on a solid support, removing nucleic acid in the sample which is not hybridised to the probe, and then detecting nucleic acid which has hybridised to the probe. Alternatively, the sample nucleic acid may be immobilised on a solid support, and the amount of probe bound to such a support can be detected. Suitable assay methods of this and other formats can be found in for example WO89/03891 and WO90/13667.

Tests for sequencing nucleotides, for example, the CACNA2D3, CAMK2D, KCNIP4, ANGPT1, NAALADL2, ZFHX3 or MPHOSPH10 nucleic acids, involve bringing a biological sample containing target DNA or RNA into contact with a probe comprising a polynucleotide or primer under hybridising conditions and determining the sequence by, for example the Sanger dideoxy chain termination method (see Sambrook *et al*.).

Such a method generally comprises elongating, in the presence of suitable reagents, the primer by synthesis of a strand complementary to the target DNA or RNA and selectively terminating the elongation reaction at one or more of an A, C, G or T/U residue; allowing strand elongation and termination reaction to occur; separating out according to size the elongated products to determine the sequence of the nucleotides at which selective termination has occurred. Suitable reagents include a DNA polymerase enzyme, the deoxynucleotides dATP, dCTP, dGTP and dTTP, a buffer and ATP. Dideoxynucleotides are used for selective termination.

### Control Regions

For some purposes, it may be necessary to utilise or investigate control regions of CACNA2D3, CAMK2D, KCNIP4, ANGPT1, NAALADL2, ZFHX3 or MPHOSPH10. Such control regions include promoters, enhancers and locus control regions. By a control region we mean a nucleic acid sequence or structure which is capable of modulating the expression of a coding sequence which is operatively linked to it.

For example, control regions are useful in generating transgenic animals expressing the relevant gene. Furthermore, control regions may be used to generate expression constructs for such expression. This is described in further detail below.

Identification of control regions of CACNA2D3, CAMK2D, KCNIP4, ANGPT1, NAALADL2, ZFHX3 or MPHOSPH10 is straightforward, and may be carried out in a number of ways. For example, the coding sequence of the relevant gene may be obtained from an organism, by screening a cDNA library using a human or mouse cDNA sequence as a probe. 5' sequences may be obtained by screening an appropriate genomic library, or by primer extension as known in the art. Database searching of genome databases may also be employed. Such 5' sequences which are particularly of interest include non-coding regions. The 5' regions may be examined by eye, or with the aid of computer programs, to identify sequence motifs which indicate the presence of promoter and/or enhancer regions.

Furthermore, sequence alignments may be conducted of the relevant nucleic acid sequences from two or more organisms. By aligning CACNA2D3, CAMK2D, KCNIP4, ANGPT1, NAALADL2, ZFHX3 or MPHOSPH10 sequences from different species, it is possible to determine which regions of the amino acid sequence are conserved between different species. Such conserved regions are likely to contain control regions for the gene in question. The mouse and human genomic sequences as disclosed here, for example, a mouse genomic sequence, may be employed for this purpose. Furthermore, homologues from other organisms may be obtained using standard methods of screening using appropriate probes generated from the mouse and human sequences. The genome of the pufferfish (*Takifugu rubripes*) or zebrafish may also be screened to identify a homologue. Comparison of the 5' non-coding region of the Fugu or zebrafish gene with a mouse or human genomic sequence may be used to identify conserved regions containing control regions.

Deletion studies may also be conducted to identify promoter and/or enhancer regions for CACNA2D3, CAMK2D, KCNIP4, ANGPT1, NAALADL2, ZFHX3 or MPHOSPH10.

The identity of putative control regions may be confirmed by molecular biology experiments, in which the candidate sequences are linked to a reporter gene and the expression of the reporter detected.

### TREATMENT AND DIAGNOSIS

The term "treatment" or "treating" as used in this document should be understood to include any indicia of success in the treatment, alleviation or amelioration of an injury, pathology or condition, including any objective or subjective parameter such as abatement; remission; diminishing of symptoms or making the injury, pathology or condition more tolerable to the patient; slowing in the rate of degeneration or decline; making the final point of degeneration less debilitating; improving a patient's physical or mental well-being; or, in some situations, preventing the onset of disease such as Kawasaki Disease.

"Diagnose" or "diagnosis", as this term is used in this document, refers to determining the nature or the identity of a condition (disease). A diagnosis may be accompanied by a determination as to the severity of the disease. "Prognostic" or "prognosis" may be used to refer to predicting the outcome or prognosis of a disease, such as to give a chance of survival based on observations and results of clinical tests. "Predisposition" may be used to refer to the likelihood of being diagnosed with, or susceptibility to a particular disease.

Diagnosis in general may thus comprise any kind of assessment of the presence of absence of a medically relevant condition. Diagnosis may thus comprise processes such as screening for the predisposition for a medically relevant condition; screening for the precursor of a medically relevant condition, screening for a medically relevant condition, clinical or pathological diagnosis of a medically relevant condition, etc. Diagnosis may comprise examination of any condition, that is detectable on a cytological, histological, biochemical or molecular biological level, that may be useful in respect to the human health and/or body. Such examinations may comprise e.g. medically diagnostic methods and research studies in life sciences. In one embodiment of the invention, the method is used for diagnosis of medically relevant conditions such as e.g. diseases. Such diseases may for example comprise disorders characterized by non-wild type proliferation of cells or tissues.

### DETECTION AND DIAGNOSTIC METHODS

### DETECTION OF POLYMORPHISMS

A few different methods are commonly used to analyze DNA for polymorphisms or mutations. The most definitive method is to sequence the DNA to determine the actual base sequence (Maxam and Gilbert, 1977; Sanger et al., 1977). Although such a method is the most definitive it is also the most expensive and time consuming method.

Restriction mapping analysis may also be used in analyzing DNA for polymorphisms. If one is looking for a known polymorphism at a site which will change the recognition site for a restriction enzyme it is possible simply to digest DNA with this restriction enzyme and analyze the fragments on a gel or with a Southern blot to determine the presence or absence of the polymorphism. This type of analysis is also useful for determining the presence or absence of gross insertions or deletions. Hybridization with allele specific oligonucleotides is yet another method for determining the presence of known polymorphisms.

Thus, specific DNA sequences in an individual, for example, a gene encoding CACNA2D3, CAMK2D, KCNIP4, ANGPT1, NAALADL2, ZFHX3 or MPHOSPH10, may undergo many different changes, such as deletion of a sequence of DNA, insertion of a sequence that was duplicated, inversion of a sequence, or conversion of a single nucleotide to another. Changes in a specific DNA sequence may be traced by using restriction enzymes that recognize specific DNA sequences of 4-6 nucleotides. Restriction enzymes cut (digest) the DNA at their specific recognized sequence, resulting in one million or so pieces. When a difference exists that changes a sequence recognized by a restriction enzyme to one not recognized, the piece of DNA produced by cutting the region are of a different size. The various possible fragment sizes from a given region therefore depend on the precise sequence of DNA in the region. Variation in the fragments produced is termed "restriction fragment length polymorphism" (RFLP). The different sized-fragments reflecting different variant DNA sequences may be visualized by separating the digested DNA according to its size on an agarose gel and visualizing the individual fragments by annealing to a radioactively labeled DNA "probe". Each individual may carry two different forms of the specific sequence. When the two homologues carry the same form of the polymorphism, one band is seen. More than two forms of a polymorphism may exist for a specific DNA marker in the population, but in one family just four forms are possible; two from each parent. Each child inherits one form of the polymorphism from each parent. Thus, the origin of each chromosome region may be traced (maternal or paternal origin).

Furthermore, RT-PCR may be carried out, followed by restriction endonuclease fingerprinting (REF). REF is a modification of the single-strand conformation polymorphism (SSCP) method, and enables efficient detection of sequence alterations in DNA fragments up to 2 kb in length (Liu and Sommer, 1995).

The use of mass spectrometry to determine the presence of polymorphisms within known genes is disclosed in United States Patent No. 5,869,242.

Single strand conformational polymorphism (SSCP) analysis is a rapid and efficacious method for detecting polymorphisms (Dean et al., Cell 61:863, 1990; Glavac and Dean, Hum. Mutation 2:404, 1993; Poduslo et al., Am. J. Hum. Genet. 49:106, 1992). In SSCP, abnormal strand motility on a gel is associated with mutational events in the gene.

Genetic analysis of polymorphisms is disclosed in detail in United States Patent Nos. 5,552,28, 5,654,13, 5,670,33, 5,807,67, 5,858,66, 5,691,15, 5,922,57, 5,972,60, 6,136,53 and 5,955,26, among others.

Any of these methods described in the above references may be used in the diagnostic methods of our invention.

### LINKAGE DISEQUILIBRIUM ANALYSIS

Linkage disequilibrium (LD) analysis is a powerful tool for mapping disease genes and may be particularly useful for investigating complex traits. LD mapping is based on the following expectations: for any two members of a population, it is expected that recombination events occurring over several generations will have shuffled their genomes, so that they share little in common with their ancestors. However, if these individuals are affected with a disease inherited from a common ancestor, the gene responsible for the disease and the markers that immediately surround it will likely be inherited without change, or IBD ("identical by descent"), from that ancestor. The size of the regions that remain shared (i.e. IBD) are inversely proportional to the number of generations separating the affected individuals and their common ancestor. Thus, "old" populations are suitable for fine scale mapping and recently founded ones are appropriate for using LD to roughly localize disease genes. (Houwen et al., 1994, in particular FIG. 3 and accompanying text). Because isolated populations have typically had a small number of founders, they are particularly suitable for LD approaches, as indicated by several successful LD studies conducted in Finland (de la Chapelle, 1993).

LD analysis has been used in several positional cloning efforts (Kerem et al., 1989; MacDonald et al., 1992; Petrukhin et al., 1993; Hastbacka et al., 1992 and 1994), but in each case the initial localization had been achieved using conventional linkage methods. Positional cloning is the isolation of a gene solely on the basis of its chromosomal location, without regard to its biochemical function. Lander and Botstein (1986) proposed that LD mapping could be used to screen the human genome for disease loci, without conventional linkage analyses. This approach was not practical until a set of mapped markers covering the genome became available (Weissenbach et al., 1992). The feasibility of genome screening using LD mapping is now demonstrated by the applicants.

Identification of the chromosomal location of a gene responsible for causing a disease associated with increased or reduced cell-cell adhesion in the epithelium can facilitate diagnosis, treatment and genetic counseling of individuals in affected families.

Due to the severity of the disorder and the limitations of a purely phenotypic diagnosis of such diseases, there is a tremendous need to genetically subtype individuals to confirm clinical diagnoses and to determine appropriate therapies based on their genotypic subtype.

### DETECTION OF EXPRESSION

We show in the Examples that the expression of CACNA2D3, CAMK2D, KCNIP4, ANGPT1, NAALADL2, ZFHX3 and MPHOSPH10 in Kawasaki Disease tissue is modulated, such as up-regulated when compared to normal tissue.

Accordingly, we provide for a method of diagnosis of Kawasaki Disease, comprising detecting modulation of expression of any one or more of CACNA2D3, CAMK2D, KCNIP4, ANGPT1, NAALADL2, ZFHX3 and MPHOSPH10, such as up-regulation of expression of CACNA2D3, CAMK2D, KCNIP4, ANGPT1, NAALADL2, ZFHX3 or MPHOSPH10 in a cell or tissue of an individual.

Detection of CACNA2D3, CAMK2D, KCNIP4, ANGPT1, NAALADL2, ZFHX3 and MPHOSPH 10 expression, activity or amount may be used to provide a method of determining the state of a cell. Thus, the methods may be used to detect a cell with high levels of CACNA2D3, CAMK2D, KCNIP4, ANGPT1, NAALADL2, ZFHX3 or MPHOSPH10 expression, activity or amount compared to a normal cell. Similarly, the methods may be used to detect a cell with low levels CACNA2D3, CAMK2D, KCNIP4, ANGPT1, NAALADL2, ZFHX3 or MPHOSPH10 expression, activity or amount compared to a normal cell.

Such detection may also be used to determine whether a cell will become a Kawasaki Disease cell (or that an individual harbouring the cell, or from whom the cell is derived, will develop Kawasaki Disease). Thus, detection of a high level of CACNA2D3, CAMK2D, KCNIP4, ANGPT1, NAALADL2, ZFHX3 or MPHOSPH10 expression, amount or activity in the cell may indicate that the cell is likely to be or become a Kawasaki Disease cell, or that the individual is likely to develop Kawasaki Disease. Similarly, if a cell has a low level of CACNA2D3, CAMK2D, KCNIP4, ANGPT1, NAALADL2, ZFHX3 or MPHOSPH10 expression, amount or activity, the cell is not or is not likely to be a Kawasaki Disease cell (or the individual is not likely to develop Kawasaki Disease).

It will be appreciated that if the level of CACNA2D3, CAMK2D, KCNIP4, ANGPT1, NAALADL2, ZFHX3 or MPHOSPH10 varies with the likelihood of developing Kawasaki Disease, that detection of CACNA2D3, CAMK2D, KCNIP4, ANGPT1, NAALADL2, ZFHX3 and MPHOSPH 10 expression, amount or activity may also be used to predict a survival rate of an individual with Kawasaki Disease, i.e., high levels of CACNA2D3, CAMK2D, KCNIP4, ANGPT1, NAALADL2, ZFHX3 or MPHOSPH10 indicating a lower survival rate or probability and low levels of CACNA2D3, CAMK2D, KCNIP4, ANGPT1, NAALADL2, ZFHX3 or MPHOSPH 10 indicating a higher survival rate or probability, both as compared to individuals or cognate populations with normal levels of CACNA2D3, CAMK2D, KCNIP4, ANGPT1, NAALADL2, ZFHX3 or MPHOSPH10 (as the case may be). Detection of expression, amount or activity of CACNA2D3, CAMK2D, KCNIP4, ANGPT1, NAALADL2, ZFHX3 or MPHOSPH 10 may therefore be used as a method of prognosis of an individual with Kawasaki Disease.

Typically, Kawasaki Disease is diagnosed by the appearance of one or more symptoms as described above in the section "Kawasaki Disease" under "Symptoms", "Signs and Tests", "Blood Tests" and "Other Tests". The methods described here may be used to supplement or replace these tests.

Detection of CACNA2D3, CAMK2D, KCNIP4, ANGPT1, NAALADL2, ZFHX3 or MPHOSPH10 expression, amount or level may be used to determine the likelihood of success of a particular therapy in an individual with Kawasaki Disease.

The diagnostic methods described in this document may be combined with the therapeutic methods described. Thus, we provide for a method of treatment, prophylaxis or alleviation of Kawasaki Disease in an individual, the method comprising detecting modulation of expression, amount or activity of CACNA2D3, CAMK2D, KCNIP4, ANGPT1, NAALADL2, ZFHX3 or MPHOSPH10 in a cell of the individual and administering an appropriate therapy to the individual based on the likelihood of developing Kawasaki Disease. The therapy may comprise an anti-Kawasaki Disease agent as described above.

The presence and quantity of CACNA2D3, CAMK2D, KCNIP4, ANGPT I, NAALADL2, ZFHX3 and MPHOSPH 10 polypeptides and nucleic acids may be detected in a sample as described in further detail below. Thus, Kawasaki Disease can be diagnosed by methods comprising determining from a sample derived from a subject an abnormally decreased or increased expression, amount or activity, such as a increased expression, amount or activity, of the CACNA2D3, CAMK2D, KCNIP4, ANGPT1, NAALADL2, ZFHX3 or MPHOSPH10 polypeptide or CACNA2D3, CAMK2D, KCNIP4, ANGPT1, NAALADL2, ZFHX3 or MPHOSPH10 mRNA.

The sample may comprise a cell or tissue sample from an organism or individual suffering or suspected to be suffering from a disease associated with increased, reduced or otherwise abnormal CACNA2D3, CAMK2D, KCNIP4, ANGPT1, NAALADL2, ZFHX3 or MPHOSPH10 expression, amount or activity, including spatial or temporal changes in level or pattern of expression, amount or activity, such as Kawasaki Disease. The level or pattern of expression, amount or activity of CACNA2D3, CAMK2D, KCNIP4, ANGPT1, NAALADL2, ZFHX3 or MPHOSPH10 in an organism suffering from or suspected to be suffering from such a disease may be usefully compared with the level or pattern of expression, amount or activity in a normal organism as a means of diagnosis of disease.

The sample may comprise a cell or tissue sample from an individual suffering or suspected to be suffering from Kawasaki Disease, such as a heart tissue or cell sample.

In some embodiments, an increased level of expression, amount or activity of CACNA2D3, CAMK2D, KCNIP4, ANGPT1, NAALADL2, ZFHX3 or MPHOSPH10 is detected in the sample. The level of CACNA2D3, CAMK2D, KCNIP4, ANGPT1, NAALADL2, ZFHX3 or MPHOSPH 10 may be increased to a significant extent when compared to normal cells, or cells known not to be from Kawasaki Disease individuals. Such cells may be obtained from the individual being tested, or another individual, such as those matched to the tested individual by age, weight, lifestyle, etc.

In some embodiments, the level of expression, amount or activity of CACNA2D3, CAMK2D, KCNIP4, ANGPT1, NAALADL2, ZFHX3 or MPHOSPH10 is increased by 10%, 20%, 30% or 40% or more. In some embodiments, the level of expression, amount or activity of CACNA2D3, CAMK2D, KCNIP4, ANGPT1, NAALADL2, ZFHX3 or MPHOSPH10 is increased by 45% or more, such as 50% or more, as judged by cDNA hybridisation.

The expression, amount or activity of CACNA2D3, CAMK2D, KCNIP4, ANGPT1, NAALADL2, ZFHX3 or MPHOSPH 10 may be detected in a number of ways, as known in the art, and as described in further detail below. Typically, the amount of CACNA2D3, CAMK2D, KCNIP4, ANGPT1, NAALADL2, ZFHX3 or MPHOSPH10 in a sample of tissue from an individual is measured, and compared with a sample from an unaffected individual. Both CACNA2D3, CAMK2D, KCNIP4, ANGPT1, NAALADL2, ZFHX3 or MPHOSPH10 nucleic acid, as well as CACNA2D3, CAMK2D, KCNIP4, ANGPT1, NAALADL2, ZFHX3 or MPHOSPH10 polypeptide levels may be measured.

Detection of the amount, activity or expression of CACNA2D3, CAMK2D, KCNIP4, ANGPT1, NAALADL2, ZFHX3 or MPHOSPH10 may be used to grade Kawasaki Disease. For example, a high level of amount, activity or expression of CACNA2D3, CAMK2D, KCNIP4, ANGPT1, NAALADL2, ZFHX3 or MPHOSPH10 may indicate an acute stage of Kawasaki Disease. Similarly, a low level of amount, activity or expression of CACNA2D3, CAMK2D, KCNIP4, ANGPT1, NAALADL2, ZFHX3 or MPHOSPH10 may indicate a convalescent phase of Kawasaki Disease.

Levels of CACNA2D3, CAMK2D, KCNIP4, ANGPT1, NAALADL2, ZFHX3 or MPHOSPH 10 expression may be determined using a number of different techniques.

### MEASURING EXPRESSION AT THE RNA LEVEL

CACNA2D3, CAMK2D, KCNIP4, ANGPT1, NAALADL2, ZFHX3 or MPHOSPH10 expression may be detected at the RNA level.

We therefore disclose a method of detecting the presence of a nucleic acid comprising a CACNA2D3, CAMK2D, KCNIP4, ANGPT1, NAALADL2, ZFHX3 or MPHOSPH10 nucleic acid in a sample, by contacting the sample with at least one nucleic acid probe which is specific for the CACNA2D3, CAMK2D, KCNIP4, ANGPT1, NAALADL2, ZFHX3 or MPHOSPH10 (as the case may be) nucleic acid and monitoring the sample for the presence of the CACNA2D3, CAMK2D, KCNIP4, ANGPT1, NAALADL2, ZFHX3 or MPHOSPH10 nucleic acid. For example, the nucleic acid probe may specifically bind to the CACNA2D3, CAMK2D, KCNIP4, ANGPT1, NAALADL2, ZFHX3 or MPHOSPH10 nucleic acid, or a portion of it, and binding between the two detected; the presence of the complex itself may also be detected.

Thus, the amount of CACNA2D3, CAMK2D, KCNIP4, ANGPT1, NAALADL2, ZFHX3 or MPHOSPH10 nucleic acid in the form of CACNA2D3, CAMK2D, KCNIP4, ANGPT1, NAALADL2, ZFHX3 or MPHOSPH10 mRNA may be measured in a sample. CACNA2D3, CAMK2D, KCNIP4, ANGPT1, NAALADL2, ZFHX3 or MPHOSPH10 mRNA may be assayed by *in situ* hybridization, Northern blotting and reverse transcriptase-polymerase chain reaction. Nucleic acid sequences may be identified by *in situ* hybridization, Southern blotting, single strand conformational polymorphism, PCR amplification and DNA-chip analysis using specific primers.

The above techniques are described in detail in Kawasaki ES. Amplification of RNA. In: PCR protocols: A Guide to Methods and Applications, Innis MA, Gelfand DH, Sninsky JJ, White TJ, eds. Academic Press, 1990, pp21-27.; Sambrook et al., Molecular Cloning: A Laboratory Manual, Cold Spring Harbor Laboratory Press, New York (1989); Lichter et al., High-resolution mapping of human chromosome 11 by in situ hybridization with cosmid clones Science 247:64-69 (1990); Orita et al. Detection of polymorphisms of human DNA by gel electrophoresis as single-strand conformation polymorphisms. Proc Natl Acad Sci USA 1989; 86:2766-2770; Fodor et al, Multiplexed biochemical assays with biological chips, Nature 364:555-556 (1993); Pease et al., Light-generated oligonucleotide arrays for rapid DNA sequence analysis, Proc. Natl. Acad. Sci. USA 91(11): 5022-5026 (1994).

CACNA2D3, CAMK2D, KCNIP4, ANGPT1, NAALADL2, ZFHX3 or MPHOSPH10 RNA may be extracted from cells using RNA extraction techniques including, for example, using acid phenol/guanidine isothiocyanate extraction (RNAzol B; Biogenesis), or RNeasy RNA preparation kits (Qiagen).Typical assay formats utilising ribonucleic acid hybridisation include nuclear run-on assays, RT-PCR and RNase protection assays (Melton et al., Nuc. Acids Res. 12:7035. Methods for detection which can be employed include radioactive labels, enzyme labels, chemiluminescent labels, fluorescent labels and other suitable labels.

Each of these methods allows quantitative determinations to be made, and are well known in the art. Decreased or increased CACNA2D3, CAMK2D, KCNIP4, ANGPT1, NAALADL2, ZFHX3 or MPHOSPH10 expression, amount or activity can therefore be measured at the RNA level using any of the methods well known in the art for the quantitation of polynucleotides. Any suitable probe from a CACNA2D3, CAMK2D, KCNIP4, ANGPT1, NAALADL2, ZFHX3 or MPHOSPH 10 sequence, for example, any portion of a suitable human CACNA2D3, CAMK2D, KCNIP4, ANGPT1, NAALADL2, ZFHX3 or MPHOSPH10 sequence may be used as a probe.

Typically, RT-PCR is used to amplify RNA targets. In this process, the reverse transcriptase enzyme is used to convert RNA to complementary DNA (cDNA) which can then be amplified to facilitate detection.

Many DNA amplification methods are known, most of which rely on an enzymatic chain reaction (such as a polymerase chain reaction, a ligase chain reaction, or a self-sustained sequence replication) or from the replication of all or part of the vector into which it has been cloned.

Many target and signal amplification methods have been described in the literature, for example, general reviews of these methods in Landegren, U. et al., Science 242:229-237 (1988) and Lewis, R., Genetic Engineering News 10:1, 54-55 (1990).

For example, the polymerase chain reaction may be employed to detect CACNA2D3, CAMK2D, KCNIP4, ANGPT1, NAALADL2, ZFHX3 or MPHOSPH10 mRNA.

The "polymerase chain reaction" or "PCR" is a nucleic acid amplification method described *inter alia* in U.S. Pat. Nos. 4,683,195 and 4,683,202. PCR can be used to amplify any known nucleic acid in a diagnostic context (Mok et al., 1994, Gynaecologic Oncology 52:247-252). Self-sustained sequence replication (3SR) is a variation of TAS, which involves the isothermal amplification of a nucleic acid template via sequential rounds of reverse transcriptase (RT), polymerase and nuclease activities that are mediated by an enzyme cocktail and appropriate oligonucleotide primers (Guatelli et al., 1990, Proc. Natl. Acad. Sci. USA 87:1874). Ligation amplification reaction or ligation amplification system uses DNA ligase and four oligonucleotides, two per target strand. This technique is described by Wu, D. Y. and Wallace, R. B., 1989, Genomics 4:560. In the Qβ Replicase technique, RNA replicase for the bacteriophage Qβ, which replicates single-stranded RNA, is used to amplify the target DNA, as described by Lizardi et al., 1988, Bio/Technology 6:1197.

A PCR procedure basically involves: (1) treating extracted DNA to form single-stranded complementary strands; (2) adding a pair of oligonucleotide primers, wherein one primer of the pair is substantially complementary to part of the sequence in the sense strand and the other primer of each pair is substantially complementary to a different part of the same sequence in the complementary antisense strand; (3) annealing the paired primers to the complementary sequence; (4) simultaneously extending the annealed primers from a 3' terminus of each primer to synthesize an extension product complementary to the strands annealed to each primer wherein said extension products after separation from the complement serve as templates for the synthesis of an extension product for the other primer of each pair; (5) separating said extension products from said templates to produce single-stranded molecules; and (6) amplifying said single-stranded molecules by repeating at least once said annealing, extending and separating steps.

Reverse transcription-polymerase chain reaction (RT-PCR) may be employed. Quantitative RT-PCR may also be used. Such PCR techniques are well known in the art, and may employ any suitable primer from a CACNA2D3, CAMK2D, KCNIP4, ANGPT1, NAALADL2, ZFHX3 or MPHOSPH10 sequence.

Alternative amplification technology can also be exploited. For example, rolling circle amplification (Lizardi et al., 1998, Nat Genet 19:225) is an amplification technology available commercially (RCAT™) which is driven by DNA polymerase and can replicate circular oligonucleotide probes with either linear or geometric kinetics under isothermal conditions. A further technique, strand displacement amplification (SDA; Walker et al., 1992, Proc. Natl. Acad. Sci. USA 80:392) begins with a specifically defined sequence unique to a specific target.

### MEASURING EXPRESSION OF AT THE POLYPEPTIDE LEVEL

CACNA2D3, CAMK2D, KCNIP4, ANGPT1, NAALADL2, ZFHX3 or MPHOSPH10 expression may be detected at the polypeptide level.

Therefore, CACNA2D3, CAMK2D, KCNIP4, ANGPT1, NAALADL2, ZFHX3 or MPHOSPH 10 expression, amount or activity may be detected by detecting the presence or amount of CACNA2D3, CAMK2D, KCNIP4, ANGPT1, NAALADL2, ZFHX3 or MPHOSPH10 polypeptide in a sample. This may be achieved by using molecules which bind to CACNA2D3, CAMK2D, KCNIP4, ANGPT1, NAALADL2, ZFHX3 or MPHOSPH10 polypeptide. Suitable molecules/agents which bind either directly or indirectly to the CACNA2D3, CAMK2D, KCNIP4, ANGPT1, NAALADL2, ZFHX3 or MPHOSPH10 polypeptide in order to detect its presence include naturally occurring molecules such as peptides and proteins, for example antibodies, or they may be synthetic molecules.

Thus, we disclose a method of detecting the presence of a CACNA2D3, CAMK2D, KCNIP4, ANGPT1, NAALADL2, ZFHX3 or MPHOSPH10 polypeptide by contacting a cell sample with an antibody capable of binding the polypeptide and monitoring said sample for the presence of the polypeptide.

For example, the CACNA2D3, CAMK2D, KCNIP4, ANGPT1, NAALADL2, ZFHX3 or MPHOSPH10 polypeptide may be detected using an anti-CACNA2D3, CAMK2D, KCNIP4, ANGPT1, NAALADL2, ZFHX3 or MPHOSPH10 antibody. Such antibodies may be made by means known in the art, as described for example in Harlow and Lane.

This may conveniently be achieved by monitoring the presence of a complex formed between the antibody and the polypeptide, or monitoring the binding between the polypeptide and the antibody. Methods of detecting binding between two entities are known in the art, and include FRET (fluorescence resonance energy transfer), surface plasmon resonance, etc.

Standard laboratory techniques such as immunoblotting as described above can be used to detect altered levels of CACNA2D3, CAMK2D, KCNIP4, ANGPT1, NAALADL2, ZFHX3 or MPHOSPH 10 protein, as compared with untreated cells in the same cell population.

Gene expression may also be determined by detecting changes in post-translational processing of CACNA2D3, CAMK2D, KCNIP4, ANGPT1, NAALADL2, ZFHX3 or MPHOSPH10 polypeptides or post-transcriptional modification of CACNA2D3, CAMK2D, KCNIP4, ANGPT1, NAALADL2, ZFHX3 or MPHOSPH10 nucleic acids. For example, differential phosphorylation of CACNA2D3, CAMK2D, KCNIP4, ANGPT1, NAALADL2, ZFHX3 or MPHOSPH10 polypeptides, the cleavage of CACNA2D3, CAMK2D, KCNIP4, ANGPT1, NAALADL2, ZFHX3 or MPHOSPH10 polypeptides or alternative splicing of CACNA2D3, CAMK2D, KCNIP4, ANGPT1, NAALADL2, ZFHX3 or MPHOSPH10 RNA, and the like may be measured. Levels of expression of gene products such as CACNA2D3, CAMK2D, KCNIP4, ANGPT1, NAALADL2, ZFHX3 or MPHOSPH10 polypeptides, as well as their post-translational modification, may be detected using proprietary protein assays or techniques such as 2D polyacrylamide gel electrophoresis.

Assay techniques that can be used to determine levels of CACNA2D3, CAMK2D, KCNIP4, ANGPT1, NAALADL2, ZFHX3 or MPHOSPH10 protein in a sample derived from a host are well-known to those of skill in the art. Antibodies can be assayed for immunospecific binding by any method known in the art.

The immunoassays which can be used include but are not limited to competitive and non-competitive assay systems using techniques such as western blots, radioimmunoassays, ELISA, sandwich immunoassays, immunoprecipitation assays, precipitin reactions, gel diffusion precipitin reactions, immunodiffusion assays, agglutination assays, complement-fixation assays, immunoradiometric assays, fluorescent immunoassays and protein A immunoassays. Such assays are routine in the art (see, for example, Ausubel et al., eds, 1994, Current Protocols in Molecular Biology, Vol. 1, John Wiley & Sons, Inc., New York, which is incorporated by reference herein in its entirety).

The specimen may be assayed for polypeptides/proteins by immunohistochemical and immunocytochemical staining (see generally Stites and Terr, Basic and Clinical Immunology, Appleton and Lange, 1994), ELISA, RIA, immunoblots, Western blotting, immunoprecipitation, functional assays and protein truncation test. Other assay methods include radioimmunoassays, competitive-binding assays, Western Blot analysis and ELISA assays.

ELISA assays are well known to those skilled in the art. Both polyclonal and monoclonal antibodies may be used in the assays. Where appropriate other immunoassays, such as radioimmunoassays (RIA) may be used as are known to those in the art. Available immunoassays are extensively described in the patent and scientific literature. See, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521 as well as Sambrook et al, 1992.

### SCREENING FOR MODULATORS

### Identifying Modulators, Agonists and Antagonists

Antagonists, in particular, small molecules may be used to specifically inhibit CACNA2D3, CAMK2D, KCNIP4, ANGPT1, NAALADL2, ZFHX3 or MPHOSPH10 for the treatment of Kawasaki Disease.

We therefore disclose CACNA2D3, CAMK2D, KCNIP4, ANGPT1, NAALADL2, ZFHX3 and MPHOSPH10 antagonists and small molecule inhibitors, as well as assays for screening for these. Antagonists of CACNA2D3, CAMK2D, KCNIP4, ANGPT1, NAALADL2, ZFHX3 or MPHOSPH10 may be screened by detecting modulation, such as down regulation, of binding or other relevant activity. We therefore provide a compound capable of down-regulating the expression, amount or activity of CACNA2D3, CAMK2D, KCNIP4, ANGPT1, NAALADL2, ZFHX3 or MPHOSPH10 polypeptide. Such a compound may be used in the methods and compositions described here for treating or preventing Kawasaki Disease.

CACNA2D3, CAMK2D, KCNIP4, ANGPT1, NAALADL2, ZFHX3 or MPHOSPH10 may therefore be used to assess the binding of small molecule substrates and ligands in, for example, cells, cell-free preparations, chemical libraries, and natural product mixtures. These substrates and ligands may be natural substrates and ligands or may be structural or functional mimetics. See Coligan et al., Current Protocols in Immunology 1(2):Chapter 5 (1991). Furthermore, screens may be conducted to identify factors which influence the expression of CACNA2D3, CAMK2D, KCNIP4, ANGPT1, NAALADL2, ZFHX3 or MPHOSPH10.

In general, the assays for agonists and antagonists rely on determining the effect of candidate molecules on one or more activities of CACNA2D3, CAMK2D, KCNIP4, ANGPT1, NAALADL2, ZFHX3 or MPHOSPH10. An assay may involve assaying polypeptide activity in the presence of a candidate molecule, and optionally in the absence of the candidate molecule, or in the presence of a molecule known to inhibit or activate an activity.

We demonstrate that expression of CACNA2D3, CAMK2D, KCNIP4, ANGPT1, NAALADL2, ZFHX3 or MPHOSPH10 is modulated in Kawasaki Disease cells or cells derived from Kawasaki Disease patients. Accordingly, control of expression of CACNA2D3, CAMK2D, KCNIP4, ANGPT1, NAALADL2, ZFHX3 or MPHOSPH10 may be employed to treat Kawasaki Disease. Therefore, it is desirous to find compounds and drugs which stimulate, reduce or otherwise modulate the expression and/or activity of CACNA2D3, CAMK2D, KCNIP4, ANGPT1, NAALADL2, ZFHX3 or MPHOSPH10, or which can inhibit the function of the protein. In general, agonists and antagonists are employed for therapeutic and prophylactic purposes for Kawasaki Disease.

By "down-regulation" we include any negative effect on the behaviour being studied; this may be total or partial. Thus, where binding is being detected, candidate antagonists are capable of reducing, ameliorating, or abolishing the binding between two entities. The down-regulation of binding (or any other activity) achieved by the candidate molecule may be at least 10%, such as at least 20%, at least 30%, at least 40%, at least 50%, at least 60%, at least 70%, at least 80%, at least 90% or more compared to binding (or which ever activity) in the absence of the candidate molecule. Thus, a candidate molecule suitable for use as an antagonist is one which is capable of reducing by 10% more the binding or other activity.

The term "compound" refers to a chemical compound (naturally occurring or synthesised), such as a biological macromolecule (e.g., nucleic acid, protein, non-peptide, or organic molecule), or an extract made from biological materials such as bacteria, plants, fungi, or animal (particularly mammalian) cells or tissues, or even an inorganic element or molecule. The compound may be an antibody.

Examples of potential antagonists of CACNA2D3, CAMK2D, KCNIP4, ANGPT1, NAALADL2, ZFHX3 or MPHOSPH10 include antibodies, small molecules, nucleotides and their analogues, including purines and purine analogues, oligonucleotides or proteins which are closely related to a binding partner of the relevant polypeptide, e.g., a fragment of the binding partner, or small molecules which bind to the polypeptide but do not elicit a response, so that the activity of the polypeptide is prevented, etc.

### Screening Kits

The materials necessary for such screening to be conducted may be packaged into a screening kit.

Such a screening kit is useful for identifying agonists, antagonists, ligands, receptors, substrates, enzymes, etc. for CACNA2D3, CAMK2D, KCNIP4, ANGPT1, NAALADL2, ZFHX3 or MPHOSPH 10 polypeptides or compounds which decrease or enhance the production of the relevant polypeptide. The screening kit may comprise: (a) a CACNA2D3, CAMK2D, KCNIP4, ANGPT1, NAALADL2, ZFHX3 or MPHOSPH10 polypeptide; (b) a recombinant cell expressing a CACNA2D3, CAMK2D, KCNIP4, ANGPT1, NAALADL2, ZFHX3 or MPHOSPH10 polypeptide; or (c) an antibody to CACNA2D3, CAMK2D, KCNIP4, ANGPT1, NAALADL2, ZFHX3 or MPHOSPH10 polypeptide. The screening kit may comprise a library. The screening kit may comprise any one or more of the components needed for screening, as described below. The screening kit may optionally comprise instructions for use.

Screening kits may also be provided which are capable of detecting CACNA2D3, CAMK2D, KCNIP4, ANGPT1, NAALADL2, ZFHX3 or MPHOSPH10 expression at the nucleic acid level. Such kits may comprise a primer for amplification of CACNA2D3, CAMK2D, KCNIP4, ANGPT1, NAALADL2, ZFHX3 or MPHOSPH10, or a pair of primers for amplification. The primer or primers may be chosen from any suitable sequence, for example a portion of the CACNA2D3, CAMK2D, KCNIP4, ANGPT1, NAALADL2, ZFHX3 or MPHOSPH 10 sequence. Methods of identifying primer sequences are well known in the art, and the skilled person will be able to design such primers with ease. The kits may comprise a nucleic acid probe for CACNA2D3, CAMK2D, KCNIP4, ANGPT1, NAALADL2, ZFHX3 or MPHOSPH 10 expression, as described in this document. The kits may also optionally comprise instructions for use.

### Rational Design

Rational design of candidate compounds likely to be able to interact with CACNA2D3, CAMK2D, KCNIP4, ANGPT1, NAALADL2, ZFHX3 or MPHOSPH10 may be based upon structural studies of the molecular shapes of a CACNA2D3, CAMK2D, KCNIP4, ANGPT1, NAALADL2, ZFHX3 or MPHOSPH10 polypeptide. One means for determining which sites interact with specific other proteins is a physical structure determination, e.g., X-ray crystallography or two-dimensional NMR techniques. These will provide guidance as to which amino acid residues form molecular contact regions. For a detailed description of protein structural determination, see, e.g., Blundell and Johnson (1976) Protein Crystallography, Academic Press, New York.

### Polypeptide Binding Assays

Modulators and antagonists of CACNA2D3, CAMK2D, KCNIP4, ANGPT1, NAALADL2, ZFHX3 or MPHOSPH10 activity or expression may be identified by any means known in the art.

In their simplest form, the assays may simply comprise the steps of mixing a candidate compound with a solution containing a CACNA2D3, CAMK2D, KCNIP4, ANGPT1, NAALADL2, ZFHX3 or MPHOSPH 10 polypeptide to form a mixture, measuring activity of CACNA2D3, CAMK2D, KCNIP4, ANGPT1, NAALADL2, ZFHX3 or MPHOSPH10 polypeptide in the mixture, and comparing the activity of the mixture to a standard.

Furthermore, molecules may be identified by their binding to CACNA2D3, CAMK2D, KCNIP4, ANGPT1, NAALADL2, ZFHX3 or MPHOSPH10, in an assay which detects binding between CACNA2D3, CAMK2D, KCNIP4, ANGPT1, NAALADL2, ZFHX3 or MPHOSPH10 and the putative molecule.

One type of assay for identifying substances that bind to a CACNA2D3, CAMK2D, KCNIP4, ANGPT1, NAALADL2, ZFHX3 or MPHOSPH10 polypeptide described here involves contacting the CACNA2D3, CAMK2D, KCNIP4, ANGPT1, NAALADL2, ZFHX3 or MPHOSPH10 polypeptide, which is immobilised on a solid support, with a non-immobilised candidate substance determining whether and/or to what extent the CACNA2D3, CAMK2D, KCNIP4, ANGPT1, NAALADL2, ZFHX3 or MPHOSPH10 polypeptide of interest and candidate substance bind to each other. Alternatively, the candidate substance may be immobilised and the CACNA2D3, CAMK2D, KCNIP4, ANGPT1, NAALADL2, ZFHX3 or MPHOSPH10 polypeptide as set out in this document non-immobilised.

The binding of the substance to the CACNA2D3, CAMK2D, KCNIP4, ANGPT1, NAALADL2, ZFHX3 or MPHOSPH 10 polypeptide can be transient, reversible or permanent. The substance may bind to the polypeptide with a Kd value which is lower than the Kd value for binding to control polypeptides (e.g., polypeptides known to not be involved in Kawasaki Disease). The Kd value of the substance may be 2 fold less than the Kd value for binding to control polypeptides, such as a Kd value 100 fold less or a Kd 1000 fold less than that for binding to the control polypeptide.

In an example assay method, the CACNA2D3, CAMK2D, KCNIP4, ANGPT1, NAALADL2, ZFHX3 or MPHOSPH 10 polypeptide may be immobilised on beads such as agarose beads. Typically this may be achieved by expressing the CACNA2D3, CAMK2D, KCNIP4, ANGPT1, NAALADL2, ZFHX3 or MPHOSPH 10 polypeptide as a GST-fusion protein in bacteria, yeast or higher eukaryotic cell lines and purifying the GST-polypeptide fusion protein from crude cell extracts using glutathione-agarose beads (Smith and Johnson, 1988; Gene 67(10):31-40). As a control, binding of the candidate substance, which is not a GST-fusion protein, to an immobilised polypeptide may be determined in the absence of the CACNA2D3, CAMK2D, KCNIP4, ANGPT1, NAALADL2, ZFHX3 or MPHOSPH10 polypeptide. The binding of the candidate substance to the immobilised CACNA2D3, CAMK2D, KCNIP4, ANGPT1, NAALADL2, ZFHX3 or MPHOSPH10 polypeptide may then be determined. This type of assay is known in the art as a GST pulldown assay. Again, the candidate substance may be immobilised and the CACNA2D3, CAMK2D, KCNIP4, ANGPT1, NAALADL2, ZFHX3 or MPHOSPH10 polypeptide non-immobilised.

It is also possible to perform this type of assay using different affinity purification systems for immobilising one of the components, for example Ni-NTA agarose and histidine-tagged components.

Binding of the polypeptide to the candidate substance may be determined by a variety of methods well-known in the art. For example, the non-immobilised component may be labeled (with for example, a radioactive label, an epitope tag or an enzyme-antibody conjugate). Alternatively, binding may be determined by immunological detection techniques. For example, the reaction mixture can be Western blotted and the blot probed with an antibody that detects the non-immobilised component. ELISA techniques may also be used.

Candidate substances are typically added to a final concentration of from 1 to 1000 nmol/ml, such as from 1 to 100 nmol/ml. In the case of antibodies, the final concentration used is typically from 100 to 500 µg/ml, such as from 200 to 300 µg/ml.

Modulators and antagonists of CACNA2D3, CAMK2D, KCNIP4, ANGPT1, NAALADL2, ZFHX3 or MPHOSPH10 may also be identified by detecting modulation of binding between CACNA2D3, CAMK2D, KCNIP4, ANGPT1, NAALADL2, ZFHX3 or MPHOSPH10 and any molecule to which this polypeptide binds, or modulation of any activity consequential on such binding or release.

### Cell Based Assays

A cell based assay may simply test binding of a candidate compound wherein adherence to the cells bearing the CACNA2D3, CAMK2D, KCNIP4, ANGPT1, NAALADL2, ZFHX3 or MPHOSPH10 polypeptide is detected by means of a label directly or indirectly associated with the candidate compound or in an assay involving competition with a labeled competitor.

Further, these assays may test whether the candidate compound results in a signal generated by binding to the CACNA2D3, CAMK2D, KCNIP4, ANGPT1, NAALADL2, ZFHX3 or MPHOSPH 10 polypeptide, using detection systems appropriate to the cells bearing the polypeptides at their surfaces. Inhibitors of activation are generally assayed in the presence of a known agonist and the effect on activation by the agonist by the presence of the candidate compound is observed.

Another method of screening compounds utilises eukaryotic or prokaryotic host cells which are stably transformed with recombinant DNA molecules expressing a library of compounds. Such cells, either in viable or fixed form, can be used for standard binding-partner assays. See also Parce et al. (1989) Science 246:243-247; and Owicki et al. (1990) Proc. Nat'l Acad. Sci. USA 87;4007-4011, which describe sensitive methods to detect cellular responses.

Competitive assays are particularly useful, where the cells expressing the library of compounds are contacted or incubated with a labelled antibody known to bind to a CACNA2D3, CAMK2D, KCNIP4, ANGPT1, NAALADL2, ZFHX3 or MPHOSPH10 polypeptide, such as ¹²⁵I-antibody, and a test sample such as a candidate compound whose binding affinity to the binding composition is being measured. The bound and free labelled binding partners for the CACNA2D3, CAMK2D, KCNIP4, ANGPT1, NAALADL2, ZFHX3 or MPHOSPH10 polypeptide are then separated to assess the degree of binding. The amount of test sample bound is inversely proportional to the amount of labelled antibody binding to the CACNA2D3, CAMK2D, KCNIP4, ANGPT1, NAALADL2, ZFHX3 or MPHOSPH10 polypeptide.

Any one of numerous techniques can be used to separate bound from free binding partners to assess the degree of binding. This separation step could typically involve a procedure such as adhesion to filters followed by washing, adhesion to plastic following by washing, or centrifugation of the cell membranes.

The assays may involve exposing a candidate molecule to a cell and assaying expression of CACNA2D3, CAMK2D, KCNIP4, ANGPT1, NAALADL2, ZFHX3 or MPHOSPH10 by any suitable means. Molecules which down-regulate the expression of CACNA2D3, CAMK2D, KCNIP4, ANGPT1, NAALADL2, ZFHX3 or MPHOSPH10 in such assays may be optionally chosen for further study, and used as drugs to down-regulate CACNA2D3, CAMK2D, KCNIP4, ANGPT1, NAALADL2, ZFHX3 or MPHOSPH10 expression. Such drugs may be usefully employed to treat or prevent Kawasaki Disease.

cDNA encoding CACNA2D3, CAMK2D, KCNIP4, ANGPT1, NAALADL2, ZFHX3 or MPHOSPH 10 protein and antibodies to the proteins may also be used to configure assays for detecting the effect of added compounds on the production of CACNA2D3, CAMK2D, KCNIP4, ANGPT1, NAALADL2, ZFHX3 or MPHOSPH10 mRNA and protein in cells. For example, an ELISA may be constructed for measuring secreted or cell associated levels of CACNA2D3, CAMK2D, KCNIP4, ANGPT1, NAALADL2, ZFHX3 or MPHOSPH10 polypeptide using monoclonal and polyclonal antibodies by standard methods known in the art, and this can be used to discover agents which may inhibit or enhance the production of CACNA2D3, CAMK2D, KCNIP4, ANGPT1, NAALADL2, ZFHX3 or MPHOSPH10 protein (also called antagonist or agonist, respectively) from suitably manipulated cells or tissues. Standard methods for conducting screening assays are well understood in the art.

### Activity Assays

Assays to detect modulators or antagonists typically involve detecting modulation of any activity of CACNA2D3, CAMK2D, KCNIP4, ANGPT1, NAALADL2, ZFHX3 or MPHOSPH10, in the presence, optionally together with detection of modulation of activity in the absence, of a candidate molecule.

Assays which detect specific biological activities of CACNA2D3, CAMK2D, KCNIP4, ANGPT1, NAALADL2, ZFHX3 or MPHOSPH10 may be used. The assays typically involve contacting a candidate molecule (e.g., in the form of a library) with CACNA2D3, CAMK2D, KCNIP4, ANGPT1, NAALADL2, ZFHX3 or MPHOSPH10 whether in the form of a polypeptide, a nucleic acid encoding the polypeptide, or a cell, organelle, extract, or other material comprising such, with a candidate modulator. The relevant activity of CACNA2D3, CAMK2D, KCNIP4, ANGPT1, NAALADL2, ZFHX3 or MPHOSPH10 (such as phosphatase activity, as described below) may be detected, to establish whether the presence of the candidate modulator has any effect.

Relevant activities of CACNA2D3, CAMK2D, KCNIP4, ANGPT1, NAALADL2, ZFHX3 or MPHOSPH 10 which may be assayed are described in the sections above relating to the relevant polypeptide. Assays for such activities are known in the art and may be employed for detecting the biological activities of CACNA2D3, CAMK2D, KCNIP4, ANGPT1, NAALADL2, ZFHX3 or MPHOSPH10 for screening.

Promoter binding assays to detect candidate modulators which bind to and/or affect the transcription or expression of CACNA2D3, CAMK2D, KCNIP4, ANGPT1, NAALADL2, ZFHX3 or MPHOSPH10 may also be used. Candidate modulators may then be chosen for further study, or isolated for use. Details of such screening procedures are well known in the art, and are for example described in, Handbook of Drug Screening, edited by Ramakrishna Seethala, Prabhavathi B. Fernandes (2001, New York, NY, Marcel Dekker, ISBN 0-8247-0562-9).

The screening methods described here may employ *in vivo* assays, although they may be configured for *in vitro* use. *In vivo* assays generally involve exposing a cell comprising CACNA2D3, CAMK2D, KCNIP4, ANGPT1, NAALADL2, ZFHX3 or MPHOSPH10 to the candidate molecule. In *in vitro* assays, CACNA2D3, CAMK2D, KCNIP4, ANGPT1, NAALADL2, ZFHX3 or MPHOSPH10 is exposed to the candidate molecule, optionally in the presence of other components, such as crude or semi-purified cell extract, or purified proteins. Where *in vitro* assays are conducted, these may employ arrays of candidate molecules (for example, an arrayed library). *In vivo* assays may be employed. Therefore, the CACNA2D3, CAMK2D, KCNIP4, ANGPT1, NAALADL2, ZFHX3 or MPHOSPH10 polypeptide may be comprised in a cell, such as heterologously. Such a cell may be a transgenic cell, which has been engineered to express CACNA2D3, CAMK2D, KCNIP4, ANGPT1, NAALADL2, ZFHX3 or MPHOSPH 10 as described above.

Where an extract is employed, it may comprise a cytoplasmic extract or a nuclear extract, methods of preparation of which are well known in the art.

It will be appreciated that any component of a cell comprising CACNA2D3, CAMK2D, KCNIP4, ANGPT1, NAALADL2, ZFHX3 or MPHOSPH10 may be employed, such as an organelle. One embodiment utilises a cytoplasmic or nuclear preparation, e.g., comprising a cell nucleus which comprises CACNA2D3, CAMK2D, KCNIP4, ANGPT1, NAALADL2, ZFHX3 or MPHOSPH 10 as described. The nuclear preparation may comprise one or more nuclei, which may be permeabilised or semi-permeabilised, by detergent treatment, for example.

Thus, in a specific embodiment, an assay format may include the following: a multiwell microtitre plate is set up to include one or more cells expressing CACNA2D3, CAMK2D, KCNIP4, ANGPT1, NAALADL2, ZFHX3 or MPHOSPH10 polypeptide in each well; individual candidate molecules, or pools of candidate molecules, derived for example from a library, may be added to individual wells and modulation of CACNA2D3, CAMK2D, KCNIP4, ANGPT1, NAALADL2, ZFHX3 or MPHOSPH10 activity measured. Where pools are used, these may be subdivided in to further pools and tested in the same manner. CACNA2D3, CAMK2D, KCNIP4, ANGPT1, NAALADL2, ZFHX3 or MPHOSPH10 activity, for example binding activity or transcriptional co-activation activity, as described elsewhere in this document may then be assayed.

Alternatively or in addition to the assay methods described above, "subtractive" procedures may also be used to identify modulators or antagonists of CACNA2D3, CAMK2D, KCNIP4, ANGPT1, NAALADL2, ZFHX3 or MPHOSPH10. Under such "subtractive" procedures, a plurality of molecules is provided, which comprises one or more candidate molecules capable of functioning as a modulator (e.g., cell extract, nuclear extract, library of molecules, etc), and one or more components is removed, depleted or subtracted from the plurality of molecules. The "subtracted" extract, etc, is then assayed for activity, by exposure to a cell comprising CACNA2D3, CAMK2D, KCNIP4, ANGPT1, NAALADL2, ZFHX3 or MPHOSPH10 (or a component thereof) as described.

Thus, for example, an 'immunodepletion' assay may be conducted to identify such modulators as follows. A cytoplasmic or nuclear extract may be prepared from a suitable cell. The extract may be depleted or fractionated to remove putative modulators, such as by use of immunodepletion with appropriate antibodies. If the extract is depleted of a modulator, it will lose the ability to affect CACNA2D3, CAMK2D, KCNIP4, ANGPT1, NAALADL2, ZFHX3 or MPHOSPH10 function or activity or expression. A series of subtractions and/or depletions may be required to identify the modulators or antagonists.

It will also be appreciated that the above "depletion" or "subtraction" assay may be used as a preliminary step to identify putative modulatory factors for further screening. Furthermore, or alternatively, the "depletion" or "subtraction" assay may be used to confirm the modulatory activity of a molecule identified by other means (for example, a "positive" screen as described elsewhere in this document) as a putative modulator.

Candidate molecules subjected to the assay and which are found to be of interest may be isolated and further studied. Methods of isolation of molecules of interest will depend on the type of molecule employed, whether it is in the form of a library, how many candidate molecules are being tested at any one time, whether a batch procedure is being followed, etc.

The candidate molecules may be provided in the form of a library. In one embodiment, more than one candidate molecule may be screened simultaneously. A library of candidate molecules may be generated, for example, a small molecule library, a polypeptide library, a nucleic acid library, a library of compounds (such as a combinatorial library), a library of antisense molecules such as antisense DNA or antisense RNA, an antibody library etc, by means known in the art. Such libraries are suitable for high-throughput screening. Different cells comprising CACNA2D3, CAMK2D, KCNIP4, ANGPT1, NAALADL2, ZFHX3 or MPHOSPH10 may be exposed to individual members of the library, and effect on the CACNA2D3, CAMK2D, KCNIP4, ANGPT1, NAALADL2, ZFHX3 or MPHOSPH10 activity determined. Array technology may be employed for this purpose. The cells may be spatially separated, for example, in wells of a microtitre plate.

In an embodiment, a small molecule library is employed. By a "small molecule", we refer to a molecule whose molecular weight may be less than about 50 kDa. In particular embodiments, a small molecule may have a molecular weight which is less than about 30 kDa, such as less than about 15 kDa or less than 10 kDa or so. Libraries of such small molecules, here referred to as "small molecule libraries" may contain polypeptides, small peptides, for example, peptides of 20 amino acids or fewer, for example, 15, 10 or 5 amino acids, simple compounds, etc.

Alternatively or in addition, a combinatorial library, as described in further detail below, may be screened for modulators or antagonists of CACNA2D3, CAMK2D, KCNIP4, ANGPT1, NAALADL2, ZFHX3 or MPHOSPH10. Assays for CACNA2D3, CAMK2D, KCNIP4, ANGPT1, NAALADL2, ZFHX3 or MPHOSPH10 activity are described above.

### Libraries

Libraries of candidate molecules, such as libraries of polypeptides or nucleic acids, may be employed in the screens for CACNA2D3, CAMK2D, KCNIP4, ANGPT1, NAALADL2, ZFHX3 or MPHOSPH 10 antagonists and inhibitors described here. Such libraries are exposed to CACNA2D3, CAMK2D, KCNIP4, ANGPT1, NAALADL2, ZFHX3 or MPHOSPH10 protein, and their effect, if any, on the activity of the protein determined.

Selection protocols for isolating desired members of large libraries are known in the art, as typified by phage display techniques. Such systems, in which diverse peptide sequences are displayed on the surface of filamentous bacteriophage (Scott and Smith (1990 *supra*), have proven useful for creating libraries of antibody fragments (and the nucleotide sequences that encoding them) for the *in vitro* selection and amplification of specific antibody fragments that bind a target antigen. The nucleotide sequences encoding the V_{H} and V_{L} regions are linked to gene fragments which encode leader signals that direct them to the periplasmic space of *E. coli* and as a result the resultant antibody fragments are displayed on the surface of the bacteriophage, typically as fusions to bacteriophage coat proteins (e.g., pIII or pVIII). Alternatively, antibody fragments are displayed externally on lambda phage capsids (phagebodies). An advantage of phage-based display systems is that, because they are biological systems, selected library members can be amplified simply by growing the phage containing the selected library member in bacterial cells. Furthermore, since the nucleotide sequence that encodes the polypeptide library member is contained on a phage or phagemid vector, sequencing, expression and subsequent genetic manipulation is relatively straightforward.

Methods for the construction of bacteriophage antibody display libraries and lambda phage expression libraries are well known in the art (McCafferty *et al*. (1990) **supra**; Kang et al. (1991) Proc. Natl. Acad. Sci. U.S.A., 88: 4363; Clackson et al. (1991) Nature, 352: 624; Lowman et al. (1991) Biochemistry, 30: 10832; Burton et al. (1991) Proc. Natl. Acad Sci U.S.A., 88: 10134; Hoogenboom et al. (1991) Nucleic Acids Res., 19: 4133; Chang et al. (1991) J. Immunol., 147: 3610; Breitling et al. (1991) Gene, 104: 147; Marks *et al.* (1991) supra; Barbas *et al.* (1992) supra; Hawkins and Winter (1992) J. Immunol., 22: 867; Marks et al., 1992, J. Biol. Chem., 267: 16007; Lerner et al. (1992) Science, 258: 1313, incorporated herein by reference). Such techniques may be modified if necessary for the expression generally of polypeptide libraries.

One particularly advantageous approach has been the use of scFv phage-libraries (Bird, R.E., et al. (1988) Science 242: 423-6, Huston et al., 1988, Proc. Natl. Acad. Sci U.S.A., 85: 5879-5883; Chaudhary et al. (1990) Proc. Natl. Acad. Sci U.S.A., 87: 1066-1070; McCafferty *et al*. (1990) supra; Clackson *et al.* (1991) supra; Marks *et al.* (1991) supra; Chiswell et al. (1992) Trends Biotech., 10: 80; Marks *et al.* (1992) supra). Various embodiments ofscFv libraries displayed on bacteriophage coat proteins have been described. Refinements of phage display approaches are also known, for example as described in WO96/06213 and WO92/01047 (Medical Research Council *et al*.) and WO97/08320 (Morphosys, supra), which are incorporated herein by reference.

Alternative library selection technologies include bacteriophage lambda expression systems, which may be screened directly as bacteriophage plaques or as colonies of lysogens, both as previously described (Huse et al. (1989) Science, 246: 1275; Caton and Koprowski (1990) Proc. Natl. Acad. Sci. U.S.A., 87; Mullinax et al. (1990) Proc. Natl. Acad. Sci. U.S.A., 87: 8095; Persson et al. (1991) Proc. Natl. Acad. Sci. U.S.A., 88: 2432) and are of use in the methods and compositions described here. These expression systems may be used to screen a large number of different members of a library, in the order of about 10⁶ or even more. Other screening systems rely, for example, on direct chemical synthesis of library members. One early method involves the synthesis of peptides on a set of pins or rods, such as described in WO84/03564. A similar method involving peptide synthesis on beads, which forms a peptide library in which each bead is an individual library member, is described in U.S. Patent No. 4,631,211 and a related method is described in WO92/00091. A significant improvement of the bead-based methods involves tagging each bead with a unique identifier tag, such as an oligonucleotide, so as to facilitate identification of the amino acid sequence of each library member. These improved bead-based methods are described in WO93/06121.

Another chemical synthesis method involves the synthesis of arrays of peptides (or peptidomimetics) on a surface in a manner that places each distinct library member (e.g., unique peptide sequence) at a discrete, predefined location in the array. The identity of each library member is determined by its spatial location in the array. The locations in the array where binding interactions between a predetermined molecule (e.g., a receptor) and reactive library members occur is determined, thereby identifying the sequences of the reactive library members on the basis of spatial location. These methods are described in U.S. Patent No. 5,143,854; WO90/15070 and WO92/10092; Fodor et al. (1991) Science, 251: 767; Dower and Fodor (1991) Ann. Rep. Med. Chem., 26: 271.

Other systems for generating libraries of polypeptides or nucleotides involve the use of cell-free enzymatic machinery for the *in vitro* synthesis of the library members. In one method, RNA molecules are selected by alternate rounds of selection against a target ligand and PCR amplification (Tuerk and Gold (1990) Science, 249: 505; Ellington and Szostak (1990) Nature, 346: 818). A similar technique may be used to identify DNA sequences which bind a predetermined human transcription factor (Thiesen and Bach (1990) Nucleic Acids Res., 18: 3203; Beaudry and Joyce (1992) Science, 257: 635; WO92/05258 and WO92/14843). In a similar way, *in vitro* translation can be used to synthesise polypeptides as a method for generating large libraries. These methods which generally comprise stabilised polysome complexes, are described further in WO88/08453, WO90/05785, WO90/07003, WO91/02076, WO91/05058, and WO92/02536. Alternative display systems which are not phage-based, such as those disclosed in WO95/22625 and WO95/11922 (Affymax) use the polysomes to display polypeptides for selection. These and all the foregoing documents also are incorporated herein by reference.

### Combinatorial Libraries

Libraries, in particular, libraries of candidate molecules, may suitably be in the form of combinatorial libraries (also known as combinatorial chemical libraries).

A "combinatorial library", as the term is used in this document, is a collection of multiple species of chemical compounds that consist of randomly selected subunits. Combinatorial libraries may be screened for molecules which are capable of inhibiting CACNA2D3, CAMK2D, KCNIP4, ANGPT1, NAALADL2, ZFHX3 or MPHOSPH10.

Various combinatorial libraries of chemical compounds are currently available, including libraries active against proteolytic and non-proteolytic enzymes, libraries of agonists and antagonists of G-protein coupled receptors (GPCRs), libraries active against non-GPCR targets (e.g., integrins, ion channels, domain interactions, nuclear receptors, and transcription factors) and libraries of whole-cell oncology and anti-infective targets, among others. A comprehensive review of combinatorial libraries, in particular their construction and uses is provided in Dolle and Nelson (1999), Journal of Combinatorial Chemistry, Vol 1 No 4, 235-282. Reference is also made to Combinatorial peptide library protocols (edited by Shmuel Cabilly, Totowa, N.J.: Humana Press, c1998. Methods in Molecular Biology v. 87). Specific combinatorial libraries and methods for their construction are disclosed in United States Patent 6,168,914 (Campbell, et al), as well as in Baldwin et al. (1995), "Synthesis of a Small Molecule Library Encoded with Molecular Tags," J. Am. Chem. Soc. 117:5588-5589, and in the references mentioned in those documents.

In one embodiment, the combinatorial library which is screened is one which is designed to potentially include molecules which interact with a component of the cell to influence gene expression. For example, combinatorial libraries against chromatin structural proteins may be screened. Other libraries which are useful for this embodiment include combinatorial libraries against histone modification enzymes (e.g., histone acetylation or histone metylation enzymes), or DNA modification, for example, DNA methylation or demethylation.

Further references describing chemical combinatorial libraries, their production and use include those available from the URL http://www.netsci.org/Science/Combichem/, including The Chemical Generation of Molecular Diversity. Michael R. Pavia, Sphinx Pharmaceuticals, A Division of Eli Lilly (Published July, 1995); Combinatorial Chemistry: A Strategy for the Future - MDL Information Systems discusses the role its Project Library plays in managing diversity libraries (Published July, 1995); Solid Support Combinatorial Chemistry in Lead Discovery and SAR Optimization, Adnan M. M. Mjalli and Barry E. Toyonaga, Ontogen Corporation (Published July, 1995); Non-Peptidic Bradykinin Receptor Antagonists From a Structurally Directed Non-Peptide Library. Sarvajit Chakravarty, Babu J. Mavunkel, Robin Andy, Donald J. Kyle*, Scios Nova Inc. (Published July, 1995); Combinatorial Chemistry Library Design using Pharmacophore Diversity Keith Davies and Clive Briant, Chemical Design Ltd. (Published July, 1995); A Database System for Combinatorial Synthesis Experiments - Craig James and David Weininger, Daylight Chemical Information Systems, Inc. (Published July, 1995); An Information Management Architecture for Combinatorial Chemistry, Keith Davies and Catherine White, Chemical Design Ltd. (Published July, 1995); Novel Software Tools for Addressing Chemical Diversity, R. S. Pearlman, Laboratory for Molecular Graphics and Theoretical Modeling, College of Pharmacy, University of Texas (Published June/July, 1996); Opportunities for Computational Chemists Afforded by the New Strategies in Drug Discovery: An Opinion, Yvonne Connolly Martin, Computer Assisted Molecular Design Project, Abbott Laboratories (Published June/July, 1996); Combinatorial Chemistry and Molecular Diversity Course at the University of Louisville: A Description, Arno F. Spatola, Department of Chemistry, University of Louisville (Published June/July, 1996); Chemically Generated Screening Libraries: Present and Future. Michael R. Pavia, Sphinx Pharmaceuticals, A Division of Eli Lilly (Published June/July, 1996); Chemical Strategies For Introducing Carbohydrate Molecular Diversity Into The Drug Discovery Process.. Michael J. Sofia, Transcell Technologies Inc. (Published June/July, 1996); Data Management for Combinatorial Chemistry. Maryjo Zaborowski, Chiron Corporation and Sheila H. DeWitt, Parke-Davis Pharmaceutical Research, Division of Warner-Lambert Company (Published November, 1995); and The Impact of High Throughput Organic Synthesis on R&D in Bio-Based Industries, John P. Devlin (Published March, 1996).

Techniques in combinatorial chemistry are gaining wide acceptance among modem methods for the generation of new pharmaceutical leads (Gallop, M. A. et al., 1994, J. Med. Chem. 37:1233-1251; Gordon, E. M. et al., 1994, J. Med. Chem. 37:1385-1401.). One combinatorial approach in use is based on a strategy involving the synthesis of libraries containing a different structure on each particle of the solid phase support, interaction of the library with a soluble receptor, identification of the 'bead' which interacts with the macromolecular target, and determination of the structure carried by the identified 'bead' (Lam, K. S. et al., 1991, Nature 354:82-84). An alternative to this approach is the sequential release of defined aliquots of the compounds from the solid support, with subsequent determination of activity in solution, identification of the particle from which the active compound was released, and elucidation of its structure by direct sequencing (Salmon, S. E. et al., 1993, Proc.Natl.Acad.Sci.USA 90:11708-11712), or by reading its code (Kerr, J. M. et al., 1993, J.Am.Chem.Soc. 115:2529-2531; Nikolaiev, V. et al., 1993, Pept. Res. 6:161-170; Ohlmeyer, M. H. J. et al., 1993, Proc.Natl.Acad.Sci.USA 90:10922-10926).

Soluble random combinatorial libraries may be synthesized using a simple principle for the generation of equimolar mixtures of peptides which was first described by Furka (Furka, A. et al., 1988, Xth International Symposium on Medicinal Chemistry, Budapest 1988; Furka, A. et al., 1988, 14th International Congress of Biochemistry, Prague 1988; Furka, A. et al., 1991, Int. J. Peptide Protein Res. 37:487-493). The construction of soluble libraries for iterative screening has also been described (Houghten, R. A. et al.1991, Nature 354:84-86). K. S. Lam disclosed the novel and unexpectedly powerful technique of using insoluble random combinatorial libraries. Lam synthesized random combinatorial libraries on solid phase supports, so that each support had a test compound of uniform molecular structure, and screened the libraries without prior removal of the test compounds from the support by solid phase binding protocols (Lam, K. S. et al., 1991, Nature 354:82-84).

Thus, a library of candidate molecules may be a synthetic combinatorial library (e.g., a combinatorial chemical library), a cellular extract, a bodily fluid (e.g., urine, blood, tears, sweat, or saliva), or other mixture of synthetic or natural products (e.g., a library of small molecules or a fermentation mixture).

A library of molecules may include, for example, amino acids, oligopeptides, polypeptides, proteins, or fragments of peptides or proteins; nucleic acids (e.g., antisense; DNA; RNA; or peptide nucleic acids, PNA); aptamers; or carbohydrates or polysaccharides. Each member of the library can be singular or can be a part of a mixture (e.g., a compressed library). The library may contain purified compounds or can be "dirty" (i.e., containing a significant quantity of impurities).

Commercially available libraries (e.g., from Affymetrix, ArQule, Neose Technologies, Sarco, Ciddco, Oxford Asymmetry, Maybridge, Aldrich, Panlabs, Pharmacopoeia, Sigma, or Tripose) may also be used with the methods described here.

In addition to libraries as described above, special libraries called diversity files can be used to assess the specificity, reliability, or reproducibility of the new methods. Diversity files contain a large number of compounds (e.g., 1000 or more small molecules) representative of many classes of compounds that could potentially result in nonspecific detection in an assay. Diversity files are commercially available or can also be assembled from individual compounds commercially available from the vendors listed above.

### ANTIBODIES

CACNA2D3, CAMK2D, KCNIP4, ANGPT1, NAALADL2, ZFHX3 or MPHOSPH10 modulators, including antagonists or modulators of CACNA2D3, CAMK2D, KCNIP4, ANGPT1, NAALADL2, ZFHX3 or MPHOSPH10, which may be used to regulate the activity of the protein (for example, for methods of treating or preventing diseases such as Kawasaki Disease as described in this document) may include antibodies against the relevant protein.

We therefore provide for antibodies which bind to aCACNA2D3, CAMK2D, KCNIP4, ANGPT1, NAALADL2, ZFHX3 or MPHOSPH10 polypeptide, fragment, homologue, variant or derivative thereof. Such antibodies are useful in detectingCACNA2D3, CAMK2D, KCNIP4, ANGPT1, NAALADL2, ZFHX3 or MPHOSPH10 expression, and in particular in diagnosing a CACNA2D3, CAMK2D, KCNIP4, ANGPT1, NAALADL2, ZFHX3 or MPHOSPH10 associated disease such as Kawasaki Disease. Other antibodies include those which have therapeutic activity, i.e., which are may be used in a therapeutic manner to treat, manage or prevent anyCACNA2D3, CAMK2D, KCNIP4, ANGPT1, NAALADL2, ZFHX3 or MPHOSPH10 associated disease, including Kawasaki Disease.

An antibody CACNA2D3, CAMK2D, KCNIP4, ANGPT21, NAALADL2, ZFHX3 or MPHOSPH10 may be generated by immunisation with a peptide corresponding to amino acid residues of human polypeptide.

Antibodies which are specific for CACNA2D3, CAMK2D, KCNIP4, ANGPT1, NAALADL2, ZFHX3 or MPHOSPH10 may be generated against any suitable epitope, for example, an epitope derived from the CACNA2D3, CAMK2D, KCNIP4, ANGPT1, NAALADL2, ZFHX3 or MPHOSPH 10 protein.

For the purposes of this document, the term "antibody"refers to complete antibodies or antibody fragments capable of binding to a selected target. Unless specified to the contrary, the term includes but is not limited to, polyclonal, monoclonal, natural or engineered antibodies including chimeric, CDR-grafted and humanised antibodies, and artificially selected antibodies produced using phage display or alternative techniques. The term also includes single chain, Fab fragments and fragments produced by a Fab expression library. Such fragments include fragments of whole antibodies which retain their binding activity for a target substance, Fv, F(ab') and F(ab') fragments, as well as single chain antibodies (scFv), fusion proteins and other synthetic proteins which comprise the antigen-binding site of the antibody. Small fragments, such as Fv and ScFv, possess advantageous properties for diagnostic and therapeutic applications on account of their small size and consequent superior tissue distribution.

The antibodies and fragments thereof may be humanised antibodies, for example as described in EP-A-239400. Furthermore, antibodies with fully human variable regions (or their fragments), for example, as described in US Patent Nos. 5,545,807 and 6,075,181 may also be used. Neutralizing antibodies, i.e., those which inhibit any biological activity of CACNA2D3, CAMK2D, KCNIP4, ANGPT1, NAALADL2, ZFHX3 or MPHOSPH10, may be used for diagnostics and therapeutics.

The antibodies described here may be altered antibodies comprising an effector protein such as a label. Labels which allow the imaging of the distribution of the antibody *in vivo* or *in vitro* may be used Such labels may be radioactive labels or radioopaque labels, such as metal particles, which are readily visualisable within an embryo or a cell mass. Moreover, they may be fluorescent labels or other labels which are visualisable on tissue samples.

Antibodies may be produced by standard techniques, such as by immunisation or by using a phage display library. Such an antibody may be capable of binding specifically to the CACNA2D3, CAMK2D, KCNIP4, ANGPT1, NAALADL2, ZFHX3 or MPHOSPH10 protein or homologue, fragment, etc.

### Polyclonal Antibodies

If polyclonal antibodies are desired, a selected mammal (e.g., mouse, rabbit, goat, horse, etc.) may be immunised with an immunogenic composition comprising a CACNA2D3, CAMK2D, KCNIP4, ANGPT1, NAALADL2, ZFHX3 or MPHOSPH10 polypeptide or peptide. Depending on the host species, various adjuvants may be used to increase immunological response. Such adjuvants include, but are not limited to, Freund's, mineral gels such as aluminium hydroxide, and surface active substances such as lysolecithin, pluronic polyols, polyanions, peptides, oil emulsions, keyhole limpet hemocyanin, and dinitrophenol. BCG (*Bacilli Calmette-Guerin*) and *Corynebacterium parvum* are potentially useful human adjuvants which may be employed if purified the substance amino acid sequence is administered to immunologically compromised individuals for the purpose of stimulating systemic defence.

Serum from the immunised animal is collected and treated according to known procedures. If serum containing polyclonal antibodies to an epitope obtainable from a CACNA2D3, CAMK2D, KCNIP4, ANGPT1, NAALADL2, ZFHX3 or MPHOSPH10 polypeptide contains antibodies to other antigens, the polyclonal antibodies can be purified by immunoaffinity chromatography. Techniques for producing and processing polyclonal antisera are known in the art. In order that such antibodies may be made, we also provide CACNA2D3, CAMK2D, KCNIP4, ANGPT1, NAALADL2, ZFHX3 or MPHOSPH10 amino acid sequences or fragments thereof haptenised to another amino acid sequence for use as immunogens in animals or humans.

### Monoclonal Antibodies

Monoclonal antibodies directed against epitopes obtainable from a CACNA2D3, CAMK2D, KCNIP4, ANGPT1, NAALADL2, ZFHX3 or MPHOSPH10 polypeptide or peptide can also be readily produced by one skilled in the art. The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. Panels of monoclonal antibodies produced against orbit epitopes can be screened for various properties; i.e., for isotype and epitope affinity.

Monoclonal antibodies may be prepared using any technique which provides for the production of antibody molecules by continuous cell lines in culture. These include, but are not limited to, the hybridoma technique originally described by Koehler and Milstein (1975 Nature 256:495-497), the trioma technique, the human B-cell hybridoma technique (Kosbor et al (1983) Immunol Today 4:72; Cote et al (1983) Proc Natl Acad Sci 80:2026-2030) and the EBV-hybridoma technique (Cole et al., Monoclonal Antibodies and Cancer Therapy, pp. 77-96, Alan R. Liss, Inc., 1985).

Recombinant DNA technology may be used to improve the antibodies as described here. Thus, chimeric antibodies may be constructed in order to decrease the immunogenicity thereof in diagnostic or therapeutic applications. Such techniques comprise splicing of mouse antibody genes to human antibody genes to obtain a molecule with appropriate antigen specificity and biological activity (Morrison et al (1984) Proc Natl Acad Sci 81:6851-6855; Neuberger et al (1984) Nature 312:604-608; Takeda et al (1985) Nature 314:452-454). Moreover, immunogenicity may be minimised by humanising the antibodies by CDR grafting [see European Patent Application 0 239 400 (Winter)] and, optionally, framework modification [EP 0 239 400].

Alternatively, techniques described for the production of single chain antibodies (US Patent No. 4,946,779) can be adapted to produce the substance specific single chain antibodies.

Antibodies, both monoclonal and polyclonal, which are directed against epitopes obtainable from a CACNA2D3, CAMK2D, KCNIP4, ANGPT1, NAALADL2, ZFHX3 or MPHOSPH 10 polypeptide or peptide are particularly useful in diagnosis. Monoclonal antibodies, in particular, may be used to raise anti-idiotype antibodies. Anti-idiotype antibodies are immunoglobulins which carry an "internal image" of the substance and/or agent against which protection is desired. Techniques for raising anti-idiotype antibodies are known in the art. These anti-idiotype antibodies may also be useful in therapy.

Antibodies may also be produced by inducing *in vivo* production in the lymphocyte population or by screening recombinant immunoglobulin libraries or panels of highly specific binding reagents as disclosed in Orlandi et al (1989, Proc Natl Acad Sci 86: 3833-3837), and Winter G and Milstein C (1991; Nature 349:293-299).

Antibody fragments which contain specific binding sites for the polypeptide or peptide may also be generated. For example, such fragments include, but are not limited to, the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed to allow rapid and easy identification of Monoclonal Fab fragments with the desired specificity (Huse WD et al (1989) Science 256:1275-128 1).

Techniques for the production of single chain antibodies (U.S. Pat. No. 4,946,778) can also be adapted to produce single chain antibodies to CACNA2D3, CAMK2D, KCNIP4, ANGPT1, NAALADL2, ZFHX3 or MPHOSPH10 polypeptides. Also, transgenic mice, or other organisms including other mammals, may be used to express humanized antibodies.

The above-described antibodies may be employed to isolate or to identify clones expressing the polypeptide or to purify the polypeptides by affinity chromatography.

### Recombinant Techniques of Antibody Production

Recombinant DNA technology may be used to produce the antibodies according to established procedure, in bacterial or mammalian cell culture. The selected cell culture system may secrete the antibody product.

Therefore, we disclose a process for the production of an antibody comprising culturing a host, e.g. *E. coli* or a mammalian cell, which has been transformed with a hybrid vector comprising an expression cassette comprising a promoter operably linked to a first DNA sequence encoding a signal peptide linked in the proper reading frame to a second DNA sequence encoding said antibody protein, and isolating said protein.

Multiplication of hybridoma cells or mammalian host cells *in vitro* is carried out in suitable culture media, which are the customary standard culture media, for example Dulbecco's Modified Eagle Medium (DMEM) or RPMI 1640 medium, optionally replenished by a mammalian serum, e.g. foetal calf serum, or trace elements and growth sustaining supplements, e.g. feeder cells such as normal mouse peritoneal exudate cells, spleen cells, bone marrow macrophages, 2-aminoethanol, insulin, transferrin, low density lipoprotein, oleic acid, or the like. Multiplication of host cells which are bacterial cells or yeast cells is likewise carried out in suitable culture media known in the art, for example for bacteria in medium LB, NZCYM, NZYM, NZM, Terrific Broth, SOB, SOC, 2 x YT, or M9 Minimal Medium, and for yeast in medium YPD, YEPD, Minimal Medium, or Complete Minimal Dropout Medium.

*In vitro* production provides relatively pure antibody preparations and allows scale-up to give large amounts of the desired antibodies. Techniques for bacterial cell, yeast or mammalian cell cultivation are known in the art and include homogeneous suspension culture, e.g. in an airlift reactor or in a continuous stirrer reactor, or immobilised or entrapped cell culture, e.g. in hollow fibres, microcapsules, on agarose microbeads or ceramic cartridges.

Large quantities of the desired antibodies can also be obtained by multiplying mammalian cells *in vivo.* For this purpose, hybridoma cells producing the desired antibodies are injected into histocompatible mammals to cause growth of antibody-producing tumours. Optionally, the animals are primed with a hydrocarbon, especially mineral oils such as pristane (tetramethyl-pentadecane), prior to the injection. After one to three weeks, the antibodies are isolated from the body fluids of those mammals. For example, hybridoma cells obtained by fusion of suitable myeloma cells with antibody-producing spleen cells from Balb/c mice, or transfected cells derived from hybridoma cell line Sp2/0 that produce the desired antibodies are injected intraperitoneally into Balb/c mice optionally pre-treated with pristane, and, after one to two weeks, ascitic fluid is taken from the animals.

The foregoing, and other, techniques are discussed in, for example, Kohler and Milstein, (1975) Nature 256:495-497; US 4,376,110; Harlow and Lane, Antibodies: a Laboratory Manual, (1988) Cold Spring Harbor, incorporated herein by reference. Techniques for the preparation of recombinant antibody molecules are described in the above references and also in, for example, EP 0623679; EP 0368684 and EP 0436597, which are incorporated herein by reference.

The cell culture supernatants are screened for the desired antibodies, preferentially by immunofluorescent staining of PGCs or other pluripotent cells, such as ES or EG cells, by immunoblotting, by an enzyme immunoassay, e.g. a sandwich assay or a dot-assay, or a radioimmunoassay.

For isolation of the antibodies, the immunoglobulins in the culture supernatants or in the ascitic fluid may be concentrated, e.g. by precipitation with ammonium sulphate, dialysis against hygroscopic material such as polyethylene glycol, filtration through selective membranes, or the like. If necessary and/or desired, the antibodies are purified by the customary chromatography methods, for example gel filtration, ion-exchange chromatography, chromatography over DEAE-cellulose and/or (immuno-) affinity chromatography, e.g. affinity chromatography with the antigen, or fragments thereof, or with Protein-A.

Hybridoma cells secreting the monoclonal antibodies are also provided. Hybridoma cells may be genetically stable, secrete monoclonal antibodies of the desired specificity and can be activated from deep-frozen cultures by thawing and recloning.

Also included is a process for the preparation of a hybridoma cell line secreting monoclonal antibodies directed to the CACNA2D3, CAMK2D, KCNIP4, ANGPT1, NAALADL2, ZFHX3 or MPHOSPH10 polypeptide, characterised in that a suitable mammal, for example a Balb/c mouse, is immunised with a one or more CACNA2D3, CAMK2D, KCNIP4, ANGPT1, NAALADL2, ZFHX3 or MPHOSPH10 polypeptides, or antigenic fragments thereof; antibody-producing cells of the immunised mammal are fused with cells of a suitable myeloma cell line, the hybrid cells obtained in the fusion are cloned, and cell clones secreting the desired antibodies are selected. For example spleen cells of Balb/c mice immunised with CACNA2D3, CAMK2D, KCNIP4, ANGPT1, NAALADL2, ZFHX3 or MPHOSPH10 are fused with cells of the myeloma cell line PAI or the myeloma cell line Sp2/0-Ag14, the obtained hybrid cells are screened for secretion of the desired antibodies, and positive hybridoma cells are cloned.

We describe a process for the preparation of a hybridoma cell line, characterised in that Balb/c mice are immunised by injecting subcutaneously and/or intraperitoneally between 10 and 10⁷ and 10⁸ cells expressing CACNA2D3, CAMK2D, KCNIP4, ANGPT1, NAALADL2, ZFHX3 or MPHOSPH 10 and a suitable adjuvant several times, e.g. four to six times, over several months, e.g. between two and four months, and spleen cells from the immunised mice are taken two to four days after the last injection and fused with cells of the myeloma cell line PAI in the presence of a fusion promoter, such as polyethylene glycol. The myeloma cells may be fused with a three-to twentyfold excess of spleen cells from the immunised mice in a solution containing about 30 % to about 50 % polyethylene glycol of a molecular weight around 4000. After the fusion the cells are expanded in suitable culture media as described hereinbefore, supplemented with a selection medium, for example HAT medium, at regular intervals in order to prevent normal myeloma cells from overgrowing the desired hybridoma cells.

Recombinant DNAs comprising an insert coding for a heavy chain variable domain and/or for a light chain variable domain of antibodies directed to CACNA2D3, CAMK2D, KCNIP4, ANGPT1, NAALADL2, ZFHX3 or MPHOSPH10 as described hereinbefore are also disclosed. By definition such DNAs comprise coding single stranded DNAs, double stranded DNAs consisting of said coding DNAs and of complementary DNAs thereto, or these complementary (single stranded) DNAs themselves.

Furthermore, DNA encoding a heavy chain variable domain and/or for a light chain variable domain of antibodies directed to CACNA2D3, CAMK2D, KCNIP4, ANGPT1, NAALADL2, ZFHX3 or MPHOSPH10 can be enzymatically or chemically synthesised DNA having the authentic DNA sequence coding for a heavy chain variable domain and/or for the light chain variable domain, or a mutant thereof. A mutant of the authentic DNA is a DNA encoding a heavy chain variable domain and/or a light chain variable domain of the above-mentioned antibodies in which one or more amino acids are deleted or exchanged with one or more other amino acids. The modification(s) may be outside the CDRs of the heavy chain variable domain and/or of the light chain variable domain of the antibody. Such a mutant DNA is also intended to be a silent mutant wherein one or more nucleotides are replaced by other nucleotides with the new codons coding for the same amino acid(s). Such a mutant sequence is also a degenerated sequence. Degenerated sequences are degenerated within the meaning of the genetic code in that an unlimited number of nucleotides are replaced by other nucleotides without resulting in a change of the amino acid sequence originally encoded. Such degenerated sequences may be useful due to their different restriction sites and/or frequency of particular codons which are preferred by the specific host, particularly *E. coli,* to obtain an optimal expression of the heavy chain murine variable domain and/or a light chain murine variable domain.

The term mutant is intended to include a DNA mutant obtained by in vitro mutagenesis of the authentic DNA according to methods known in the art.

For the assembly of complete tetrameric immunoglobulin molecules and the expression of chimeric antibodies, the recombinant DNA inserts coding for heavy and light chain variable domains are fused with the corresponding DNAs coding for heavy and light chain constant domains, then transferred into appropriate host cells, for example after incorporation into hybrid vectors.

Also disclosed are recombinant DNAs comprising an insert coding for a heavy chain murine variable domain of an antibody directed to CACNA2D3, CAMK2D, KCNIP4, ANGPT1, NAALADL2, ZFHX3 or MPHOSPH 10 fused to a human constant domain g, for example γ1,γ2, γ3 or γ4, such as γ1 or γ4. Likewise recombinant DNAs comprising an insert coding for a light chain murine variable domain of an antibody directed to CACNA2D3, CAMK2D, KCNIP4, ANGPT1, NAALADL2, ZFHX3 or MPHOSPH10 fused to a human constant domain κ or λ, such as κ are also disclosed.

In another embodiment, we disclose recombinant DNAs coding for a recombinant polypeptide wherein the heavy chain variable domain and the light chain variable domain are linked by way of a spacer group, optionally comprising a signal sequence facilitating the processing of the antibody in the host cell and/or a DNA coding for a peptide facilitating the purification of the antibody and/or a cleavage site and/or a peptide spacer and/or an effector molecule.

The DNA coding for an effector molecule is intended to be a DNA coding for the effector molecules useful in diagnostic or therapeutic applications. Thus, effector molecules which are toxins or enzymes, especially enzymes capable of catalysing the activation of prodrugs, are particularly indicated. The DNA encoding such an effector molecule has the sequence of a naturally occurring enzyme or toxin encoding DNA, or a mutant thereof, and can be prepared by methods well known in the art.

### Use

Anti-CACNA2D3, CAMK2D, KCNIP4, ANGPT1, NAALADL2, ZFHX3 or MPHOSPH10 antibodies may be used in method of detecting a relevant polypeptide present in biological samples by a method which comprises: (a) providing an anti-CACNA2D3, CAMK2D, KCNIP4, ANGPT1, NAALADL2, ZFHX3 or MPHOSPH10 antibody; (b) incubating a biological sample with said antibody under conditions which allow for the formation of an antibody-antigen complex; and (c) determining whether antibody-antigen complex comprising said antibody is formed.

Suitable samples include extracts tissues such as brain, breast, ovary, lung, colon, pancreas, testes, liver, muscle and bone tissues or from neoplastic growths derived from such tissues. In particular, a sample may comprise a tissue from an individual suspected to be suffering from Kawasaki Disease.

Antibodies may be bound to a solid support and/or packaged into kits in a suitable container along with suitable reagents, controls, instructions and the like.

### Antibody Delivery

The antibodies against the CACNA2D3, CAMK2D, KCNIP4, ANGPT1, NAALADL2, ZFHX3 or MPHOSPH 10 protein may be delivered into a cell by means of techniques known in the art, for example by the use of liposomes, polymers, (e.g., polyethylene glycol (PEG), N-(2-hydroxypropyl) methacrylamide (HPMA) copolymers, polyamidoamine (PAMAM) dendrimers, HEMA, linear polyamidoamine polymers etc) etc. The immunoglobulins and/or antibodies may also be delivered into cells as protein fusions or conjugates with a protein capable of crossing the plasma membrane and/or the nuclear membrane. For example, the immunoglobulin and/or target may be fused or conjugated to a domain or sequence from such a protein responsible for the translocational activity. Translocation domains and sequences may include domains and sequences from the HIV-1-trans-activating protein (Tat), *Drosophila* Antennapedia homeodomain protein and the herpes simplex-1 virus VP22 protein.

### DIAGNOSTIC KITS

We also provide diagnostic kits for detecting Kawasaki Disease in an individual, or susceptibility to Kawasaki Disease in an individual.

The diagnostic kit may comprise means for detecting expression, amount or activity of CACNA2D3, CAMK2D, KCNIP4, ANGPT1, NAALADL2, ZFHX3 or MPHOSPH10 in the individual, by any means as described in this document. The diagnostic kit may therefore comprise any one or more of the following: a CACNA2D3, CAMK2D, KCNIP4, ANGPT1, NAALADL2, ZFHX3 or MPHOSPH10 polynucleotide or a fragment thereof; a complementary nucleotide sequence to CACNA2D3, CAMK2D, KCNIP4, ANGPT1, NAALADL2, ZFHX3 or MPHOSPH10 nucleic acid or a fragment thereof; a CACNA2D3, CAMK2D, KCNIP4, ANGPT1, NAALADL2, ZFHX3 or MPHOSPH10 polypeptide or a fragment thereof.

The diagnostic kit may comprise any one or more of the following: an anti-CACNA2D3 antibody, an anti-CAMK2D antibody, an anti-KCNIP4 antibody, an anti-ANGPT1 antibody, an anti-NAALADL2 antibody, an anti-ZFHX3 antibody and an anti-MPHOSPH10 antibody. The diagnostic kit may comprise e.g., an anti-peptide antibody human CACNA2D3, CAMK2D, KCNIP4, ANGPT1, NAALADL2, ZFHX3 or MPHOSPH10 antibody.

The diagnostic kit may comprise instructions for use, or other indicia. The diagnostic kit may further comprise means for treatment or prophylaxis of Kawasaki Disease, such as any of the compositions described in this document, or any means known in the art for treating Kawasaki Disease. In particular, the diagnostic kit may comprise an anti-CACNA2D3, CAMK2D, KCNIP4, ANGPT1, NAALADL2, ZFHX3 or MPHOSPH10 agent as described, for example obtained by screening. Yhe diagnostic kit may comprise Pregabalin as described, and/or a modulator of f CACNA2D3, CAMK2D, KCNIP4, ANGPT I, NAALADL2, ZFHX3 or MPHOSPH10.

The diagnostic kit may comprise a therapeutic drug such as salicylate, corticosteroid, Pregabalin or immunoglobulin (IVIV). The therapeutic drug may also comprise an anti-CACNA2D3, CAMK2D, KCNIP4, ANGPT1, NAALADL2, ZFHX3 or MPHOSPHIO antibody.

### PROPHYLACTIC AND THERAPEUTIC METHODS

CACNA2D3, CAMK2D, KCNIP4, ANGPT1, NAALADL2, ZFHX3 or MPHOSPH10 or a modulator thereof (including Pregabalin) may be used for treatment of disease in humans or other animals.

We disclose methods of treating Kawasaki Disease. Methods of preventing Kawasaki Disease (i.e., prophylaxis) also suitably employ the same or similar approaches.

Accordingly, we provide for the use of CACNA2D3, CAMK2D, KCNIP4, ANGPT1, NAALADL2, ZFHX3 or MPHOSPH10 and modulators thereof in the treatment or prevention of Kawasaki Disease. The CACNA2D3, CAMK2D, KCNIP4, ANGPT1, NAALADL2, ZFHX3 or MPHOSPH10 or a modulator thereof (including Pregabalin) may be used as drugs or therapies to treat Kawasaki Disease. They may be used to prevent such onset or progress of the disease.

In general terms, our methods involve manipulation of cells, by modulating (such as down-regulating) the expression, amount or activity of CACNA2D3, CAMK2D, KCNIP4, ANGPT1, NAALADL2, ZFHX3 or MPHOSPH10. The treatment may comprise generally contacting an Kawasaki Disease cell, or a cell suspected of being a Kawasaki Disease cell, with CACNA2D3, CAMK2D, KCNIP4, ANGPT1, NAALADL2, ZFHX3 or MPHOSPH10 or a modulator thereof (including Pregabalin). The methods may involve exposing a patient to an CACNA2D3, CAMK2D, KCNIP4, ANGPT1, NAALADL2, ZFHX3 or MPHOSPH10 or a modulator thereof (including Pregabalin) as described here.

It may or in addition be exposed to CACNA2D3, CAMK2D, KCNIP4, ANGPT1, NAALADL2, ZFHX3 or MPHOSPH 10 or a modulator thereof (including Pregabalin) or other molecule known to have effect in preventing or treating a Kawasaki Disease disease. Where this is so, the cell may be exposed to both the CACNA2D3, CAMK2D, KCNIP4, ANGPT1, NAALADL2, ZFHX3 or MPHOSPH 10 or a modulator thereof (including Pregabalin) and the agent together, or individually in sequence. The exposure may be repeated a number of times. Any combination of CACNA2D3, CAMK2D, KCNIP4, ANGPT1, NAALADL2, ZFHX3 or MPHOSPH10 or a modulator thereof (including Pregabalin) and an other agent in whatever amount or relative amount, in whatever timing of exposure, may be used.

We therefore provide for the use of combinations of CACNA2D3, CAMK2D, KCNIP4, ANGPT1, NAALADL2, ZFHX3 or MPHOSPH10 or a modulator thereof (including Pregabalin) and anti-Kawasaki Disease agents, as described above, in the treatment of Kawasaki Disease.

The cell may be an individual cell, or it may be in a cell mass. The cell may be inside the body of an organism. The organism may be one which is known to be suffering from Kawasaki Disease, or it could be one in which Kawasaki Disease is suspected, or it could be one which is susceptible to Kawasaki Disease. The treatment may comprise administering the CACNA2D3, CAMK2D, KCNIP4, ANGPT1, NAALADL2, ZFHX3 or MPHOSPH10 or a modulator thereof (including Pregabalin) to the organism. As above, a single polypeptide/nucleic acid/modulator may be administered, or a combination of any of CACNA2D3, CAMK2D, KCNIP4, ANGPT1, NAALADL2, ZFHX3 or MPHOSPH10 and a modulator thereof (including Pregabalin) may be administered. The administration may be simultaneous or sequential, as described above. Thus, the treatment may comprise administering an anti-CACNA2D simultaneously or sequentially with for example an anti-CAMK2D to the individual.

For this purpose, a number of criteria may be designated, which reflect the progress of treatment or prophylaxis or the well-being of the patient. Useful criteria in the case of Kawasaki Disease may include any of the symptoms set out above in the section "Kawasaki Disease" under "Symptoms".

Thus, as an example, a treated individual may show a decrease in such a symptom as measured by an appropriate assay or test. A treated individual may for example show a 1%, 2%, 3%, 4%, 5%, 6%, 7%, 8%, 9%, 10%, 15%, 20%, 25%, 30%, 40%, 50%, 60%, 70%, 80%, 90%, 100% or more decrease in one or more symptoms, compared to an individual who has not been treated.

For example, a patient disease may be defined as being "treated" if a condition associated with the disease is significantly inhibited (i.e., by 50% or more) relative to controls. The inhibition may be by at least 75% relative to controls, such as by 90%, by 95% or 100% relative to controls. By the term "treatment" we mean to also include prophylaxis or alleviation of Kawasaki Disease.

The antibody approach to therapy involving use of CACNA2D3, CAMK2D, KCNIP4, ANGPT1, NAALADL2, ZFHX3 or MPHOSPH10 or a modulator thereof (including Pregabalin) may be combined with other approaches for therapy of such disorders including conventional drug based approaches.

### PHARMACEUTICAL COMPOSITIONS

As disclosed herein, CACNA2D3, CAMK2D, KCNIP4, ANGPT1, NAALADL2, ZFHX3, MPHOSPH10 and modulators thereof may be used to treat or prevent Kawasaki Disease.

CACNA2D3, CAMK2D, KCNIP4, ANGPT1, NAALADL2, ZFHX3, MPHOSPH10 and modulators thereof can be administered in a variety of ways including enteral, parenteral and topical routes of administration. For example, suitable modes of administration include oral, subcutaneous, transdermal, transmucosal, iontophoretic, intravenous, intramuscular, intraperitoneal, intranasal, subdural, rectal, and the like.

In accordance with other embodiments, there is provided a composition comprising CACNA2D3, CAMK2D, KCNIP4, ANGPT1, NAALADL2, ZFHX3, MPHOSPH10 or a modulator thereof (including Pregabalin), together with a pharmaceutically acceptable carrier or excipient for the treatment or prevention of Kawasaki Disease.

Suitable pharmaceutically acceptable excipients include processing agents and drug delivery modifiers and enhancers, such as, for example, calcium phosphate, magnesium stearate, talc, monosaccharides, disaccharides, starch, gelatin, cellulose, methyl cellulose, sodium carboxymethyl cellulose, dextrose, hydroxypropyl-p-cyclodextrin, polyvinylpyrrolidinone, low melting waxes, ion exchange resins, and the like, as well as combinations of any two or more thereof. Other suitable pharmaceutically acceptable excipients are described in "Remington's Pharmaceutical Sciences," Mack Pub. Co., New Jersey (1991), incorporated herein by reference.

Pharmaceutical compositions containing CACNA2D3, CAMK2D, KCNIP4, ANGPT1, NAALADL2, ZFHX3, MPHOSPH10 or a modulator thereof (including Pregabalin) may be in any form suitable for the intended method of administration, including, for example, a solution, a suspension, or an emulsion. Liquid carriers are typically used in preparing solutions, suspensions, and emulsions. Liquid carriers contemplated for use in the practice include, for example, water, saline, pharmaceutically acceptable organic solvent (s), pharmaceutically acceptable oils or fats, and the like, as well as mixtures of two or more thereof. The liquid carrier may contain other suitable pharmaceutically acceptable additives such as solubilizers, emulsifiers, nutrients, buffers, preservatives, suspending agents, thickening agents, viscosity regulators, stabilizers, and the like. Suitable organic solvents include, for example, monohydric alcohols, such as ethanol, and polyhydric alcohols, such as glycols.

Suitable oils include, for example, soybean oil, coconut oil, olive oil, safflower oil, cottonseed oil, and the like. For parenteral administration, the carrier can also be an oily ester such as ethyl oleate, isopropyl myristate, and the like. Compositions may also be in the form of microparticles, microcapsules, liposomal encapsulates, and the like, as well as combinations of any two or more thereof.

The CACNA2D3, CAMK2D, KCNIP4, ANGPT1, NAALADL2, ZFHX3, MPHOSPH10 or modulator thereof (including Pregabalin) may be administered orally, parenterally, sublingually, by inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or ionophoresis devices. The term parenteral as used herein includes subcutaneous injections, intravenous, intramuscular, intrasternal injection, or infusion techniques.

Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-propanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono-or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols that are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as is normal practice, additional substances other than inert diluents, e. g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, cyclodextrins, and sweetening, flavoring, and perfuming agents.

In accordance with yet other embodiments, we provide methods for inhibiting or enhancing any activity of CACNA2D3, CAMK2D, KCNIP4, ANGPT1, NAALADL2, ZFHX3 or MPHOSPH10, in a human or animal subject, the method comprising administering to a subject an amount of a modulator of CACNA2D3, CAMK2D, KCNIP4, ANGPT1, NAALADL2, ZFHX3 or MPHOSPH10 (or composition comprising such compound) effective to inhibit the relevant activity in the subject. Other embodiments provide methods for treating Kawasaki Disease, in a human or animal subject, comprising administering to the cell or to the human or animal subject an amount of a compound or composition as described here effective to inhibit or enhance the activity, amount or expression of CACNA2D3, CAMK2D, KCNIP4, ANGPT1, NAALADL2, ZFHX3 or MPHOSPH10 in the cell or subject. The subject may be a human or non-human animal subject. Inhibition of protein activity includes detectable suppression of the relevant protein activity either as compared to a control or as compared to expected protein activity.

Effective amounts of the CACNA2D3, CAMK2D, KCNIP4, ANGPT1, NAALADL2, ZFHX3, MPHOSPH 10 or a modulator thereof (including Pregabalin) generally include any amount sufficient to detectably inhibit the relevant protein activity by any of the assays described herein, by other assays known to those having ordinary skill in the art or by detecting an alleviation of symptoms in a subject afflicted with Kawasaki Disease, such as any one or more of the symptoms described above in the section "Kawasaki Disease" under "Symptoms".

The amount of active ingredient that may be combined with the carrier materials to produce a single dosage form will vary depending upon the host treated and the particular mode of administration. It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy. The therapeutically effective amount for a given situation can be readily determined by routine experimentation and is within the skill and judgment of the ordinary clinician.

A therapeutically effective dose will generally be from about 10µg/kg/day to 100mg/kg/day, for example from about 25µg/kg/day to about 20 mg/kg/day or from about 50µg/kg/day to about 2mg/kg/day of an CACNA2D3, CAMK2D, KCNIP4, ANGPT1, NAALADL2, ZFHX3, MPHOSPH10 or a modulator thereof (including Pregabalin), which may be administered in one or multiple doses.

The CACNA2D3, CAMK2D, KCNIP4, ANGPT1, NAALADL2, ZFHX3, MPHOSPH10 or a modulator thereof (including Pregabalin) can also be administered in the form of liposomes. As is known in the art, liposomes are generally derived from phospholipids or other lipid substances. Liposomes are formed by mono-or multilamellar hydrated liquid crystals that are dispersed in an aqueous medium. Any non-toxic, physiologically acceptable and metabolizable lipid capable of forming liposomes can be used. The present compositions in liposome form can contain, in addition to a compound, stabilizers, preservatives, excipients, and the like. Lipids which may be used include the phospholipids and phosphatidyl cholines (lecithins), both natural and synthetic. Methods to form liposomes are known in the art. See, for example, Prescott, Ed., Methods in Cell Biology, Volume XIV, Academic Press, New York, N. W., p. 33 et seq (1976).

While the CACNA2D3, CAMK2D, KCNIP4, ANGPT1, NAALADL2, ZFHX3, MPHOSPH10 or a modulator thereof (including Pregabalin) can be administered as the sole active pharmaceutical agent, they can also be used in combination with one or more other agents used in the treatment of disorders. Representative agents useful in combination with CACNA2D3, CAMK2D, KCNIP4, ANGPT1, NAALADL2, ZFHX3, MPHOSPH10 or a modulator thereof (including Pregabalin) for the treatment of Kawasaki Disease include, for example, immunoglobulin (IVIV), salicylate, corticosteroid treatment, etc, as described above in the section "Kawasaki Disease" under "Treatment".

When additional active agents are used in combination with the CACNA2D3, CAMK2D, KCNIP4, ANGPT1, NAALADL2, ZFHX3, MPHOSPH10 or a modulator thereof (including Pregabalin), the additional active agents may generally be employed in therapeutic amounts as indicated in the PHYSICIANS'DESK REFERENCE (PDR) 53rd Edition (1999), which is incorporated herein by reference, or such therapeutically useful amounts as would be known to one of ordinary skill in the art.

The CACNA2D3, CAMK2D, KCNIP4, ANGPT1, NAALADL2, ZFHX3, MPHOSPH10 or a modulator thereof (including Pregabalin) and the other therapeutically active agents can be administered at the recommended maximum clinical dosage or at lower doses. Dosage levels of the active CACNA2D3, CAMK2D, KCNIP4, ANGPT1, NAALADL2, ZFHX3, MPHOSPH10 or a modulator thereof (including Pregabalin) in the compositions may be varied so as to obtain a desired therapeutic response depending on the route of administration, severity of the disease and the response of the patient. The combination can be administered as separate compositions or as a single dosage form containing both agents. When administered as a combination, the therapeutic agents can be formulated as separate compositions that are given at the same time or different times, or the therapeutic agents can be given as a single composition.

### Bioavailability

The compounds disclosed here (and combinations) are in some embodiments orally bioavailable. Oral bioavailablity refers to the proportion of an orally administered drug that reaches the systemic circulation. The factors that determine oral bioavailability of a drug are dissolution, membrane permeability and metabolic stability. Typically, a screening cascade of firstly in vitro and then in vivo techniques is used to determine oral bioavailablity.

Dissolution, the solubilisation of the drug by the aqueous contents of the gastro-intestinal tract (GIT), can be predicted from in vitro solubility experiments conducted at appropriate pH to mimic the GIT. The CACNA2D3, CAMK2D, KCNIP4, ANGPT1, NAALADL2, ZFHX3, MPHOSPH10 or a modulator thereof (including Pregabalin) may in some embodiments have a minimum solubility of 50 mg/ml. Solubility can be determined by standard procedures known in the art such as described in Adv. Drug Deliv. Rev. 23, 3-25, 1997.

Membrane permeability refers to the passage of the compound through the cells of the GIT. Lipophilicity is a key property in predicting this and is defined by in vitro Log D_{7.4} measurements using organic solvents and buffer. The CACNA2D3, CAMK2D, KCNIP4, ANGPT1, NAALADL2, ZFHX3, MPHOSPH10 or a modulator thereof (including Pregabalin) may have a Log D_{7.4} of -2 to +4 or -1 to +2. The log D can be determined by standard procedures known in the art such as described in J. Pharm. Pharmacol. 1990, 42:144.

Cell monolayer assays such as CaCO₂ add substantially to prediction of favourable membrane permeability in the presence of efflux transporters such as p-glycoprotein, so-called caco-2 flux. The CACNA2D3, CAMK2D, KCNIP4, ANGPT1, NAALADL2, ZFHX3, MPHOSPH 10 or a modulator thereof (including Pregabalin) may have a caco-2 flux of greater than 2x 10⁻⁶cms⁻¹, for example greater than 5x 10⁻⁶cms⁻¹. The caco flux value can be determined by standard procedures known in the art such as described in J. Pharm. Sci, 1990, 79, 595-600.

Metabolic stability addresses the ability of the GIT or the liver to metabolise compounds during the absorption process: the first pass effect. Assay systems such as microsomes, hepatocytes etc are predictive of metabolic liability. The compounds of the Examples may in some embodiments show metabolic stability in the assay system that is commensurate with an hepatic extraction of less than 0.5. Examples of assay systems and data manipulation are described in Curr. Opin. Drug Disc. Devel., 201, 4, 36-44, Drug Met. Disp.,2000, 28, 1518-1523.

Because of the interplay of the above processes further support that a drug will be orally bioavailable in humans can be gained by in vivo experiments in animals. Absolute bioavailability is determined in these studies by administering the compound separately or in mixtures by the oral route. For absolute determinations (% absorbed) the intravenous route is also employed. Examples of the assessment of oral bioavailability in animals can be found in Drug Met. Disp.,2001, 29, 82-87; J. Med Chem , 1997, 40, 827-829, Drug Met. Disp.,1999, 27, 221-226.

The term "pharmaceutically acceptable carrier" as used herein generally refers to organic or inorganic materials, which cannot react with active ingredients. The carriers include but are not limited to sugars, such as lactose, glucose and sucrose; starches such as corn starch and potato starch; cellulose and its derivatives such as sodium carboxymethycellulose, ethylcellulose and cellulose acetates; powdered tragancanth; malt; gelatin; talc; stearic acids; magnesium stearate; calcium sulfate; vegetable oils, such as peanut oil, cotton seed oil, sesame oil, olive oil, corn oil and oil of theobroma; polyols such as propylene glycol, glycerine, sorbitol, mannitol, and polyethylene glycol; agar; alginic acids; pyrogen-free water; isotonic saline; and phosphate buffer solution; skim milk powder; as well as other non-toxic compatible substances used in pharmaceutical formulations. Wetting agents and lubricants such as sodium lauryl sulfate, as well as coloring agents, flavoring agents, lubricants, excipients, tabletting agents, stabilizers, antioxidants and preservatives, can also be present.

The term "therapeutically effective amount" as used herein generally refers to an amount of an agent, for example the amount of a compound as an active ingredient, that is sufficient to effect treatment as defined herein when administered to a subject in need of such treatment. A therapeutically effective amount of a compound, salt, derivative, isomer or enantiomer of the present invention will depend upon a number of factors including, for example, the age and weight of the subject, the precise condition requiring treatment and its severity, the nature of the formulation, and the route of administration, and will ultimately be at the discretion of the attendant physician or veterinarian. However, an effective amount of a compound of the present invention for the treatment of disorders associated with bacterial or viral infection, in particular bacterial meningitis, will generally be in the range of about 10 to about 40 mg/kg body weight of recipient (mammal) per day and more usually about 40 mg/kg body weight per day. Thus, for a 70 kg adult subject, the actual amount per day would typically be about 2,800 mg, and this amount may be given in a single dose per day or more usually in a number (such as two, three, four, five or six) of sub-doses per day such that the total daily dose is the same. An effective amount of a salt of the present invention may be determined as a proportion of the effective amount of the compound per se.

The term "treatment" as used herein refers to any treatment of a condition or disease in an animal, particularly a mammal, more particularly a human, and includes: preventing the disease or condition from occurring in a subject which may be predisposed to the disease but has not yet been diagnosed as having it; inhibiting the disease or condition, i.e. arresting its development; relieving the disease or condition, i.e. causing regression of the condition; or relieving the conditions caused by the disease, i.e. symptoms of the disease.

### Chemical derivative

The term "derivative" or "derivatised" as used herein includes chemical modification of a compound. Illustrative of such chemical modifications would be replacement of hydrogen by a halo group, an alkyl group, an acyl group or an amino group.

### Chemical modification

In one embodiment, the compound may be a chemically modified compound.

The chemical modification of a compound may either enhance or reduce hydrogen bonding interaction, charge interaction, hydrophobic interaction, Van Der Waals interaction or dipole interaction between the compound and the target.

In one aspect, the identified compound may act as a model (for example, a template) for the development of other compounds.

### Individual

The compounds are delivered to individuals. As used herein, the term "individual" refers to vertebrates, particularly members of the mammalian species. The term includes but is not limited to domestic animals, sports animals, primates and humans.

### EXAMPLES

### Example 1. Materials and Methods: Study design

We used a staged study design, performing the initial genome-wide association analyses in a Dutch Caucasian case-control sample and re-testing the most significantly associated SNPs and haplotypes in an independent sample of Kawasaki Disease trios from Australia, the US and the UK, using a different genotyping technology.

### Example 2. Materials and Methods: Phenotypic Definition and Case Ascertainment

KD shares many features of other infectious diseases of young children, including fever, rash and changes to the mucous membranes. There is no diagnostic test and laboratory parameters individually have insufficient sensitivity or specificity for diagnosis.²

In all study cohorts we employed a conservative and widely accepted Kawasaki Disease case definition in an attempt to maximize phenotypic homogeneity and diagnostic specificity. Kawasaki Disease is defined by the presence of prolonged fever, together with at least four of the five classical diagnostic criteria. ¹³ Children with at least five days of fever and two diagnostic criteria with echocardiographic changes of coronary artery damage during the acute and/or convalescent phases of Kawasaki Disease are also included, as these coronary artery manifestations are likely pathognomonic for Kawasaki Disease. ¹³ Cases of incomplete Kawasaki Disease, for whom fever and less than 4 diagnostic criteria are present and which constitute approximately 15% of Kawasaki Disease cases receiving clinical treatment, ¹⁴ are excluded.

In all cohorts clinical and laboratory data are obtained directly from patient medical files and supplemented with parental questionnaires. Phenotypic data are reviewed in all cases by experienced pediatricians. Ethical approval is obtained from the appropriate national and regional institutional review boards for each study population (Academic Medical Center [AMC], Amsterdam (Dutch cohort), UK Multi-Centre Research Ethics Committee (UK cohort), each participating tertiary paediatric hospital's ethics committee (Australian cohort) and the University of California at San Diego, The Children's Memorial Hospital of Chicago, Northwestern University Feinberg School of Medicine, and Boston Children's Hospital (US cohort)). Informed consent and assent as appropriate are obtained from participating families.

### Example 3. Materials and Methods: Subjects

The initial GWAS is performed on 119 Dutch Caucasian Kawasaki Disease cases and 135 healthy controls. The cases are identified by collaborating pediatricians and sent for cardiological evaluation during the acute stage and subsequent follow-up to the AMC. The controls are unrelated adult Caucasian blood donors residing in the same geographical area. Ethnicity is determined by self or parental ethnic identification. Assessment for possible population stratification is performed with Eigenstrat. ¹⁵ A second independent cohort, which consisted of 583 Kawasaki Disease-affected families, including complete and incomplete trios, from Australia, the US and the UK, is used to verify the most significantly associated variants identified in the GWAS. Family Kawasaki Disease cases in each country are identified from pediatric hospital databases, through Kawasaki Disease parent support groups and through media releases. Biological parents (where available) and unaffected siblings (to reconstruct missing parental genotypes) are recruited.

### Example 4. Materials and Methods: DNA and RNA Collection and Extraction

Blood is collected into EDTA (Dutch, UK and US cohorts) and ADC (Australian cohorts, on whom Buffy coats are separated). Shed buccal cells are collected as previously described. ¹⁶ Genomic DNA is extracted by standard protocols. DNA quality is assessed by visual inspection after running 1.2% agarose gels and by calculating absorbance ratio OD_{260nm/280nm}. DNA quantification is measured using Picogreen^{™} dsDNA reagent. Degraded or low concentrated samples are excluded.

RNA samples from acute Kawasaki Disease cases are obtained prior to intravenous immunoglobulin treatment and again during convalescence (within one year n = 17, after one year n = 10) from 27 US children who fulfilled the Kawasaki Disease case definition. These Kawasaki Disease patients are also analyzed as part of the genotyping follow-up cohort. Whole blood is collected into the PaxGene^{™} tubes, according to manufacturer's instructions and RNA is extracted and stored at -80°C for batch analysis. RNA quantification is performed by optical density (260nM).

### Example 5. Materials and Methods: Genotyping in GWAS and Follow-up Studies

We genotyped the Dutch case-control samples using the Affymetrix^{™} 250K NSP chip in accordance with the manufacturer's instructions. Samples with call rates below 93% at p = 0.33 after running a BRLMM algorithm (Bayesian Robust Linear Model with Mahalanobis distance classifier, Affymetrix^{™}), are re-hybridized and are excluded from further analysis if they failed to achieve the established threshold.

Associated individual SNPs from the GWAS are ranked by nominal significance using a combination of allelic association, Armitage trend test and a recessive-dominant model. SNPs that deviated significantly from HWE in the control group after applying a Bonferroni correction, or failed genotyping QC (call rate < 93%, or monomorphic) are excluded from further analysis. The 1176 most significantly associated SNPs are selected for genotyping in the Australian, UK and US trios by a custom Illumina^{™} Oligo Pool Assay. For 28 SNPs genotyping assays could not be designed, leaving 1148 SNPs for the follow-up study. Families are excluded if no familial relationship is detected by RelPair ¹⁷, if there is sample duplication, if the proband genotype is unavailable or if they had more than 3 Mendelian errors. Analysis of the successfully genotyped SNPs is performed with Illumina^{™} BeadStudio software. In addition haplotypic analysis of the GWA data is performed, using a multi-marker sliding window. ¹⁸ Genotyping of all 166 SNPs identified by haplotypic analyses is performed by Sequenom iPLEX^{™} in the family-based follow-up cohort.

### Example 6. Materials and Methods: Identifying Associated Genes

For each disease associated polymorphism verified in the follow-up family study, we investigated whether the variant is within an annotated gene. When the SNP is in an inter-genic region, we analyzed the closest annotated genes situated within 50kb up- and downstream.

### Example 7. Materials and Methods: Gene Ontology Clustering

Associated genes are analyzed for gene ontology information using *Ingenuity Pathways Analysis* (IPA) software (Ingenuity^{™} Systems, www.ingenuity.com). To start building networks, IPA queries the Ingenuity Pathways Knowledge Base for interactions between identified 'Focus Genes', in our case genes detected in the GWAS, and all other gene objects stored in the knowledge base, to generate a set of networks with a maximum network size of 35 genes/proteins. Networks are displayed graphically as genes/gene products ('nodes') and the biological relationships between the nodes ('edges'). All edges are supported by at least one reference from the literature, or from canonical information stored in the Ingenuity Pathways Knowledge Base. In addition, IPA computes a score for each network according to the fit of the user's set of significant genes. The score, representing the -log (p-value), indicates the likelihood of the Focus Genes in a network from Ingenuity's knowledge base being found together due to random chance.

### Example 8. Materials and Methods: Gene Expression

Genes corresponding to associations or identified by IPA are investigated for differential expression (between acute and convalescent Kawasaki Disease) using Taqman^{™} low density array (TLDA; Applied Biosystems, Foster City, CA, USA) on 2µg total RNA as per manufacturer's instructions. Sample RNA concentrations are normalized using 18S RNA as an internal control. Data are analyzed by SDS RQ Manager software (Applied Biosystems). Gene expression data are imported into Genespring Software GXV 7.3 (Silicon Genetics, Redwood City, CA and analysed by an ANOVA t-test (assuming unequal variances) to identify differentially expressed genes.

### Example 9. Materials and Methods: Statistical Analyses

In the initial GWAS, HWE in the control group and allelic association analysis are calculated using *HelixTree*^{™} *v4.4.1* (GoldenHelix Inc., Bozeman, MT, United States) and *Exemplar*^{™} (Sapio Sciences, LLC, York, PA, United States). Allelic P-values are calculated by means of a 2x2 chi-square table and an Armitage trend test is used to derive genotypic p-values. Genotype association analysis and odds ratios are calculated using *Exemplar.* A two-sided Fisher's Exact test is used when counts in any cell fell below five. Allelic analysis of the X chromosome SNPs is performed with *Haploview v3.31* ¹⁹ and a likelihood ratio test is applied to calculate genotypic associations. Quantile-quantile (Q-Q) plots are constructed by ranking a set of values of -log p-value and plotting them against their expected values, given the assumption that the values had been sampled from a chi-squared distribution, with one degree of freedom. Deviations from the line of equality indicated either that the theoretical distribution is incorrect, or that the sample is contaminated with values generated in some other manner (for example, by a true association). The family-based association analysis is performed using FBAT, ²⁰ allowing transmission disequilibrium analysis in extended families. Combined p-values and odds ratios are calculated by Fisher's combined probability test which allows pooled information across several tests that share the same null hypothesis. ²¹

### Example 10. Materials and Methods: Haplotypic Analysis

Analysis of haplotypic associations is performed using a recently described method, the VSSWRR (Variable-Sized Sliding Window via Regularized Regression), a haplotype analysis method for population-based case-control association studies. ¹⁸ It uses a variable-size sliding window where the maximum window size is determined by local haplotype diversity and the sample size and a combined analysis of all the haplotypes of different lengths (up to the maximum window size) at the same starting position is performed using L₁-regularized regression method, adjusting for the dependency and complementariness among the haplotypes. It allows efficient management of a large number of haplotypes and is powerful in the detection of disease associations. ¹⁸

### Example 11. Results: Genome-wide Association Study

119 Kawasaki Disease cases and 135 controls were analyzed in the initial GWAS (Table 1).

| **Characteristics** | **Genome-Wide Association** | **Family-based Follow-up** |
|---|---|---|
| | **Cohort** | **Cohort** |
| Cases (n) | 119 | 583 |
| Controls (n) | 135 | 1357 |
| Males (%) | 145/254 (57.1) | 1031/1940 (53.1) |
| Caucasian ethnicity (%) | 241/254 (94.5) | - |
| Coronary artery damage (%) | 24/119 (20.1) | 155/583 (26.5) |
| Intravenous immunoglobulin received (%) | 96/102 (94.1)^{a} | 432/583 (74.1) |

| | | |
|---|---|---|
| ^{a} No information on 17 patients. **Table 1.** Demographic and Clinical Characteristics of Subjects in the GWAS (Dutch case-control samples) and Follow-up Study (Australian, US and UK families). | | |

Ten non-Caucasian individuals are excluded following admixture analysis by Eigenstrat. ¹⁵ Three samples are excluded due to genotyping call rates <93%. Thus the final GWA analysis consisted of 107 Kawasaki Disease patients and 134 controls. Of the 262,264 SNPs on the Affymetrix^{™} 250K NSP chip, 18,211 had a call rate of <93%, 18,981 are monomorphic (MAF <0.1%) and 1,150 deviated significantly from HWE in the control group; 223,922 SNPs are therefore available for analysis, of which 5,571 are on the X chromosome. A total of 14,065 SNPs are significantly associated (p <0.05), 1915 more SNPs than would be expected to be associated at this level of significance by chance alone. The quantile-quantile plot between observed and expected allele frequencies showed deviation from expected with p <∼10⁻⁴, suggesting the presence of true associations (Figure 1).

### Example 12. Results: Follow-up Study in Independent Cohorts

After verifying family relationships and checking sample duplications, 63 samples are excluded from further analysis. Thus, 1,903 members of 583 Kawasaki Disease families, including 498 trios, are tested in the follow-up association analysis.

### Example 13. Results: Individual SNP Associations

The 1,148 most significantly associated SNPs variants (corresponding to a minimum significance level of p <0.0024) are selected by a combination of Armitage trend test, recessive-dominant and allelic association analysis (Figure 2). SNPs are genotyped in our follow-up cohort by a custom Illumina^{™} Oligo Pool Assay, in which 1,116 SNPs are successfully genotyped (Table S1). Fifteen SNPs failed quality filters, leaving a total of 1,101 SNPs.

Forty SNPs are replicated and showed significant association with Kawasaki Disease susceptibility (Table 2). Twenty eight lay either in, or within 50 kb of known genes.

The most highly associated SNP is located in the gene for N-acetylated alpha-linked acidic dipeptidase-like 2 (*NAALADL2*), (combined OR from case-control and family analyses = 1.43 (1.32-1.53); p_{combined} =1.13x10⁻⁶).

Three SNPs are located in introns of the gene for AT-binding transcription factor 1 (*ZFHX3*), all of which had a protective effect (OR_{combined} = 0.64 (0.52-0.75), 0.68 (0.57-0.79), 0.73 (0.62-0.84); p_{combined} = 2.37x10⁻⁶, 7.06x10⁻⁵, 5.35x10⁻⁴, respectively). Two of these SNPs are only 647bp apart and in high linkage disequilibrium (LD) (r² = 0.93), whereas the third is ∼10kb distant and in less LD (r² = 0.46).

Two SNPs, which are 325bp apart and in complete LD (r² = 1), are situated ∼10kb 3' of the angiopoietin 1 gene (*ANGPT1*) (OR_{combined} = 0.58 (0.43-0.73) and 0.6 (0.45-0.76); p_{combined} 3.39x10⁻⁵ and 5.44x10⁻⁵).

**Table 2. Significantly Associated Variants Confirmed in the Follow-up Study.**

| | | | **Genome-Wide Association** | | **Family-based Follow-up study** | | | **Combined values** | |
|---|---|---|---|---|---|---|---|---|---|
| **Marker** | **Chr.** | **Genes^{a}** | **p value** | **OR (95%CI)** | **p value** | **OR (95%CI)^{b}** | **Info.fam^{c}** | **p value** | **OR (95%CI)** |
| rs3842967 | 1 | MARK1 | 2.69*10⁻³ | 0.55 (0.34-0.8) | 4.68*10⁻² | 0.8 (0.58-1.1) | 278 | 1.26*10⁻³ | 0.70 (0.58-0.83) |
| rs10183521 | 2 | - | 4.15*10⁻⁴ | 1.37 (0.94-1.99) | 1.79*10⁻² | 1.24 (0.97-1.58) | 366 | 9.50*10⁻⁵ | 1.28 (1.17-1.39) |
| rs6432366 | 2 | - | 1.05*10⁻³ | 1.81 (1.28-2.7) | 1.03*10⁻² | 1.28 (0.98-1.66) | 337 | 1.35*10⁻⁴ | 1.44 (1.33-1.55) |
| rs10519011 | 2 | CEP68 | 2.39*10⁻³ | 10.5(1.3-85.2) | 3.08*10⁻² | 2 (0.87-4.57) | 40 | 7.73*10⁻⁴ | 2.58 (2.16-3.01) |
| rs10153799 | 2 | - | 1.52*10⁻³ | 4 (1.6-9.9) | 4.23*10⁻² | 1.92 (0.83-4.45) | 40 | 6.84*10⁻⁴ | 3.09 (2.76-3.42) |
| rs9834548^{d} | 3 | - | 2.72*10⁻⁴ | 0.5 (0.34-0.73) | 2.35*10⁻³ | 0.73 (0.55-0.97) | 343 | 9.77*10⁻⁶ | 0.64 (0.53-0.75) |
| rs358056 | 3 | CACNA2D3 | 6.14*10⁻⁴ | 1.92 (1.22-3) | 2.82*10⁻² | 1.26 (0.94-1.69) | 281 | 2.07*10⁻⁴ | 1.43 (1.31-1.56) |
| rs16849640 | 3 | CLSTN2 | 4.48*10⁻⁴ | 0.26 (0.11-0.62) | 4.46*10⁻² | 0.8 (0.5-1.28) | 142 | 2.36*10⁻⁴ | 0.62 (0.41-0.82) |
| rs17531088 | 3 | NAALADL2 | 3.39*10⁻⁴ | 1.46 (1.01-2.1) | 1.64*10⁻⁴ | 1.41 (1.1-1.8) | 375 | 1.13*10⁻⁶ | 1.43 (1.32-1.53) |
| rs937130 | 3 | GABRR3 | 1.42*10⁻³ | 1.66 (1.16-2.39) | 3.81*10⁻² | 1.19 (0.93-1.53) | 373 | 5.87*10⁻⁴ | 1.33 (1.22-1.43) |
| rs17531554 | 4 | CAMK2D | 1.34*10⁻³ | 2.1(1.3-3.3) | 3.27*10⁻² | 1.29 (0.93-1.77) | 246 | 4.82*10⁻⁴ | 1.52 (1.38-1.65) |
| rs16869942 | 4 | KCNIP4 | 1.19*10⁻³ | 8 (1.77-36.34) | 8.15*10⁻³ | 2 (1.01-3.93) | 63 | 1.22*10⁻⁴ | 2.62 (2.28-2.96) |
| rs4864471 | 4 | LNX1,LOC441016 | 6.05*10⁻⁴ | 2.47 (1.3-4.5) | 4.01*10⁻³ | 1.5 (1.03-2.19) | 186 | 3.38*10⁻⁵ | 1.74 (1.57-1.91) |
| rs4333331 | 5 | PDZD2 | 8.59*10⁻⁴ | 0.82 (0.56-1.18) | 2.78*10⁻² | 0.81 (0.62-1.06) | 360 | 2.78*10⁻⁴ | 0.81 (0.71-0.92) |
| rs7739088 | 6 | C6orf70, C6orf122 | 1.51*10⁻³ | 0.49 (0.31-0.77) | 1.77*10⁻² | 0.77 (0.56-1.05) | 303 | 3.09*10⁻⁴ | 0.67 (0.54-0.80) |
| rs7755143 | 6 | PI16,MTCH1 | 1.07*10⁻³ | 2.25 (1.37-3.68) | 4.42*10⁻² | 1.27 (0.92-1.77) | 229 | 5.17*10⁻⁴ | 1.53 (1.38-1.67) |
| rs1408505 | 6 | PPP1R14C | 2.15*10⁻³ | 0.51 (0.33-0.79) | 4.29*10⁻² | 0.79 (0.57-1.09) | 263 | 9.46*10⁻⁴ | 0.68 (0.55-0.81) |
| rs2273828 | 6 | TCP1 | 6.35*10⁻⁴ | 0.6 (0.4-0.87) | 4.15*10⁻² | 0.82 (0.63-1.07) | 355 | 3.04*10⁻⁴ | 0.74 (0.63-0.85) |
| rs9392158 | 6 | RIOK1 | 7.89*10⁻⁴ | 2.4 (1.38-4.26) | 8.30*10⁻³ | 1.48 (0.99-2.19) | 172 | 8.47*10⁻⁵ | 1.75 (1.58-1.92) |
| rs17157642 | 7 | - | 1.30*10⁻³ | 2.37 (1.38-4.0) | 4.26*10⁻² | 1.28 (0.92-1.79) | 225 | 5.99*10⁻⁴ | 1.53 (1.38-1.68) |
| rs196751 | 7 | DGKB | 1.34*10⁻³ | 0.55 (0.38-0.89) | 4.99*10⁻² | 0.83 (0.64-1.08) | 362 | 7.12*10⁻⁴ | 0.73 (0.62-0.83) |
| rs17167055 | 7 | COL28A1 | 1.84*10⁻³ | 5.02 (1.64-15.37) | 2.33*10⁻² | 1.61 (0.92-2.79) | 88 | 4.73*10⁻⁴ | 2.06 (1.79-2.33) |
| rs10046555 | 7 | TNS3 | 1.78*10⁻³ | 0.52 (0.31-0.86) | 4.66*10⁻² | 0.78 (0.55-1.11) | 242 | 8.61*10⁻⁴ | 0.69 (0.54-0.83) |
| rs1010824 | 8 | ANGPT1 | 4.08*10⁻⁴ | 0.38 (0.22-0.66) | 5.96*10⁻³ | 0.69 (0.47-1) | 228 | 3.39*10⁻⁴ | 0.58 (0.43-0.73) |
| rs6469101 | 8 | ANGPT1 | 1.12*10⁻³ | 0.39 (0.21-0.73) | 3.62*10⁻³ | 0.69 (0.48-1) | 230 | 5.44*10⁻⁵ | 0.60 (0.45-0.76) |
| rs2912272 | 8 | CSMD1 | 2.52*10⁻³ | 2.54 (1.36-4.74) | 4.60*10⁻² | 1.37 (0.89-2.11) | 144 | 1.17*10⁻³ | 1.69 (1.51-1.88) |
| rs328879 | 9 | - | 1.10*10⁻³ | 0.53 (0.36-0.79) | 7.27*10⁻⁴ | 0.72 (0.55-0.95) | 351 | 1.20*10⁻⁵ | 0.65 (0.54-0.77) |
| rs10984642 | 9 | - | 4.27*10⁻⁵ | 0.27 (0.14-0.52) | 4.28*10⁻² | 0.75 (0.5-1.12) | 190 | 2.60*10⁻⁵ | 0.57 (0.4-0.74) |
| rs16915269 | 9 | - | 7.84*10⁻⁴ | 3.09 (1.55-6.14) | 5.51*10⁻³ | 1.56 (1.02-2.39) | 147 | 5.77*10⁻⁵ | 1.92 (1.72-2.11) |
| rs11063543 | 12 | - | 1.19*10⁻³ | 2.5 (1.42-4.44) | 4.19*10⁻² | 1.28 (0.92-1.79) | 231 | 5.43*10⁻⁴ | 1.53 (1.38-1.68) |
| rs10862477 | 12 | ZDHHC17 | 2.55*10⁻³ | 1.33 (0.91-1.95) | 4.97*10⁻² | 1.20 (0.93-1.54) | 354 | 1.27*10⁻³ | 1.24 (1.13-1.35) |
| rs285032 | 13 | FARP1 | 3.15*10⁻⁴ | 0.52 (0.36-0.75) | 3.71*10⁻³ | 0.77 (0.6-0.99) | 381 | 1.71*10⁻⁵ | 0.68 (0.58-0.79) |
| rs7151781 | 14 | - | 1.70*10⁻³ | 1.58 (1.09-2.29) | 1.09*10⁻² | 1.29 (1-1.68) | 332 | 2.20*10⁻⁴ | 1.38 (1.27-1.49) |
| rs2899383 | 15 | - | 2.00*10⁻³ | 0.71 (0.49-1.04) | 4.37*10⁻² | 0.83 (0.64-1.08) | 354 | 9.66*10⁻⁴ | 0.79 (0.68-0.90) |
| rs4775110 | 15 | RNF111 | 1.91*10⁻³ | 1.8 (1.2-2.71) | 1.14*10⁻² | 1.26 (0.98-1.63) | 353 | 2.56*10⁻⁴ | 1.40 (1.29-1.51) |
| rs7199343 | 16 | ZFHX3 | 8.73*10⁻⁵ | 0.73 (0.55-0.97) | 1.16*10⁻³ | 0.73 (0.55-0.97) | 332 | 2.37*10⁻⁶ | 0.64 (0.52-0.75) |
| rs10852516 | 16 | ZFHX3 | 2.33*10⁻³ | 0.55 (0.37-0.81) | 2.31*10⁻³ | 0.75 (0.57-0.98) | 354 | 7.06*10⁻⁵ | 0.68 (0.57-0.79) |
| rs11075953 | 16 | ZFHX3 | 2.27*10⁻³ | 0.59 (0.4-0.87) | 2.16*10⁻² | 0.8 (0.61-1.05) | 349 | 5.35*10⁻⁴ | 0.73 (0.62-0.84) |
| rs8059315 | 16 | GLG1 | 1.38*10⁻⁴ | 1.4 (0.87-2.26) | 4.74*10⁻² | 1.27 (0.91-1.75) | 241 | 8.45*10⁻⁵ | 1.31 (1.17-1.45) |
| rs7208206 | 17 | - | 2.15*10⁻³ | 0.23 (0.08-0.66) | 3.50*10⁻² | 0.58 (0.27-1.24) | 65 | 8.89*10⁻⁴ | 0.43 (0.13-0.73) |
| Haplotype analysis | | | | | | | | | |
| rs17348835, | 2 | - | 2.51*10⁻³ | 1.85 (1.25-2.73) | 2.22*10⁻² | 1.50 (1.05-2.13) | 206 | 5.99*10⁻⁴ | 1.64 (1.27-2.13) |
| rs17348849 | | | | | | | | | |
| rs7558220, rs357752 | 2 | MPHOSPH10 | 4.90*10⁻³ | 0.38 (0.19-0.75) | 7.10*10⁻³ | 0.40 (0.20-0.80) | 114 | 3.91*10⁻⁴ | 0.39 (0.24-0.63) |
| rs7558220, rs357752, rs1458868, rs357729, rs11674899 | 2 | MPHOSPH10 | 1.68*10⁻² | 3.61 (1.22-7.46) | 2.50*10⁻² | 3.42 (1.09-10.7) | 78 | 3.69*10⁻³ | 3.52 (1.60-7.75) |
| rs7572970, rs716615, rs12692592 | 2 | RBMS1 | 1.37*10⁻³ | 0.67 (0.53-0.86) | 3.60*10⁻³ | 0.71 (0.56-0.89) | 297 | 6.51*10⁻⁵ | 0.69 (0.58-0.82) |
| rs224666, rs 10767911, rs224618290 | 11 | - | 1.80*10⁻² | 0.43 (0.22-0.87) | 3.50*10⁻² | 0.45 (0.21-0.97) | 87 | 5.27*10⁻³ | 0.44 (0.26-0.74) |
| rs6034368, rs202479 | 20 | LOC441938 | 9.32*10⁻³ | 0.73 (0.57-0.92) | 2.00*10⁻² | 0.75 (0.59 -0.96) | 290 | 1.79*10⁻³ | 0.74 (0.62-0.88) |

| | | | | | | | | | |
|---|---|---|---|---|---|---|---|---|---|
| ^{a} Genes containing the SNP or the closest gene up to 50 kb up and downstream of the SNP. ^{b} Odds ratio for haplotype TDT was calculated based on haplotypes reconstructed from FBAT software viewhap command. ^{c} Number of informative families. ^{d} build35 rs 11711042. | | | | | | | | | |

### Example 14. Results: Haplotypic Associations

166 SNPs within the most highly associated thirty five haplotype blocks (corresponding to p < 2x10⁻³) are genotyped using a Sequenom iPLEX platform in the follow-up cohort (Table S2). Twenty-nine SNPs could not be genotyped, leaving 137 SNPs for further analysis. Significant associations are verified for six haplotypes, of which four are within known genes.

Two haplotypes lay within the gene encoding M-phase phosphoprotein 10 (U3 small nucleolar ribonucleoprotein), *MPHOSPH10*. One of these haplotypes is associated with increased susceptibility (OR_{combined} = 3.52 (1.6-7.75); p = 3.69x10⁻³) and the other with protection from Kawasaki Disease (OR_{combined} = 0.39 (0.24-0.63); p = 3.91x 10⁻⁴) (Table 2).

### Example 15. Results: Network Analysis

The individual SNP and haplotypic analysis together identified a total of 45 disease associations (40 SNPs and 6 haplotypes). We identified 31 genes in relation to these 45 associations (Table 2); 14 of the variants are within 10 genes, 17 associations had 21 genes within 50 kb, whereas 14 associations are not close to known annotated genes.

These 31 genes are loaded into the IPA *Knowledge Base.* While all genes are identified by this software, only 29 are mapped in the database and of these only 21 are known to have network connections by IPA.

We identified a single network of 35 genes with a highly significant score of 29 (p <10⁻²⁹), that included 12 of the 21 associated genes identified by the GWAS and incorporated 24 genes, later referred as network-implicated genes. Highlights from this network are shown in Figure 3 and Figure 4. This network is classified by IPA as related to development and function of the cardiovascular system.

The genes in Figure 3 include ANGPT1, TNF, PCZD2, TREM1, CACNA2D3, NFYB, HMGN3, AK2, MYC, SERBP1, TPP2, RBMS1, ZFHX3, CCND1, GLG1, Heparin, CAMK2D, NUMB, LNX2, LNX1, TGFBETA and RNF11.

The genes in Figure 4 include ANGPT 1, LNX2, NUMB, CAMK2D, FXYD1, IL1B, ELK1, IFNGR2, KLRG1, CCL2, IL2, CDKN1A, IL13, IL4, CASP1, CCL8, IL10, FCGR2A and Caspase.

### Example 16. Results: Gene Expression

We investigated 27 of the 31 genetically associated genes, and 15 of the 24 network-implicated genes for differential expression (acute *vs*. convalescence Kawasaki Disease) using 27 acute and convalescent paired whole blood samples from US children fulfilling the study case definition (Table 3).

Of the 54 RNA samples, two pairs are excluded because of inadequate RNA quality. Expression of these 42 genes is assessed by real-time reverse transcription PCR (Table 3). Four genes are not detected by the TLDA and may not be expressed in peripheral blood. Of the associated genes identified by the GWAS that are expressed in peripheral blood, 15 are significantly differentially expressed in Kawasaki Disease patients in the acute phase, with *NAALADL2* showing the largest fold change (FC) with -6.6 (p = 3.5x 10-4), while *CAMK2D* showed the most significant difference (p = 6.3x10⁻⁸, FC = -2.6) (Table 3).

Among the 15 tested genes identified by IPA, 10 are differentially expressed in acute versus convalescent Kawasaki Disease (Table 3).

**Table 3. Differential Gene Expression of Associated Genes and Those Identified by the Ingenuity Pathway Analysis in Acute and Convalescent Kawasaki Disease Samples from the Same Individuals.**

| **Gene Name** | **Accession ID** | **IPA^{a}** | **Expression** | **Differential Expression** | **Fold Change** |
|---|---|---|---|---|---|
| LOC441016 | XM_496693 | AG | No assay | - | - |
| LOC441938 | - | AG | No assay | - | - |
| C6orf122 | NM_207502 | AGR | Yes | NS^{b} | - |
| C6orf70 | NM_018341 | AGR | Not done | - | - |
| CEP68 | NM_015147 | AGR | Yes | 9.8*10⁻⁶ | -1.7 |
| COL28A1 | NM_001037763 | AGR | Yes | 4.3*10⁻³ | -3.4 |
| CSMD1 | NM_015336 | AGR | Yes | 3.4*10⁻² | -3.3 |
| FARP1 | NM_005766 | AGR | Yes | 7.3*10⁻³ | -3.2 |
| GABRR3 | NM_001105580 | AGR | Not done | - | - |
| PI16 | NM_153370 | AGR | Yes | 9.2*10⁻⁶ | -3.5 |
| CLSTN2 | NM_022131 | AGRN | No | - | - |
| DGKB | NM_145695 | AGRN | No | - | - |
| KCNIP4 | NM_025221 | AGRN | No | - | - |
| MPHOSPH10 | NM_005791 | AGRN | Yes | 2.9*10⁻⁴ | -1.8 |
| MTCH1 | NM_014341 | AGRN | Yes | NS | - |
| PPP1R14C | NM_030949 | AGRN | Yes | NS | - |
| RIOK1 | NM_153005 | AGRN | Yes | 1.4*10⁻³ | -2.1 |
| TNS3 | NM_022748 | AGRN | Yes | NS | - |
| ZDHHC17 | NM_033225 | AGRN | Yes | 1.2*10⁻³ | -1.4 |
| ANGPT1 | NM_001146 | AGRNI | Yes | 1.6*10⁻⁵ | -3.2 |
| CACNA2D3 | NM_018398 | AGRNI | Yes | 2.4*10⁻⁶ | -2.3 |
| CAMK2D | NM_001221 | AGRNI | Yes | 6.3*10⁻⁸ | -2.6 |
| GLG1 | NM_012201 | AGRNI | Yes | 7.9*10⁻³ | -1.5 |
| LNX1 | NM_032622 | AGRNI | Yes | NS | - |
| MARK1 | NM_018650 | AGRNI | Yes | 3.6*10⁻³ | -2.1 |
| NAALADL2 | NM_207015 | AGRNI | Yes | 3.5*10⁻⁴ | -6.6 |
| PDZD2 | NM_178140 | AGRNI | Yes | NS | - |
| RBMS1 | NM_016839 | AGRNI | Yes | 3.9*10⁻⁷ | 1.9 |
| RNF111 | NM_017610 | AGRNI | Yes | NS | - |
| TCP1 | NM_030752 | AGRNI | Yes | 1.7*10⁻² | -1.5 |
| ZFHX3 | NM_006885 | AGRNI | Yes | NS | - |
| AK2 | NM_013411 | I | Yes | 6.7*10⁻³ | -1.7 |
| Aminoacids | NA^{c} | I | - | - | - |
| CCND1 | NM_053056 | I | Yes | 2.3*10⁻² | -1.5 |
| CDC2L1 | NM_033486 | I | Not done | - | - |
| CHST4 | NM_005769 | I | Not done | - | - |
| heparin | NA | I | - | - | - |
| HMGN3 | NM_004242 | I | Yes | 2.6*10⁻² | -2.1 |
| ICK | NM_016513 | I | Not done | - | - |
| IL3 | NM_000588 | I | Yes | NS | - |
| ILKAP | NM_030768 | I | Not done | - | - |
| LNX2 | NM_153371 | I | Yes | 1.0*10⁻² | -1.8 |
| MYC | NM_002467 | I | Yes | 3.4*10⁻² | -2.0 |
| NFYB | NM_006166 | I | Yes | NS | - |
| NUMB | NM_001005743 | I | Yes | 1.5*10⁻² | -1.7 |
| PRL2C3 | NM_011118 | I | No assay | - | - |
| RPL41 | NM_001035267 | I | Yes | NS | - |
| RPS18 | NM_018342 | I | No asssay | - | - |
| SDF2 | NM_006923 | I | Not done | - | - |
| SERBP1 | NM_001018067 | I | Yes | 3.9*10⁻² | -1.3 |
| TGFB1 | NM_000660 | I | Yes | 5.3*10⁻³ | 1.3 |
| TGFB2 | NM_003238 | I | Yes | NS | - |
| TNF | NM_000594 | I | Yes | NS | - |
| TPP2 | NM_003291 | I | Yes | 3.0*10⁻⁵ | -1.7 |
| TREM1 | NM_018643 | I | Yes | 1.9*10⁻² | 1.6 |

| | | | | | |
|---|---|---|---|---|---|
| ^{a} AG-associated gene but not recognized in IPA; AGR-associated gene and recognized in IPA; AGRN-associated gene, recognized in IPA and network eligible; AGRNI- associated gene, recognized in IPA, network eligible and involved in forming significant network; I-gene inserted to form significant network.^{b} Not significant.^{c} Not available. | | | | | |

### Example 17. Discussion

To our knowledge, this is the first GWAS of any infectious disease and the largest genetic study of Kawasaki Disease to date. We have identified a number of novel SNPs and haplotypes that are associated with Kawasaki Disease susceptibility.

These include variants within or close to genes whose functions are plausible in Kawasaki Disease pathogenesis, as well as those that lie in apparent 'gene deserts' that lack annotated expressed genes. These findings are in keeping with recent large-scale GWAS in other complex diseases, such as type 2 diabetes, obesity and Crohn's disease, where markers within known expressed genes and also within genomic regions without annotated genes have been reliably identified. ^{22,23}

The magnitude of the effect sizes for Kawasaki Disease susceptibility is comparable to that reported from other GWAS, although our most significantly associated variants affect susceptibility by up to 3.5 fold, higher than the effect generally reported for most complex diseases. ²²

Fine-mapping of these associated SNPs, which is on-going, is likely to identify the disease-modifying variants, or those in LD with these variants, so the effect sizes are likely to increase for those regions that represent genuine associations. The assertion that the associated variants lie in or close to biologically relevant loci involved in Kawasaki Disease susceptibility is supported by; (i) a number of genes containing two or three independently associated variants in LD, (ii) the differential gene expression of 15 of the 23 blood-expressed associated genes during acute versus convalescent Kawasaki Disease, (iii) the network analyses that suggest plausible functional relationships between a number of the associated loci and a putative functional association of genes that is extremely unlikely to have occurred by chance.

Thirty one of the associated variants are in, or close to, expressed genes, many of which have functions that may be relevant to early events in Kawasaki Disease, particularly those linking an immunological or infectious insult with prolonged and abnormal inflammation. ²⁴ Reference is made to the text above for discussion of *ZFHX3, ANGPT1, NAALADL2* and *KCNIP4*.

In addition to individually associated genes, we investigated putative relationships between associated genes using the IPA, which identifies networks from a knowledge-base review of published literature. This analysis linked 12 of the genetically associated genes to 23 other nodes in a highly significant network described as 'cardiovascular system development and function'. The network indicates a number of interactions between inflammation and heart-specific molecules, with calcium signaling playing a central role.

Inflammation mediates the vascular damage in Kawasaki Disease. ² Mediators such as the pro-inflammatory cytokine tumor necrosis factor (TNF) and its negative regulator transforming growth factor-beta (TGFβ) show reciprocal expression in Kawasaki Disease and are suggested by animal models to be involved in the pathogenesis of coronary vasculitis. ³⁹⁻⁴² The gene *RNF11*, identified by the current study, encodes a known regulator of TGFβ that has a number of other cell-signaling functions. ⁴³ The putative network suggests that TNF and TGFβ may exert pathogenic effects via the transcription factors NFYB (nuclear transcription factor Y, beta) and MYC (v-myc myelocytomatosis viral oncogene homolog (avian)), which is also a regulator of VEGF and ANGPT1 and 2. ⁴⁴ Both NFYB and MYC are involved in calcium signaling, which is central to the function of many of the genes identified by the network.

Two genes associated with MYC and NFYB are particularly appealing candidates in Kawasaki Disease; (i) CACNA2D3 (calcium channel, voltage-dependent, alpha 2/delta 3 subunit), which is expressed in the heart and may bind plasma ligands such as cytokines and lead to calcium signaling ⁴⁵ and; (ii) CAMK2D (calcium/calmodulin-dependent protein kinase (CaM kinase) II delta), which is also expressed in the heart and implicated in calcium signaling. See the text above for discussion of CACNA2D3 and CAMK2D.

Further genomic and gene expression investigations are underway to explore these putative functional relationships.

Our sample of 702 cases represents the largest genetic Kawasaki Disease cohort and is drawn predominantly from a single ethnic group. We used a staged approach to avoid conservative correction for multiple statistical comparisons that might mask associations of moderate effect size in this modestly sized sample. We are aware that the genomic coverage and power of the GWAS are limited and calculate that the initial GWAS has only approximately 50% power to detect an OR of 2.0 at p <0.05. Our approach therefore aimed to reduce the risk of type I errors by ensuring that a large and independent replication cohort is included as part of the initial design, as we did not expect the associated variants to reach GW significance, given the cohort size in the GWAS. ⁵⁴ The initial GWAS identified 1915 more SNPs that are significantly associated at p <0.05 than would be expected by chance alone. The Q-Q analysis indicated that the observed associations deviated significantly from the expected results (in the absence of significant association) at approximately p <10⁻⁴. To minimize the chances of spurious associations arising from population admixture, we employed detailed stratification analysis in the case-control samples, excluding samples that are not definitely Caucasian. In the replication study, also predominantly Caucasian, we used family-based association analysis, which is less sensitive to the effects of population stratification ⁵⁵ and gives similar estimates of association. ⁵⁶ However power is reduced by the exclusion of non-informative parents. In addition family-based analysis can identify only allelic and not genotypic association and therefore recessive effects will be missed. We selected SNPs for the replication study by both genotypic and allelic methods, but genotypic associations would have to be verified in a case-control design and population controls are not available for our cohorts. It is therefore likely that although the associations verified by family-based methods may be more robust than if we had employed a case-control design, there are potentially biologically important associations that we cannot confirm by this study design.

We chose to attempt to replicate directly SNPs that are associated by the GWAS, rather than fine-mapping putatively associated loci with additional variants around those identified by the GWAS. It has been suggested that this is the most powerful and efficient strategy for replication, as including additional local variants in the follow-up stage increases the chances of false positive associations. ⁵⁷ We used a different genotyping technology for each stage of the study to minimize carry-over of false positive associations arising from genotyping errors.

Although it is impossible to calculate accurately, the genome coverage of our study is likely to be less than 50%. Our aim is to identify novel genetic determinants of Kawasaki Disease that are not obvious candidate loci. It is therefore not surprising that neither previously reported and credible candidate gene associations in Kawasaki Disease, such as *IL-4*, ⁵⁸ *VEGF,* ^{59,60} *CCR5,* ¹⁶ and *MBL,* ⁶¹ nor the recently reported *ITPKC* variant ⁴⁹ are replicated by the GWAS. Our study has almost certainly failed to identify these and other as yet unidentified variants that represent additional major determinants of Kawasaki Disease susceptibility. The epidemiological data in Kawasaki Disease suggest that the infectious trigger(s) are widely distributed and most individuals would encounter the causative pathogen(s) during early childhood. ³ Kawasaki Disease may result either from relatively few rare genetic variants that exert major effect, or from several more common variants whose combination is relatively rare. The markedly increased incidence in North Eastern Asian populations implies that some genetic determinants are either unique to these populations or their frequency is much higher than in Caucasians. The *ITPKC* variant ⁴⁹ is associated in both US Caucasians and Japanese, implying genetic homogeneity, at least within this haplotype block. Few other studies have investigated individuals from different ethnic groups.

In conclusion, this is the first GWAS of an infectious disease and the largest genetic study of Kawasaki Disease. Despite the relatively modest power and genomic coverage, we have identified a number of novel associated SNPs, many of which lie within or close to previously unrecognized candidates for Kawasaki Disease. The effect sizes, independent verification in different populations, differential gene expression and network analyses all indicate that at least a proportion of these variants represent novel genetic risk factors for Kawasaki Disease. Some of the associated genes may interact to mediate the deleterious effects of inflammation on the cardiovascular system. Further characterization of the associated genes and their functional interactions may lead to the identification of novel diagnostic and therapeutic targets in Kawasaki Disease and may be informative about early pathogenic processes in atherosclerosis.

### FURTHER ASPECTS

Further aspects and embodiments of the invention are now set out in the following numbered paragraphs; it is to be understood that the invention encompasses these aspects:
Paragraph 1. A method for the diagnosis, detection, treatment or prevention of Kawasaki Disease, the method comprising detecting or modulating a gene, polypeptide encoded by a gene, marker, polymorphism or haplotype shown in Table 2, Table 3, Table 4, Table S1, Table S2, Figure 3 or Figure 4 or a sequence having at least 90% sequence identity thereto.
Paragraph 2. A method according to Claim 1 for the diagnosis or detection of Kawasaki Disease, in which the method comprises detecting a polymorphism shown in Table 2.
Paragraph 3. A method according to Paragraph 1 or 2, in which the polymorphism is in or around a gene selected from NAALADL2, ANGPT1 and ZFHX3 or a sequence having at least 90% sequence identity thereto.
Paragraph 4. A method according to Paragraph 1, 2 or 3, in which the polymorphism comprises a single nucleotide polymorphism (SNP) selected from the group consisting of: rs17531088, rs1010824, rs6469101, rs7199343, rs10852516 and rs 11075953.
Paragraph 5. A method according to Paragraph 1 for the diagnosis or detection of Kawasaki Disease, in which the method comprises detecting a haplotype shown in Table 2 or a sequence having at least 90% sequence identity thereto.
Paragraph 6. A method according to Paragraph 6, in which the haplotype is in or around a gene selected from MPHOSPH10, RBMS1 and LOC441938 or a sequence having at least 90% sequence identity thereto.
Paragraph 7. A method according to Paragraph 6 or 7, in which the haplotype is selected from the group consisting of: rs7558220, rs357752; and rs7558220, rs357752, rs1458868, rs357729 and rs11674899.
Paragraph 8. A method according to Paragraph 1 for the diagnosis or detection of Kawasaki Disease, in which the method comprises detecting expression, such as a change in the expression pattern or level, of a gene shown in Table 3, or a polypeptide encoded by such a gene or a sequence having at least 90% sequence identity thereto.
Paragraph 9. A method according to Paragraph 8, in which the gene comprises NAALADL2 or CAMK2D.
Paragraph 10. A method according to any preceding Paragraph, comprising detecting a plurality of genes, polypeptides encoded by the genes, markers, polymorphisms or haplotypes.
Paragraph 11. A method according to any preceding Paragraph, comprising generating polymorphism profile, a haplotype profile or an expression profile of a plurality of genes.
Paragraph 12. A method according to any preceding Paragraph, in which 5 or more polymorphisms or haplotypes, such as all the polymorphisms or haplotypes in the relevant table, are detected
Paragraph 13. A method according to any preceding Paragraph, in which the expression level of 5 or more genes, such as all of the genes in the relevant table, is detected.
Paragraph 14. A method according to any preceding Paragraph, in which the gene, polypeptide encoded by a gene, marker, polymorphism or haplotype is detected, or the profile is derived, from microarray hybridisation such as hybridisation to an Affymetrix microarray, or by real time polymerase chain reaction (RT-PCR).
Paragraph 15. A method according to any preceding Paragraph for the diagnosis or detection of Kawasaki Disease, in which the method is a computer implemented method comprising processing expression or polymorphism data for one or more genes, polypeptides encoded by the genes, markers, polymorphisms or haplotypes set out in Table 2, Table 3, Table 4, Table S1, Table S2, Figure 3 or Figure 4 in a computer.
Paragraph 16. A method according to Paragraph 1 for the treatment or prevention of Kawasaki Disease in an individual, in which the method comprises modulating the expression of a gene shown in Table 3 or the amount or activity of a polypeptide encoded by such a gene or a sequence having at least 90% sequence identity thereto.
Paragraph 17. A method according to Paragraph 17, in which the gene comprises CAMK2D, CACNA2D3 or KCNIP4.
Paragraph 18. A method according to Paragraph 16 or 17, in which the method comprises administering an agonist or antagonist of the polypeptide.
Paragraph 19. A method according to Paragraph 17 or 18, which comprises administering a therapeutically effective amount of Pregabalin ((3S)-3-(aminomethyl)-5-methyl-hexanoic acid), a compound similar in structure to, a compound functionally equivalent to, a chemical derivative of, a chemical modification of, a substituted, a pharmaceutically acceptable salt of, a polymorphic form of, an isotopic variation of, a prodrug of, a pro-moiety of or a salt of Pregabalin, to an individual.
Paragraph 20. A method of identifying a molecule suitable for the treatment, prophylaxis or alleviation of Kawasaki Disease, the method comprising determining if a candidate molecule is an agonist or antagonist of a polypeptide shown in or encoded by a nucleic acid sequence shown in Table 2, Table 3, Table 4, Table S1, Table S2, Figure 3 or Figure 4 or a sequence having at least 90% sequence identity thereto.
Paragraph 21. Use of a polypeptide shown in or encoded by a nucleic acid sequence shown in Table 2, Table 3, Table 4, Table S1, Table S2, Figure 3 or Figure 4 or a sequence having at least 90% sequence identity thereto in a method of identifying a molecule suitable for the treatment, prophylaxis or alleviation of Kawasaki Disease.
Paragraph 22. A method according to Paragraph 20 or 21, in which the candidate molecule is exposed to a the polypeptide in order to determine if the candidate molecule is an agonist or antagonist thereof.
Paragraph 23. A method according to Paragraph 20, 21 or 22, in which the candidate molecule is exposed to a cell expressing the polypeptide.
Paragraph 24. Use of a nucleic acid sequence shown in Table 2, Table 3, Table 4, Table S1, Table S2, Figure 3 or Figure 4 or a sequence having at least 90% sequence identity thereto, for the identification of a molecule suitable for the treatment, prophylaxis or alleviation of Kawasaki Disease.
Paragraph 25. A method of identifying an agonist or antagonist of a polypeptide shown in or encoded by a nucleic acid sequence shown in Table 2, Table 3, Table 4, Table S1, Table S2, Figure 3 or Figure 4 or a sequence having at least 90% sequence identity thereto, the method comprising administering a candidate molecule to an animal suffering from Kawasaki Disease and determining whether the animal exhibits an alleviated symptom of Kawasaki Disease.
Paragraph 26. A method according to Paragraph 25, in which the animal is a rodent, such as a mouse.
Paragraph 27. A method according to any preceding Paragraph, in which the agonist or antagonist comprises an immunoglobulin, such as an antibody, or a small molecule for example capable of binding specifically to a polypeptide shown in or encoded by a nucleic acid sequence shown in Table 2, Table 3, Table 4, Table S1, Table S2, Figure 3 or Figure 4 or a sequence having at least 90% sequence identity thereto.
Paragraph 28. An agonist or antagonist of a polypeptide identified by a method or use according to any preceding Paragraph, the polypeptide being shown in or encoded by a nucleic acid sequence shown in Table 2, Table 3, Table 4, Table S1, Table S2, Figure 3 or Figure 4 or a sequence having at least 90% sequence identity thereto.
Paragraph 29. A molecule according to Paragraph 28 for use in the treatment, prophylaxis or alleviation of Kawasaki Disease.
Paragraph 30. Use of a molecule according to Paragraph 28 for the preparation of a pharmaceutical composition for the treatment of Kawasaki Disease in an individual.
Paragraph 31. A diagnostic kit for Kawasaki Disease or susceptibility thereto comprising any one or more of the following: a molecule capable of binding to a polypeptide shown in Table 2, Table 3, Table 4, Table S1, Table S2, Figure 3 or Figure 4 or a sequence having at least 90% sequence identity thereto, such as an antibody; a nucleic acid capable of encoding such; a nucleic acid capable of binding to a nucleic acid sequence shown in Table 2, Table 3, Table 4, Table S1, Table S2, Figure 3 or Figure 4 or a sequence having at least 90% sequence identity thereto, such as a nucleic acid capable of discriminating between polymorphisms for example by being capable of binding to one polymorphism at a particular location, but not capable of binding to another polymorphism at that location.
Paragraph 32. A combination comprising a plurality of markers, polymorphisms or haplotypes set out in Table 2, Table 3, Table 4, Table S1, Table S2, Figure 3 or Figure 4.
Paragraph 33. A combination according to Paragraph 32 in the form of an array, such as a microarray.
Paragraph 34. A data carrier comprising information set out in Table 2, Table 3, Table 4, Table S1, Table S2, Figure 3 or Figure 4.
Paragraph 35. Use of Pregabalin ((3S)-3-(aminomethyl)-5-methyl-hexanoic acid) for the treatment or prevention of Kawasaki Disease in an individual.
Paragraph 37. A pharmaceutical composition for the treatment or prevention of Kawasaki Disease in an individual, the pharmaceutical composition comprising Pregabalin ((3S)-3-(aminomethyl)-5-methyl-hexanoic acid) together with a pharmaceutically acceptable excipient, carrier or diluent.

### REFERENCES

1. Cheung YF, Wong SJ, Ho MH (2007) Relationship between carotid intima-media thickness and arterial stiffness in children after Kawasaki disease. Arch Dis Child 92:43-47
2. Bums JC, Glode MP (2004) Kawasaki syndrome. Lancet 364:533-544
3. Burgner D, Harnden A (2005) Kawasaki disease: what is the epidemiology telling us about the etiology? Int J Infect Dis 9:185-194
4. Park YW, Han JW, Park IS, Kim CH, Yun YS, Cha SH, Ma JS, Lee SB, Kim CH, Lee HJ, et al. (2005) Epidemiologic picture of Kawasaki disease in Korea, 2000-2002. Pediatr Int 47:382-387
5. Holman RC, Curns AT, Belay ED, Steiner CA, Effler PV, Yorita KL, Miyamura J, Forbes S, Schonberger LB, Melish M (2005) Kawasaki syndrome in Hawaii. Pediatr Infect Dis J 24:429-433
6. Gardner-Medwin JM, Dolezalova P, Cummins C, Southwood TR (2002) Incidence of Henoch-Schonlein purpura, Kawasaki disease, and rare vasculitides in children of different ethnic origins. Lancet 360:1197-1202.
7. Bronstein DE, Dille AN, Austin JP, Williams CM, Palinkas LA, Bums JC (2000) Relationship of climate, ethnicity and socioeconomic status to Kawasaki disease in San Diego County, 1994 through 1998. Pediatr Infect Dis J 19:1087-1091.
8. Dergun M, Kao A, Hauger SB, Newburger JW, Bums JC (2005) Familial occurrence of Kawasaki syndrome in North America. Arch Pediatr Adolesc Med 159:876-881
9. Fujita Y, Nakamura Y, Sakata K, Hara N, Kobayashi M, Nagai M, Yanagawa H, Kawasaki T (1989) Kawasaki disease in families. Pediatrics 84:666-669
10. Uehara R, Yashiro M, Nakamura Y, Yanagawa H (2003) Kawasaki disease in parents and children. Acta Paediatr 92:694-697
11. Onouchi Y, Tamari M, Takahashi A, Tsunoda T, Yashiro M, Nakamura Y, Yanagawa H, Wakui K, Fukushima Y, Kawasaki T, et al. (2007) A genomewide linkage analysis of Kawasaki disease: evidence for linkage to chromosome 12. J Hum Genet 52:179-190
12. Onouchi Y, Gunji T, Bums JC, Shimizu C, Newburger JW, Yashiro M, Nakamura Y, Yanagawa H, Wakui K, Fukushima Y, et al. (2008) ITPKC functional polymorphism associated with Kawasaki disease susceptibility and formation of coronary artery aneurysms. Nat Genet 40:35-42
13. Newburger JW, Takahashi M, Gerber MA, Gewitz MH, Tani LY, Bums JC, Shulman ST, Bolger AF, Ferrieri P, Baltimore RS, et al. (2004) Diagnosis, treatment, and long-term management of Kawasaki disease: a statement for health professionals from the Committee on Rheumatic Fever, Endocarditis, and Kawasaki Disease, Council on Cardiovascular Disease in the Young, American Heart Association. Pediatrics 114:1708-1733
14. Rowley AH (2002) Incomplete (atypical) Kawasaki disease. Pediatr Infect Dis J 21:563-565
15. Price AL, Patterson NJ, Plenge RM, Weinblatt ME, Shadick NA, Reich D (2006) Principal components analysis corrects for stratification in genome-wide association studies. Nat Genet 38:904-909
16. Bums JC, Shimizu C, Gonzalez E, Kulkarni H, Patel S, Shike H, Sundel RS, Newburger JW, Ahuja SK (2005) Genetic variations in the receptor-ligand pair CCR5 and CCL3L1 are important determinants of susceptibility to Kawasaki disease. J Infect Dis 192:344-349
17. Zhu J, Shearer GM, Norman JE, Pinto LA, Marincola FM, Prasad A, Waclawiw MA, Csako G, Quyyumi AA, Epstein SE (2000) Host response to cytomegalovirus infection as a determinant of susceptibility to coronary artery disease: sex-based differences in inflammation and type of immune response. Circulation 102:2491-2496.
18. Li Y, Sung WK, Liu JJ (2007) Association mapping via regularized regression analysis of single-nucleotide-polymorphism haplotypes in variable-sized sliding windows. Am J Hum Genet 80:705-715
19. Barrett JC, Fry B, Maller J, Daly MJ (2005) Haploview: analysis and visualization of LD and haplotype maps. Bioinformatics 21:263-265
20. Laird NM, Horvath S, Xu X (2000) Implementing a unified approach to family-based tests of association. Genet Epidemiol 19 Suppl 1:S36-42
21. Fisher RA (1932) Statistical methods for research workers. Oliver & Boyd, Edinburgh
22. The Wellcome Trust Case Control Consortium (2007) Genome-wide association study of 14,000 cases of seven common diseases and 3,000 shared controls. Nature 447:661-678
23. Libioulle C, Louis E, Hansoul S, Sandor C, Farnir F, Franchimont D, Vermeire S, Dewit O, de Vos M, Dixon A, et al. (2007) Novel Crohn disease locus identified by genome-wide association maps to a gene desert on 5p13.1 and modulates expression of PTGER4. PLoS Genet 3:e58
24. Cheung YF, O K, Tam SC, Siow YL (2005) Induction of MCP1, CCR2, and iNOS expression in THP-1 macrophages by serum of children late after Kawasaki disease. Pediatr Res 58:1306-1310
25. Morinaga T, Yasuda H, Hashimoto T, Higashio K, Tamaoki T (1991) A human alpha-fetoprotein enhancer-binding protein, ATBF1, contains four homeodomains and seventeen zinc fingers. Mol Cell Biol 11:6041-6049
26. Nojiri S, Joh T, Miura Y, Sakata N, Nomura T, Nakao H, Sobue S, Ohara H, Asai K, Ito M (2004) ATBF1 enhances the suppression of STAT3 signaling by interaction with PIAS3. Biochem Biophys Res Commun 314:97-103
27. Ueno Y, Takano N, Kanegane H, Yokoi T, Yachie A, Miyawaki T, Taniguchi N (1989) The acute phase nature of interleukin 6: studies in Kawasaki disease and other febrile illnesses. Clin Exp Immunol 76:337-342
28. Brindle NP, Saharinen P, Alitalo K (2006) Signaling and functions of angiopoietin-1 in vascular protection. Circ Res 98:1014-1023
29. Hunsaker DM, Hunsaker JC, Adams KC, Noonan JA, Ackermann DM (2003) Fatal Kawasaki disease due to coronary aneurysm rupture with massive cardiac tamponade. J Ky Med Assoc 101:233-238
30. Terai M, Honda T, Yasukawa K, Higashi K, Hamada H, Kohno Y (2003) Prognostic impact of vascular leakage in acute Kawasaki disease. Circulation 108:325-330
31. DeBusk LM, Hallahan DE, Lin PC (2004) Akt is a major angiogenic mediator downstream of the Ang1/Tie2 signaling pathway. Exp Cell Res 298:167-177
32. Higashi K, Terai M, Hamada H, Honda T, Kanazawa M, Kohno Y (2007) Impairment of angiogenic activity in the serum from patients with coronary aneurysms due to Kawasaki disease. Circ J 71:1052-1059
33. Freeman AF, Crawford SE, Cornwall ML, Garcia FL, Shulman ST, Rowley AH (2005) Angiogenesis in fatal acute Kawasaki disease coronary artery and myocardium. Pediatr Cardiol 26:578-584
34. Tonkin ET, Smith M, Eichhorn P, Jones S, Imamwerdi B, Lindsay S, Jackson M, Wang TJ, Ireland M, Burn J, et al. (2004) A giant novel gene undergoing extensive alternative splicing is severed by a Cornelia de Lange-associated translocation breakpoint at 3q26.3. Hum Genet 115:139-148
35. Pruunsild P, Timmusk T (2005) Structure, alternative splicing, and expression of the human and mouse KCNIP gene family. Genomics 86:581-593
36. Lin YL, Lin SR, Wu TT, Chang LS (2004) Evidence showing an intermolecular interaction between KChIP proteins and Taiwan cobra cardiotoxins. Biochem Biophys Res Commun 319:720-724
37. Kawada H, Niwano S, Niwano H, Yumoto Y, Wakisaka Y, Yuge M, Kawahara K, Izumi T (2006) Tumor necrosis factor-alpha downregulates the voltage gated outward K+ current in cultured neonatal rat cardiomyocytes: a possible cause of electrical remodeling in diseased hearts. Circ J 70:605-609
38. Haney I, Beghetti M, McCrindle BW, Gow RM (1995) Ventricular arrhythmia complicating Kawasaki disease. Can J Cardiol 11:931-933
39. Ahn HJ, Maruo S, Tomura M, Mu J, Hamaoka T, Nakanishi K, Clark S, Kurimoto M, Okamura H, Fujiwara H (1997) A mechanism underlying synergy between IL-12 and IFN-gamma-inducing factor in enhanced production of IFN-gamma. J Immunol 159:2125-2131
40. Matsubara T, Umezawa Y, Tsuru S, Motohashi T, Yabuta K, Furukawa S (1997) Decrease in the concentrations of transforming growth factor-beta 1 in the sera of patients with Kawasaki disease. Scand J Rheumatol 26:314-317
41. Kimura J, Takada H, Nomura A, Ohno T, Mizuno Y, Saito M, Kusuhara K, Hara T (2004) Th1 and Th2 cytokine production is suppressed at the level of transcriptional regulation in Kawasaki disease. Clin Exp Immunol 137:444-449
42. Hui-Yuen JS, Duong TT, Yeung RS (2006) TNF-alpha is necessary for induction of coronary artery inflammation and aneurysm formation in an animal model of Kawasaki disease. J Immunol 176:6294-6301
43. Azmi P, Seth A (2005) RNF11 is a multifunctional modulator of growth factor receptor signalling and transcriptional regulation. Eur J Cancer 41:2549-2560
44. Baudino TA, McKay C, Pendeville-Samain H, Nilsson JA, Maclean KH, White EL, Davis AC, Ihle JN, Cleveland JL (2002) c-Myc is essential for vasculogenesis and angiogenesis during development and tumor progression. Genes Dev 16:2530-2543
45. Qin N, Yagel S, Momplaisir ML, Codd EE, D'Andrea MR (2002) Molecular cloning and characterization of the human voltage-gated calcium channel alpha(2)delta-4 subunit. Mol Pharmacol 62:485-496
46. Little GH, Bai Y, Williams T, Poizat C (2007) Nuclear calcium/calmodulin-dependent protein kinase IIdelta preferentially transmits signals to histone deacetylase 4 in cardiac cells. J Biol Chem 282:7219-7231
47. Mishra S, Mishra JP, Gee K, McManus DC, LaCasse EC, Kumar A (2005) Distinct role of calmodulin and calmodulin-dependent protein kinase-II in lipopolysaccharide and tumor necrosis factor-alpha-mediated suppression of apoptosis and antiapoptotic c-IAP2 gene expression in human monocytic cells. J Biol Chem 280:37536-37546
48. Tsujimoto H, Takeshita S, Nakatani K, Kawamura Y, Tokutomi T, Sekine I (2001) Delayed apoptosis of circulating neutrophils in Kawasaki disease. Clin Exp Immunol 126:355-364
49. Onouchi Y, Gunji T, Bums JC, Shimizu C, Newburger JW, Yashiro M, Nakamura Y, Yanagawa H, Wakui K, Fukushima Y, et al. (2007) ITPKC functional polymorphism associated with Kawasaki disease susceptibility and formation of coronary artery aneurysms. Nat Genet
50. Mirza M, Hreinsson J, Strand ML, Hovatta O, Soder O, Philipson L, Pettersson RF, Sollerbrant K (2006) Coxsackievirus and adenovirus receptor (CAR) is expressed in male germ cells and forms a complex with the differentiation factor JAM-C in mouse testis. Exp Cell Res 312:817-830
51. Yanagawa B, Spiller OB, Proctor DG, Choy J, Luo H, Zhang HM, Suarez A, Yang D, McManus BM (2004) Soluble recombinant coxsackievirus and adenovirus receptor abrogates coxsackievirus b3-mediated pancreatitis and myocarditis in mice. J Infect Dis 189:1431-1439
52. Andreoletti L, Venteo L, Douche-Aourik F, Canas F, Lorin de la Grandmaison G, Jacques J, Moret H, Jovenin N, Mosnier JF, Matta M, et al. (2007) Active Coxsackieviral B infection is associated with disruption of dystrophin in endomyocardial tissue of patients who died suddenly of acute myocardial infarction. J Am Coll Cardiol 50:2207-2214
53. Armbruester V, Sauter M, Roemer K, Best B, Hahn S, Nty A, Schmid A, Philipp S, Mueller A, Mueller-Lantzsch N (2004) Np9 protein of human endogenous retrovirus K interacts with ligand of numb protein X. J Virol 78:10310-10319
54. Forner K, Lamarine M, Guedj M, Dauvillier J, Wojcik J (2007) Universal False Discovery Rate Estimation Methodology for Genome-Wide Association Studies. Hum Hered 65:183-194
55. Schulze TG, McMahon FJ (2002) Genetic association mapping at the crossroads: which test and why? Overview and practical guidelines. Am J Med Genet 114:1-11
56. Ackerman H, Usen S, Jallow M, Sisay-JoofF, Pinder M, Kwiatkowski DP (2005) A comparison of case-control and family-based association methods: the example of sickle-cell and malaria. Ann Hum Genet 69:559-565
57. Clarke GM, Carter KW, Palmer LJ, Morris AP, Cardon LR (2007) Fine mapping versus replication in whole-genome association studies. Am J Hum Genet 81:995-1005
58. Bums JC, Shimizu C, Shike H, Newburger JW, Sundel RP, Baker AL, Matsubara T, Ishikawa Y, Brophy VA, Cheng S, et al. (2005) Family-based association analysis implicates IL-4 in susceptibility to Kawasaki disease. Genes Immun 6:438-444
59. Kariyazono H, Ohno T, Khajoee V, Ihara K, Kusuhara K, Kinukawa N, Mizuno Y, Hara T (2004) Association of Vascular Endothelial Growth Factor (VEGF) and VEGF Receptor Gene Polymorphisms with Coronary Artery Lesions of Kawasaki Disease. Pediatr Res
60. Breunis WB, Biezeveld MH, Geissler J, Ottenkamp J, Kuipers IM, Lam J, Hutchinson A, Welch R, Chanock SJ, Kuijpers TW (2006) Vascular endothelial growth factor gene haplotypes in Kawasaki disease. Arthritis Rheum 54:1588-1594
61. Biezeveld MH, Kuipers IM, Geissler J, Lam J, Ottenkamp JJ, Hack CE, Kuijpers TW (2003) Association of mannose-binding lectin genotype with cardiovascular abnormalities in Kawasaki disease. Lancet 361:1268-1270.

The following numbered references [1] to [17] relate to the section headed "Kawasaki Disease" in the Detailed Description
[1]. Merck Manual, Online edition, Kawasaki Disease]
[2]. Kawasaki T (1967). "[Acute febrile mucocutaneous syndrome with lymphoid involvement with specific desquamation of the fingers and toes in children]" (in Japanese). Arerugi 16 (3): 178-222. PMID 6062087.
[3]. a b "Kawasaki Disease - Signs and Symptoms".
[4]. a b c d e "Who Kawasaki Disease Affects". Children's Hospital Boston. Retrieved on 2009-01-04.
[5]. Rowley AH, Baker SC, Orenstein JM, Shulman ST (May 2008). "Searching for the cause of Kawasaki disease--cytoplasmic inclusion bodies provide new insight". Nat. Rev. Microbiol. 6 (5): 394-401. doi:10.1038/nrmicro1853. PMID 18364728.
[6]. "Kawasaki Disease". American Heart Association. Retrieved on 3 January 2009.
[7]. "Kawasaki Disease: Causes". Mayo Clinic. Retrieved on 3 January 2009.
[8]. Nakamura Y, Yashiro M, Uehara R, Oki I, Watanabe M, Yanagawa H (2008). "Monthly observation of the number of patients with Kawasaki disease and its incidence rates in Japan: chronological and geographical observation from nationwide surveys" ([dead link] - Scholar search). J Epidemiol 18 (6): 273-9. doi:10.2188/jea.JE2008030. PMID 19075496.
[9]. Freeman AF, Shulman ST (June 2001). "Recent developments in Kawasaki disease". Curr Opin Infect Dis 14 (3): 357-61. PMID 11964855.
[10]. Onouchi Y, Gunji T, Bums JC, et al (January 2008). "ITPKC functional polymorphism associated with Kawasaki disease susceptibility and formation of coronary artery aneurysms". Nat. Genet. 40 (1): 35-42. doi:10.1038/ng.2007.59. PMID 18084290.
[11]. Keren G, Danon YL, Orgad S, Kalt R, Gazit E (August 1982). "HLA Bw51 is increased in mucocutaneous lymph node syndrome in Israeli patients". Tissue Antigens 20 (2): 144-6. PMID 6958087.
[12]. Park AH, Batchra N, Rowley A, Hotaling A (May 1997). "Patterns of Kawasaki syndrome presentation". Int. J. Pediatr. Otorhinolaryngol. 40 (1): 41-50. doi:10.1016/S0165-5876(97)01494-8. PMID 9184977.
[13]. "Kawasaki Disease - June 1999 - American Academy of Family Physicians".
[14]. Oates-Whitehead RM, Baumer JH, Haines L, et al (2003). "Intravenous immunoglobulin for the treatment of Kawasaki disease in children". Cochrane Database Syst Rev (4): CD004000. doi: 10.1002/14651858.CD004000. PMID 14584002.
[15]. Hsieh KS, Weng KP, Lin CC, Huang TC, Lee CL, Huang SM (December 2004). "Treatment of acute Kawasaki disease: aspirin's role in the febrile stage revisited". Pediatrics 114 (6): e689-93. doi:10.1542/peds.2004-1037. PMID 15545617.
[16]. Sundel RP, Baker AL, Fulton DR, Newburger JW (June 2003). "Corticosteroids in the initial treatment of Kawasaki disease: report of a randomized trial". J. Pediatr. 142 (6): 611-6. doi:10.1067/mpd.2003.191. PMID 12838187.
[17]. Newburger JW et al, Randomized trial of pulsed corticosteroid therapy for primary treatment of Kawasaki disease, N Engl J Med. 2007 Feb 25;356(7):663-75

Morinaga, T, Yasuda, H, Hashimoto, T, Higashio, K, Tamaoki, T. A human alpha-fetoprotein enhancer-binding protein, ATBF 1, contains four homeodomains and seventeen zinc fingers. Molec. Cell. Biol. 11: 6041-6049, 1991.
Qi, Y, Ranish, J. A, Zhu, X, Krones, A, Zhang, J, Aebersold, R, Rose, D. W, Rosenfeld, M. G, Carriere, C. Atbf1 is required for the Pit1 gene early activation. Proc. Nat. Acad. Sci. 105: 2481-2486, 2008.
Sun, X, Frierson, H. F, Chen, C, Li, C, Ran, Q, Otto, K. B, Cantarel, B. M, Vessella, R. L, Gao, A. C, Petros, J, Miura, Y, Simons, J. W, Dong, J.-T. Frequent somatic mutations of the transcription factor ATBF1 in human prostate cancer. Nature Genet. 37: 407-412, 2005.
Yamada, K, Ma, D, Miura, Y, Ido, A, Tamaoki, T, Yoshida, M. C. Assignment of the ATBF1 transcription factor gene (Atbf1) to mouse chromosome band 8E1 by in situ hybridization. Cytogenet. Cell Genet. 75: 30-31, 1996.
Yamada, K, Mirua, Y, Scheidl, T, Yoshida, M. C, Tamaoki, T. Assignment of the human ATBF1 transcription factor gene to chromosome 16q22.3-q23.1. Genomics 29: 552-553, 1995.
Arai, F, Hirao, A, Ohmura, M, Sato, H, Matsuoka, S, Takubo, K, Ito, K, Koh, G. Y, Suda, T. Tie2/Angiopoietin-1 signaling regulates hematopoietic stem cell quiescence in the bone marrow niche. Cell 118: 149-161, 2004.
Cheung, A. H, Stewart, R. J, Marsden, P. A. Endothelial Tie2/Tek ligands angiopoietin-1 (ANGPT1) and angiopoietin-2 (ANGPT2): regional localization of the human genes to 8q22.3-q23 and 8p23. Genomics 48: 389-391, 1998.
Davis, S, Aldrich, T. H, Jones, P. F, Acheson, A, Compton, D. L, Jain, V, Ryan, T. E, Bruno, J, Radziejewski, C, Maisonpierre, P. C, Yancopoulos, G. D. Isolation of angiopoietin-1, a ligand for the TIE2 receptor, by secretion-trap expression cloning. Cell 87: 1161-1169, 1996.
Folkman, J, D'Amore, P. A. Blood vessel formation: what is its molecular basis? Cell 87: 1153-1155, 1996.
Geva, E, Ginzinger, D. G, Zaloudek, C. J, Moore, D. H, Byrne, A, Jaffe, R. B.: Human placental vascular development: vasculogenic and angiogenic (branching and nonbranching) transformation is regulated by vascular endothelial growth factor-A, angiopoietin-1, and angiopoietin-2. J. Clin. Encodr. Metab. 87: 4213-4224, 2002.
Grosios, K, Leek, J. P, Markham, A. F, Yancopoulos, G. D, Jones, P. F. Assignment of ANGPT4, ANGPT1, and ANGPT2 encoding Angiopoietins 4, 1 and 2 to human chromosome bands 20p13, 8q22.3-q23 and 8p23.1, respectively, by in situ hybridization and radiation hybrid mapping. Cytogenet. Cell Genet. 84: 118-120, 1999.
Hanahan, D. Signaling vascular morphogenesis and maintenance. Science 277: 48-50, 1997.
Holash, J, Maisonpierre, P. C, Compton, D, Boland, P, Alexander, C. R, Zagzag, D, Yancopoulos, G. D, Wiegand, S. J. Vessel cooption, regression, and growth in tumors mediated by angiopoietins and VEGF. Science 284: 1994-1998, 1999.
Loughna, S, Sato, T. N. A combinatorial role of angiopoietin-1 and orphan receptor TIE1 pathways in establishing vascular polarity during angiogenesis. Molec. Cell 7: 233-239, 2001.
Marziliano, N, Crovella, S, Audero, E, Pecile, V, Bussolino, F, Amoroso, A, Garagna, S. Genetic mapping of the mouse homologue of the human angiopoietin-1 gene (Agpt) to mouse chromosome 9E2 by in situ hybridization. Cytogenet. Cell Genet. 87: 199-200, 1999.
Sato, T. N, Tozawa, Y, Deutsch, U, Wolburg-Buchholz, K, Fujiwara, Y, Gendron-Maguire, M, Gridley, T, Wolburg, H, Risau, W, Qin, Y. Distinct roles of the receptor tyrosine kinases Tie-1 and Tie-2 in blood vessel formation. Nature 376: 70-73, 1995.
Suri, C, Jones, P. F, Patan, S, Bartunkova, S, Maisonpierre, P. C, Davis, S, Sato, T. N, Yancopoulos, G. D. Requisite role of angiopoietin-1, a ligand for the TIE2 receptor, during embryonic angiogenesis. Cell 87: 1171-1180, 1996.
Suri, C, McClain, J, Thurston, G, McDonald, D. M, Zhou, H, Oldmixon, E. H, Sato, T. N, Yancopoulos, G. D. Increased vascularization in mice overexpressing angiopoietin-1. Science 282: 468-471, 1998.
Thurston, G, Suri, C, Smith, K, McClain, J, Sato, T. N, Yancopoulos, G. D, McDonald, D. M. Leakage-resistant blood vessels in mice transgenically overexpressing angiopoietin-1. Science 286: 2511-2514, 1999.
Valenzuela, D. M, Griffiths, J. A, Rojas, J, Aldrich, T. H, Jones, P. F, Zhou, H, McClain, J, Copeland, N. G, Gilbert, D. J, Jenkins, N. A, Huang, T, Papadopoulos, N, Maisonpierre, P. C, Davis, S, Yancopoulos, G. D. Angiopoietins 3 and 4: diverging gene counterparts in mice and humans. Proc. Nat. Acad. Sci. 96: 1904-1909, 1999.
Vikkula, M, Boon, L. M, Carraway, K. L., III; Calvert, J. T, Diamonti, A. J, Goumnerov, B, Pasyk, K. A, Marchuk, D. A, Warman, M. L, Cantley, L. C, Mulliken, J. B, Olsen, B. R. Vascular dysmorphogenesis caused by an activating mutation in the receptor tyrosine kinase TIE2. Cell 87: 1181-1190, 1996.
Ward, E. G, Grosios, K, Markham, A. F, Jones, P. F. Genomic structures of the human angiopoietins show polymorphism in angiopoietin-2. Cytogenet. Cell Genet. 94: 147-154, 2001.
Tonkin, E. T, Smith, M, Eichhorn, P, Jones, S, Imamwerdi, B, Lindsay, S, Jackson, M, Wang, T.-J, Ireland, M, Burn, J, Krantz, I. D, Carr, P, Strachan, T. A giant novel gene undergoing extensive alternative splicing is severed by a Cornelia de Lange-associated translocation breakpoint at 3q26.3. Hum. Genet. 115: 139-148, 2004.
Holmqvist, M. H, Cao, J, Hernandez-Pineda, R, Jacobson, M. D, Carroll, K. I, Sung, M. A, Betty, M, Ge, P, Gilbride, K. J, Brown, M. E, Jurman, M. E, Lawson, D, Silos-Santiago, I, Xie, Y, Covarrubias, M, Rhodes, K. J, Distefano, P. S, An, W. F. Elimination of fast inactivation in Kv4 A-type potassium channels by an auxiliary subunit domain. Proc. Nat. Acad. Sci. 99: 1035-1040, 2002.
Morohashi, Y, Hatano, N, Ohya, S, Takikawa, R, Watabiki, T, Takasugi, N, Imaizumi, Y, Tomita, T, Iwatsubo, T. Molecular cloning and characterization of CALP/KChIP4, a novel EF-hand protein interacting with presenilin 2 and voltage-gated potassium channel subunit Kv4. J. Biol. Chem. 277: 14965-14975, 2002.
Hanke, S, Bugert, P, Chudek, J, Kovacs, G. Cloning a calcium channel alpha-2-delta-3 subunit gene from a putative tumor suppressor gene region at chromosome 3p21.1 in conventional renal cell carcinoma. Gene 264: 69-75, 2001.
Hoch, B, Meyer, R, Hetzer, R, Krause, E.-G, Karczewski, P. Identification and expression of delta-isoforms of the multifunctional Ca(2+)/calmodulin-dependent protein kinase in failing and nonfailing human myocardium. Circ. Res. 84: 713-721, 1999.
Tombes, R. M, Krystal, G. W. Identification of novel human tumor cell-specific CaMK-II variants. Biochim. Biophys. Acta 1355: 281-292, 1997.
Xu, X, Yang, D, Ding, J.-H, Wang, W, Chu, P.-H, Dalton, N. D, Wang, H.-Y, Bermingham, J. R., Jr, Ye, Z, Liu, F, Rosenfeld, M. G, Manley, J. L, Ross, J., Jr, Chen, J, Xiao, R.-P, Cheng, H, Fu, X.-D. ASF/SF2-regulated CaMKII-delta alternative splicing temporally reprograms excitation-contraction coupling in cardiac muscle. Cell 120: 59-72, 2005.
Zhang, R, Khoo, M. S. C, Wu, Y, Yang, Y, Grueter, C. E, Ni, G, Price, E. E., Jr, Thiel, W, Guatimosim, S, Song, L.-S, Madu, E. C, Shah, A. N, Vishnivetskaya, T. A, Atkinson, J. B, Gurevich, V. V, Salama, G, Lederer, W. J, Colbran, R. J, Anderson, M. E. Calmodulin kinase II inhibition protects against structural heart disease. Nature Med. 11: 409-417, 2005.
Zhang, T, Maier, L. S, Dalton, N. D, Miyamoto, S, Ross, J., Jr, Bers, D. M, Brown, J. H. The delta-c isoform of CaMKII is activated in cardiac hypertrophy and induces dilated cardiomyopathy and heart failure. Circ. Res. 92: 912-919, 2003.
Zhu, W.-Z, Wang, S.-Q, Chakir, K, Yang, D, Zhang, T, Brown, J. H, Devic, E, Kobilka, B. K, Cheng, H, Xiao, R.-P. Linkage of beta-1-adrenergic stimulation to apoptotic heart cell death through protein kinase A-independent activation of Ca(2+)/calmodulin kinase II. J. Clin. Invest. 111: 617-625, 2003.
Matsumoto-Taniura, N, Pirollet, F, Monroe, R, Gerace, L, Westendorf, J. M. :Identification of novel M phase phosphoproteins by expression cloning. Molec. Biol. Cell 7: 1455-1469, 1996.
Westendorf, J. M, Konstantinov, K. N, Wormsley, S, Shu, M.-D, Matsumoto-Taniura, N, Pirollet, F, Klier, F. G, Gerace, L, Baserga, S. J. M phase phosphoprotein 10 is a human U3 small nucleolar ribonucleoprotein component. Molec. Biol. Cell 9: 437-449, 1998.

Each of the applications and patents mentioned in this document, and each document cited or referenced in each of the above applications and patents, including during the prosecution of each of the applications and patents ("application cited documents") and any manufacturer's instructions or catalogues for any products cited or mentioned in each of the applications and patents and in any of the application cited documents, are hereby incorporated herein by reference. Furthermore, all documents cited in this text, and all documents cited or referenced in documents cited in this text, and any manufacturer's instructions or catalogues for any products cited or mentioned in this text, are hereby incorporated herein by reference.

Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the claims.

### TABLE S1

Table S1 (Supplementary Table 1) below shows the Chromosomal Location and Allele Frequencies of 1,116 Genotyped SNPs in the Follow-up Study.

**Table S1. Chromosomal Location and Allele Frequencies of 1,116 Genotyped SNPs in the Follow-up Study.**

| **rs number** | **Chr** | **Location** | **allele1(freq)** | **allele2(freq)** | **samples failed** |
|---|---|---|---|---|---|
| rs17361670 | 1 | 6282734 | A (0.902) | T (0.098) | 0 |
| rs2076608 | 1 | 17294889 | G (0.617) | A (0.383) | 0 |
| rs2235918 | 1 | 17295197 | C (0.615) | T (0.385) | 0 |
| rs9661678 | 1 | 18353084 | C (0.875) | A (0.125) | 0 |
| rs900489 | 1 | 21921238 | G (0.739) | T (0.261) | 0 |
| rs215814 | 1 | 37440103 | A (0.732) | G (0.268) | 0 |
| rs17508197 | 1 | 37452463 | C (0.816) | A (0.184) | 0 |
| rs4246510 | 1 | 38990481 | G (0.535) | C (0.465) | 6 |
| rs1108989 | 1 | 53702051 | A (0.729) | T (0.271) | 0 |
| rs880385 | 1 | 55592687 | G (0.529) | T (0.471) | 0 |
| rs10493241 | 1 | 58556559 | C (0.854) | T (0.146) | 0 |
| rs621517 | 1 | 59749607 | A (0.643) | G (0.357) | 0 |
| rs403597 | 1 | 60640529 | C (0.863) | A (0.137) | 8 |
| rs386468 | 1 | 60641125 | G (0.863) | A (0.137) | 0 |
| rs11208108 | 1 | 63285233 | A (0.62) | T (0.38) | 0 |
| rs2273122 | 1 | 63644346 | T (0.851) | C (0.149) | 0 |
| rs10493345 | 1 | 63673489 | G (0.853) | A (0.147) | 0 |
| rs2036867 | 1 | 63679468 | G (0.852) | T (0.148) | 0 |
| rs12078371 | 1 | 63755548 | C (0.852) | G (0.148) | 1 |
| rs9804078 | 1 | 63770221 | A (0.852) | T (0.148) | 0 |
| rs7528129 | 1 | 63849402 | A (0.866) | G (0.134) | 1 |
| rs3790861 | 1 | 63860032 | T (0.905) | C (0.095) | 0 |
| rs17395543 | 1 | 69745492 | C (0.709) | T (0.291) | 0 |
| rs10493558 | 1 | 75434361 | A (0.868) | G (0.132) | 0 |
| rs12410373 | 1 | 76170837 | A (0.642) | T (0.358) | 0 |
| rs2060361 | 1 | 82976694 | G (0.89) | A (0.11) | 0 |
| rs12032086 | 1 | 88446200 | T (0.841) | C (0.159) | 2 |
| rs2039615 | 1 | 95517923 | T (0.693) | C (0.307) | 0 |
| rs12047766 | 1 | 95636445 | C (0.836) | T (0.164) | 0 |
| rs17374320 | 1 | 96446475 | G (0.678) | T (0.322) | 0 |
| rs1413229 | 1 | 97433341 | G (0.637) | A (0.363) | 0 |
| rs2197445 | 1 | 101981633 | A (0.537) | G (0.463) | 3 |
| rs11184211 | 1 | 104916711 | A (0.567) | G (0.433) | 3 |
| rs994239 | 1 | 105307475 | C (0.559) | A (0.441) | 0 |
| rs570718 | 1 | 107831897 | T (0.614) | G (0.386) | 1 |
| rs4839283 | 1 | 110615698 | C (0.56) | T (0.44) | 3 |
| rs10494252 | 1 | 145483560 | G (0.925) | T (0.075) | 0 |
| rs1027423 | 1 | 145485153 | C (0.927) | G (0.073) | 0 |
| rs10494250 | 1 | 145490671 | C (0.928) | T (0.072) | 0 |
| rs2932505 | 1 | 146261815 | A (0.7) | C (0.3) | 1 |
| rs7520184 | 1 | 153520207 | G (0.708) | A (0.292) | 0 |
| rs2023631 | 1 | 156423692 | A (0.563) | G (0.437) | 0 |
| rs11265498 | 1 | 159094909 | C (0.758) | T (0.242) | 0 |
| rs2255847 | 1 | 159193561 | G (0.813) | A (0.187) | 1 |
| rs6683580 | 1 | 159951193 | A (0.689) | G (0.311) | 0 |
| rs2815287 | 1 | 161399589 | A (0.642) | C (0.358) | 0 |
| rs2815286 | 1 | 161401663 | T (0.642) | C (0.358) | 1 |
| rs2661353 | 1 | 161402368 | G (0.643) | A (0.357) | 0 |
| rs9628673 | 1 | 161405125 | A (0.642) | C (0.358) | 0 |
| rs1415678 | 1 | 166920955 | C (0.72) | T (0.28) | 0 |
| rs12029713 | 1 | 169129334 | A (0.846) | G (0.154) | 0 |
| rs10919525 | 1 | 169142151 | G (0.845) | C (0.155) | 0 |
| rs10800571 | 1 | 169144622 | A (0.844) | T (0.156) | 0 |
| rs10800572 | 1 | 169152187 | A (0.845) | G (0.155) | 2 |
| rs2068871 | 1 | 172062792 | A (0.895) | G (0.105) | 2 |
| rs2206503 | 1 | 174720725 | A (0.891) | G (0.109) | 1 |
| rs6659737 | 1 | 175154142 | C (0.504) | G (0.496) | 0 |
| rs602988 | 1 | 180852826 | C (0.948) | T (0.052) | 7 |
| rs2383574 | 1 | 185347822 | G (0.813) | A (0.187) | 0 |
| rs673503 | 1 | 186290345 | C (0.825) | T (0.175) | 0 |
| rs10919781 | 1 | 198184713 | C (0.974) | T (0.026) | 0 |
| rs7539189 | 1 | 201848954 | G (0.908) | A (0.092) | 0 |
| rs7539296 | 1 | 201849106 | G (0.908) | A (0.092) | 0 |
| rs2485885 | 1 | 208422636 | T (0.792) | C (0.208) | 0 |
| rs7529770 | 1 | 209175356 | A (0.627) | G (0.373) | 0 |
| rs17049199 | 1 | 217504436 | G (0.696) | A (0.304) | 0 |
| rs11118329 | 1 | 217768584 | A (0.679) | T (0.321) | 0 |
| rs3842967 | 1 | 218725512 | A (0.771) | G (0.229) | 1 |
| rs12406084 | 1 | 220087883 | A (0.906) | G (0.094) | 0 |
| rs17011009 | 1 | 220121557 | A (0.847) | G (0.153) | 0 |
| rs2378598 | 1 | 221036800 | A (0.518) | G (0.482) | 0 |
| rs6659283 | 1 | 232348663 | G (0.963) | A (0.037) | 0 |
| rs12757197 | 1 | 233657182 | G (0.913) | A (0.087) | 0 |
| rs1842079 | 1 | 235691820 | G (0.806) | A (0.194) | 0 |
| rs7538106 | 1 | 238186372 | C (0.504) | T (0.496) | 0 |
| rs2817506 | 1 | 243993108 | G (0.853) | A (0.147) | 0 |
| rs4925735 | 1 | 246066942 | T (0.841) | C (0.159) | 0 |
| rs1529011 | 2 | 2973996 | A (0.608) | T (0.392) | 0 |
| rs955146 | 2 | 5411499 | T (0.709) | C (0.291) | 1 |
| rs6432366 | 2 | 5913808 | C (0.626) | G (0.374) | 0 |
| rs3755261 | 2 | 10471349 | G (0.799) | A (0.201) | 0 |
| rs16859194 | 2 | 12700041 | T (0.696) | C (0.304) | 0 |
| rs17312849 | 2 | 13976875 | C (0.917) | T (0.083) | 1 |
| rs4832645 | 2 | 16558217 | G (0.734) | C (0.266) | 6 |
| rs4240230 | 2 | 16570348 | A (0.686) | G (0.314) | 6 |
| rs6745357 | 2 | 16576876 | C (0.669) | G (0.331) | 0 |
| rs7593098 | 2 | 16581786 | C (0.674) | G (0.326) | 0 |
| rs7552 | 2 | 16597409 | A (0.668) | G (0.332) | 1 |
| rs7573656 | 2 | 16598913 | G (0.689) | A (0.311) | 1 |
| rs806645 | 2 | 31661444 | C (0.69) | T (0.31) | 0 |
| rs13032137 | 2 | 35850977 | G (0.551) | C (0.449) | 0 |
| rs7568352 | 2 | 35862789 | T (0.515) | C (0.485) | 0 |
| rs7568855 | 2 | 35863118 | T (0.559) | G (0.441) | 0 |
| rs1518670 | 2 | 35863769 | G (0.543) | A (0.457) | 0 |
| rs1518663 | 2 | 35876659 | G (0.558) | C (0.442) | 2 |
| rs2949076 | 2 | 35938693 | G (0.575) | A (0.425) | 0 |
| rs1519704 | 2 | 35939111 | T (0.563) | A (0.437) | 1 |
| rs2110745 | 2 | 40354855 | A (0.609) | G (0.391) | 1 |
| rs1873555 | 2 | 43633169 | C (0.853) | T (0.147) | 11 |
| rs7596052 | 2 | 43635330 | G (0.706) | A (0.294) | 4 |
| rs17037853 | 2 | 49000793 | A (0.956) | G (0.044) | 0 |
| rs11893174 | 2 | 49835492 | C (0.897) | G (0.103) | 2 |
| rs3850333 | 2 | 50856433 | C (0.585) | T (0.415) | 0 |
| rs6749959 | 2 | 52519666 | A (0.771) | G (0.229) | 0 |
| rs1533076 | 2 | 53063583 | C (0.845) | T (0.155) | 0 |
| rs7595561 | 2 | 53076659 | T (0.787) | A (0.213) | 0 |
| rs1861772 | 2 | 56315410 | A (0.561) | C (0.439) | 0 |
| rs1861776 | 2 | 56510398 | T (0.521) | C (0.479) | 2 |
| rs1424649 | 2 | 57223628 | T (0.655) | C (0.345) | 0 |
| rs10153799 | 2 | 57633546 | T (0.977) | G (0.023) | 1 |
| rs10519011 | 2 | 65160865 | C (0.974) | G (0.026) | 0 |
| rs2553055 | 2 | 65960337 | T (0.73) | C (0.27) | 0 |
| rs10490192 | 2 | 66790990 | A (0.62) | T (0.38) | 1 |
| rs7600609 | 2 | 66791511 | C (0.615) | T (0.385) | 0 |
| rs2081399 | 2 | 66925563 | C (0.714) | T (0.286) | 0 |
| rs2861182 | 2 | 66927348 | A (0.707) | G (0.293) | 1 |
| rs17631953 | 2 | 66951054 | C (0.761) | G (0.239) | 0 |
| rs4852511 | 2 | 79765398 | C (0.511) | A (0.489) | 1 |
| rs2241436 | 2 | 86214320 | C (0.514) | T (0.486) | 0 |
| rs13017143 | 2 | 86320060 | T (0.61) | C (0.39) | 0 |
| rs 10489973 | 2 | 101832572 | C (0.833) | A (0.167) | 0 |
| rs4851526 | 2 | 101984802 | A (0.608) | G (0.392) | 1 |
| rs10187099 | 2 | 104077457 | A (0.645) | G (0.355) | 0 |
| rs6722922 | 2 | 113557986 | C (0.611) | T (0.389) | 1 |
| rs17046609 | 2 | 117539769 | G (0.874) | A (0.126) | 2 |
| rs6542556 | 2 | 120474323 | G (0.686) | A (0.314) | 8 |
| rs10183521 | 2 | 123479012 | C (0.551) | T (0.449) | 1 |
| rs10198627 | 2 | 137794706 | T (0.773) | C (0.227) | 0 |
| rs9677983 | 2 | 158502910 | C (0.975) | T (0.025) | 2 |
| rs1425039 | 2 | 160136369 | C (0.525) | A (0.475) | 1 |
| rs918969 | 2 | 160174902 | G (0.522) | A (0.478) | 0 |
| rs11899419 | 2 | 160253615 | A (0.521) | G (0.479) | 0 |
| rs10929982 | 2 | 160944523 | T (0.79) | C (0.21) | 0 |
| rs3769941 | 2 | 165893486 | A (0.75) | G (0.25) | 0 |
| rs16850467 | 2 | 165916123 | G (0.764) | C (0.236) | 0 |
| rs6744911 | 2 | 165916295 | A (0.759) | G (0.241) | 0 |
| rs6432834 | 2 | 166083178 | A (0.754) | C (0.246) | 1 |
| rs10184828 | 2 | 172981755 | G (0.649) | C (0.351) | 0 |
| rs10189510 | 2 | 183199011 | A (0.898) | G (0.102) | 3 |
| rs2696115 | 2 | 183236915 | G (0.672) | A (0.328) | 0 |
| rs825662 | 2 | 183245644 | T (0.667) | C (0.333) | 1 |
| rs288267 | 2 | 183279231 | T (0.644) | A (0.356) | 0 |
| rs10205822 | 2 | 187577487 | A (0.75) | T (0.25) | 0 |
| rs16828607 | 2 | 187664978 | G (0.803) | A (0.197) | 0 |
| rs1487400 | 2 | 187712914 | G (0.802) | T (0.198) | 0 |
| rs10931278 | 2 | 187777521 | G (0.812) | A (0.188) | 0 |
| rs1728673 | 2 | 187864697 | C (0.684) | G (0.316) | 1 |
| rs860860 | 2 | 187867518 | G (0.684) | A (0.316) | 0 |
| rs840600 | 2 | 187867995 | A (0.684) | C (0.316) | 0 |
| rs840616 | 2 | 187904714 | C (0.659) | T (0.341) | 0 |
| rs698590 | 2 | 187923068 | T (0.685) | A (0.315) | 0 |
| rs3821183 | 2 | 187964403 | C (0.809) | T (0.191) | 0 |
| rs3821181 | 2 | 187977176 | G (0.808) | C (0.192) | 0 |
| rs11888904 | 2 | 196872939 | G (0.679) | A (0.321) | 0 |
| rs6434836 | 2 | 196881381 | T (0.635) | C (0.365) | 1 |
| rs6713102 | 2 | 196882632 | G (0.671) | A (0.329) | 0 |
| rs7558972 | 2 | 196884227 | C (0.633) | T (0.367) | 0 |
| rs11900095 | 2 | 210862256 | A (0.889) | G (0.111) | 0 |
| rs4261668 | 2 | 210870557 | T (0.804) | C (0.196) | 0 |
| rs6725545 | 2 | 211742060 | T (0.7) | C (0.3) | 0 |
| rs6435607 | 2 | 211742427 | A (0.7) | T (0.3) | 0 |
| rs1437933 | 2 | 211744920 | C (0.7) | T (0.3) | 0 |
| rs6435608 | 2 | 211746572 | C (0.7) | A (0.3) | 2 |
| rs 1344906 | 2 | 211746920 | C (0.7) | A (0.3) | 0 |
| rs1370613 | 2 | 211749923 | C (0.7) | T (0.3) | 1 |
| rs 1250224 | 2 | 216033519 | A (0.701) | C (0.299) | 0 |
| rs12613007 | 2 | 223025456 | G (0.682) | A (0.318) | 0 |
| rs895774 | 2 | 223594807 | C (0.625) | T (0.375) | 0 |
| rs895772 | 2 | 223601208 | C (0.64) | T (0.36) | 0 |
| rs10171599 | 2 | 224474816 | T (0.769) | A (0.231) | 0 |
| rs10432452 | 2 | 224898026 | G (0.773) | A (0.227) | 0 |
| rs1562012 | 2 | 225826057 | G (0.999) | A (0.001) | 6 |
| rs10498205 | 2 | 227109839 | T (0.833) | C (0.167) | 1 |
| rs2102101 | 2 | 227118593 | A (0.836) | G (0.164) | 1 |
| rs17196337 | 2 | 227155742 | C (0.835) | T (0.165) | 0 |
| rs6437001 | 2 | 231721548 | G (0.654) | T (0.346) | 0 |
| rs7558609 | 2 | 233408623 | G (0.637) | A (0.363) | 0 |
| rs4553820 | 2 | 241592248 | T (0.748) | C (0.252) | 0 |
| rs1143977 | 3 | 2334149 | G (0.692) | A (0.308) | 1 |
| rs 1032783 | 3 | 2336802 | T (0.694) | A (0.306) | 0 |
| rs1666325 | 3 | 2358336 | G (0.681) | A (0.319) | 0 |
| rs6808240 | 3 | 2397321 | T (0.73) | C (0.27) | 0 |
| rs9866915 | 3 | 3947118 | A (0.831) | C (0.169) | 0 |
| rs1038482 | 3 | 3986128 | T (0.903) | C (0.097) | 1 |
| rs17039550 | 3 | 3999446 | C (0.896) | G (0.104) | 3 |
| rs1444060 | 3 | 4205231 | G (0.682) | C (0.318) | 0 |
| rs6769118 | 3 | 4212101 | C (0.518) | T (0.482) | 1 |
| rs996402 | 3 | 4212188 | C (0.683) | T (0.317) | 4 |
| rs310692 | 3 | 4287735 | A (0.552) | G (0.448) | 0 |
| rs9864388 | 3 | 8119521 | C (0.62) | A (0.38) | 0 |
| rs39667 | 3 | 10458958 | A (0.846) | G (0.154) | 2 |
| rs4684078 | 3 | 11747784 | C (0.582) | A (0.418) | 1 |
| rs357125 | 3 | 13312476 | G (0.61) | A (0.39) | 0 |
| rs17015415 | 3 | 24908893 | A (1) | G (0) | 4 |
| rs17576085 | 3 | 25139629 | T (0.809) | C (0.191) | 0 |
| rs6549938 | 3 | 29420467 | A (0.71) | G (0.29) | 0 |
| rs347128 | 3 | 32422309 | A (0.623) | G (0.377) | 0 |
| rs10510678 | 3 | 36215909 | A (0.856) | G (0.144) | 0 |
| rs1165958 | 3 | 36247401 | C (0.716) | T (0.284) | 1 |
| rs10514693 | 3 | 36272792 | G (0.85) | A (0.15) | 0 |
| rs285330 | 3 | 36285414 | T (0.747) | C (0.253) | 0 |
| rs17034777 | 3 | 36290576 | G (0.845) | A (0.155) | 0 |
| rs285326 | 3 | 36290671 | C (0.739) | T (0.261) | 0 |
| rs1585287 | 3 | 39989394 | C (0.598) | T (0.402) | 0 |
| rs17637178 | 3 | 45379802 | G (0.687) | A (0.313) | 0 |
| rs358056 | 3 | 55038668 | G (0.763) | T (0.237) | 0 |
| rs1303938 | 3 | 55945191 | T (0.627) | C (0.373) | 1 |
| rs923128 | 3 | 56088596 | G (0.825) | A (0.175) | 4 |
| rs813800 | 3 | 64851817 | C (0.637) | T (0.363) | 0 |
| rs7634969 | 3 | 65151156 | A (0.787) | G (0.213) | 0 |
| rs17073075 | 3 | 65496864 | G (0.768) | T (0.232) | 0 |
| rs9840574 | 3 | 65505289 | C (0.773) | T (0.227) | 1 |
| rs17006293 | 3 | 69777596 | C (0.95) | T (0.05) | 0 |
| rs17635394 | 3 | 69784496 | T (0.748) | C (0.252) | 0 |
| rs937130 | 3 | 99194687 | C (0.525) | T (0.475) | 2 |
| rs10212176 | 3 | 99338828 | C (0.848) | T (0.152) | 0 |
| rs2460037 | 3 | 103332789 | T (0.727) | C (0.273) | 0 |
| rs16844786 | 3 | 103358583 | G (0.771) | C (0.229) | 0 |
| rs17352589 | 3 | 103363790 | A (0.845) | C (0.155) | 0 |
| rs2619283 | 3 | 103373585 | C (0.59) | T (0.41) | 0 |
| rs17723244 | 3 | 119192786 | A (0.719) | G (0.281) | 0 |
| rs9865576 | 3 | 124561849 | T (0.565) | A (0.435) | 0 |
| rs 1920006 | 3 | 133273696 | T (0.684) | C (0.316) | 8 |
| rs2600960 | 3 | 134079612 | A (0.501) | C (0.499) | 1 |
| rs9827596 | 3 | 139134883 | A (0.642) | G (0.358) | 0 |
| rs1429771 | 3 | 139135241 | T (0.638) | C (0.362) | 2 |
| rs1991561 | 3 | 139135724 | A (0.64) | G (0.36) | 0 |
| rs1429772 | 3 | 139136229 | A (0.635) | G (0.365) | 0 |
| rs16847696 | 3 | 139176774 | T (0.847) | C (0.153) | 3 |
| rs 16849640 | 3 | 141202055 | A (0.918) | T (0.082) | 8 |
| rs 16849687 | 3 | 141214982 | G (0.903) | A (0.097) | 0 |
| rs800082 | 3 | 145822895 | C (0.578) | T (0.422) | 0 |
| rs800084 | 3 | 145823184 | A (0.578) | G (0.422) | 0 |
| rs7616299 | 3 | 160075874 | G (0.835) | A (0.165) | 0 |
| rs16847010 | 3 | 163477687 | T (0.912) | C (0.088) | 1 |
| rs2173068 | 3 | 163845916 | G (0.515) | A (0.485) | 1 |
| rs9834548 | 3 | 166622641 | G (0.657) | C (0.343) | 0 |
| rs6443824 | 3 | 168032542 | A (0.586) | G (0.414) | 1 |
| rs6443825 | 3 | 168032644 | T (0.586) | G (0.414) | 1 |
| rs10490783 | 3 | 170522888 | G (0.949) | A (0.051) | 1 |
| rs 12492606 | 3 | 171291048 | G (0.86) | A (0.14) | 4 |
| rs3772186 | 3 | 171372025 | T (0.86) | A (0.14) | 0 |
| rs17531088 | 3 | 176376469 | C (0.557) | T (0.443) | 0 |
| rs7647833 | 3 | 177976361 | T (0.919) | G (0.081) | 0 |
| rs 13064299 | 3 | 189347794 | T (0.534) | C (0.466) | 0 |
| rs1711004 | 3 | 189348113 | T (0.534) | C (0.466) | 0 |
| rs1846957 | 3 | 193752631 | T (0.703) | C (0.297) | 0 |
| rs6444717 | 3 | 194604017 | C (0.895) | T (0.105) | 0 |
| rs13072883 | 3 | 196476043 | A (0.549) | G (0.451) | 0 |
| rs2688515 | 3 | 197011868 | A (0.533) | G (0.467) | 0 |
| rs1045887 | 4 | 424079 | C (0.649) | T (0.35 1) | 0 |
| rs6599350 | 4 | 463898 | G (0.671) | C (0.329) | 0 |
| rs2878610 | 4 | 7424737 | A (0.567) | G (0.433) | 0 |
| rs6449174 | 4 | 9575520 | C (0.58) | T (0.42) | 2 |
| rs4345174 | 4 | 10668941 | T (0.634) | C (0.366) | 0 |
| rs12509320 | 4 | 11881474 | C (0.561) | T (0.439) | 1 |
| rs1454881 | 4 | 11917909 | C (0.571) | T (0.429) | 0 |
| rs17358384 | 4 | 12129840 | T (0.921) | C (0.079) | 0 |
| rs16887182 | 4 | 12164369 | A (0.922) | G (0.078) | 0 |
| rs3815466 | 4 | 15349390 | G (0.877) | T (0.123) | 1 |
| rs16868299 | 4 | 15741519 | G (0.763) | T (0.237) | 0 |
| rs4279206 | 4 | 17365371 | A (0.749) | G (0.251) | 0 |
| rs 16869942 | 4 | 20376800 | C (0.965) | G (0.035) | 0 |
| rs6814624 | 4 | 35681646 | T (0.709) | G (0.291) | 0 |
| rs7664766 | 4 | 35689904 | T (0.944) | A (0.056) | 0 |
| rs2375743 | 4 | 35840895 | A (0.815) | G (0.185) | 0 |
| rs12641222 | 4 | 37831102 | C (0.736) | T (0.264) | 1 |
| rs17619330 | 4 | 39796815 | C (0.71) | T (0.29) | 0 |
| rs7683275 | 4 | 41221779 | A (0.698) | G (0.302) | 1 |
| rs16854256 | 4 | 42052968 | C (0.999) | T (0.001) | 1 |
| rs1492839 | 4 | 45490652 | C (0.525) | T (0.475) | 0 |
| rs7670127 | 4 | 45495094 | G (0.533) | A (0.467) | 0 |
| rs7697851 | 4 | 48178687 | T (0.527) | C (0.473) | 0 |
| rs4695391 | 4 | 48277666 | G (0.507) | A (0.493) | 1 |
| rs796555 | 4 | 48331352 | C (0.506) | T (0.494) | 2 |
| rs13149083 | 4 | 48504702 | C (0.558) | T (0.442) | 1 |
| rs4864471 | 4 | 54120941 | G (0.883) | T (0.117) | 0 |
| rs11941778 | 4 | 63100524 | G (0.858) | A (0.142) | 0 |
| rs10866161 | 4 | 66324704 | G (0.643) | A (0.357) | 0 |
| rs1497435 | 4 | 67914643 | G (0.528) | C (0.472) | 1 |
| rs6532120 | 4 | 75870047 | C (0.738) | T (0.262) | 0 |
| rs6813112 | 4 | 76482429 | T (0.869) | C (0.131) | 1 |
| rs346515 | 4 | 87062528 | T (0.626) | C (0.374) | 1 |
| rs1481011 | 4 | 89355781 | C (0.983) | A (0.017) | 0 |
| rs1039147 | 4 | 91744589 | A (0.737) | C (0.263) | 0 |
| rs1365492 | 4 | 101011975 | G (0.57) | A (0.43) | 1 |
| rs3796917 | 4 | 106697698 | A (0.818) | T (0.182) | 0 |
| rs718820 | 4 | 108270949 | T (0.589) | C (0.411) | 1 |
| rs7684254 | 4 | 112464239 | G (0.698) | A (0.302) | 1 |
| rs2349577 | 4 | 112466783 | C (0.608) | T (0.392) | 0 |
| rs1508218 | 4 | 112497172 | G (0.608) | T (0.392) | 0 |
| rs17531554 | 4 | 114723610 | C (0.823) | T (0.177) | 0 |
| rs2620424 | 4 | 116109214 | G (0.652) | A (0.348) | 1 |
| rs6820993 | 4 | 116116504 | G (0.521) | T (0.479) | 1 |
| rs2583523 | 4 | 116147641 | G (0.808) | A (0.192) | 0 |
| rs17584238 | 4 | 116191330 | T (0.823) | C (0.177) | 0 |
| rs509777 | 4 | 116232506 | T (0.699) | C (0.301) | 0 |
| rs2892665 | 4 | 116392566 | C (0.519) | G (0.481) | 4 |
| rs343168 | 4 | 122035951 | T (0.87) | C (0.13) | 1 |
| rs1452494 | 4 | 122408343 | T (0.597) | A (0.403) | 1 |
| rs12642727 | 4 | 122438683 | T (0.702) | C (0.298) | 0 |
| rs1511498 | 4 | 132613842 | C (0.68) | T (0.32) | 0 |
| rs1425559 | 4 | 134431169 | G (0.602) | A (0.398) | 1 |
| rs13128113 | 4 | 136361141 | A (0.602) | G (0.398) | 0 |
| rs5006660 | 4 | 136361839 | T (0.602) | C (0.398) | 1 |
| rs5006661 | 4 | 136361948 | C (0.602) | T (0.398) | 0 |
| rs12500209 | 4 | 141863122 | G (0.663) | T (0.337) | 2 |
| rs4956494 | 4 | 142090098 | A (0.5) | G (0.5) | 1 |
| rs11100924 | 4 | 147536489 | A (0.825) | G (0.175) | 2 |
| rs3849035 | 4 | 147538816 | T (0.825) | C (0.175) | 0 |
| rs2714900 | 4 | 148464252 | A (0.786) | T (0.214) | 0 |
| rs1429134 | 4 | 148465085 | C (0.787) | T (0.213) | 0 |
| rs2163012 | 4 | 148487477 | A (0.757) | G (0.243) | 0 |
| rs1366691 | 4 | 148501262 | T (0.764) | C (0.236) | 1 |
| rs1155422 | 4 | 156628123 | G (0.515) | T (0.485) | 0 |
| rs9991982 | 4 | 159001480 | C (0.545) | T (0.455) | 0 |
| rs10857308 | 4 | 159137447 | C (0.741) | A (0.259) | 0 |
| rs6819071 | 4 | 167887265 | C (0.648) | T (0.352) | 0 |
| rs7658503 | 4 | 167887816 | G (0.648) | T (0.352) | 0 |
| rs10013756 | 4 | 181547116 | A (0.65) | G (0.35) | 1 |
| rs10780063 | 4 | 181547198 | G (0.567) | A (0.433) | 0 |
| rs17090674 | 4 | 185156464 | C (0.726) | T (0.274) | 0 |
| rs27061 | 5 | 1415793 | G (0.59) | A (0.41) | 0 |
| rs7735656 | 5 | 2203273 | T (0.71) | G (0.29) | 2 |
| rs11957345 | 5 | 8542452 | C (0.762) | G (0.238) | 0 |
| rs900535 | 5 | 10759152 | A (0.608) | G (0.392) | 0 |
| rs267985 | 5 | 10766395 | T (0.6) | C (0.4) | 0 |
| rs437652 | 5 | 24417939 | G (0.879) | A (0.121) | 0 |
| rs4333331 | 5 | 32064406 | G (0.57) | C (0.43) | 0 |
| rs366050 | 5 | 34661418 | G (0.58) | A (0.42) | 1 |
| rs4869648 | 5 | 36371148 | G (0.716) | C (0.284) | 1 |
| rs7730020 | 5 | 40693709 | C (0.663) | T (0.337) | 0 |
| rs298023 | 5 | 58979217 | A (0.879) | T (0.121) | 0 |
| rs12517174 | 5 | 60004772 | T (0.563) | C (0.437) | 0 |
| rs7701440 | 5 | 60656737 | T (0.525) | C (0.475) | 0 |
| rs7734879 | 5 | 60747389 | C (0.66) | A (0.34) | 1 |
| rs7714712 | 5 | 60768309 | C (0.659) | A (0.341) | 1 |
| rs6449535 | 5 | 60797342 | T (0.638) | G (0.362) | 1 |
| rs6882136 | 5 | 60797829 | G (0.638) | A (0.362) | 1 |
| rs11952146 | 5 | 60854620 | T (0.638) | C (0.362) | 1 |
| rs6861728 | 5 | 62246496 | C (0.543) | A (0.457) | 0 |
| rs6899143 | 5 | 67142008 | A (0.844) | C (0.156) | 0 |
| rs7737876 | 5 | 68215089 | C (1) | A (0) | 1 |
| rs3903314 | 5 | 71076065 | T (0.792) | C (0.208) | 3 |
| rs1106471 | 5 | 71082963 | C (0.79) | G (0.21) | 0 |
| rs3915457 | 5 | 71086332 | G (0.854) | T (0.146) | 0 |
| rs266695 | 5 | 95823834 | T (0.677) | C (0.323) | 2 |
| rs502025 | 5 | 104050496 | C (0.726) | T (0.274) | 6 |
| rs17157943 | 5 | 104716994 | C (0.957) | T (0.043) | 0 |
| rs13189318 | 5 | 104818430 | T (0.959) | G (0.041) | 0 |
| rs13183836 | 5 | 104830760 | G (0.894) | A (0.106) | 0 |
| rs4254949 | 5 | 109622167 | A (0.788) | G (0.212) | 3 |
| rs6890286 | 5 | 110206809 | T (0.922) | G (0.078) | 0 |
| rs17136136 | 5 | 113324058 | T (0.745) | C (0.255) | 2 |
| rs10900751 | 5 | 120939209 | A (0.742) | T (0.258) | 2 |
| rs2407110 | 5 | 121620597 | G (0.772) | A (0.228) | 6 |
| rs11241637 | 5 | 121642763 | T (0.77) | A (0.23) | 2 |
| rs13175503 | 5 | 121651840 | C (0.769) | G (0.231) | 2 |
| rs2045902 | 5 | 121915188 | C (0.74) | T (0.26) | 4 |
| rs1382309 | 5 | 121922955 | A (0.729) | G (0.271) | 1 |
| rs17764730 | 5 | 127385425 | C (0.746) | T (0.254) | 1 |
| rs9327479 | 5 | 127990740 | G (0.728) | A (0.272) | 0 |
| rs4836487 | 5 | 129379398 | A (0.661) | G (0.339) | 3 |
| rs574654 | 5 | 134482588 | C (0.511) | T (0.489) | 0 |
| rs6896456 | 5 | 134605656 | G (0.853) | A (0.147) | 0 |
| rs874158 | 5 | 134807316 | G (0.888) | A (0.112) | 5 |
| rs245117 | 5 | 134929260 | T (0.946) | C (0.054) | 3 |
| rs11958545 | 5 | 135651835 | C (0.886) | T (0.114) | 0 |
| rs3777149 | 5 | 135698334 | T (0.885) | C (0.115) | 0 |
| rs2673925 | 5 | 135733573 | C (0.701) | T (0.299) | 1 |
| rs7705405 | 5 | 135780907 | G (0.859) | A (0.141) | 0 |
| rs6886081 | 5 | 139139145 | T (0.906) | C (0.094) | 0 |
| rs12653538 | 5 | 143664477 | G (0.822) | A (0.178) | 0 |
| rs11750184 | 5 | 148826711 | G (0.753) | A (0.247) | 0 |
| rs2431718 | 5 | 148833314 | G (0.632) | A (0.368) | 0 |
| rs10035492 | 5 | 149234692 | C (0.898) | T (0.102) | 0 |
| rs7723957 | 5 | 153616381 | A (0.676) | G (0.324) | 0 |
| rs473214 | 5 | 153627422 | A (0.677) | G (0.323) | 1 |
| rs256835 | 5 | 155875323 | A (0.514) | G (0.486) | 3 |
| rs6897279 | 5 | 155894494 | A (0.533) | T (0.467) | 0 |
| rs4382149 | 5 | 155933832 | G (0.523) | T (0.477) | 0 |
| rs934418 | 5 | 155935397 | G (0.524) | A (0.476) | 0 |
| rs6879255 | 5 | 161952476 | A (0.685) | G (0.315) | 1 |
| rs1963837 | 5 | 161997183 | G (0.906) | A (0.094) | 0 |
| rs297878 | 5 | 168543070 | G (0.772) | C (0.228) | 0 |
| rs12652441 | 5 | 168803228 | T (0.648) | G (0.352) | 1 |
| rs2126237 | 6 | 2667496 | T (0.893) | C (0.107) | 0 |
| rs9501885 | 6 | 2686186 | G (0.927) | A (0.073) | 10 |
| rs9378794 | 6 | 3383425 | G (0.795) | A (0.205) | 0 |
| rs7773169 | 6 | 4233389 | T (0.53) | G (0.47) | 0 |
| rs1999022 | 6 | 4242285 | T (0.621) | C (0.379) | 1 |
| rs9392158 | 6 | 7372349 | T (0.89) | G (0.11) | 0 |
| rs1890954 | 6 | 8331801 | G (0.732) | T (0.268) | 1 |
| rs3798751 | 6 | 11050950 | A (0.948) | G (0.052) | 0 |
| rs2327335 | 6 | 11227033 | C (0.826) | T (0.174) | 0 |
| rs7775964 | 6 | 11915660 | G (0.939) | C (0.061) | 0 |
| rs7739436 | 6 | 11924176 | A (0.939) | G (0.061) | 0 |
| rs7740866 | 6 | 11927869 | C (0.939) | A (0.061) | 5 |
| rs10949552 | 6 | 18947116 | G (0.584) | A (0.416) | 0 |
| rs1555108 | 6 | 22802808 | T (0.793) | G (0.207) | 0 |
| rs12195633 | 6 | 23424572 | T (0.636) | C (0.364) | 1 |
| rs2817027 | 6 | 35886697 | C (0.678) | A (0.322) | 1 |
| rs612262 | 6 | 35999586 | C (0.645) | T (0.355) | 0 |
| rs4713951 | 6 | 36370969 | C (0.556) | T (0.444) | 1 |
| rs7755143 | 6 | 37041148 | C (0.835) | T (0.165) | 1 |
| rs11969402 | 6 | 56554765 | A (0.613) | G (0.387) | 0 |
| rs9370539 | 6 | 56562340 | A (0.602) | G (0.398) | 2 |
| rs6459166 | 6 | 56590760 | C (0.599) | G (0.401) | 0 |
| rs2230862 | 6 | 56592717 | G (0.613) | A (0.387) | 0 |
| rs4470833 | 6 | 63746733 | C (0.641) | G (0.359) | 0 |
| rs7750310 | 6 | 63767918 | A (0.65) | T (0.35) | 0 |
| rs4235961 | 6 | 63781337 | G (0.65) | T (0.35) | 0 |
| rs1341175 | 6 | 63790311 | G (0.651) | A (0.349) | 0 |
| rs1459651 | 6 | 63941094 | T (0.68) | G (0.32) | 2 |
| rs9446459 | 6 | 72509427 | A (0.503) | G (0.497) | 2 |
| rs9446461 | 6 | 72509624 | C (0.504) | T (0.496) | 0 |
| rs4079357 | 6 | 74371962 | A (0.915) | G (0.085) | 0 |
| rs2313753 | 6 | 76708983 | G (0.637) | C (0.363) | 0 |
| rs7745251 | 6 | 84228475 | T (0.643) | G (0.357) | 0 |
| rs16876114 | 6 | 86156062 | A (0.762) | T (0.238) | 0 |
| rs9450667 | 6 | 88151105 | C (0.705) | T (0.295) | 0 |
| rs7754397 | 6 | 91847214 | A (0.873) | G (0.127) | 0 |
| rs9342279 | 6 | 92226731 | G (0.748) | T (0.252) | 1 |
| rs1750603 | 6 | 94958120 | G (0.663) | A (0.337) | 0 |
| rs6925110 | 6 | 106120691 | A (0.541) | G (0.459) | 0 |
| rs9285409 | 6 | 113728953 | A (0.856) | C (0.144) | 5 |
| rs1743257 | 6 | 118722083 | A (0.873) | G (0.127) | 2 |
| rs794266 | 6 | 119362012 | G (0.567) | A (0.433) | 0 |
| rs9490983 | 6 | 124242283 | A (0.589) | G (0.411) | 1 |
| rs9388287 | 6 | 124242464 | A (0.582) | G (0.418) | 1 |
| rs10484734 | 6 | 124255980 | G (0.585) | C (0.415) | 2 |
| rs990802 | 6 | 124745200 | A (0.925) | C (0.075) | 0 |
| rs9491153 | 6 | 124752767 | G (0.93) | C (0.07) | 0 |
| rs9320991 | 6 | 124805484 | C (0.797) | T (0.203) | 0 |
| rs9376869 | 6 | 145242793 | G (0.824) | A (0.176) | 0 |
| rs9403621 | 6 | 145246661 | G (0.831) | C (0.169) | 1 |
| rs6913165 | 6 | 145259141 | T (0.826) | C (0.174) | 1 |
| rs 1408505 | 6 | 150512956 | G (0.799) | A (0.201) | 0 |
| rs270289 | 6 | 153783769 | C (0.65) | T (0.35) | 0 |
| rs4568464 | 6 | 154037004 | C (0.857) | T (0.143) | 0 |
| rs9480349 | 6 | 156914836 | C (0.822) | T (0.178) | 1 |
| rs9384506 | 6 | 157223884 | C (0.694) | T (0.306) | 1 |
| rs927450 | 6 | 160102086 | C (0.551) | T (0.449) | 1 |
| rs3818299 | 6 | 160126621 | G (0.553) | A (0.447) | 0 |
| rs2273828 | 6 | 160127039 | G (0.552) | A (0.448) | 0 |
| rs927451 | 6 | 160133426 | A (0.522) | C (0.478) | 0 |
| rs3019449 | 6 | 162085083 | G (0.717) | T (0.283) | 1 |
| rs7739088 | 6 | 169926308 | A (0.79) | G (0.21) | 0 |
| rs3823466 | 6 | 169931851 | G (0.875) | A (0.125) | 4 |
| rs17167055 | 7 | 7382384 | A (0.951) | G (0.049) | 0 |
| rs320785 | 7 | 9725914 | G (0.668) | C (0.332) | 0 |
| rs11974132 | 7 | 9726775 | C (0.995) | G (0.005) | 2 |
| rs1369496 | 7 | 12139698 | G (0.789) | C (0.211) | 2 |
| rs2282873 | 7 | 13957468 | A (0.502) | G (0.498) | 3 |
| rs196751 | 7 | 14443632 | A (0.523) | G (0.477) | 0 |
| rs12699619 | 7 | 14471463 | T (0.599) | C (0.401) | 0 |
| rs10236653 | 7 | 14500845 | G (0.53) | A (0.47) | 0 |
| rs12699645 | 7 | 14557877 | T (0.523) | C (0.477) | 1 |
| rs17731618 | 7 | 18304136 | T (0.75) | C (0.25) | 2 |
| rs2057986 | 7 | 22064906 | T (0.847) | C (0.153) | 0 |
| rs198733 | 7 | 24143041 | A (0.608) | T (0.392) | 2 |
| rs198731 | 7 | 24143347 | A (0.607) | T (0.393) | 1 |
| rs198728 | 7 | 24144172 | A (0.604) | G (0.396) | 0 |
| rs 198727 | 7 | 24144378 | A (0.604) | G (0.396) | 0 |
| rs198723 | 7 | 24145552 | G (0.61) | A (0.39) | 0 |
| rs198701 | 7 | 24149638 | C (0.606) | T (0.394) | 0 |
| rs2905863 | 7 | 24154144 | T (0.601) | C (0.399) | 3 |
| rs6957661 | 7 | 24345130 | G (0.892) | A (0.108) | 0 |
| rs12700921 | 7 | 29026983 | A (0.831) | G (0.169) | 0 |
| rs4720246 | 7 | 37402913 | A (0.833) | G (0.167) | 0 |
| rs7782999 | 7 | 37413218 | A (0.776) | T (0.224) | 0 |
| rs17171805 | 7 | 41030252 | T (0.966) | C (0.034) | 0 |
| rs10081385 | 7 | 41932574 | C (0.606) | T (0.394) | 1 |
| rs6952496 | 7 | 41940034 | T (0.609) | C (0.391) | 0 |
| rs7805291 | 7 | 41940312 | C (0.575) | T (0.425) | 0 |
| rs10248218 | 7 | 41941248 | G (0.575) | A (0.425) | 0 |
| rs3735361 | 7 | 41967449 | C (0.654) | T (0.346) | 0 |
| rs1427658 | 7 | 42975718 | C (0.675) | T (0.325) | 0 |
| rs965924 | 7 | 47654463 | G (0.759) | T (0.241) | 0 |
| rs10046555 | 7 | 47677313 | G (0.835) | A (0.165) | 0 |
| rs10258317 | 7 | 52709890 | G (0.817) | A (0.183) | 3 |
| rs1357884 | 7 | 54384323 | A (0.643) | G (0.357) | 1 |
| rs6593148 | 7 | 54469423 | T (0.613) | G (0.387) | 1 |
| rs10499740 | 7 | 54498075 | C (0.61) | T (0.39) | 0 |
| rs6952270 | 7 | 54503165 | C (0.612) | T (0.388) | 0 |
| rs7780516 | 7 | 67003990 | G (0.553) | A (0.447) | 0 |
| rs4719155 | 7 | 70745160 | G (0.749) | A (0.251) | 2 |
| rs10215394 | 7 | 81903176 | C (0.845) | G (0.155) | 51 |
| rs551790 | 7 | 83177960 | G (0.996) | T (0.004) | 0 |
| rs7811683 | 7 | 95049579 | T (0.557) | A (0.443) | 0 |
| rs 10264647 | 7 | 95107108 | T (0.933) | G (0.067) | 0 |
| rs17168776 | 7 | 97022171 | G (0.906) | T (0.094) | 0 |
| rs 16872046 | 7 | 105497973 | C (0.923) | T (0.077) | 0 |
| rs6466190 | 7 | 107238281 | T (0.583) | G (0.417) | 2 |
| rs10272725 | 7 | 109726403 | A (0.675) | G (0.325) | 0 |
| rs17157641 | 7 | 109805335 | G (0.837) | A (0.163) | 0 |
| rs17157642 | 7 | 109810597 | A (0.835) | G (0.165) | 0 |
| rs10250499 | 7 | 121017315 | T (0.619) | C (0.381) | 0 |
| rs13232111 | 7 | 122174203 | G (0.798) | A (0.202) | 0 |
| rs2190252 | 7 | 122176633 | A (0.833) | T (0.167) | 0 |
| rs17144945 | 7 | 122242607 | G (0.831) | T (0.169) | 0 |
| rs7787372 | 7 | 122251637 | A (0.83) | G (0.17) | 0 |
| rs6466910 | 7 | 123794350 | A (0.917) | G (0.083) | 5 |
| rs7782495 | 7 | 125067319 | A (0.535) | G (0.465) | 1 |
| rs17165389 | 7 | 130383092 | T (0.889) | C (0.111) | 0 |
| rs10267502 | 7 | 130623837 | C (0.836) | T (0.164) | 0 |
| rs2354332 | 7 | 138185511 | C (0.715) | G (0.285) | 1 |
| rs6978936 | 7 | 142898445 | C (1) | T (0) | 1 |
| rs1997089 | 7 | 144456909 | A (0.838) | G (0.162) | 85 |
| rs874607 | 7 | 150695805 | T (0.878) | C (0.122) | 1 |
| rs4367421 | 7 | 155784949 | C (0.921) | T (0.079) | 2 |
| rs2335836 | 7 | 157662662 | G (0.688) | C (0.312) | 7 |
| rs3763409 | 7 | 158217591 | T (0.801) | C (0.199) | 0 |
| rs842443 | 7 | 158263253 | T (0.738) | C (0.262) | 2 |
| rs2730237 | 7 | 158398927 | T (0.823) | C (0.177) | 0 |
| rs17754389 | 8 | 1255434 | G (0.774) | C (0.226) | 1 |
| rs2912272 | 8 | 3885737 | G (0.911) | T (0.089) | 1 |
| rs2741710 | 8 | 6751396 | G (0.679) | A (0.321) | 0 |
| rs7836248 | 8 | 11191768 | G (0.837) | A (0.163) | 0 |
| rs4831429 | 8 | 13330455 | C (0.579) | T (0.421) | 1 |
| rs1023865 | 8 | 13332518 | C (0.635) | T (0.365) | 1 |
| rs1510446 | 8 | 14567748 | T (0.508) | C (0.492) | 0 |
| rs17322021 | 8 | 14611006 | C (0.544) | G (0.456) | 8 |
| rs12681041 | 8 | 14615022 | T (0.527) | C (0.473) | 0 |
| rs972018 | 8 | 14653073 | C (0.633) | T (0.367) | 0 |
| rs2252570 | 8 | 14679483 | G (0.735) | A (0.265) | 0 |
| rs6983527 | 8 | 18504346 | G (0.954) | A (0.046) | 0 |
| rs1565142 | 8 | 18526961 | G (0.942) | C (0.058) | 0 |
| rs17051664 | 8 | 24050241 | G (0.978) | T (0.022) | 0 |
| rs7823178 | 8 | 24086776 | A (0.974) | G (0.026) | 1 |
| rs7827129 | 8 | 24086898 | T (0.981) | C (0.019) | 0 |
| rs6997885 | 8 | 24087820 | A (0.964) | G (0.036) | 0 |
| rs10106387 | 8 | 25696110 | G (0.648) | T (0.352) | 0 |
| rs11135930 | 8 | 26182054 | G (0.65) | A (0.35) | 0 |
| rs16879735 | 8 | 32649799 | G (0.921) | C (0.079) | 0 |
| rs10503925 | 8 | 32660601 | A (0.922) | T (0.078) | 0 |
| rs13273367 | 8 | 34208038 | T (0.541) | C (0.459) | 0 |
| rs7014037 | 8 | 52326067 | C (0.725) | G (0.275) | 0 |
| rs9298441 | 8 | 52352800 | C (0.696) | G (0.304) | 0 |
| rs12678378 | 8 | 53893196 | C (0.874) | T (0.126) | 0 |
| rs12677436 | 8 | 53916301 | C (0.875) | T (0.125) | 0 |
| rs2978508 | 8 | 62271508 | A (0.547) | G (0.453) | 0 |
| rs12334387 | 8 | 64709872 | A (0.547) | G (0.453) | 0 |
| rs1149464 | 8 | 64749916 | G (0.776) | T (0.224) | 0 |
| rs790564 | 8 | 64766772 | C (0.728) | A (0.272) | 0 |
| rs809339 | 8 | 64781183 | G (0.785) | A (0.215) | 0 |
| rs4737188 | 8 | 64919211 | T (0.518) | A (0.482) | 0 |
| rs12543529 | 8 | 64966773 | T (0.707) | G (0.293) | 0 |
| rs13254613 | 8 | 64967358 | A (0.639) | C (0.361) | 0 |
| rs6472127 | 8 | 65031744 | A (0.52) | G (0.48) | 4 |
| rs7001339 | 8 | 70018061 | G (0.903) | A (0.097) | 0 |
| rs7014001 | 8 | 72576843 | T (0.94) | C (0.06) | 0 |
| rs10957634 | 8 | 74059557 | C (0.652) | G (0.348) | 2 |
| rs2975842 | 8 | 74088145 | G (0.519) | A (0.481) | 0 |
| rs9886561 | 8 | 85758876 | T (0.779) | C (0.221) | 0 |
| rs39510 | 8 | 90873782 | T (0.687) | A (0.313) | 1 |
| rs39766 | 8 | 90880512 | C (0.567) | T (0.433) | 0 |
| rs40380 | 8 | 90884475 | G (0.584) | C (0.416) | 0 |
| rs4554514 | 8 | 91425144 | G (0.61) | A (0.39) | 0 |
| rs10504958 | 8 | 97028995 | C (0.764) | T (0.236) | 0 |
| rs1010824 | 8 | 108321089 | C (0.846) | T (0.154) | 3 |
| rs6469101 | 8 | 108321414 | G (0.843) | A (0.157) | 1 |
| rs2357037 | 8 | 114933324 | A (0.7) | G (0.3) | 0 |
| rs13260311 | 8 | 115905216 | G (0.758) | C (0.242) | 0 |
| rs11562770 | 8 | 119086110 | A (0.554) | G (0.446) | 0 |
| rs6993726 | 8 | 123513831 | C (0.647) | T (0.353) | 0 |
| rs4871550 | 8 | 125885031 | C (0.523) | T (0.477) | 0 |
| rs10095165 | 8 | 125885619 | G (0.521) | C (0.479) | 0 |
| rs7839993 | 8 | 127940655 | T (0.715) | C (0.285) | 0 |
| rs6985700 | 8 | 127951156 | C (0.76) | A (0.24) | 6 |
| rs1467191 | 8 | 127951377 | T (0.76) | A (0.24) | 3 |
| rs12334411 | 8 | 127952092 | C (0.76) | T (0.24) | 0 |
| rs13255292 | 8 | 129145755 | C (0.682) | T (0.318) | 0 |
| rs7820273 | 8 | 129432082 | G (0.783) | A (0.217) | 0 |
| rs9642882 | 8 | 129450806 | C (0.784) | T (0.216) | 0 |
| rs7835480 | 8 | 131593803 | A (0.687) | G (0.313) | 0 |
| rs6471231 | 8 | 135476962 | G (0.805) | A (0.195) | 0 |
| rs961369 | 8 | 137054021 | T (0.704) | A (0.296) | 0 |
| rs17547120 | 8 | 137054329 | C (0.708) | A (0.292) | 1 |
| rs2922350 | 8 | 137055200 | A (0.703) | G (0.297) | 2 |
| rs6577695 | 8 | 137056973 | G (0.71) | T (0.29) | 1 |
| rs1868368 | 8 | 137082629 | T (0.717) | C (0.283) | 0 |
| rs1448725 | 8 | 137084046 | T (0.717) | C (0.283) | 0 |
| rs10505642 | 8 | 137092988 | C (0.551) | T (0.449) | 1 |
| rs2166945 | 8 | 137093245 | C (0.551) | T (0.449) | 1 |
| rs1839179 | 8 | 137093317 | C (0.551) | G (0.449) | 0 |
| rs6994909 | 8 | 137094522 | A (0.551) | G (0.449) | 0 |
| rs4909537 | 8 | 137094596 | T (0.548) | C (0.452) | 0 |
| rs11994949 | 8 | 137102961 | G (0.611) | A (0.389) | 0 |
| rs10109871 | 8 | 137110974 | A (0.606) | G (0.394) | 0 |
| rs13266231 | 8 | 137113539 | G (0.611) | A (0.389) | 0 |
| rs12546080 | 8 | 137129495 | A (0.543) | G (0.457) | 0 |
| rs17617902 | 8 | 137187961 | G (0.645) | A (0.355) | 0 |
| rs2318337 | 8 | 139842825 | A (0.653) | G (0.347) | 1 |
| rs6577946 | 8 | 139843591 | A (0.638) | C (0.362) | 1 |
| rs11248215 | 8 | 146121517 | C (0.743) | T (0.257) | 0 |
| rs12344766 | 9 | 7424628 | A (0.801) | C (0.199) | 0 |
| rs2246687 | 9 | 7446295 | C (0.671) | T (0.329) | 1 |
| rs10976375 | 9 | 7529474 | T (0.853) | C (0.147) | 0 |
| rs2026462 | 9 | 7738664 | G (0.772) | T (0.228) | 0 |
| rs1445208 | 9 | 9152669 | T (0.645) | A (0.355) | 3 |
| rs2035987 | 9 | 14744010 | G (0.532) | A (0.468) | 2 |
| rs224021 | 9 | 20304007 | A (0.65) | G (0.35) | 0 |
| rs1412981 | 9 | 24160867 | T (0.918) | C (0.082) | 0 |
| rs16915269 | 9 | 29757803 | C (0.91) | G (0.09) | 0 |
| rs1328997 | 9 | 32793147 | A (0.925) | G (0.075) | 1 |
| rs13294227 | 9 | 37772708 | C (0.988) | T (0.012) | 0 |
| rs10116778 | 9 | 38729591 | T (0.956) | C (0.044) | 0 |
| rs11143793 | 9 | 70615043 | G (0.552) | A (0.448) | 1 |
| rs7034640 | 9 | 71067510 | C (0.748) | G (0.252) | 1 |
| rs4442205 | 9 | 72785367 | T (0.714) | G (0.286) | 0 |
| rs11143817 | 9 | 75960888 | T (0.784) | G (0.216) | 0 |
| rs11144123 | 9 | 76671799 | T (0.661) | C (0.339) | 0 |
| rs1329577 | 9 | 81802859 | A (0.809) | C (0.191) | 0 |
| rs1502683 | 9 | 84789400 | T (0.541) | C (0.459) | 0 |
| rs1393565 | 9 | 84794860 | C (0.51) | G (0.49) | 0 |
| rs12553922 | 9 | 89830581 | C (0.766) | T (0.234) | 0 |
| rs2814850 | 9 | 89988300 | G (0.503) | C (0.497) | 3 |
| rs753826 | 9 | 90065179 | C (0.812) | T (0.188) | 0 |
| rs9410428 | 9 | 90752701 | G (0.501) | T (0.499) | 0 |
| rs4877063 | 9 | 91023799 | C (0.616) | A (0.384) | 0 |
| rs9410467 | 9 | 91053269 | C (0.618) | T (0.382) | 1 |
| rs4601388 | 9 | 93587744 | A (0.958) | G (0.042) | 2 |
| rs10992504 | 9 | 94680202 | G (0.849) | A (0.151) | 3 |
| rs10992543 | 9 | 94734992 | C (0.954) | T (0.046) | 0 |
| rs2639345 | 9 | 104660705 | G (0.884) | A (0.116) | 0 |
| rs328879 | 9 | 106829073 | G (0.667) | A (0.333) | 1 |
| rs556471 | 9 | 112662034 | C (0.825) | G (0.175) | 0 |
| rs474186 | 9 | 112662287 | A (0.825) | G (0.175) | 0 |
| rs824007 | 9 | 113013602 | C (0.73) | G (0.27) | 1 |
| rs7046095 | 9 | 115518056 | C (0.714) | T (0.286) | 0 |
| rs10982427 | 9 | 116653517 | T (0.838) | C (0.162) | 0 |
| rs10982959 | 9 | 117659124 | C (0.863) | A (0.137) | 0 |
| rs2149364 | 9 | 119582809 | G (0.731) | A (0.269) | 0 |
| rs12342472 | 9 | 119582997 | T (0.735) | C (0.265) | 0 |
| rs10984642 | 9 | 121490161 | C (0.887) | T (0.113) | 0 |
| rs4837375 | 9 | 131333483 | T (0.753) | C (0.247) | 2 |
| rs4837376 | 9 | 131333575 | A (0.753) | C (0.247) | 0 |
| rs1780482 | 9 | 131333761 | C (0.753) | T (0.247) | 0 |
| rs657152 | 9 | 135129086 | G (0.658) | T (0.342) | 0 |
| rs652600 | 9 | 135300838 | T (0.665) | C (0.335) | 1 |
| rs7040581 | 9 | 135954271 | C (0.648) | G (0.352) | 1 |
| rs12339163 | 9 | 136345009 | A (0.883) | G (0.117) | 2 |
| rs7915178 | 10 | 1724473 | C (0.538) | T (0.462) | 1 |
| rs12221422 | 10 | 2666345 | C (0.918) | T (0.082) | 2 |
| rs1325586 | 10 | 3313773 | C (0.593) | T (0.407) | 0 |
| rs313438 | 10 | 4673858 | C (0.783) | A (0.217) | 0 |
| rs10904310 | 10 | 4683742 | C (0.583) | A (0.417) | 0 |
| rs11252648 | 10 | 4697250 | G (0.535) | T (0.465) | 0 |
| rs11254963 | 10 | 7115639 | C (0.93) | T (0.07) | 0 |
| rs11254971 | 10 | 7136011 | T (0.934) | C (0.066) | 1 |
| rs4749378 | 10 | 8021446 | G (0.619) | A (0.381) | 0 |
| rs11255456 | 10 | 8032624 | G (0.565) | T (0.435) | 0 |
| rs10795736 | 10 | 9791314 | T (0.578) | C (0.422) | 0 |
| rs17149684 | 10 | 11255448 | A (0.934) | G (0.066) | 0 |
| rs642349 | 10 | 11260140 | C (0.932) | G (0.068) | 0 |
| rs596406 | 10 | 11264671 | T (0.925) | C (0.075) | 0 |
| rs2815624 | 10 | 12639700 | G (0.891) | A (0.109) | 0 |
| rs11257892 | 10 | 12646058 | C (0.933) | G (0.067) | 6 |
| rs1228333 | 10 | 14369232 | C (0.538) | T (0.462) | 3 |
| rs10828176 | 10 | 21351254 | T (0.882) | G (0.118) | 2 |
| rs1148179 | 10 | 28655614 | C (0.84) | T (0.16) | 0 |
| rs1148182 | 10 | 28656425 | C (0.841) | T (0.159) | 4 |
| rs2771247 | 10 | 30675754 | C (0.725) | T (0.275) | 0 |
| rs947095 | 10 | 31099342 | C (0.787) | A (0.213) | 0 |
| rs7097979 | 10 | 32458480 | C (0.648) | T (0.352) | 0 |
| rs7091786 | 10 | 35053638 | A (0.678) | G (0.322) | 2 |
| rs1892429 | 10 | 37494403 | A (1) | G (0) | 2 |
| rs7074794 | 10 | 44726083 | C (0.863) | G (0.137) | 0 |
| rs11101285 | 10 | 49167438 | C (0.7) | T (0.3) | 0 |
| rs2221 | 10 | 51961378 | G (0.765) | A (0.235) | 0 |
| rs10740592 | 10 | 56143850 | T (0.9) | C (0.1) | 0 |
| rs17776046 | 10 | 61082207 | G (0.67) | A (0.33) | 0 |
| rs10761488 | 10 | 61775998 | A (0.767) | G (0.233) | 2 |
| rs11000150 | 10 | 73479947 | T (0.899) | G (0.101) | 0 |
| rs11000151 | 10 | 73480173 | T (0.881) | A (0.119) | 0 |
| rs11000152 | 10 | 73480408 | G (0.9) | A (0.1) | 0 |
| rs10824708 | 10 | 80467529 | G (0.932) | A (0.068) | 0 |
| rs1559554 | 10 | 82440784 | C (0.603) | T (0.397) | 0 |
| rs7069222 | 10 | 83746082 | G (0.7) | A (0.3) | 0 |
| rs7069550 | 10 | 83746268 | G (0.706) | T (0.294) | 0 |
| rs2207782 | 10 | 83747179 | C (0.7) | T (0.3) | 0 |
| rs11200841 | 10 | 85838557 | A (0.846) | T (0.154) | 0 |
| rs12768356 | 10 | 92972218 | G (0.942) | A (0.058) | 0 |
| rs7084314 | 10 | 93020188 | C (0.941) | T (0.059) | 0 |
| rs7913670 | 10 | 95013926 | A (0.78) | G (0.22) | 1 |
| rs11188956 | 10 | 98620578 | A (0.902) | G (0.098) | 1 |
| rs17112190 | 10 | 98629836 | G (0.905) | A (0.095) | 0 |
| rs7898565 | 10 | 99965717 | G (0.997) | A (0.003) | 0 |
| rsl7113768 | 10 | 102916068 | G (0.736) | A (0.264) | 0 |
| rs3802680 | 10 | 104104876 | G (0.759) | A (0.241) | 1 |
| rs1660985 | 10 | 109259862 | T (0.956) | C (0.044) | 0 |
| rs10884475 | 10 | 109323686 | T (0.949) | C (0.051) | 0 |
| rs11196100 | 10 | 114520508 | C (0.929) | G (0.071) | 0 |
| rs7914078 | 10 | 115067194 | T (0.589) | C (0.411) | 0 |
| rs17093324 | 10 | 117331154 | G (0.976) | C (0.024) | 1 |
| rs2907596 | 10 | 119819411 | A (0.999) | G (0.001) | 0 |
| rs236212 | 10 | 120108588 | A (0.753) | G (0.247) | 0 |
| rs17099092 | 10 | 121367994 | G (0.934) | A (0.066) | 1 |
| rs2279941 | 10 | 121682142 | T (0.936) | G (0.064) | 0 |
| rs4445563 | 10 | 121692000 | A (0.936) | G (0.064) | 4 |
| rs7913126 | 10 | 121917773 | G (0.831) | C (0.169) | 0 |
| rs10887005 | 10 | 123621430 | G (0.652) | C (0.348) | 0 |
| rs10887006 | 10 | 123629245 | A (0.65) | T (0.35) | 3 |
| rs1555487 | 10 | 123984146 | A (0.733) | T (0.267) | 0 |
| rs2918157 | 10 | 132906450 | T (0.526) | C (0.474) | 1 |
| rs10769353 | 11 | 5911385 | T (0.639) | C (0.361) | 0 |
| rs10839332 | 11 | 6039479 | G (0.568) | C (0.432) | 1 |
| rs11041007 | 11 | 6742577 | A (0.669) | C (0.331) | 0 |
| rs7947196 | 11 | 6749489 | G (0.786) | C (0.214) | 1 |
| rs6578793 | 11 | 6881438 | C (0.804) | T (0.196) | 1 |
| rs1507628 | 11 | 7172817 | A (0.62) | G (0.38) | 26 |
| rs4758287 | 11 | 8056489 | C (0.7) | A (0.3) | 1 |
| rs7937941 | 11 | 11322906 | A (1) | G (0) | 17 |
| rs4500466 | 11 | 11998072 | A (0.627) | G (0.373) | 0 |
| rs1945625 | 11 | 15112676 | G (0.572) | T (0.428) | 1 |
| rs4757560 | 11 | 17646738 | G (0.716) | C (0.284) | 0 |
| rs4757568 | 11 | 17672467 | A (0.68) | G (0.32) | 0 |
| rs11825693 | 11 | 18791150 | C (0.681) | G (0.319) | 5 |
| rs3935980 | 11 | 18815116 | A (0.793) | G (0.207) | 1 |
| rs2716454 | 11 | 24710115 | T (0.822) | C (0.178) | 0 |
| rs2129354 | 11 | 24735537 | C (0.71) | A (0.29) | 0 |
| rs622744 | 11 | 30179213 | A (0.624) | G (0.376) | 0 |
| rs2658577 | 11 | 32024752 | G (0.869) | A (0.131) | 0 |
| rs224631 | 11 | 32043193 | A (0.878) | G (0.122) | 1 |
| rs12221768 | 11 | 58662689 | A (0.987) | G (0.013) | 0 |
| rs526934 | 11 | 59390069 | A (0.74) | G (0.26) | 2 |
| rs1792942 | 11 | 61655137 | T (0.586) | C (0.414) | 1 |
| rs1675062 | 11 | 61655435 | C (0.625) | T (0.375) | 0 |
| rs7102454 | 11 | 65351396 | T (0.663) | C (0.337) | 1 |
| rs1385600 | 11 | 77613814 | T (0.804) | C (0.196) | 0 |
| rs4945261 | 11 | 77667908 | G (0.813) | A (0.187) | 0 |
| rs10793294 | 11 | 77674051 | A (0.731) | C (0.269) | 0 |
| rs11602622 | 11 | 77688478 | A (0.811) | G (0.189) | 2 |
| rs7115850 | 11 | 77722719 | G (0.802) | C (0.198) | 0 |
| rs2373115 | 11 | 77768798 | G (0.802) | T (0.198) | 0 |
| rs1982733 | 11 | 83372736 | G (0.933) | A (0.067) | 0 |
| rs7120017 | 11 | 96230931 | T (0.713) | C (0.287) | 0 |
| rs7927859 | 11 | 100888916 | G (0.546) | A (0.454) | 0 |
| rs10895127 | 11 | 100889822 | G (0.546) | A (0.454) | 0 |
| rs4285844 | 11 | 100893168 | C (0.543) | G (0.457) | 0 |
| rs11226015 | 11 | 103169269 | A (0.859) | G (0.141) | 1 |
| rs900517 | 11 | 103175016 | T (0.857) | C (0.143) | 1 |
| rs1488956 | 11 | 103735129 | T (0.832) | C (0.168) | 2 |
| rs10791697 | 11 | 103744339 | T (0.832) | C (0.168) | 0 |
| rs2155045 | 11 | 105143740 | A (0.623) | C (0.377) | 0 |
| rs11226856 | 11 | 105204258 | A (0.622) | G (0.378) | 1 |
| rs2513606 | 11 | 105823275 | T (0.78) | C (0.22) | 1 |
| rs7948284 | 11 | 107088095 | G (0.69) | A (0.31) | 0 |
| rs11215271 | 11 | 114272263 | C (0.842) | T (0.158) | 0 |
| rs4142640 | 11 | 117109417 | G (0.964) | A (0.036) | 0 |
| rs516678 | 11 | 120544119 | A (0.998) | C (0.002) | 1 |
| rs11828777 | 11 | 122551915 | C (0.565) | T (0.435) | 0 |
| rs7103696 | 11 | 123195003 | G (0.577) | C (0.423) | 0 |
| rs601813 | 11 | 124511563 | A (0.856) | G (0.144) | 1 |
| rs10893678 | 11 | 126782050 | C (0.803) | T (0.197) | 0 |
| rs691197 | 11 | 129124330 | A (0.635) | G (0.365) | 1 |
| rs2434936 | 11 | 129124497 | G (0.643) | A (0.357) | 1 |
| rs948083 | 11 | 130137398 | G (0.964) | A (0.036) | 0 |
| rs6590761 | 11 | 134093450 | A (0.791) | C (0.209) | 2 |
| rs11063263 | 12 | 61880 | A (0.534) | G (0.466) | 2 |
| rs635332 | 12 | 3256534 | G (0.666) | A (0.334) | 1 |
| rs10849208 | 12 | 5127842 | A (0.845) | G (0.155) | 0 |
| rs11063543 | 12 | 5142385 | A (0.839) | C (0.161) | 0 |
| rs11615654 | 12 | 5145032 | G (0.575) | A (0.425) | 0 |
| rs7303158 | 12 | 5145801 | T (0.569) | C (0.431) | 0 |
| rs4930739 | 12 | 5582739 | A (0.696) | G (0.304) | 0 |
| rs3210140 | 12 | 7546404 | C (0.7) | T (0.3) | 0 |
| rs2416673 | 12 | 11477909 | C (0.5) | T (0.5) | 0 |
| rs2416671 | 12 | 11478200 | T (0.501) | C (0.499) | 0 |
| rs2965710 | 12 | 11478437 | C (0.528) | T (0.472) | 0 |
| rs11055699 | 12 | 14020103 | C (0.934) | T (0.066) | 0 |
| rs10846461 | 12 | 17089175 | C (0.858) | T (0.142) | 0 |
| rs7974666 | 12 | 17135652 | G (0.753) | A (0.247) | 5 |
| rs12370697 | 12 | 21139891 | T (0.825) | G (0.175) | 2 |
| rs17328763 | 12 | 21173837 | T (0.823) | C (0.177) | 0 |
| rs5484 | 12 | 21422704 | C (0.835) | T (0.165) | 1 |
| rs12825420 | 12 | 22066058 | C (0.703) | G (0.297) | 3 |
| rs10844025 | 12 | 31882773 | T (0.634) | G (0.366) | 0 |
| rs2128614 | 12 | 31890261 | A (0.633) | T (0.367) | 0 |
| rs7303356 | 12 | 33524866 | C (0.562) | G (0.438) | 1 |
| rs17128779 | 12 | 39454516 | G (0.906) | A (0.094) | 2 |
| rs281839 | 12 | 39831495 | T (0.642) | G (0.358) | 0 |
| rs2567034 | 12 | 39831992 | A (0.68) | G (0.32) | 0 |
| rs281843 | 12 | 39832839 | G (0.64) | A (0.36) | 0 |
| rs6582240 | 12 | 39866444 | T (0.641) | A (0.359) | 0 |
| rs2043134 | 12 | 39888622 | G (0.662) | A (0.338) | 1 |
| rs1922765 | 12 | 41883491 | G (0.716) | A (0.284) | 2 |
| rs10880439 | 12 | 41898082 | T (0.793) | C (0.207) | 1 |
| rs11525670 | 12 | 44737959 | C (0.557) | T (0.443) | 1 |
| rs3911501 | 12 | 50819085 | A (0.929) | G (0.071) | 0 |
| rs1488047 | 12 | 53689461 | C (0.872) | A (0.128) | 0 |
| rs11172831 | 12 | 57618799 | A (0.89) | T (0.11) | 0 |
| rs11172837 | 12 | 57631844 | A (0.886) | G (0.114) | 0 |
| rs7969029 | 12 | 62405176 | G (0.512) | C (0.488) | 0 |
| rs10878938 | 12 | 67872706 | C (0.76) | T (0.24) | 0 |
| rs1807989 | 12 | 71360679 | A (0.684) | C (0.316) | 9 |
| rs10879910 | 12 | 74090951 | G (0.552) | A (0.448) | 1 |
| rs10862477 | 12 | 75658282 | C (0.641) | G (0.359) | 0 |
| rs1382635 | 12 | 76931673 | C (0.542) | T (0.458) | 0 |
| rs17008548 | 12 | 80346893 | G (1) | A (0) | 0 |
| rs2246396 | 12 | 80970103 | C (0.738) | T (0.262) | 0 |
| rs2246421 | 12 | 80970551 | C (0.819) | T (0.181) | 1 |
| rs1897636 | 12 | 80971899 | G (0.738) | A (0.262) | 1 |
| rs1511591 | 12 | 82586860 | G (0.542) | T (0.458) | 0 |
| rs2520511 | 12 | 89529284 | A (0.676) | G (0.324) | 0 |
| rs10431437 | 12 | 95602230 | C (0.812) | G (0.188) | 0 |
| rs4762359 | 12 | 96031673 | G (0.609) | T (0.391) | 1 |
| rs11111589 | 12 | 102375658 | T (0.85) | C (0.15) | 0 |
| rs752471 | 12 | 104130800 | A (0.559) | G (0.441) | 0 |
| rs10861372 | 12 | 104202653 | T (0.717) | C (0.283) | 2 |
| rs11112447 | 12 | 104206462 | G (0.715) | A (0.285) | 0 |
| rs12228258 | 12 | 104206732 | T (0.716) | C (0.284) | 2 |
| rs4578495 | 12 | 104210151 | C (0.716) | A (0.284) | 0 |
| rs1426466 | 12 | 106296697 | G (0.783) | A (0.217) | 0 |
| rs1863508 | 12 | 107079969 | C (0.821) | T (0.179) | 4 |
| rs7310199 | 12 | 107096646 | A (0.823) | G (0.177) | 0 |
| rs10774613 | 12 | 110034548 | T (0.603) | C (0.397) | 1 |
| rs1247932 | 12 | 113177514 | C (0.662) | T (0.338) | 0 |
| rs12302135 | 12 | 113191009 | C (0.736) | T (0.264) | 0 |
| rs4298970 | 12 | 118053074 | G (0.685) | A (0.315) | 0 |
| rs11612464 | 12 | 125126042 | G (0.716) | A (0.284) | 1 |
| rs9943743 | 12 | 126284872 | C (0.687) | T (0.313) | 0 |
| rs2932174 | 13 | 18963041 | A (0.999) | C (0.001) | 3 |
| rs9315353 | 13 | 19627842 | T (0.661) | C (0.339) | 0 |
| rs4769957 | 13 | 19630487 | G (0.659) | A (0.341) | 0 |
| rs1323889 | 13 | 21087435 | A (0.615) | C (0.385) | 114 |
| rs3117963 | 13 | 21432122 | A (0.824) | C (0.176) | 0 |
| rs17331447 | 13 | 22990328 | G (0.911) | C (0.089) | 0 |
| rs9507300 | 13 | 23758240 | G (0.809) | A (0.191) | 0 |
| rs3899581 | 13 | 28476098 | A (1) | G (0) | 2 |
| rs9508822 | 13 | 30179695 | A (0.954) | T (0.046) | 0 |
| rs17074898 | 13 | 30183532 | G (0.954) | A (0.046) | 0 |
| rs9566215 | 13 | 36841825 | C (0.509) | T (0.491) | 1 |
| rs4941866 | 13 | 36880118 | T (0.56) | C (0.44) | 2 |
| rs1853329 | 13 | 36880413 | C (0.558) | T (0.442) | 1 |
| rs7323267 | 13 | 40102015 | T (0.799) | C (0.201) | 0 |
| rs7325144 | 13 | 40972712 | T (0.502) | A (0.498) | 4 |
| rs9567011 | 13 | 42117467 | G (0.705) | A (0.295) | 0 |
| rs9533256 | 13 | 42302854 | C (0.525) | T (0.475) | 6 |
| rs9533832 | 13 | 43830514 | C (0.729) | T (0.271) | 8 |
| rs6561363 | 13 | 46734039 | A (0.976) | C (0.024) | 0 |
| rs9535083 | 13 | 48282047 | G (0.513) | C (0.487) | 13 |
| rs9536559 | 13 | 53353061 | T (0.738) | C (0.262) | 2 |
| rs7998997 | 13 | 56149616 | T (0.616) | C (0.384) | 0 |
| rs9537493 | 13 | 56333987 | G (0.623) | A (0.377) | 2 |
| rs9569813 | 13 | 57558569 | C (0.769) | G (0.231) | 0 |
| rs1986272 | 13 | 61914887 | A (0.69) | G (0.31) | 1 |
| rs1445204 | 13 | 62010635 | G (0.703) | T (0.297) | 1 |
| rs9572529 | 13 | 70142290 | T (0.757) | C (0.243) | 1 |
| rs1337985 | 13 | 72032913 | T (0.652) | C (0.348) | 0 |
| rs1929734 | 13 | 75691821 | C (0.754) | T (0.246) | 1 |
| rs4884068 | 13 | 76965482 | G (0.791) | C (0.209) | 0 |
| rs9574076 | 13 | 76983280 | G (0.854) | A (0.146) | 2 |
| rs2256157 | 13 | 77820106 | T (0.622) | A (0.378) | 1 |
| rs2329072 | 13 | 77858815 | T (0.609) | C (0.391) | 0 |
| rs1176291 | 13 | 80104028 | G (0.902) | C (0.098) | 0 |
| rs7317522 | 13 | 81290693 | C (0.597) | T (0.403) | 0 |
| rs4112703 | 13 | 81378893 | G (0.653) | T (0.347) | 0 |
| rs2329247 | 13 | 82160171 | G (0.878) | A (0.122) | 0 |
| rs1412852 | 13 | 83029226 | C (0.607) | T (0.393) | 5 |
| rs9635051 | 13 | 83307071 | C (0.801) | T (0.199) | 0 |
| rs10775092 | 13 | 89304509 | T (0.719) | A (0.281) | 0 |
| rs4324007 | 13 | 92040264 | T (0.716) | A (0.284) | 0 |
| rs 160823 | 13 | 92556862 | A (0.533) | G (0.467) | 0 |
| rs9523932 | 13 | 92586357 | A (0.531) | G (0.469) | 0 |
| rs7995706 | 13 | 93030876 | A (0.876) | G (0.124) | 1 |
| rs11070146 | 13 | 95098788 | C (0.895) | G (0.105) | 0 |
| rs11070147 | 13 | 95098938 | C (0.895) | G (0.105) | 1 |
| rs2793711 | 13 | 97211632 | G (0.785) | A (0.215) | 1 |
| rs285032 | 13 | 97584533 | T (0.528) | C (0.472) | 1 |
| rs9513515 | 13 | 98394451 | A (0.605) | G (0.395) | 0 |
| rs9557290 | 13 | 99206337 | A (0.878) | G (0.122) | 0 |
| rs673892 | 13 | 100338016 | G (0.582) | A (0.418) | 0 |
| rs558322 | 13 | 108617962 | A (0.797) | G (0.203) | 0 |
| rs7338868 | 13 | 113479759 | A (0.6) | C (0.4) | 4 |
| rs7399637 | 13 | 113586171 | G (0.597) | A (0.403) | 5 |
| rs1953993 | 14 | 19590795 | C (0.555) | T (0.445) | 0 |
| rs9322961 | 14 | 20822050 | G (0.738) | A (0.262) | 0 |
| rs8009917 | 14 | 25016313 | G (0.596) | A (0.404) | 1 |
| rs10483301 | 14 | 25046977 | C (0.584) | G (0.416) | 0 |
| rs1103563 | 14 | 25565566 | T (0.624) | C (0.376) | 0 |
| rs17097735 | 14 | 30648513 | C (0.858) | T (0.142) | 1 |
| rs6571396 | 14 | 30750978 | G (0.593) | T (0.407) | 0 |
| rs1956964 | 14 | 36070532 | C (0.78) | T (0.22) | 0 |
| rs1953503 | 14 | 40136834 | C (0.587) | T (0.413) | 0 |
| rs1959270 | 14 | 40138973 | A (0.589) | T (0.411) | 2 |
| rs10140209 | 14 | 40139964 | C (0.588) | T (0.412) | 1 |
| rs2415627 | 14 | 40152666 | T (0.587) | C (0.413) | 0 |
| rs2415628 | 14 | 40152757 | C (0.587) | T (0.413) | 0 |
| rs7155547 | 14 | 40153278 | G (0.587) | T (0.413) | 0 |
| rs17605241 | 14 | 40633859 | A (0.525) | G (0.475) | 0 |
| rs1037298 | 14 | 42504914 | G (0.796) | T (0.204) | 1 |
| rs7151781 | 14 | 42556718 | T (0.63) | C (0.37) | 1 |
| rs12879964 | 14 | 42756711 | C (0.926) | T (0.074) | 2 |
| rs8009311 | 14 | 42896422 | A (0.729) | G (0.271) | 2 |
| rs2146228 | 14 | 43524696 | C (0.755) | G (0.245) | 0 |
| rs10873028 | 14 | 48964500 | C (0.966) | T (0.034) | 1 |
| rs2071047 | 14 | 53488161 | C (0.578) | T (0.422) | 0 |
| rs178497 | 14 | 57598866 | C (0.817) | A (0.183) | 0 |
| rs10148587 | 14 | 59758913 | C (0.685) | T (0.315) | 0 |
| rs17102844 | 14 | 65213533 | A (0.97) | G (0.03) | 0 |
| rs9323464 | 14 | 65240886 | G (0.97) | A (0.03) | 0 |
| rs10132765 | 14 | 65312067 | A (0.891) | G (0.109) | 0 |
| rs2195103 | 14 | 80485215 | T (0.577) | C (0.423) | 0 |
| rs2268451 | 14 | 80493871 | T (0.571) | G (0.429) | 0 |
| rs12590408 | 14 | 84945827 | C (0.659) | T (0.341) | 0 |
| rs1286920 | 14 | 89609412 | G (0.562) | C (0.438) | 0 |
| rs4900107 | 14 | 91823565 | C (0.822) | A (0.178) | 2 |
| rs2369898 | 14 | 96308969 | C (0.562) | T (0.438) | 0 |
| rs2057191 | 14 | 98204264 | C (0.554) | T (0.446) | 0 |
| rs7177398 | 15 | 23614127 | C (0.698) | A (0.302) | 0 |
| rs8031119 | 15 | 23940780 | T (0.919) | C (0.081) | 2 |
| rs7177955 | 15 | 23972678 | A (0.714) | C (0.286) | 0 |
| rs11636093 | 15 | 34131083 | C (0.682) | T (0.318) | 0 |
| rs1453960 | 15 | 34605105 | G (0.912) | A (0.088) | 0 |
| rs8025834 | 15 | 35428810 | A (0.692) | G (0.308) | 0 |
| rs17447128 | 15 | 35593647 | G (0.622) | A (0.378) | 0 |
| rs2929644 | 15 | 36824627 | T (0.913) | C (0.087) | 1 |
| rs4622487 | 15 | 37496071 | T (0.753) | G (0.247) | 1 |
| rs8030833 | 15 | 37931532 | G (0.604) | A (0.396) | 0 |
| rs2278860 | 15 | 41446338 | A (0.862) | G (0.138) | 0 |
| rs1814538 | 15 | 41547066 | A (0.865) | G (0.135) | 0 |
| rs2899383 | 15 | 43884343 | G (0.633) | A (0.367) | 8 |
| rs16973929 | 15 | 45183002 | T (0.958) | C (0.042) | 2 |
| rs11070753 | 15 | 48226570 | G (0.701) | C (0.299) | 0 |
| rs3794541 | 15 | 50228603 | T (0.661) | C (0.339) | 0 |
| rs4775110 | 15 | 57103176 | G (0.636) | C (0.364) | 1 |
| rs902117 | 15 | 66099717 | A (0.502) | G (0.498) | 0 |
| rs17308514 | 15 | 66100225 | A (0.501) | G (0.499) | 0 |
| rs8028430 | 15 | 72632798 | A (0.829) | G (0.171) | 0 |
| rs4073149 | 15 | 72685472 | C (0.877) | T (0.123) | 0 |
| rs6495126 | 15 | 72962079 | G (0.713) | A (0.287) | 0 |
| rs11857574 | 15 | 83638964 | G (0.651) | A (0.349) | 2 |
| rs10520676 | 15 | 86510642 | G (0.792) | A (0.208) | 0 |
| rs8025158 | 15 | 86514696 | G (0.781) | A (0.219) | 0 |
| rs1545143 | 15 | 91070944 | G (0.717) | A (0.283) | 0 |
| rs7494848 | 15 | 91109026 | A (0.581) | C (0.419) | 0 |
| rs10163001 | 15 | 91825462 | T (0.606) | C (0.394) | 5 |
| rs4777893 | 15 | 91825747 | G (0.608) | A (0.392) | 0 |
| rs1026453 | 15 | 93377084 | C (0.739) | T (0.261) | 0 |
| rs17637117 | 15 | 93705068 | T (0.925) | G (0.075) | 0 |
| rs1369941 | 15 | 94066648 | T (0.52) | C (0.48) | 0 |
| rs2639193 | 15 | 94084399 | G (0.579) | A (0.421) | 1 |
| rs2639185 | 15 | 94185748 | T (0.676) | C (0.324) | 0 |
| rs9920491 | 15 | 96417284 | T (0.528) | C (0.472) | 1 |
| rs1383149 | 15 | 96427654 | A (0.527) | G (0.473) | 1 |
| rs2089 | 15 | 96428340 | C (0.69) | T (0.31) | 1 |
| rs4638553 | 15 | 98893149 | G (0.513) | A (0.487) | 0 |
| rs12438080 | 15 | 98899586 | C (0.516) | A (0.484) | 1 |
| rs9788652 | 15 | 98899692 | C (0.517) | G (0.483) | 3 |
| rs12916234 | 15 | 99347739 | C (0.613) | A (0.387) | 0 |
| rs516667 | 15 | 100021554 | T (0.699) | C (0.301) | 0 |
| rs8046007 | 16 | 3945522 | T (0.51) | C (0.49) | 0 |
| rs4786153 | 16 | 7391240 | T (0.815) | G (0.185) | 0 |
| rs4352066 | 16 | 7391727 | T (0.813) | G (0.187) | 0 |
| rs1532927 | 16 | 8014023 | G (0.6) | A (0.4) | 0 |
| rs9922383 | 16 | 10065179 | G (0.898) | A (0.102) | 0 |
| rs7188291 | 16 | 10068537 | A (0.898) | G (0.102) | 0 |
| rs17671033 | 16 | 10068727 | G (0.898) | A (0.102) | 0 |
| rs13336632 | 16 | 10078155 | A (0.896) | C (0.104) | 3 |
| rs194355 | 16 | 10159292 | A (0.668) | G (0.332) | 1 |
| rs12596456 | 16 | 10467336 | A (0.728) | G (0.272) | 0 |
| rs 17229044 | 16 | 10970437 | C (0.792) | T (0.208) | 0 |
| rs4567695 | 16 | 19025362 | A (0.609) | G (0.391) | 0 |
| rs12596903 | 16 | 20162376 | T (0.86) | C (0.14) | 0 |
| rs11074446 | 16 | 20162624 | T (0.86) | C (0.14) | 0 |
| rs 105284 | 16 | 22730109 | A (0.769) | T (0.231) | 0 |
| rs12447130 | 16 | 49031626 | A (0.932) | G (0.068) | 1 |
| rs2193097 | 16 | 49693901 | T (0.773) | C (0.227) | 1 |
| rs8061957 | 16 | 49694053 | C (0.773) | T (0.227) | 0 |
| rs1558680 | 16 | 50123656 | G (0.752) | C (0.248) | 0 |
| rs3095634 | 16 | 52098891 | G (0.876) | A (0.124) | 0 |
| rs11076397 | 16 | 59210721 | T (0.66) | A (0.34) | 0 |
| rs 10500495 | 16 | 62768048 | G (0.871) | C (0.129) | 0 |
| rs7199582 | 16 | 65678216 | T (0.92) | A (0.08) | 0 |
| rs11075953 | 16 | 71557266 | A (0.659) | G (0.341) | 2 |
| rs7199343 | 16 | 71566525 | A (0.702) | T (0.298) | 0 |
| rs10852516 | 16 | 71567199 | T (0.682) | C (0.318) | 0 |
| rs8059315 | 16 | 73063948 | G (0.816) | C (0.184) | 0 |
| rs7197903 | 16 | 77172009 | C (0.7) | T (0.3) | 32 |
| rs8064208 | 16 | 79005790 | A (0.547) | T (0.453) | 0 |
| rs11642541 | 16 | 79006741 | T (0.501) | G (0.499) | 0 |
| rs8059530 | 16 | 79717993 | A (0.552) | G (0.448) | 0 |
| rs9941266 | 16 | 80381806 | C (0.617) | A (0.383) | 0 |
| rs17679500 | 16 | 81865050 | T (0.93) | C (0.07) | 2 |
| rs4238696 | 16 | 82150264 | C (0.778) | T (0.222) | 0 |
| rs867425 | 16 | 83256233 | A (0.82) | C (0.18) | 0 |
| rs9937119 | 16 | 84633042 | C (0.545) | T (0.455) | 1 |
| rs12149906 | 16 | 85608124 | G (0.999) | A (0.001) | 42 |
| rs9940496 | 16 | 86774393 | T (0.887) | C (0.113) | 3 |
| rs9907608 | 17 | 5844015 | C (0.821) | T (0.179) | 0 |
| rs9303196 | 17 | 5848167 | T (0.852) | A (0.148) | 0 |
| rs10521231 | 17 | 13455247 | C (0.796) | G (0.204) | 0 |
| rs1125373 | 17 | 13507806 | T (0.837) | G (0.163) | 0 |
| rs2058226 | 17 | 14680628 | C (1) | T (0) | 72 |
| rs8073642 | 17 | 15135200 | A (0.671) | T (0.329) | 0 |
| rs9906280 | 17 | 24771580 | T (0.726) | C (0.274) | 1 |
| rs8079280 | 17 | 27942295 | T (0.514) | C (0.486) | 0 |
| rs321153 | 17 | 27972666 | T (0.54) | A (0.46) | 1 |
| rs7213646 | 17 | 28773841 | C (0.612) | T (0.388) | 1 |
| rs7208206 | 17 | 49828551 | T (0.971) | G (0.029) | 0 |
| rs8079872 | 17 | 72627221 | A (0.685) | G (0.315) | 2 |
| rs4798773 | 18 | 922503 | G (0.518) | C (0.482) | 0 |
| rs7226622 | 18 | 21045556 | C (0.867) | T (0.133) | 0 |
| rs17703962 | 18 | 22886548 | A (0.856) | G (0.144) | 1 |
| rs1231268 | 18 | 23944009 | C (0.831) | T (0.169) | 0 |
| rs1997021 | 18 | 25930660 | A (0.779) | G (0.221) | 4 |
| rs9950246 | 18 | 27203850 | T (0.721) | C (0.279) | 0 |
| rs1031732 | 18 | 27209158 | C (0.723) | T (0.277) | 4 |
| rs3859361 | 18 | 27218632 | T (0.716) | C (0.284) | 0 |
| rs10502673 | 18 | 33316161 | G (0.751) | A (0.249) | 0 |
| rs4611540 | 18 | 33740912 | T (0.59) | C (0.41) | 0 |
| rs4799526 | 18 | 35940745 | T (0.813) | C (0.187) | 4 |
| rs8094466 | 18 | 38436886 | T (0.585) | C (0.415) | 0 |
| rs1944405 | 18 | 38783085 | A (0.907) | T (0.093) | 0 |
| rs4986228 | 18 | 42849257 | A (0.624) | G (0.376) | 1 |
| rs3813086 | 18 | 46442790 | G (0.745) | A (0.255) | 0 |
| rs7227964 | 18 | 47836380 | A (0.609) | G (0.391) | 0 |
| rs17747515 | 18 | 48138948 | C (0.692) | T (0.308) | 1 |
| rs1942103 | 18 | 48392344 | T (0.983) | G (0.017) | 0 |
| rs12456800 | 18 | 52268092 | T (0.79) | C (0.21) | 2 |
| rs629737 | 18 | 53301269 | A (0.646) | C (0.354) | 0 |
| rs630250 | 18 | 53301411 | T (0.647) | C (0.353) | 1 |
| rs2279096 | 18 | 53308956 | A (0.645) | G (0.355) | 0 |
| rs8098520 | 18 | 53320727 | A (0.765) | G (0.235) | 1 |
| rs11152081 | 18 | 54364418 | G (0.664) | A (0.336) | 3 |
| rs4327152 | 18 | 59994664 | C (0.64) | T (0.36) | 0 |
| rs1618942 | 18 | 60864766 | C (0.904) | G (0.096) | 0 |
| rs470490 | 18 | 61182216 | T (0.62) | G (0.38) | 0 |
| rs2850696 | 18 | 61209698 | T (0.751) | C (0.249) | 0 |
| rs2587427 | 18 | 61739825 | G (0.637) | A (0.363) | 1 |
| rs6566183 | 18 | 61764669 | C (0.679) | T (0.321) | 0 |
| rs8091446 | 18 | 62834159 | C (0.641) | T (0.359) | 0 |
| rs1942200 | 18 | 66856528 | G (0.873) | A (0.127) | 0 |
| rs1879682 | 18 | 67648221 | T (0.653) | A (0.347) | 0 |
| rs1893626 | 18 | 67862047 | G (0.629) | C (0.371) | 1 |
| rs4892005 | 18 | 68225792 | A (0.798) | T (0.202) | 0 |
| rs6566673 | 18 | 68661887 | C (0.902) | T (0.098) | 0 |
| rs4891489 | 18 | 68662302 | G (0.635) | A (0.365) | 0 |
| rs8098624 | 18 | 68669199 | C (0.681) | T (0.319) | 0 |
| rs1109070 | 18 | 68674559 | A (0.696) | G (0.304) | 0 |
| rs 17797002 | 18 | 68719599 | T (0.768) | C (0.232) | 0 |
| rs12959128 | 18 | 68757575 | T (0.564) | G (0.436) | 1 |
| rs12959259 | 18 | 68757648 | T (0.564) | G (0.436) | 0 |
| rs2469005 | 18 | 68861758 | C (0.787) | T (0.213) | 0 |
| rs1108149 | 18 | 68864609 | G (0.617) | A (0.383) | 0 |
| rs8099793 | 18 | 70629026 | C (0.733) | T (0.267) | 3 |
| rs2628125 | 18 | 70722593 | T (0.554) | A (0.446) | 0 |
| rs8085262 | 18 | 70725167 | C (0.711) | A (0.289) | 0 |
| rs4891199 | 18 | 70729556 | C (0.717) | T (0.283) | 0 |
| rs486131 | 18 | 72775661 | T (0.71) | C (0.29) | 0 |
| rs9305032 | 19 | 8644378 | C (0.896) | T (0.104) | 1 |
| rs2139528 | 19 | 21630954 | T (0.818) | C (0.182) | 1 |
| rs1125039 | 19 | 34402560 | T (0.589) | A (0.411) | 1 |
| rs17834073 | 19 | 38658293 | T (0.854) | C (0.146) | 0 |
| rs967987 | 19 | 40030096 | A (0.539) | G (0.461) | 0 |
| rs1836011 | 19 | 44765798 | G (0.746) | A (0.254) | 0 |
| rs10853822 | 19 | 55939506 | A (0.754) | G (0.246) | 1 |
| rs1803254 | 19 | 56434956 | G (0.902) | C (0.098) | 0 |
| rs8103085 | 19 | 56435857 | G (0.905) | T (0.095) | 4 |
| rs10412123 | 19 | 56454393 | T (0.896) | C (0.104) | 1 |
| rs4802789 | 19 | 56545280 | T (0.827) | C (0.173) | 0 |
| rs10853838 | 19 | 56858647 | C (0.723) | T (0.277) | 2 |
| rs4802895 | 19 | 57330646 | T (0.58) | C (0.42) | 2 |
| rs2074059 | 19 | 62677272 | A (0.636) | T (0.364) | 0 |
| rs2074060 | 19 | 62677378 | C (0.636) | G (0.364) | 0 |
| rs6108256 | 20 | 909527 | A (0.924) | C (6.076) | 0 |
| rs202479 | 20 | 1627534 | A (0.634) | G (0.366) | 0 |
| rs202478 | 20 | 1627805 | A (0.635) | G (0.365) | 0 |
| rs1438099 | 20 | 1629670 | A (0.887) | T (0.113) | 1 |
| rs1438098 | 20 | 1629929 | T (0.888) | C (0.112) | 6 |
| rs6045666 | 20 | 1888504 | T (0.682) | C (0.318) | 1 |
| rs6136667 | 20 | 1926301 | G (0.829) | C (0.171) | 0 |
| rs4815645 | 20 | 4024588 | T (0.781) | G (0.219) | 0 |
| rs6035292 | 20 | 19239116 | A (0.96) | G (0.04) | 0 |
| rs6046024 | 20 | 19294184 | T (0.938) | C (0.062) | 0 |
| rs3790285 | 20 | 19590681 | G (0.68) | T (0.32) | 5 |
| rs200184 | 20 | 19999890 | C (0.522) | T (0.478) | 1 |
| rs6136938 | 20 | 20006992 | G (0.612) | A (0.388) | 0 |
| rs6138482 | 20 | 25007442 | C (0.781) | T (0.219) | 1 |
| rs6011973 | 20 | 35397612 | C (0.973) | T (0.027) | 1 |
| rs6093784 | 20 | 41224584 | T (0.589) | C (0.411) | 0 |
| rs6073299 | 20 | 42109444 | G (0.731) | A (0.269) | 0 |
| rs6017252 | 20 | 42112605 | G (0.698) | A (0.302) | 0 |
| rs736232 | 20 | 49346133 | T (0.723) | C (0.277) | 0 |
| rs6015215 | 20 | 56178326 | C (0.616) | T (0.384) | 1 |
| rs1555364 | 20 | 57644265 | T (0.747) | C (0.253) | 2 |
| rs1006644 | 20 | 59663684 | C (0.8) | T (0.2) | 2 |
| rs926184 | 21 | 15267502 | G (0.979) | A (0.021) | 0 |
| rs1474513 | 21 | 15587206 | C (0.804) | T (0.196) | 3 |
| rs7279678 | 21 | 15608029 | T (0.857) | C (0.143) | 5 |
| rs197586 | 21 | 18246841 | C (0.726) | T (0.274) | 0 |
| rs12483463 | 21 | 18279661 | A (0.64) | G (0.36) | 2 |
| rs2826415 | 21 | 20948523 | A (0.749) | G (0.251) | 0 |
| rs2826432 | 21 | 20955734 | G (0.834) | A (0.166) | 0 |
| rs1156324 | 21 | 20956024 | A (0.836) | T (0.164) | 1 |
| rs2826436 | 21 | 20958402 | A (0.856) | T (0.144) | 0 |
| rs2826443 | 21 | 20963774 | T (0.851) | G (0.149) | 0 |
| rs2826472 | 21 | 20977898 | C (0.843) | G (0.157) | 1 |
| rs1910608 | 21 | 24517741 | C (0.672) | T (0.328) | 1 |
| rs12483123 | 21 | 24603321 | C (0.683) | T (0.317) | 0 |
| rs2832878 | 21 | 30738720 | C (0.565) | A (0.435) | 0 |
| rs1892861 | 21 | 30740565 | A (0.563) | G (0.437) | 1 |
| rs2252898 | 21 | 30741247 | A (0.563) | T (0.437) | 0 |
| rs7281360 | 21 | 30753600 | C (0.563) | T (0.437) | 1 |
| rs2832892 | 21 | 30753928 | T (0.562) | C (0.438) | 1 |
| rs2832896 | 21 | 30755532 | A (0.563) | G (0.437) | 1 |
| rs2832907 | 21 | 30770217 | T (0.561) | G (0.439) | 1 |
| rs2832908 | 21 | 30774051 | T (0.564) | C (0.436) | 4 |
| rs2244305 | 21 | 33574828 | C (0.603) | T (0.397) | 2 |
| rs2065317 | 21 | 40174501 | C (0.645) | T (0.355) | 1 |
| rs7575 | 22 | 17803250 | G (0.867) | A (0.133) | 0 |
| rs8136108 | 22 | 47211457 | G (0.527) | A (0.473) | 0 |
| rs5768736 | 22 | 47347102 | C (0.58) | T (0.42) | 1 |
| rs2688098 | 22 | 48068960 | T (0.604) | C (0.396) | 1 |

### TABLE S2

Table S2 (Supplementary Table 2) below shows the Chromosomal Location, Allele Frequencies and Haplotype Blocks of 137 Genotyped SNPs in the Follow-up Study Derived from Haplotype Analysis.

**Table S2. Chromosomal Location, Allele Frequencies and Haplotype Blocks of 137 Genotyped SNPs in the Follow-up Study Derived from Haplotype Analysis.**

| **Haplotype block** | **SNP ID** | **Chr** | **Location** | **allele1(freq)** | **allele2(freq)** | **samples failed** |
|---|---|---|---|---|---|---|
| 1 | rs11208183 | 1 | 63542669 | A (0.901) | T (0.099) | 27 |
| 1 | rs6675284 | 1 | 63590981 | C (0.596) | T (0.404) | 117 |
| 1 | rs10789136 | 1 | 63602934 | A (0.511) | C (0.489) | 29 |
| 2 | rs6681513 | 1 | 179308984 | C (0.624) | G (0.376) | 29 |
| 2 | rs9787100 | 1 | 179310895 | A (0.758) | G (0.242) | 36 |
| 2 | rs3845424 | 1 | 179404443 | T (1) | C (0) | 25 |
| 3 | rs6704572 | 2 | 2885615 | A (0.71) | G (0.29) | 114 |
| 3 | rs1448621 | 2 | 2908588 | T (0.91) | A (0.09) | 28 |
| 3 | rs1463146 | 2 | 2913362 | G (0.908) | A (0.092) | 15 |
| 3 | rs2385432 | 2 | 2919128 | G (0.848) | T (0.152) | 43 |
| 3 | SNP_A-4235588 | 2 | 2930794 | C (0.803) | T (0.197) | 15 |
| 3 | rs1667036 | 2 | 2937414 | A (0.593) | G (0.407) | 41 |
| 4 | rs17348835 | 2 | 14629872 | T (0.84) | C (0.16) | 51 |
| 4 | rs17348849 | 2 | 14630177 | T (0.834) | A (0.166) | 24 |
| 4 | rs16861918 | 2 | 14660925 | G (0.893) | A (0.107) | 62 |
| 4 | rs7559666 | 2 | 14700592 | A (0.681) | G (0.319) | 34 |
| 4 | rs7604626 | 2 | 14728248 | A (0.959) | G (0.041) | 18 |
| 4 | rs2571642 | 2 | 14774365 | A (0.651) | G (0.349) | 20 |
| 5 | rs7572753 | 2 | 33241044 | T (0.704) | C (0.296) | 23 |
| 5 | rs17012628 | 2 | 33245587 | T (0.973) | A (0.027) | 60 |
| 6 | rs13394665 | 2 | 64244935 | C (0.531) | A (0.469) | 32 |
| 6 | rs17754672 | 2 | 64254112 | G (0.573) | A (0.427) | 49 |
| 6 | rs6710681 | 2 | 64266725 | C (0.577) | T (0.423) | 32 |
| 6 | rs2901646 | 2 | 64268396 | A (0.598) | G (0.402) | 32 |
| 6 | rs6758601 | 2 | 64278598 | G (0.656) | A (0.344) | 157 |
| 7 | rs7558220 | 2 | 71183839 | C (0.927) | T (0.073) | 20 |
| 7 | rs357752 | 2 | 71224326 | T (0.683) | A (0.317) | 21 |
| 7 | rs1458868 | 2 | 71229709 | T (0.507) | C (0.493) | 22 |
| 7 | rs357729 | 2 | 71235448 | T (0.907) | A (0.093) | 25 |
| 7 | rs11674899 | 2 | 71236003 | G (0.83) | A (0.17) | 46 |
| 8 | rs7572970 | 2 | 160844902 | G (0.71) | A (0.29) | 27 |
| 8 | rs716615 | 2 | 160845236 | T (0.694) | C (0.306) | 38 |
| 8 | rs12692592 | 2 | 160871627 | T (0.781) | G (0.219) | 93 |
| 8 | rs7593730 | 2 | 160879700 | C (0.775) | T (0.225) | 18 |
| 8 | rs1430 | 2 | 160882939 | C (0.724) | T (0.276) | 37 |
| 8 | rs4589705 | 2 | 160884382 | A (0.777) | T (0.223) | 40 |
| 8 | rs4664013 | 2 | 160892410 | C (0.67) | G (0.33) | 21 |
| 8 | rs10165319 | 2 | 160901051 | C (0.644) | T (0.356) | 29 |
| 10 | rs1530271 | 4 | 116081334 | C (0.889) | G (0.111) | 65 |
| 10 | rs2165543 | 4 | 116108687 | C (0.884) | T (0.116) | 118 |
| 10 | rs2620424 | 4 | 116109214 | G (0.656) | A (0.344) | 46 |
| 10 | rs7676598 | 4 | 116168165 | T (0.876) | A (0.124) | 33 |
| 11 | rs340686 | 5 | 8440200 | G (0.769) | A (0.231) | 37 |
| 11 | rs17213196 | 5 | 8459914 | T (0.84) | A (0.16) | 30 |
| 11 | rs17279186 | 5 | 8460305 | T (0.83) | C (0.17) | 30 |
| 11 | rs4702551 | 5 | 8477283 | A (0.698) | G (0.302) | 50 |
| 11 | rs4461605 | 5 | 8492356 | C (0.845) | T (0.155) | 25 |
| 11 | rs10475432 | 5 | 8505249 | A (0.936) | C (0.064) | 120 |
| 12 | rs2162769 | 5 | 132568008 | C (0.573) | T (0.427) | 217 |
| 12 | rs3828692 | 5 | 132573958 | G (0.586) | A (0.414) | 28 |
| 13 | rs17110060 | 5 | 148780327 | C (0.859) | T (0.141) | 17 |
| 13 | rs353249 | 5 | 148804949 | T (0.916) | C (0.084) | 28 |
| 13 | rs10062536 | 5 | 148805862 | C (0.91) | T (0.09) | 22 |
| 13 | rs386763 | 5 | 148826750 | C (0.586) | A (0.414) | 28 |
| 14 | rs11742763 | 5 | 168543160 | A (0.826) | G (0.174) | 22 |
| 15 | rs7780145 | 7 | 19164657 | T (0.713) | G (0.287) | 35 |
| 15 | rs17436262 | 7 | 19165050 | T (0.757) | A (0.243) | 21 |
| 15 | rs11762869 | 7 | 19167144 | C (0.733) | T (0.267) | 56 |
| 15 | rs17141090 | 7 | 19300515 | C (0.935) | G (0.065) | 43 |
| 15 | rs11763201 | 7 | 19304734 | T (0.796) | C (0.204) | 53 |
| 16 | rs1721383 | 7 | 38069864 | G (0.855) | T (0.145) | 22 |
| 16 | rs1464713 | 7 | 38075522 | G (0.856) | T (0.144) | 51 |
| 16 | rs2037604 | 7 | 38078323 | C (0.637) | T (0.363) | 122 |
| 16 | rs1403986 | 7 | 38078601 | A (0.813) | G (0.187) | 117 |
| 16 | rs2037603 | 7 | 38078742 | A (0.634) | C (0.366) | 31 |
| 16 | rs10280461 | 7 | 38116457 | A (0.532) | C (0.468) | 129 |
| 16 | rs7801794 | 7 | 38176362 | G (0.792) | A (0.208) | 55 |
| 17 | rs2127187 | 7 | 95200791 | C (0.892) | T (0.108) | 39 |
| 17 | rs6465477 | 7 | 95227449 | T (0.677) | C (0.323) | 151 |
| 17 | rs1382620 | 7 | 95242508 | G (0.636) | A (0.364) | 18 |
| 17 | rs983350 | 7 | 95242854 | T (0.638) | A (0.362) | 89 |
| 17 | rs4729206 | 7 | 95258613 | C (0.929) | G (0.071) | 22 |
| 17 | rs2706888 | 7 | 95262393 | T (0.634) | C (0.366) | 50 |
| 18 | rs3802108 | 7 | 156713827 | T (0.836) | G (0.164) | 33 |
| 18 | rs1182389 | 7 | 156731564 | G (0.612) | A (0.388) | 61 |
| 18 | rs1182385 | 7 | 156733344 | T (0.615) | C (0.385) | 24 |
| 18 | rs1182426 | 7 | 156769361 | G (0.927) | A (0.073) | 50 |
| 18 | rs1182421 | 7 | 156771665 | C (0.931) | G (0.069) | 98 |
| 18 | rs10254807 | 7 | 156820915 | G (0.572) | C (0.428) | 50 |
| 19 | rs16879814 | 8 | 32693125 | T (0.692) | C (0.308) | 124 |
| 19 | rs10089599 | 8 | 32717763 | G (0.841) | A (0.159) | 23 |
| 20 | rs17313886 | 8 | 65346519 | C (0.85) | T (0.15) | 41 |
| 20 | rs6982872 | 8 | 65355691 | T (0.823) | C (0.177) | 52 |
| 20 | rs190937 | 8 | 65379408 | T (0.89) | C (0.11) | 20 |
| 20 | rs9298082 | 8 | 65391007 | T (0.687) | A (0.313) | 28 |
| 20 | rs1438230 | 8 | 65411763 | T (0.932) | A (0.068) | 23 |
| 20 | rs17315206 | 8 | 65416726 | G (0.803) | A (0.197) | 44 |
| 20 | rs10110341 | 8 | 65421657 | T (0.642) | C (0.358) | 149 |
| 21 | rs7833605 | 8 | 72569276 | A (0.581) | T (0.419) | 24 |
| 23 | rs3122359 | 9 | 89987672 | A (0.906) | G (0.094) | 37 |
| 23 | rs2814828 | 9 | 90001002 | C (0.751) | T (0.249) | 22 |
| 23 | rs2814829 | 9 | 90003284 | C (0.853) | T (0.147) | 17 |
| 23 | rs2814839 | 9 | 90009583 | C (0.861) | T (0.139) | 102 |
| 23 | rs2578237 | 9 | 90009661 | A (0.859) | G (0.141) | 79 |
| 23 | rs2492849 | 9 | 90009833 | C (0.861) | A (0.139) | 29 |
| 23 | rs2251622 | 9 | 90013426 | A (0.741) | T (0.259) | 26 |
| 23 | rs4877421 | 9 | 90028369 | G (0.896) | A (0:104) | 25 |
| 22 | rs4242598 | 9 | 90761702 | T (0.625) | C (0.375) | 32 |
| 22 | rs6559326 | 9 | 90771352 | G (0.556) | C (0.444) | 35 |
| 22 | rs11137472 | 9 | 90781500 | A (0.849) | G (0.151) | 32 |
| 22 | rs6559331 | 9 | 90797689 | C (0.577) | T (0.423) | 166 |
| 22 | rs9410299 | 9 | 90829608 | C (0.51) | G (0.49) | 116 |
| 25 | rs224666 | 11 | 31980648 | G (0.906) | A (0.094) | 29 |
| 25 | rs10767911 | 11 | 31999948 | T (0.525) | C (0.475) | 19 |
| 25 | rs224618 | 11 | 32021225 | T (0.865) | G (0.135) | 28 |
| 25 | rs7938107 | 11 | 32025034 | G (0.676) | T (0.324) | 40 |
| 25 | rs7938229 | 11 | 32025117 | C (0.679) | T (0.321) | 50 |
| 25 | rs85074 | 11 | 32033408 | G (0.71) | T (0.29) | 49 |
| 26 | ras11110315 | 12 | 99165532 | T (0.794) | A (0.206) | 98 |
| 26 | rs17476256 | 12 | 99191180 | A (0.835) | G (0.165) | 139 |
| 27 | rs7399367 | 13 | 113586246 | C (0.752) | G (0.248) | 21 |
| 28 | rs7170842 | 15 | 96440938 | T (0.935) | C (0.065) | 39 |
| 29 | rs12596992 | 16 | 71536544 | C (0.884) | G (0.116) | 28 |
| 29 | rs6499601 | 16 | 71538731 | C (0.653) | T (0.347) | 24 |
| 29 | rs13336412 | 16 | 71539450 | A (0.627) | C (0.373) | 27 |
| 29 | rs756718 | 16 | 71553497 | T (0.681) | C (0.319) | 44 |
| 29 | rs756717 | 16 | 71553663 | G (0.616) | A (0.384) | 41 |
| 30 | rs9910028 | 17 | 71761805 | A (0.581) | G (0.419) | 41 |
| 30 | rs2279053 | 17 | 71798984 | T (0.686) | C (0.314) | 21 |
| 30 | rs346790 | 17 | 71811890 | C (0.978) | T (0.022) | 146 |
| 30 | rs2279056 | 17 | 71821069 | A (0.658) | G (0.342) | 27 |
| 32 | rs7230449 | 18 | 61174731 | T (0.931) | C (0.069) | 23 |
| 32 | rs470574 | 18 | 61189812 | A (0.786) | T (0.214) | 36 |
| 31 | rs2156025 | 18 | 66885986 | A (0.769) | G (0.231) | 20 |
| 33 | rs17685936 | 19 | 34337760 | A (0.771) | G (0.229) | 52 |
| 33 | rs7257235 | 19 | 34394722 | C (0.922) | A (0.078) | 21 |
| 33 | rs4805347 | 19 | 34402073 | T (0.59) | C (0.41) | 20 |
| 33 | rs17691420 | 19 | 34422716 | G (0.663) | C (0.337) | 28 |
| 34 | rs6054014 | 20 | 6170240 | T (0.771) | C (0.229) | 60 |
| 34 | rs6085463 | 20 | 6200538 | C (0.62) | T (0.38) | 102 |
| 34 | rs237695 | 20 | 6237592 | G (0.582) | T (0.418) | 55 |
| 34 | rs1571216 | 20 | 6264328 | T (0.665) | G (0.335) | 35 |
| 34 | rs7265934 | 20 | 6264437 | C (0.693) | A (0.307) | 68 |
| 35 | rs7345130 | 20 | 1604854 | C (0.731) | T (0.269) | 76 |
| 35 | rs6034368 | 20 | 1606258 | C (0.728) | T (0.272) | 74 |
| 35 | rs4814511 | 20 | 1658272 | T (0.748) | C (0.252) | 69 |
| 35 | rs6135821 | 20 | 1665028 | T (0.906) | C (0.094) | 68 |

## Claims

1. A molecule for use in a method of treatment or diagnosis of Kawasaki Disease in an individual, the molecule comprising: (a) CACNA2D3; (b) CAMK2D; (c) KCNIP4; (d) ANGPT1; (e) NAALADL2; (f) ZFHX3; (g) MPHOSPH10; (h) a sequence having at least 90% sequence identity to any of (a) to (g); or (i) a modulator of any of (a) to (e), for example in the form of a pharmaceutical composition comprising the molecule together with a pharmaceutically acceptable excipient, carrier or diluent.

2. A molecule according to Claim 1 for use as specified therein, in which the molecule comprises an antagonist of CACNA2D3 such as Pregabalin ((3S)-3-(aminomethyl)-5-methyl-hexanoic acid), and in which the method preferably comprises administering the antagonist of CACNA2D3 to an individual.

3. A method of identifying a molecule suitable for the treatment, prophylaxis or alleviation of Kawasaki Disease, the method comprising determining if a candidate molecule is an agonist or antagonist of a polypeptide selected from the group consisting of: (a) CACNA2D3; (b) CAMK2D; (c) KCNIP4; (d) ANGPT1; (e) NAALADL2; (f) ZFHX3; (g) MPHOSPH10; and (h) a sequence having at least 90% sequence identity to any of (a) to (g), preferably in which the candidate molecule is exposed to the polypeptide, or a cell expressing the polypeptide, in order to determine if the candidate molecule is a modulator thereof.

4. A method of identifying a modulator of a polypeptide selected from the group consisting of: (a) CACNA2D3; (b) CAMK2D; (c) KCNIP4; (d) ANGPT1; (e) NAALADL2; (f) ZFHX3; (g) MPHOSPH10; and (h) a sequence having at least 90% sequence identity to any of (a) to (g), the method comprising administering a candidate molecule to an animal, such as a rodent, for example a mouse, suffering from Kawasaki Disease and determining whether the animal exhibits an alleviated symptom of Kawasaki Disease.

5. A molecule or modulator of a polypeptide identified by a method according to Claim 3 or 4, preferably for use in the treatment of Kawasaki Disease.

6. Pregabalin ((3S)-3-(aminomethyl)-5-methyl-hexanoic acid) for use in the treatment or prevention of Kawasaki Disease in an individual.

7. Use of a molecule according to Claim 5 or 6 for the preparation of a pharmaceutical composition for the treatment of Kawasaki Disease in an individual.

8. A pharmaceutical composition for the treatment or prevention of Kawasaki Disease in an individual, the pharmaceutical composition comprising Pregabalin ((3S)-3-(aminomethyl)-5-methyl-hexanoic acid) together with a pharmaceutically acceptable excipient, carrier or diluent.

9. A method for the diagnosis or detection of Kawasaki Disease, in which the method comprises:
(a) detecting a polymorphism in or around a gene selected from NAALADL2, ANGPT1 and ZFHX3 or a sequence having at least 90% sequence identity thereto;
(b) detecting a single nucleotide polymorphism (SNP) selected from the group consisting of: rs17531088, rs1010824, rs6469101, rs7199343, rs 10852516 and rs11075953 as set out in Table 2 or Table S1;
(c) detecting a haplotype in or around a gene selected from MPHOSPH10, RBMS1 and LOC441938 or a sequence having at least 90% sequence identity thereto;
(d) detecting a haplotype selected from the group consisting of: rs7558220, rs357752; and rs7558220, rs357752, rs1458868, rs357729 and rs11674899 as set out in Table 2 or Table S2; or
(e) any combination of (a) to (d) above.

10. A method according to Claim 9, which comprises any one or more of the following features:
(a) detecting a plurality of polymorphisms, such as 5 or more polymorphisms, for example all the polymorphisms such as single nucleotide polymorphisms (SNPs) as specified in (b) of Claim 9;
(b) detecting a plurality of haplotypes, such as 5 or more haplotypes, for example all the haplotypes as specified in (d) of Claim 9;
(c) detecting a plurality of genes, polypeptides encoded by the genes or markers;
(d) generating a polymorphism profile, a haplotype profile or an expression profile of a plurality of genes;
(e) detecting, or deriving a profile from, a gene, polypeptide encoded by a gene, marker, polymorphism or haplotype, by microarray hybridisation such as hybridisation to an Affymetrix microarray, or by real time polymerase chain reaction (RT-PCR); or (f) performing a computer implemented method comprising processing expression or polymorphism data for one or more genes, polypeptides encoded by the genes, markers, polymorphisms or haplotypes set out in Claim 9 in a computer.

11. A method for the diagnosis or detection of Kawasaki Disease, in which the method comprises detecting expression, such as a change in the expression pattern or level, of a gene comprising NAALADL2 or CAMK2D, or a polypeptide encoded by such a gene or a sequence having at least 90% sequence identity thereto.

12. A diagnostic kit for Kawasaki Disease or susceptibility thereto comprising any one or more of the following: a molecule capable of binding to a polypeptide (a) CACNA2D3; (b) CAMK2D; (c) KCNIP4; (d) ANGPT1; (e) NAALADL2; (f) ZFHX3; (g) MPHOSPH10 or a sequence having at least 90% sequence identity thereto, such as an antibody; a nucleic acid capable of encoding such; a nucleic acid capable of binding to a nucleic acid selected from the group consisting of: (a) CACNA2D3; (b) CAMK2D; (c) KCNIP4; (d) ANGPT1; (e) NAALADL2; (f) ZFHX3; (g) MPHOSPH10 or a sequence having at least 90% sequence identity thereto, such as a nucleic acid capable of discriminating between polymorphisms for example by being capable of binding to one polymorphism at a particular location, but not capable of binding to another polymorphism at that location.

13. A combination comprising a plurality of markers, polymorphisms or haplotypes set out in (a) to (d) of Claim 9 preferably in the form of an array, such as a microarray.

14. A data carrier comprising information set out in (a) to (d) of Claim 9.

15. A method according to Claim 9, 10 or 11, further comprising detecting or modulating a gene, polypeptide encoded by a gene, marker, polymorphism or haplotype shown in Table 2, Table 3, Table 4, Table S1, Table S2, Figure 3 or Figure 4 or a sequence having at least 90% sequence identity thereto.
